(19) Europäisches Patentamt
European Patent Office
Office européen des brevets

(11) EP 3 953 331 B1

(12) **EUROPEAN PATENT SPECIFICATION**

(45) Date of publication and mention of the grant of the patent:
**20.11.2024 Bulletin 2024/47**

(21) Application number: **20788312.5**

(22) Date of filing: **06.04.2020**

(51) International Patent Classification (IPC):
***C07D 401/14*** (2006.01) ***C07D 403/12*** (2006.01)
***C07D 403/14*** (2006.01) ***C07D 405/14*** (2006.01)
***C07D 409/14*** (2006.01) ***A61K 31/416*** (2006.01)
***A61P 35/00*** (2006.01)

(52) Cooperative Patent Classification (CPC):
**C07D 403/12; A61P 35/00; C07D 401/14; C07D 403/14; C07D 405/14; C07D 409/14**

(86) International application number:
**PCT/US2020/026799**

(87) International publication number:
**WO 2020/210139 (15.10.2020 Gazette 2020/42)**

(54) **PYRAZOLESULFONAMIDES AS ANTITUMOR AGENTS**

PYRAZOLSULFONAMIDE ALS ANTITUMORMITTEL

PYRAZOLESULFONAMIDES EN TANT QU'AGENTS ANTITUMORAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.04.2019 US 201962832125 P**

(43) Date of publication of application:
**16.02.2022 Bulletin 2022/07**

(73) Proprietor: **Peloton Therapeutics, Inc.**
**Rahway, NJ 07065 (US)**

(72) Inventors:
- **ESTRADA, Michelle Ann**
  **Dallas, Texas 75235-7323 (US)**
- **GRINA, Jonas**
  **Dallas, Texas 75235-7323 (US)**
- **WEHN, Paul**
  **Dallas, Texas 75235-7323 (US)**
- **XU, Rui**
  **Dallas, Texas 75235-7323 (US)**

(74) Representative: **Moré, Solveig Helga**
**Kroher Strobel**
**Rechts- und Patentanwälte PartmbB**
**Bavariaring 20**
**80336 München (DE)**

(56) References cited:
**WO-A1-2019/147783 US-B2- 6 638 964**
**US-B2- 8 957 070**

- **DATABASE PubChem COMPOUND [online] 19 October 2012 (2012-10-19), "COMPOUND SUMMARY N-(1H-Indazol-7-yl)-1H-pyrazole-4-sulfonamide | C10H9N5O2S", XP055748013, Database accession no. CID 60911247**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 953 331 B1

## Description

### BACKGROUND OF THE INVENTION

The frequent observation of aberrant pre-mRNA splicing in many cancers may offer opportunities for the development of novel therapeutic agents. For example, RBM39 protein is associated with core components of the spliceosome. Loss or reduction of amount of RBM39 protein can alter the frequency of alternative splicing events resulting in exon skipping and intron retention. Such events may trigger selective lethality in cancer cells reliant on altered splicing or induce expression of splicing-derived neoantigens that can be exploited for therapy.

RBM39 protein is required for acute myeloid leukemia (AML) maintenance through misspicing of HOXA9 target genes and it has been found that RBM39 loss alters splicing of mRNAs essential for AML cell growth (See E. Wang et al., "Targeting an RNA-Binding Protein network in Acute Myeliod Leukemia", Cancer Cell 35, 369-382, (2019) and D. Hsiehchen, et al., "Biomarkers for RBM39 degradation in acute myeloid leukemia", Springer Nature, Feb. 2020)). It is also believed that compounds able to degregrate RBM39 may be effective in treating cancers such as colon, EZH2 mutant limphomas, and melanomia.

US 8 957 070 B2 discloses fused heterocyclic compounds having glukinase activating activity and their use for the treatment of diabetes and cancers such as, e.g., leukemia, breast cancer, prostate cancer or skin cancer.

US 6 638 964 B2 discloses sulfonamide-containing indole compounds having an antiangiogenic effect and their use as antitumor agents.

PubChem database entry 60911247 discloses the pyrazole-4-sulfonamide derivative N-(1H-indazol-7-yl)-1H-pyrazole-4-sulfonamide but does not specify any therapeutic uses thereof.

WO 2019/147783 A1 relates to sulfonamide derivatives that selectively target certain RNA splicing pathways implicated in cancer cell growth by, e.g., degradation of RRM family proteins including RBM39.

### SUMMARY OF THE INVENTION

The present invention relates to novel compounds, to pharmaceutical compositions comprising the compounds, to a process for making the compounds and to the use of the compounds in therapy. More particularly, it relates to certain pyrazole-4-sulfonamide derivatives useful as RBM39 degraders for the treatment of cancer.

In one aspect, the present invention provides a compound of Formula I:

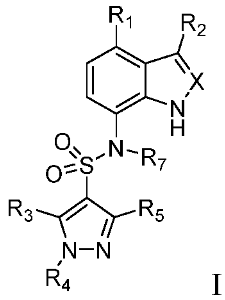

I

or a pharmaceutically acceptable salt thereof.

In another aspect, the present disclosure provides a pharmaceutical composition comprising a compound of Formula I or a pharmaceutically acceptable salt thereof, and a pharmaceutically acceptable diluent or carrier.

In another aspect, the present disclosure provides a use of a compound according to Formula I or a pharmaceutically acceptable salt thereof in the preparation of a medicament for modulating the degradation of RBM39 protein.

In another aspect, the present disclosure provides a a compound according to Formula I for use in a method of treating cancer in a subject in need thereof, comprising administering to the subject an effective amount of a compound of Formula I, or a pharmaceutically acceptable salt thereof.

In another aspect, the present invention provides a compound of Formula I, or a pharmaceutically acceptable salt thereof, for use as a medicament for the treatment of cancer.

### DETAILED DESCRIPTION OF THE INVENTION

In one aspect, the present invention provides a compound of Formula I:

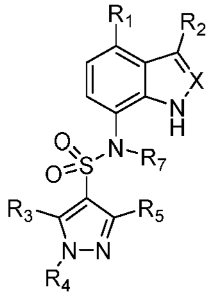

I

or a pharmaceutically acceptable salt thereof, wherein

X is N or C**$R_6$**;

**$R_1$** is hydrogen, C1-C4 alkyl, C1-C4 fluoroalkyl, or halo;

**$R_2$** is halo or cyano;

**$R_3$** is hydrogen, C1-C4 alkyl, halo, cyano, C1-C4 fluoroalkyl, -(C1-C4 alkyl)OH, or $-NR^aR^b$, wherein $R^a$ or $R^b$ is independently hydrogen, C3-C6 cycloalkyl, heterocycloalkyl or C1-C4 alkyl, or $R^a/R^b$ and the nitrogen atom they are attached to form a 5 or 6-membered ring optionally independently substituted by one or more substituents selected from halo, C1-C4 alkyl, cyano, hydroxy and -O(C1-C4 alkyl);

**$R_4$** is hydrogen, C1-C6 alkyl, C1-C10heteroalkyl, C1-C4 fluoroalkyl, -(C1-C4 alkyl)$NH_2$, C3-C6 cycloalkyl, -(C1-C4 alkyl)(C3-C6 cycloalkyl), -(C1-C4 alkyl)phenyl, phenyl, heteroaryl, -(C1-C4 alkyl)heteroaryl, C2-C5 alkenyl, -(C1-C4 alkyl)heterocycloalkyl, heterocycloalkyl, -C0-C4 alkyl$S(=O)_2$(C1-C4 alkyl), $-S(=O)_2$phenyl, $-S(=O)_2$heteroaryl, -C0-C4 alkylC(=O)heterocycloalkyl, -C0-C4 alkyl $C(=O)NR^aR^b$, -(C0-C4 alkyl)C(=O)O(C1-C4 alkyl), -(C0-C4 alkyl)C(=O)C1-C4 alkyl, -(C0-C4 alkyl)C(=O)OH or -(C1-C8 alkyl)OH, wherein **$R_4$** is substituted with 0, 1, 2, or 3 substituents selected from deuterium, halo, hydroxy, -(C0-C4alkyl)O(C1-C4 alkyl), -C1-C4 alkyl, -(C0-C4alkyl)cyano, $-S(=O)_2$(C1-C4 alkyl), - C0-C4 alkyl$C(=O)NR^aR^b$, -C(=O)O(C1-C4 alkyl), C1-C4 fluoroalkyl oxo, -(C1-C4 alkyl)OH, and $-NH_2$, wherein each of $R^a$ and $R^b$ is independently substituted with 0, 1, 2, or 3 hydrogen, C3-C6 cycloalkyl, heterocycloalkyl or C1-C4 alkyl;

**$R_5$** is hydrogen, halo, $-NH_2$ or C1-C4 alkyl;

**$R_6$** is hydrogen, halo or C1-C4 alkyl; and

**$R_7$** is hydrogen, halo, heteroalkyl, C1-C4 alkyl, -(C0-C4 alkyl)O( C1-C4 alkyl), -(C1-C4 alkyl)(C3-C6 cycloalkyl), C3-C6 cycloalkyl, -(C1-C4 alkyl)heterocycloalkyl, or heterocycloalkyl.

In certain embodiment of Formula I, X is N. In another further embodiment, X is $CR_6$.

In certain embodiments of Formula I, $R_1$ is C1-C4 alkyl or halo. In a further embodiment, $R_1$ is methyl. In another further embodiment, $R_1$ is chloro. In another further embodiment, $R_1$ is fluoro.

In certain embodiments of Formula I, $R_1$ is C1-C4 fluoroalkyl. In a further embodiment, $R_1$ is trifluoromethyl.

In certain embodiments of Formula I, $R_1$ is hydrogen.

In certain embodiments of Formula I, $R_1$ is hydrogen, fluoro, chloro, methyl, or trifluoromethyl.

In certain embodiments of Formula I, $R_2$ is halo. In a further embodiment, $R_2$ is chloro. In certain embodiments of Formula I, $R_2$ is fluoro. In certain other embodiments, $R_2$ is cyano.

In certain embodiments of Formula I, $R_2$ is cyano, chloro or fluoro.

In certain embodiments of Formula I, $R_3$ is hydrogen.

In certain embodiments of Formula I, $R_3$ is cyano.

In certain embodiments of Formula I, $R_3$ is halo. In a further embodiment, $R_3$ is fluoro.

In certain embodiments of Formula I, $R_3$ is C1-C4 alkyl. In a further embodiment, $R_3$ is methyl. In another further embodiments, $R_3$ is $CHF_2$.

In certain embodiments of Formula I, $R_3$ is -(C1-C4 alkyl)OH. In a further embodiment, $R_3$ is $-C_2H_4OH$.

In certain embodiments of Formula I, $R_3$ is $-NR^aR^b$. In a further embodiment, $R_3$ is $-NH_2$.

In certain embodiments of Formula I, $R_3$ is hydrogen, methyl, fluoro, chloro, difluoromethyl, $-NH_2$, or $-C_2H_4OH$.

In certain embodiment of Formula I, $R_5$ is hydrogen.

In certain embodiments of Formula I, $R_5$ is halo. In a further embodiment, $R_5$ is fluoro. In another embodiment, $R_5$ is chloro.

In certain embodiments of Formula I, $R_5$ is C1-C4 alkyl. In another embodiment of Formula I, $R_5$ is methyl.

In certain embodiments of Formula I, $R_5$ is $-NH_2$.

In certain embodiments of Formula I, $R_5$ is hydrogen, methyl, fluoro, chloro, or $-NH_2$.

In certain embodiments of Formula I, $R_6$ is hydrogen.

In certain embodiments of Formula I, $R_6$ is halo. In another embodiment of Formula I, $R_5$ is fluoro.

In certain embodiments of Formula I, $R_6$ is hydrogen or fluoro.

In certain embodiments of Formula I, $R_7$ is hydrogen, -(C0-C4 alkyl)O( C1-C4 alkyl), or-(C1-C4 alkyl)(C3-C6 cycloalkyl).

In certain embodiments of Formula I, $R_7$ is hydrogen.

In certain embodiments of Formula I, $R_7$ is -(C0-C4 alkyl)O( C1-C4 alkyl). In another embodiment of Formula I, $R_7$ is methoxyethyl ($-CH_2-CH_2-O-CH_3$).

In certain embodiments of Formula I, $R_7$ is -(C1-C4 alkyl)(C3-C6 cycloalkyl). In another embodiment of Formula I, $R_7$ is cyclopropylmethyl.

In certain embodiments of Formula I, $R_7$ is hydrogen, cyclopropylmethyl or methoxyethyl.

In certain embodiments of Formula I, $R_4$ is hydrogen, C1-C6 alkyl, C1-C10heteroalkyl, C1-C4 fluoroalkyl, -(C1-C4 alkyl)$NH_2$, C3-C6 cycloalkyl, -(C1-C4 alkyl)(C3-C6 cycloalkyl), -(C1-C4 alkyl)phenyl, phenyl, heteroaryl, C2-C5 alkenyl, -(C1-C4 alkyl)heterocycloalkyl, heterocycloalkyl, -C0-C4 alkylS$(=O)_2$(C1-C4 alkyl), -S$(=O)_2$phenyl, -S$(=O)_2$heteroaryl, -C0-C4 alkyl C(=O)$NR^aR^b$, -(C0-C4 alkyl)C(=O)O(C1-C4 alkyl), -(C0-C4 alkyl)C(=O)C1-C4 alkyl, -(C0-C4 alkyl)C(=O)OH or -(C1-C8 alkyl)OH, wherein $\mathbf{R_4}$ is substituted with 0, 1, 2, or 3 substituents selected from deuterium, halo, hydroxy, -(C0-C4alkyl)O(C1-C4 alkyl), -C1-C4 alkyl, -(C0-C4alkyl)cyano, - C0-C4 alkylC(=O)$NR^aR^b$, -C(=O)O(C1-C4 alkyl), C1-C4 fluoroalkyl, oxo, -(C1-C4 alkyl)OH, and $-NH_2$, wherein each of $R^a$ and $R^b$ is independently substituted with 0, 1, 2, or 3 hydrogen, C3-C6 cycloalkyl, heterocycloalkyl or C1-C4 alkyl.

In certain embodiments of Formula I, $\mathbf{R_4}$ is hydrogen, methyl, ethyl, propyl, isopropyl, tert-butyl, fluoromethyl, difluoromethyl, difluoroethyl, trifluoroethyl, fluoropropyl, methoxyethyl, methoxyethoxyethyl, cyclopropyl, cyclobutyl, cyclopentyl, cyclohexyl, 3-bicyclo[1.1.1]pentanyl, cyclopropylmethyl, cyclobutylmethyl, cyclopentylmethyl, cyclohexylmethyl, cyclopropylethyl, cyclobutylethyl, cyclopentylethyl and cyclohexylmethyl, tetrahydrofuranyl, azetidinyl, tetrahydropyranyl, piperidinyl, oxetanyl, thietanyl, pyrrolidinylethyl, pyrrolidinyl, pyrrolidinylmethyl, azetidinylmethyl, tetrahydrothiopyranylmethyl, piperidinylmethyl, tetrahydropyranylmethyl, tetrahydrothiophenyl, oxetanylethyl, oxaspiro[3.3]heptyl, (methylsulfonyl)ethyl, 2-methyl-2-(methylsulfonyl)propyl, pyrazolyl(methylsulfonyl), methylsulfonyl, (methylsulfonyl)methyl, phenylsulfonyl, hydroxyethyl, aminocarbonylmethyl, carboxymethyl, cyclobutylaminocarbonylmethyl, pyrrolidinylcarbonylmethyl, methylaminocarbonylmethyl, (dimethylamino)carbonylmethyl, aminocarbonyl(C1-C4alkyl), methoxycarbonyl(C1-C4alkyl), (aminocarbonyl)C1-C4alkyl, phenyl, pyridinyl, propenyl,

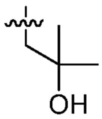 , 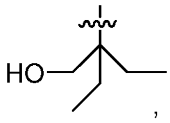 , 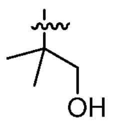 , 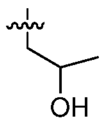 , 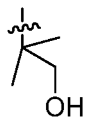 ,

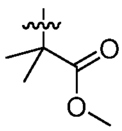 , 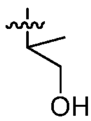 , 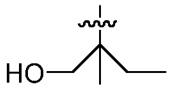 , 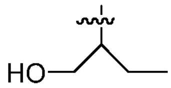 ,

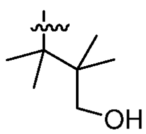 , 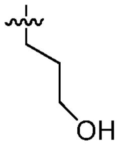 , 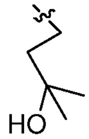 , 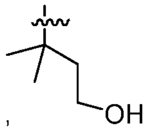 , 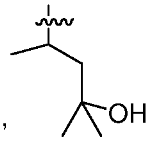 , 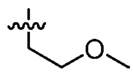 ,

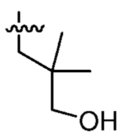 , 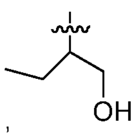 , 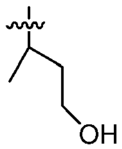 , 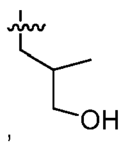 , 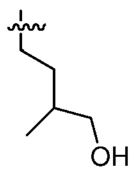 ,

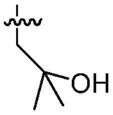 , 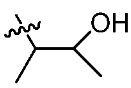 , 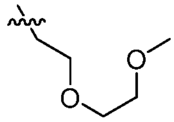 or 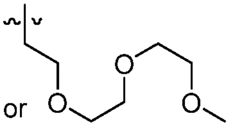 ,

wherein $\mathbf{R_4}$ is substituted with 0, 1, 2, or 3 substituents selected from deuterium, halo, hydroxy, -(C0-C4alkyl)O(C1-C4 alkyl), -C1-C4 alkyl, -(C0-C4alkyl)cyano, - C0-C4 alkylC(=O)NR$^a$R$^b$, -C(=O)O(C1-C4 alkyl), C1-C4 fluoroalkyl, oxo, -(C1-C4 alkyl)OH, and -NH$_2$, wherein each of R$^a$ and R$^b$ is independently substituted with 0, 1, 2, or 3 hydrogen, C3-C6 cycloalkyl, heterocycloalkyl or C1-C4 alkyl.

In certain embodiments of Formula I, $\mathbf{R_4}$ is substituted with 0, 1, 2, or 3 substituents selected from deuterium, halo, hydroxy, methoxy, hydroxy, cyanomethyl, cyano, trifluoromethyl, trifluoroethyl, amino, fluoromethyl, *tert*-butyloxycarbonyl, oxo, aminocarbonylmethyl, difluoromethyl, hydroxymethyl, methylcarbonyl, aminocarbonylethyl, and methoxymethyl.

In certain embodiments of Formula I, $R_1$ is C1-C4 alkyl or C1-C4 fluoroalkyl, and $R_2$ is cyano. In a further embodiment, $R_1$ is methyl or trifluoromethyl.

In certain embodiments of Formula I, $R_1$ is hydrogen and $R_2$ is halo. In a further embodiment, $R_1$ is chloro.

In certain embodiments of Formula I, $R_1$ is hydrogen and $R_2$ is cyano.

In certain embodiments of Formula I, $R_1$ is halo and $R_2$ is cyano. In a further embodiment, $R_1$ is chloro. In another further embodiment, $R_1$ is fluoro.

In certain embodiments of Formula I, each of $R_1$ and $R_2$ is halo. In a further embodiment, each of $R_1$ and $R_2$ is chloro.

In certain embodiments of Formula I, $R_1$ is C1-C4 alkyl or C1-C4 fluoroalkyl, $R_2$ is cyano and $R_3$ is hydrogen. In a further embodiment, $R_1$ is methyl or trifluoromethyl. In a still further embodiment, $R_5$ is hydrogen.

In certain embodiments of Formula I, $R_1$ is hydrogen, $R_2$ is halo and $R_3$ is hydrogen. In a further embodiment, $R_2$ is chloro. In a still further embodiment, $R_5$ is hydrogen.

In certain embodiments of Formula I, $R_1$ is halo, $R_2$ is cyano and $R_3$ is hydrogen. In a further embodiment, $R_1$ is chloro. In a still further embodiment, $R_5$ is hydrogen.

The invention is also directed to a compound, or a pharmaceutically acceptable salt thereof, selected from the following exemplified compounds:

N-(3-chloro-1H-indol-7-yl)-1-methyl-pyrazole-4-sulfonamide;
N-(3-chloro-1H-indol-7-yl)-1,3,5-trimethyl-pyrazole-4-sulfonamide;
N-(3-cyano-4-methyl-1H-indol-7-yl)-1-methyl-pyrazole-4-sulfonamide;
N-(3-chloro-1H-indol-7-yl)-1-ethyl-pyrazole-4-sulfonamide;
N-(3-chloro-1H-indol-7-yl)-1,3-dimethyl-pyrazole-4-sulfonamide;
N-(3-chloro-1H-indol-7-yl)-1-(difluoromethyl)pyrazole-4-sulfonamide;
N-(3-chloro-1H-indol-7-yl)-1-(2,2-difluoroethyl)pyrazole-4-sulfonamide;
N-(3-chloro-1H-indol-7-yl)-1-tetrahydrofuran-3-yl-pyrazole-4-sulfonamide;
N-(3-chloro-1H-indol-7-yl)-1-(2-methylsulfonylethyl)pyrazole-4-sulfonamide;
N-(3-chloro-1H-indol-7-yl)-1H-pyrazole-4-sulfonamide;
N-(3-cyano-4-methyl-1H-indol-7-yl)-1-(difluoromethyl)pyrazole-4-sulfonamide;
tert-butyl 3-[4-[(3-chloro-1H-indol-7-yl)sulfamoyl]pyrazol-1-yl]azetidine-1-carboxylate;
N-(3-chloro-1H-indol-7-yl)-1-(2-hydroxyethyl)pyrazole-4-sulfonamide;
N-(3-chloro-1H-indol-7-yl)-1-(cyanomethyl)pyrazole-4-sulfonamide;
1-(5-chloro-3-fluoro-2-pyridyl)-N-(3-chloro-1H-indol-7-yl)pyrazole-4-sulfonamide;
2-[4-[(3-chloro-1H-indol-7-yl)sulfamoyl]pyrazol-1-yl]acetamide;
2-[4-[4-[(3-chloro-1H-indol-7-yl)sulfamoyl]pyrazol-1-yl] sulfonylpyrazol-1-yl] acetamide;
1-(azetidin-3-yl)-N-(3-chloro-1H-indol-7-yl)pyrazole-4-sulfonamide;
N-(3-chloro-1H-indol-7-yl)-1-(1H-pyrazol-4-ylsulfonyl)pyrazole-4-sulfonamide;
N-(3-chloro-1H-indol-7-yl)-1-tetrahydropyran-4-yl-pyrazole-4-sulfonamide;
N-(3-chloro-1H-indol-7-yl)-1-(5-fluoro-2-pyridyl)pyrazole-4-sulfonamide;
N-(3-chloro-1H-indol-7-yl)-1-[2-cyano-4-(trifluoromethyl)phenyl]pyrazole-4-sulfonamide;
N-(3-chloro-1H-indol-7-yl)-1-[2-(2-methoxyethoxy)ethyl]pyrazole-4-sulfonamide;
N-(3-chloro-1H-indol-7-yl)-1-[2-[2-(2-methoxyethoxy)ethoxy]ethyl]pyrazole-4-sulfonamide;
N-(3-cyano-4-methyl-1H-indol-7-yl)-1-(2-hydroxyethyl)pyrazole-4-sulfonamide;
tert-butyl 4-[4-[(3 -chloro- 1H-indol-7-yl)sulfamoyl]pyrazol-1 -yl]piperidine-1 -carboxylate;
1-(1-acetylazetidin-3-yl)-N-(3-chloro-1H-indol-7-yl)pyrazole-4-sulfonamide;
N-(3-chloro-1H-indol-7-yl)-1-[(3-methyloxetan-3-yl)methyl]pyrazole-4-sulfonamide;
N-(3-cyano-4-methyl-1H-indol-7-yl)-1-tetrahydrofuran-3-yl-pyrazole-4-sulfonamide;
N-(3-chloro-1H-indol-7-yl)-1-[5-(trifluoromethyl)-2-pyridyl]pyrazole-4-sulfonamide;
N-(3-cyano-4-methyl-1H-indol-7-yl)-1-[5-(trifluoromethyl)-2-pyridyl]pyrazole-4-sulfonamide;
N-(3-chloro-1H-indol-7-yl)-1-(2,2-dimethoxyethyl)pyrazole-4-sulfonamide;
tert-butyl N-[2-[4-[(3-chloro-1H-indol-7-yl)sulfamoyl]pyrazol-1-yl]ethyl]carbamate;

1-(2-aminoethyl)-N-(3-chloro-1H-indol-7-yl)pyrazole-4-sulfonamide;
N-(3-chloro-1H-indol-7-yl)-1-(2-fluoroethyl)pyrazole-4-sulfonamide;
N-(3-cyano-4-methyl-1H-indol-7-yl)-1-(2-fluoroethyl)pyrazole-4-sulfonamide;
N-(3-chloro-1H-indol-7-yl)-1-phenyl-pyrazole-4-sulfonamide;
N-(3-chloro-1H-indol-7-yl)-1-(2,2,2-trifluoroethyl)pyrazole-4-sulfonamide;
N-(3-chloro-1H-indol-7-yl)-1-isopropyl-pyrazole-4-sulfonamide;
N-(3-chloro-1H-indol-7-yl)-1-(oxetan-3-yl)pyrazole-4-sulfonamide;
N-(3-chloro-1H-indol-7-yl)-1-(4-piperidyl)pyrazole-4-sulfonamide;
tert-butyl 3-[[4-[(3-chloro-1H-indol-7-yl)sulfamoyl]pyrazol-1-yl]methyl]azetidine-1-carboxylate;
1-(azetidin-3-ylmethyl)-N-(3-chloro-1H-indol-7-yl)pyrazole-4-sulfonamide;
N-(3-chloro-1H-indol-7-yl)-1-[(3-methylthietan-3-yl)methyl]pyrazole-4-sulfonamide;
N-(3-chloro-1H-indol-7-yl)-1-[(3-methyl-1,1-dioxo-thietan-3-yl)methyl]pyrazole-4-sulfonamide;
N-(3-cyano-1H-indol-7-yl)-1-methyl-pyrazole-4-sulfonamide;
N-(3-chloro-1H-indol-7-yl)-1-cyclobutyl-pyrazole-4-sulfonamide;
N-(3-chloro-1H-indol-7-yl)-N,1-bis(cyclopropylmethyl)pyrazole-4-sulfonamide;
N-(3-chloro-1H-indol-7-yl)-1-(2-methoxyethyl)pyrazole-4-sulfonamide;
N-(3-chloro-1H-indol-7-yl)-N,1-bis(2-methoxyethyl)pyrazole-4-sulfonamide;
N-(3-chloro-1H-indol-7-yl)-1-(cyclopropylmethyl)pyrazole-4-sulfonamide;
N-(3-chloro-1H-indol-7-yl)-1-[(1-cyanocyclopropyl)methyl]pyrazole-4-sulfonamide;
N-(3-chloro-1H-indol-7-yl)-1-[(3,3-difluorocyclobutyl)methyl]pyrazole-4-sulfonamide;
N-(3-chloro-4-fluoro-1H-indol-7-yl)-1-methyl-pyrazole-4-sulfonamide;
N-(3-chloro-1H-indol-7-yl)-1-(fluoromethyl)pyrazole-4-sulfonamide;
N-(3-chloro-1H-indol-7-yl)-1-(3-fluoropropyl)pyrazole-4-sulfonamide;
N-(3-chloro-1H-indol-7-yl)-1-(1,1-dioxothietan-3-yl)pyrazole-4-sulfonamide;
N-(3-chloro-1H-indol-7-yl)-1-(2-cyanoethyl)pyrazole-4-sulfonamide;
N-(3-cyano-4-methyl-1H-indol-7-yl)-1-(2,2,2-trifluoroethyl)pyrazole-4-sulfonamide;
N-(3-cyano-4-methyl-1H-indol-7-yl)-1-cyclobutyl-pyrazole-4-sulfonamide;
N-(3-cyano-4-methyl-1H-indol-7-yl)-1H-pyrazole-4-sulfonamide;
N-(3-cyano-4-methyl-1H-indol-7-yl)-1-isopropyl-pyrazole-4-sulfonamide;
N-(3-cyano-4-methyl-1H-indol-7-yl)-1-(oxetan-3-yl)pyrazole-4-sulfonamide;
N-(3-cyano-4-fluoro-1H-indol-7-yl)-1-methyl-pyrazole-4-sulfonamide;
N-(3-cyano-4-methyl-1H-indol-7-yl)-1-(2,2-difluoroethyl)pyrazole-4-sulfonamide;
N-(3-cyano-4-methyl-1H-indol-7-yl)-1-(2-methylsulfonylethyl)pyrazole-4-sulfonamide;
N-(3-cyano-4-methyl-1H-indol-7-yl)-1-ethyl-pyrazole-4-sulfonamide;
N-(3-cyano-4-methyl-1H-indol-7-yl)-1-(cyclopropylmethyl)pyrazole-4-sulfonamide;
N-(3-cyano-4-methyl-1H-indol-7-yl)-1-(fluoromethyl)pyrazole-4-sulfonamide;
N-(3-cyano-4-methyl-1H-indol-7-yl)-1-[(3-methyloxetan-3-yl)methyl]pyrazole-4-sulfonamide;
N-(3-cyano-4-methyl-1H-indol-7-yl)-1-[(3-fluorooxetan-3-yl)methyl]pyrazole-4-sulfonamide;
N-(3-cyano-4-methyl-1H-indol-7-yl)-1-(3-fluoropropyl)pyrazole-4-sulfonamide;N-(3-chloro-4-methyl-1H-indol-7-yl)-1-methyl-pyrazole-4-sulfonamide;
N-(3-cyano-4-methyl-1H-indol-7-yl)-1-(2-cyano-2-methyl-propyl)pyrazole-4-sulfonamide;
1-(2-cyanoethyl)-N-(3-cyano-4-methyl-1H-indol-7-yl)pyrazole-4-sulfonamide;
N-(3-cyano-4-methyl-1H-indol-7-yl)-1-(cyclobutylmethyl)pyrazole-4-sulfonamide;
N-(3-cyano-4-methyl-1H-indol-7-yl)-1-[(2,2-difluorocyclopropyl)methyl]pyrazole-4-sulfonamide;
N-(3-cyano-4-methyl-1H-indol-7-yl)-1-[1-(fluoromethyl)vinyl]pyrazole-4-sulfonamide;
N-(3,4-dichloro-1H-indol-7-yl)-1-methyl-pyrazole-4-sulfonamide;
N-(4-chloro-3-cyano-1H-indol-7-yl)-1-methyl-pyrazole-4-sulfonamide;
N-(3-chloro-1H-indol-7-yl)-1-(3-hydroxycyclobutyl)pyrazole-4-sulfonamide;
N-(3-cyano-4-methyl-1H-indol-7-yl)-1-(2-methyl-2-methylsulfonyl-propyl)pyrazole-4-sulfonamide;
N-(3-cyano-4-methyl-1H-indol-7-yl)-1-(2-oxaspiro[3.3]heptan-6-yl)pyrazole-4-sulfonamide;
N-(3-cyano-4-methyl-1H-indol-7-yl)-1-(4,4-difluorocyclohexyl)pyrazole-4-sulfonamide;
N-(3-cyano-4-methyl-1H-indol-7-yl)-1-[1-(2,2,2-trifluoroethyl)-4-piperidyl]pyrazole-4-sulfonamide;
N-(3-cyano-4-methyl-1H-indol-7-yl)-1-cyclopentyl-pyrazole-4-sulfonamide;
1-benzyl-N-(3-cyano-4-methyl-1H-indol-7-yl)pyrazole-4-sulfonamide;
N-(3-cyano-4-methyl-1H-indol-7-yl)-1-propyl-pyrazole-4-sulfonamide;
N-(4-chloro-3-cyano-1H-indol-7-yl)-1-(2,2-difluoroethyl)pyrazole-4-sulfonamide;
N-(3-cyano-4-fluoro-1H-indol-7-yl)-1-(2,2-difluoroethyl)pyrazole-4-sulfonamide;
N-(3-cyano-4-methyl-1H-indol-7-yl)-1-methylsulfonyl-pyrazole-4-sulfonamide;

1-tert-butyl-N-(3-cyano-4-methyl-1H-indol-7-yl)pyrazole-4-sulfonamide;
1-tert-butyl-N-(3-chloro-1H-indol-7-yl)pyrazole-4-sulfonamide;N-[3-chloro-4-(trifluoromethyl)-1H-indol-7-yl]-1-methyl-pyrazole-4-sulfonamide;
N-(3-chloro-4-methyl-1H-indol-7-yl)-1-(2,2-difluoroethyl)pyrazole-4-sulfonamide;
N-(3-cyano-4-methyl-1H-indol-7-yl)-1-[[1-(trifluoromethyl)cyclopropyl]methyl]pyrazole-4-sulfonamide;
N-(3-cyano-4-methyl-1H-indol-7-yl)-1-(fluoromethylsulfonyl)pyrazole-4-sulfonamide;
1-(benzenesulfonyl)-N-(3-cyano-4-methyl-1H-indol-7-yl)pyrazole-4-sulfonamide;
N-(3-cyano-4-methyl-1H-indol-7-yl)-1-ethyl-5-fluoro-pyrazole-4-sulfonamide;
N-[3-chloro-4-(trifluoromethyl)-1H-indol-7-yl]-1-(2,2-difluoroethyl)pyrazole-4-sulfonamide;
N-[3-cyano-4-(trifluoromethyl)-1H-indol-7-yl]-1-(2,2-difluoroethyl)pyrazole-4-sulfonamide;
N-[3-cyano-4-(trifluoromethyl)-1H-indol-7-yl]-1-methyl-pyrazole-4-sulfonamide;
N-(3-cyano-4-methyl-1H-indol-7-yl)-1-[[3-(hydroxymethyl)oxetan-3-yl]methyl]pyrazole-4-sulfonamide;
N-(3-cyano-4-methyl-1H-indol-7-yl)-1-(3-methyloxetan-3-yl)pyrazole-4-sulfonamide;
N-(3-cyano-4-methyl-1H-indol-7-yl)-1-(2-hydroxy-2-methyl-propyl)pyrazole-4-sulfonamide;
N-(3-cyano-4-methyl-1H-indol-7-yl)-1-(3,3-difluorocyclobutyl)pyrazole-4-sulfonamide;
N-(3-cyano-4-methyl-1H-indol-7-yl)-1-(trideuteriomethyl)pyrazole-4-sulfonamide;
N-(3-cyano-4-methyl-1H-indol-7-yl)-1-(3-fluorocyclobutyl)pyrazole-4-sulfonamide;
N-(3-cyano-4-methyl-1H-indol-7-yl)-5-fluoro-1-methyl-pyrazole-4-sulfonamide;
N-(3-cyano-4-methyl-1H-indol-7-yl)-1,5-dimethyl-pyrazole-4-sulfonamide;
N-(3-cyano-4-methyl-1H-indol-7-yl)-1,3-dimethyl-pyrazole-4-sulfonamide;
N-(3,4-dichloro-1H-indol-7-yl)-1-(3-methyloxetan-3-yl)pyrazole-4-sulfonamide;
N-(3-cyano-4-methyl-1H-indol-7-yl)-1-[3-(hydroxymethyl)oxetan-3-yl]pyrazole-4-sulfonamide;
N-(3-cyano-4-methyl-1H-indol-7-yl)-1-[3-(fluoromethyl)oxetan-3-yl]pyrazole-4-sulfonamide;
N-(4-chloro-3-cyano-1H-indol-7-yl)-1-(3-methyloxetan-3-yl)pyrazole-4-sulfonamide;
N-(4-chloro-3-cyano-1H-indol-7-yl)-1-(difluoromethyl)pyrazole-4-sulfonamide;
N-(4-chloro-3-cyano-1H-indol-7-yl)-1-(2,2,2-trifluoroethyl)pyrazole-4-sulfonamide;
N-(4-chloro-3-cyano-1H-indol-7-yl)-1H-pyrazole-4-sulfonamide;
N-(4-chloro-3-cyano-1H-indol-7-yl)-5-fluoro-1-methyl-pyrazole-4-sulfonamide;
N-(4-chloro-3-cyano-1H-indol-7-yl)-1-(cyclopropylmethyl)pyrazole-4-sulfonamide;
N-(4-chloro-3-cyano-1H-indol-7-yl)-1-(fluoromethyl)pyrazole-4-sulfonamide;
N-(4-chloro-3-cyano-1H-indol-7-yl)-1-(oxetan-3-yl)pyrazole-4-sulfonamide;
N-(3-fluoro-4-methyl-1H-indol-7-yl)-1-methyl-pyrazole-4-sulfonamide;
N-(4-chloro-3-cyano-1H-indol-7-yl)-1-ethyl-pyrazole-4-sulfonamide;
N-(3-cyano-4-methyl-1H-indol-7-yl)-5-fluoro-1-(3-methyloxetan-3-yl)pyrazole-4-sulfonamide;
N-(3-chloro-2-fluoro-1H-indol-7-yl)-1-methyl-pyrazole-4-sulfonamide;
N-(4-chloro-3-cyano-1H-indol-7-yl)-5-fluoro-1-(3-methyloxetan-3-yl)pyrazole-4-sulfonamide;
5-cyano-N-(3-cyano-4-methyl-1H-indol-7-yl)-1-methyl-pyrazole-4-sulfonamide;
N-(4-chloro-3-cyano-1H-indol-7-yl)-5-cyano-1-methyl-pyrazole-4-sulfonamide;
methyl 2-[4-[(3 -cyano-4-methyl-1H-indol-7-yl)sulfamoyl]pyrazol-1-yl] acetate;
2-[4-[(3-cyano-4-methyl-1H-indol-7-yl)sulfamoyl]pyrazol-1-yl]acetic acid;
N-(4-chloro-3-cyano-1H-indol-7-yl)-1-(trideuteriomethyl)pyrazole-4-sulfonamide;
N-(4-chloro-3-cyano-1H-indol-7-yl)-1-(2-hydroxy-2-methyl-propyl)pyrazole-4-sulfonamide;
2-[4-[(3-cyano-4-methyl-1H-indol-7-yl)sulfamoyl]pyrazol-1-yl]-N-(3,3-difluorocyclobutyl)acetamide;
N-(3-cyano-4-methyl-1H-indol-7-yl)-1-[2-[(3R,4S)-3,4-difluoropyrrolidin-1-yl]-2-oxoethyl]pyrazole-4-sulfonamide;
2-[4-[(3-cyano-4-methyl-1H-indol-7-yl)sulfamoyl]pyrazol-1-yl]-N-methyl-acetamide;
2-[4-[(3-cyano-4-methyl-1H-indol-7-yl)sulfamoyl]pyrazol-1-yl]-N,N-dimethyl-acetamide;
2-[4-[(3-cyano-4-methyl-1H-indol-7-yl)sulfamoyl]pyrazol-1-yl]acetamide;
N-(3-cyano-4-methyl-1H-indol-7-yl)-1-(2-oxo-2-pyrrolidin-1-yl-ethyl)pyrazole-4-sulfonamide;
N-(3,4-dichloro-1H-indol-7-yl)-1-(oxetan-3-yl)pyrazole-4-sulfonamide;
N-(3-cyano-4-methyl-1H-indol-7-yl)-5-fluoro-1-(oxetan-3-yl)pyrazole-4-sulfonamide;
1-[(1-cyanocyclopropyl)methyl]-N-(3-cyano-4-methyl-1H-indol-7-yl)pyrazole-4-sulfonamide;
N-(3-cyano-4-methyl-1H-indol-7-yl)-1-(2-oxopyrrolidin-3-yl)pyrazole-4-sulfonamide;
N-(4-chloro-3-cyano-1H-indol-7-yl)-1-(2-oxopyrrolidin-3-yl)pyrazole-4-sulfonamide;
N-(3-cyano-4-methyl-1H-indol-7-yl)-3-fluoro-1-methyl-pyrazole-4-sulfonamide;
N-(3-cyano-4-methyl-1H-indol-7-yl)-5-(difluoromethyl)-1-methyl-pyrazole-4-sulfonamide;
N-(4-chloro-3-cyano-1H-indol-7-yl)-5-(difluoromethyl)-1-methyl-pyrazole-4-sulfonamide;
N-(3-cyano-4-methyl-1H-indol-7-yl)-1-[(3S,4R)-4-fluoropyrrolidin-3-yl]pyrazole-4-sulfonamide;
N-(4-chloro-3-cyano-1H-indol-7-yl)-1-[(3S,4R)-4-fluoropyrrolidin-3-yl]pyrazole-4-sulfonamide;

tert-butyl (3S,4R)-3-[4-[(3-cyano-4-methyl-1H-indol-7-yl)sulfamoyl]pyrazol-1-yl]-4-fluoro-pyrrolidine-1 -carboxylate;
2-[4-[(4-chloro-3-cyano-1H-indol-7-yl)sulfamoyl]pyrazol-1-yl]-2-methyl-propanamide;
5-chloro-N-(3-cyano-4-methyl-1H-indol-7-yl)-1-methyl-pyrazole-4-sulfonamide;
5-chloro-N-(4-chloro-3-cyano-1H-indol-7-yl)-1-methyl-pyrazole-4-sulfonamide;
N-(3-cyano-4-methyl-1H-indol-7-yl)-1-[3-(cyanomethyl)oxetan-3-yl]pyrazole-4-sulfonamide;
N-(4-chloro-3-cyano-1H-indol-7-yl)-1-[3-(cyanomethyl)oxetan-3-yl]pyrazole-4-sulfonamide;
N-(3,4-dichloro-1H-indol-7-yl)-1H-pyrazole-4-sulfonamide;
1-[3-(cyanomethyl)oxetan-3-yl]-N-(3,4-dichloro-1H-indol-7-yl)pyrazole-4-sulfonamide;
5-amino-N-(3-cyano-4-methyl-1H-indol-7-yl)-1-methyl-pyrazole-4-sulfonamide;
N-(4-chloro-3-cyano-1H-indol-7-yl)-1-(2-hydroxyethyl)pyrazole-4-sulfonamide;
N-(3,4-dichloro-1H-indol-7-yl)-1-(2-hydroxyethyl)pyrazole-4-sulfonamide;
N-(4-chloro-3-cyano-1H-indol-7-yl)-1-[1-(chloromethyl)-2-hydroxy-1-(hydroxymethyl)ethyl]pyrazole-4-sulfonamide;
N-(4-chloro-3-cyano-1H-indol-7-yl)-1-[3-(hydroxymethyl)oxetan-3-yl]pyrazole-4-sulfonamide;
1-[1-(chloromethyl)-2-hydroxy-1-(hydroxymethyl)ethyl]-N-(3,4-dichloro-1H-indol-7-yl)pyrazole-4-sulfonamide;
N-(3,4-dichloro-1H-indol-7-yl)-1-[3-(hydroxymethyl)oxetan-3-yl]pyrazole-4-sulfonamide;
methyl 2-[4-[(3,4-dichloro-1H-indol-7-yl)sulfamoyl]pyrazol-1-yl]-2-methyl-propanoate;
methyl 2-[4-[(4-chloro-3-cyano-1H-indol-7-yl)sulfamoyl]pyrazol-1-yl]-2-methyl-propanoate;
N-(3,4-dichloro-1H-indol-7-yl)-1-(2-hydroxy-1,1-dimethyl-ethyl)pyrazole-4-sulfonamide;
N-(4-chloro-3-cyano-1H-indol-7-yl)-1-(2-hydroxy-1,1-dimethyl-ethyl)pyrazole-4-sulfonamide;
N-(3,4-dichloro-1H-indol-7-yl)-1-(2-hydroxy-1-methyl-ethyl)pyrazole-4-sulfonamide;
N-(3,4-dichloro-1H-indol-7-yl)-1-[2-hydroxy-1-(hydroxymethyl)-1-methyl-ethyl]pyrazole-4-sulfonamide;
N-(4-chloro-3-cyano-1H-indol-7-yl)-1-(3-hydroxycyclobutyl)pyrazole-4-sulfonamide;
N-(4-chloro-3-cyano-1H-indol-7-yl)-1-(3-hydroxycyclobutyl)pyrazole-4-sulfonamide;
N-(3,4-dichloro-1H-indol-7-yl)-1-[2-hydroxy-1-(hydroxymethyl)ethyl]pyrazole-4-sulfonamide;
N-(3,4-dichloro-1H-indol-7-yl)-1-(2-hydroxy-2-methyl-propyl)pyrazole-4-sulfonamide;
1-(2-amino-2-methyl-propyl)-N-(4-chloro-3-cyano-1H-indol-7-yl)pyrazole-4-sulfonamide;
N-(4-chloro-3-cyano-1H-indol-7-yl)-1-[(1-hydroxycyclobutyl)methyl]pyrazole-4-sulfonamide;
5-amino-N-(4-chloro-3-cyano-1H-indol-7-yl)-1-methyl-pyrazole-4-sulfonamide;
N-(3,4-dichloro-1H-indol-7-yl)-1-(methylsulfonylmethyl)pyrazole-4-sulfonamide;
N-(4-chloro-3-cyano-1H-indol-7-yl)-1-(methylsulfonylmethyl)pyrazole-4-sulfonamide;
N-(4-chloro-3-cyano-1H-indol-7-yl)-1-[(1-cyanocyclopropyl)methyl]pyrazole-4-sulfonamide;
N-(4-chloro-3-cyano-1H-indol-7-yl)-1-[(4-hydroxytetrahydropyran-4-yl)methyl]pyrazole-4-sulfonamide;
N-(4-chloro-3-cyano-1H-indol-7-yl)-5-fluoro-1-(2-hydroxy-2-methyl-propyl)pyrazole-4-sulfonamide;
N-(4-chloro-3-cyano-1H-indol-7-yl)-1-(3-methoxycyclobutyl)pyrazole-4-sulfonamide;
N-(3-chloro-1H-indol-7-yl)-1-(3-methoxycyclobutyl)pyrazole-4-sulfonamide;
N-(4-chloro-3-cyano-1H-indol-7-yl)-1-[1-(hydroxymethyl)cyclobutyl]pyrazole-4-sulfonamide;
N-(3-cyano-4-fluoro-1H-indol-7-yl)-1-(2-hydroxy-2-methyl-propyl)pyrazole-4-sulfonamide;
N-(3-cyano-4-methyl-1H-indol-7-yl)-1-(3-hydroxycyclobutyl)pyrazole-4-sulfonamide;
N-(3-chloro-1H-indol-7-yl)-1-(3-hydroxycyclobutyl)pyrazole-4-sulfonamide;
N-(4-chloro-3-cyano-1H-indol-7-yl)-1-[[1-(hydroxymethyl)cyclopropyl]methyl]pyrazole-4-sulfonamide;
N-(4-chloro-3-cyano-1H-indol-7-yl)-1-[(2S)-2-hydroxypropyl]pyrazole-4-sulfonamide;
N-(4-chloro-3-cyano-1H-indol-7-yl)-1-[(2R)-2-hydroxypropyl]pyrazole-4-sulfonamide;
N-(3-cyano-4-methyl-1H-indol-7-yl)-1-(2-hydroxy-1,1-dimethyl-ethyl)pyrazole-4-sulfonamide;
N-(4-chloro-3-cyano-1H-indol-7-yl)-3-fluoro-1-(2-hydroxy-2-methyl-propyl)pyrazole-4-sulfonamide;
N-(3-cyano-4-methyl-1H-indol-7-yl)-3-fluoro-1-(2-hydroxy-2-methyl-propyl)pyrazole-4-sulfonamide;
N-(3-cyano-4-fluoro-1H-indol-7-yl)-3-fluoro-1-(2-hydroxy-2-methyl-propyl)pyrazole-4-sulfonamide;
N-(3-cyano-4-methyl-1H-indol-7-yl)-1-[(1R)-2-hydroxy-1-methyl-ethyl]pyrazole-4-sulfonamide;
N-(4-chloro-3-cyano-1H-indol-7-yl)-1-[(1R)-2-hydroxy-1-methyl-ethyl]pyrazole-4-sulfonamide;
N-(4-chloro-3-cyano-1H-indol-7-yl)-1-(3-hydroxypropyl)pyrazole-4-sulfonamide;
N-(3 -cyano-4-methyl-1H-indol-7-yl)-1-(3-hydroxypropyl)pyrazole-4-sulfonamide;
N-(3-cyano-4-fluoro-1H-indol-7-yl)-1-(3-hydroxypropyl)pyrazole-4-sulfonamide;
N-(3-cyano-4-fluoro-1H-indol-7-yl)-1-[(1R)-2-hydroxy-1-methyl-ethyl]pyrazole-4-sulfonamide;
(2R)-2-[4-[(4-chloro-3-cyano-1H-indol-7-yl)sulfamoyl]pyrazol-1-yl]propenamide;
N-(3-cyano-4-fluoro-1H-indol-7-yl)-1H-pyrazole-4-sulfonamide;
N-(3-cyano-4-fluoro-1H-indol-7-yl)-1-(difluoromethyl)pyrazole-4-sulfonamide;
N-(3-cyano-4-fluoro-1H-indol-7-yl)-1-(2,2,2-trifluoroethyl)pyrazole-4-sulfonamide;
N-(3-cyano-4-fluoro-1H-indol-7-yl)-1-ethyl-pyrazole-4-sulfonamide;
1-(3-bicyclo[1.1.1]pentanyl)-N-(3-cyano-4-methyl-1H-indol-7-yl)pyrazole-4-sulfonamide;

1-(3-bicyclo [1.1.1]pentanyl)-N-(3-cyano-4-fluoro-1H-indol-7-yl)pyrazole-4-sulfonamide;
N-(4-chloro-3-cyano-1H-indol-7-yl)-1-(3-hydroxy-3-methyl-butyl)pyrazole-4-sulfonamide;
N-(3-cyano-4-methyl-1H-indol-7-yl)-1-(3-hydroxy-3-methyl-butyl)pyrazole-4-sulfonamide;
N-(3-cyano-4-fluoro-1H-indol-7-yl)-1-(3-hydroxy-3-methyl-butyl)pyrazole-4-sulfonamide;
1-(3-bicyclo[1.1.1]pentanyl)-N-(4-chloro-3-cyano-1H-indol-7-yl)pyrazole-4-sulfonamide;
N-(3-cyano-4-fluoro-1H-indol-7-yl)-1-(2-hydroxy-1,1-dimethyl-ethyl)pyrazole-4-sulfonamide;
N-(3-cyano-4-fluoro-1H-indol-7-yl)-1-[(1R)-2-fluoro-1-methyl-ethyl]pyrazole-4-sulfonamide;
N-(4-chloro-3-cyano-1H-indol-7-yl)-1-[(1S)-2,2-difluoro-1-(hydroxymethyl)ethyl]pyrazole-4-sulfonamide;
N-(3-cyano-4-methyl-1H-indol-7-yl)-1-[(1S)-2,2-difluoro-1-(hydroxymethyl)ethyl]pyrazole-4-sulfonamide;
N-(3-cyano-4-fluoro-1H-indol-7-yl)-1-[(1S)-2,2-difluoro-1-(hydroxymethyl)ethyl]pyrazole-4-sulfonamide;
N-(3-cyano-4-methyl-1H-indol-7-yl)-1-(3-hydroxy-1,1-dimethyl-propyl)pyrazole-4-sulfonamide;
N-(4-chloro-3 -cyano-1H-indol-7-yl)-1-(3-hydroxy-1,1-dimethyl-propyl)pyrazole-4-sulfonamide; N-(3-cyano-4-methyl-1H-indol-7-yl)-1-(3-hydroxy-1,1,3-trimethyl-butyl)pyrazole-4-sulfonamide;
N-(4-chloro-3-cyano-1H-indol-7-yl)-1-[(1S,2R)-2-hydroxy-1-methyl-propyl]pyrazole-4-sulfonamide;
N-(3-cyano-4-fluoro-1H-indol-7-yl)-1-[(1S,2R)-2-hydroxy-1-methyl-propyl]pyrazole-4-sulfonamide;
N-(3-cyano-4-methyl-1H-indol-7-yl)-1-[(1S,2R)-2-hydroxy-1-methyl-propyl]pyrazole-4-sulfonamide;
N-(3-cyano-1H-indol-7-yl)-1-(2-hydroxy-1,1-dimethyl-ethyl)pyrazole-4-sulfonamide;
N-(3-cyano-4-methyl-1H-indol-7-yl)-1-(3-hydroxy-1-methyl-propyl)pyrazole-4-sulfonamide;
N-(4-chloro-3 -cyano-1H-indol-7-yl)-1-(3-hydroxy-1-methyl-propyl)pyrazole-4-sulfonamide;
N-(4-chloro-3-cyano-1H-indol-7-yl)-1-[(1S)-1-(fluoromethyl)-2-hydroxy-ethyl]pyrazole-4-sulfonamide;
N-(3-cyano-4-methyl-1H-indol-7-yl)-5-(2-hydroxyethyl)-1-methyl-pyrazole-4-sulfonamide;
2-[4-[(3-cyano-4-fluoro-1H-indol-7-yl)sulfamoyl]pyrazol-1-yl]-2-methyl-propanamide;
2-[4-[(3-cyano-4-methyl-1H-indol-7-yl)sulfamoyl]pyrazol-1-yl]-2-methyl-propanamide;
N-(3-cyano-4-methyl-1H-indol-7-yl)-1-(3-hydroxy-1,3-dimethyl-butyl)pyrazole-4-sulfonamide;
N-(4-chloro-3-cyano-1H-indol-7-yl)-1-(3-hydroxy-1,3-dimethyl-butyl)pyrazole-4-sulfonamide;
5-chloro-N-(3-cyano-4-methyl-1H-indol-7-yl)-1-(2-hydroxy-1,1-dimethyl-ethyl)pyrazole-4-sulfonamide;
5-chloro-N-(3-cyano-4-fluoro-1H-indol-7-yl)-1-(2-hydroxy-1,1-dimethyl-ethyl)pyrazole-4-sulfonamide;
N-(3-cyano-4-methyl-1H-indol-7-yl)-1-[(1S)-1-(fluoromethyl)-2-hydroxy-ethyl]pyrazole-4-sulfonamide;
N-(3-cyano-4-fluoro-1H-indol-7-yl)-1-[(1S)-1-(fluoromethyl)-2-hydroxy-ethyl]pyrazole-4-sulfonamide;
1-[(2S)-2-amino-3,3,3-trifluoro-propyl]-N-(4-chloro-3-cyano-1H-indol-7-yl)pyrazole-4-sulfonamide;
1-[(2S)-2-amino-3,3,3-trifluoro-propyl]-N-(3-cyano-4-methyl-1H-indol-7-yl)pyrazole-4-sulfonamide;
5-chloro-N-(4-chloro-3-cyano-1H-indol-7-yl)-1-(2-hydroxy-1,1-dimethyl-ethyl)pyrazole-4-sulfonamide;
5-chloro-N-(3-cyano-1H-indol-7-yl)-1-(2-hydroxy-1,1-dimethyl-ethyl)pyrazole-4-sulfonamide;
3-chloro-N-(3-cyano-4-methyl-1H-indol-7-yl)-1-(2-hydroxy-1,1-dimethyl-ethyl)pyrazole-4-sulfonamide;
3-chloro-N-(3-cyano-1H-indol-7-yl)-1-(2-hydroxy-1,1-dimethyl-ethyl)pyrazole-4-sulfonamide;
tert-butyl 3-[[4-[(4-chloro-3-cyano-1H-indol-7-yl)sulfamoyl]pyrazol-1-yl]methyl]-3-fluoro-azetidine-1-carboxylate;
tert-butyl 3-[[4-[(3-cyano-4-methyl-1H-indol-7-yl)sulfamoyl]pyrazol-1-yl]methyl]-3-fluoro-azetidine-1-carboxylate;
N-(4-chloro-3-cyano-1H-indol-7-yl)-1-[(3-fluoroazetidin-3-yl)methyl]pyrazole-4-sulfonamide;
N-(3-cyano-4-methyl-1H-indol-7-yl)-1-[(3-fluoroazetidin-3-yl)methyl]pyrazole-4-sulfonamide;
N-(3-cyano-4-methyl-1H-indol-7-yl)-1-(3-hydroxy-2,2-dimethyl-propyl)pyrazole-4-sulfonamide;
N-(4-chloro-3-cyano-1H-indol-7-yl)-1-(3 -hydroxy-2,2-dimethyl-propyl)pyrazole-4-sulfonamide;
3-chloro-N-(4-chloro-3-cyano-1H-indol-7-yl)-1-(2-hydroxy-1,1-dimethyl-ethyl)pyrazole-4-sulfonamide;
3 -chloro-N-(3 -cyano-4-fluoro-1H-indol-7-yl)-1-(2-hydroxy-1,1-dimethyl-ethyl)pyrazole-4-sulfonamide;
N-(4-chloro-3-cyano-1H-indol-7-yl)-1-[1-(hydroxymethyl)-2-methoxy-ethyl]pyrazole-4-sulfonamide;
N-(3-cyano-4-methyl-1H-indol-7-yl)-1-[1-(hydroxymethyl)-2-methoxy-ethyl]pyrazole-4-sulfonamide;
N-(3-cyano-4-methyl-1H-indol-7-yl)-1-[(2R)-3-hydroxy-2-methyl-propyl]pyrazole-4-sulfonamide;
N-(4-chloro-3-cyano-1H-indol-7-yl)-1-[(2R)-3-hydroxy-2-methyl-propyl]pyrazole-4-sulfonamide;
N-(4-chloro-3-cyano-1H-indol-7-yl)-1-[(1S)-2-hydroxy-1-methyl-ethyl]pyrazole-4-sulfonamide;
N-(3-cyano-4-fluoro-1H-indol-7-yl)-1-[(1S)-2-hydroxy-1-methyl-ethyl]pyrazole-4-sulfonamide;
N-(3-cyano-4-methyl-1H-indol-7-yl)-1-[1-(hydroxymethyl)cyclopropyl]pyrazole-4-sulfonamide;
N-(4-chloro-3-cyano-1H-indol-7-yl)-1-[1-(hydroxymethyl)cyclopropyl]pyrazole-4-sulfonamide;
N-(3-cyano-4-methyl-1H-indol-7-yl)-1-(3-hydroxybutyl)pyrazole-4-sulfonamide;
5-amino-N-(3-cyano-4-methyl-1H-indol-7-yl)-1-ethyl-pyrazole-4-sulfonamide;
5-amino-N-(3-cyano-4-fluoro-1H-indol-7-yl)-1-ethyl-pyrazole-4-sulfonamide;
5-amino-N-(4-chloro-3-cyano-1H-indol-7-yl)-1-ethyl-pyrazole-4-sulfonamide;
N-(3-cyano-4-methyl-1H-indol-7-yl)-1-[(1R,2S)-2-hydroxy-1-methyl-propyl]pyrazole-4-sulfonamide;
N-(4-chloro-3-cyano-1H-indol-7-yl)-1-[(1R,2S)-2-hydroxy-1-methyl-propyl]pyrazole-4-sulfonamide;
5-chloro-N-(3-cyano-4-methyl-1H-indol-7-yl)-1-(2-hydroxy-2-methyl-propyl)pyrazole-4-sulfonamide;

5-amino-N-(3 -cyano-4-methyl-1H-indol-7-yl)-1-(2,2-difluoroethyl)pyrazole-4-sulfonamide;
5-amino-N-(4-chloro-3-cyano-1H-indol-7-yl)-1-(2,2-difluoroethyl)pyrazole-4-sulfonamide;
5-amino-N-(3-cyano-4-fluoro-1H-indol-7-yl)-1-(2,2-difluoroethyl)pyrazole-4-sulfonamide;
5-chloro-N-(3-cyano-4-fluoro-1H-indol-7-yl)-1-(2-hydroxy-2-methyl-propyl)pyrazole-4-sulfonamide;
N-(3-cyano-4-methyl-1H-indol-7-yl)-1-[(1R)-2-fluoro-1-methyl-ethyl]pyrazole-4-sulfonamide; 5-amino-N-(3-cyano-4-fluoro-1H-indol-7-yl)-1-methyl-pyrazole-4-sulfonamide;
5-amino-N-(3 -cyano-4-methyl-1H-indol-7-yl)-1-(2-hydroxy-2-methyl-propyl)pyrazole-4-sulfonamide;
5-amino-N-(3-cyano-4-methyl-1H-indol-7-yl)-1-(difluoromethyl)pyrazole-4-sulfonamide;
5-amino-N-(3-cyano-4-fluoro-1H-indol-7-yl)-1-(difluoromethyl)pyrazole-4-sulfonamide;
N-(4-chloro-3-cyano-1H-indol-7-yl)-1-[(1R,2R)-2-hydroxy-1-methyl-propyl]pyrazole-4-sulfonamide;
N-(3-cyano-4-methyl-1H-indol-7-yl)-1-[(1R,2R)-2-hydroxy-1-methyl-propyl]pyrazole-4-sulfonamide;
N-(3-cyano-4-fluoro-1H-indol-7-yl)-1-[(1R,2R)-2-hydroxy-1-methyl-propyl]pyrazole-4-sulfonamide;
5-amino-N-(4-chloro-3-cyano-1H-indol-7-yl)-1-(2-hydroxy-2-methyl-propyl)pyrazole-4-sulfonamide;
3-amino-N-(3-cyano-4-methyl-1H-indol-7-yl)-1-ethyl-pyrazole-4-sulfonamide;
3-amino-N-(3-cyano-4-fluoro-1H-indol-7-yl)-1-ethyl-pyrazole-4-sulfonamide;
5-amino-N-(3-cyano-1H-indol-7-yl)-1-(difluoromethyl)pyrazole-4-sulfonamide;
N-(3-cyano-4-methyl-1H-indol-7-yl)-1-(1 -fluoro-2-hydroxy-1-methyl-ethyl)pyrazole-4-sulfonamide;
N-(3 -cyano-4-fluoro-1H-indol-7-yl)-1-(1 -fluoro-2-hydroxy-1-methyl-ethyl)pyrazole-4-sulfonamide
3-amino-N-(3-cyano-4-methyl-1H-indol-7-yl)-1H-pyrazole-4-sulfonamide;
3-amino-N-(4-chloro-3-cyano-1H-indol-7-yl)-1H-pyrazole-4-sulfonamide;
N-(3-cyano-4-methyl-1H-indol-7-yl)-1-(1-fluoro-2-hydroxy-ethyl)pyrazole-4-sulfonamide;
N-(3-cyano-4-fluoro-1H-indol-7-yl)-1-(1-fluoro-2-hydroxy-ethyl)pyrazole-4-sulfonamide;
5-amino-N-(3-cyano-1H-indol-7-yl)-1-methyl-pyrazole-4-sulfonamide;
3-amino-N-(3-cyano-4-fluoro-1H-indol-7-yl)-1H-pyrazole-4-sulfonamide;
N-(3-cyano-4-methyl-1H-indol-7-yl)-1-(1,1-difluoro-2-hydroxy-ethyl)pyrazole-4-sulfonamide;
N-(3-cyano-4-fluoro-1H-indol-7-yl)-1 -(fluoromethyl)pyrazole-4-sulfonamide;
5-amino-N-(3-cyano-4-methyl-1H-indol-7-yl)-1-[(1R)-2-hydroxy-1-methyl-ethyl]pyrazole-4-sulfonamide;
5-amino-N-(3-cyano-4-methyl-1H-indazol-7-yl)-1-methyl-pyrazole-4-sulfonamide;
N-(3-cyano-4-methyl-1H-indazol-7-yl)-1-(fluoromethyl)pyrazole-4-sulfonamide;
5-amino-N-(4-chloro-3-cyano-1H-indol-7-yl)-1-[(1R)-2-hydroxy-1-methyl-ethyl]pyrazole-4-sulfonamide;
5-amino-N-(4-chloro-3-cyano-1H-indazol-7-yl)-1-methyl-pyrazole-4-sulfonamide;
5-amino-N-(3-cyano-4-methyl-1H-indol-7-yl)-1-(fluoromethyl)pyrazole-4-sulfonamide;
5-amino-N-(3-cyano-4-fluoro-1H-indol-7-yl)-1-(fluoromethyl)pyrazole-4-sulfonamide;
N-(4-chloro-3-cyano-1H-indazol-7-yl)-1-[(1R)-1-(fluoromethyl)-2-hydroxy-ethyl]pyrazole-4-sulfonamide;
N-(4-chloro-3-cyano-1H-indol-7-yl)-1-[(1R)-1-(fluoromethyl)-2-hydroxy-ethyl]pyrazole-4-sulfonamide;
5-amino-N-(3-cyano-1H-indol-7-yl)-1-(fluoromethyl)pyrazole-4-sulfonamide;
N-(3-cyano-4-methyl-1H-indol-7-yl)-1-[(1R)-1-(fluoromethyl)-2-hydroxy-ethyl]pyrazole-4-sulfonamide;
N-(3-cyano-4-methyl-1H-indazol-7-yl)-1-[(1R)-1-(fluoromethyl)-2-hydroxy-ethyl]pyrazole-4-sulfonamide;
5-amino-N-(3-cyano-4-methyl-1H-indol-7-yl)-1-(2-hydroxy-1,1-dimethyl-ethyl)pyrazole-4-sulfonamide; and
5-amino-N-(4-chloro-3-cyano-1H-indol-7-yl)-1-(2-hydroxy-1,1-dimethyl-ethyl)pyrazole-4-sulfonamide.

While preferred embodiments of the present invention have been shown and described herein, it will be obvious to those skilled in the art that such embodiments are provided by way of example only. It is intended that the appended claims define the scope of the invention and that methods and structures within the scope of these claims and their equivalents be covered thereby.

Unless defined otherwise, all technical and scientific terms used herein have the same meaning as is commonly understood by one of skill in the art to which this invention belongs.

As used in the specification and claims, the singular form "a", "an" and "the" include plural references unless the context clearly dictates otherwise.

Unless defined otherwise, the term "Cx-Cy" when used in conjunction with a chemical moiety, such as alkyl, alkenyl, or alkynyl is meant to include groups that contain from x to y carbons in the chain. For example, the term "Cx-Cy alkyl" refers to substituted or unsubstituted saturated hydrocarbon groups, including straight-chain alkyl and branched-chain alkyl groups that contain from x to y carbons in the chain.

The term "alkyl" as used herein refers to saturated linear or branched-chain monovalent hydrocarbon radicals. In one embodiment, an alkyl group contains from about 1 to about 10 carbon atoms. The term "C1-C10 alkyl" as used herein refers to saturated linear or branched-chain monovalent hydrocarbon radicals of one to 10 carbon atoms, respectively. The term "C1-C4 alkyl" as used herein refers to saturated linear or branched-chain monovalent hydrocarbon radicals of one to four carbon atoms, respectively. Illustrative examples of C1-C4 alkyl include methyl, ethyl, 1-propyl,

2-propyl, 1-butyl, 2-methyl-1-propyl, 2-butyl, and 2-methyl-2-propyl. The term "C1-C6 alkyl" as used herein refers to saturated linear or branched-chain monovalent hydrocarbon radicals of one to six carbon atoms, respectively. Illustrative examples of C1-C6 alkyl include, but are not limited to, methyl, ethyl, 1-propyl, 2-propyl, I-butyl, 2-methyl-1-propyl, 2-butyl, 2-methyl-2-propyl, 1-hexyl, 2-hexyl, 3-hexyl, 3,3-dimethylbutabyl, 2,2-dimethylbutanyl, 2,3-dimethylbutanyl, 2-methylpentanyl, 3-methylpentanyl, and 4-methylpentanyl. Alkyl, C1-C4 alkyl or C1-C6 alkyl groups used herein can be optionally independently substituted by one or more of the following substituents: deuterium, halo, hydroxy, -O(C1-C4 alkyl), cyano, -C(=O)$NR^aR^b$, -C(=O)$OR^c$, -S(=O)$_2$(C1-C4 alkyl), -$NR^aR^b$, -$NR^a$C(=O)$OR^c$ and -$NR^a$C(=O)(C1-C4 alkyl), wherein each of $R^a$, $R^b$, and $R^c$ is independently hydrogen, C3-C6 cycloalkyl, heterocycloalkyl or C1-C4 alkyl, or $R^a$/$R^b$ and the nitrogen atom they are attached to form a 5 or 6-membered ring optionally independently substituted by one or more substituents selected from halo, C1-C4 alkyl, cyano, hydroxy and -O(C1-C4 alkyl).

The term "C1-C4 fluoroalkyl" as used herein refers to saturated linear or branched-chain monovalent hydrocarbon radicals of one to four carbon atoms substituted with one or more fluorine atoms. Illustrative examples include, but are not limited to, $CHF_2$, $CH_2F$, $CF_3$, $CH_2CF_3$, $CF_2CH_3$, $CHFCH_3$, $CF(CH_3)_2$, $CH(CF_3)_2$, $CHFCF_3$ and $CF_2CF_3$.

The term "C3-C6 cycloalkyl" as used herein refers to monocyclic non-aromatic radicals, which have 3, 4, 5, or up to 6 carbon ring atoms and may be fully saturated, or partially unsaturated. Illustrative examples of C3-C6 cycloalkyl groups include, but are not limited to, cyclopropyl, cyclobutyl, cyclopentyl and cyclohexyl. C3-C6 cycloalkyl groups used herein may be optionally independently substituted by one or more of the following substituents: deuterium, halo, hydroxy, -O(C1-C4 alkyl), cyano, -C(=O)$NR^aR^b$, -C(=O)$OR^c$, -S(=O)$_2$(C1-C4 alkyl), -$NR^aR^b$, -$NR^a$C(=O)$OR^c$ and -$NR^a$C(=O)(C1-C4 alkyl), wherein each of $R^a$, $R^b$, and $R^c$ is independently hydrogen, C3-C6 cycloalkyl, heterocycloalkyl or C1-C4 alkyl, or $R^a$/$R^b$ and the nitrogen atom they are attached to form a 5 or 6-membered ring optionally independently substituted by one or more substituents selected from halo, C1-C4 alkyl, cyano, hydroxy and -O(C1-C4 alkyl), or two substituents on the C3-C6 cycloalkyl ring form a 4-6 membered heterocycle or carbocycle.

The term "C2-C5 alkenyl" as used herein refers to straight or branched hydrocarbon chain radicals containing at least one double bond and having from two to five carbon atoms. A C2-C5 alkenyl is attached to the rest of a molecule by a single bond through an $sp^2$ hybridized carbon. Illustrative examples of C2-C5 alkenyl include, but are not limited to, ethenyl (i.e., vinyl), prop-1-enyl, but-1-enyl, pent-1-enyl, penta-1,4-dienyl. C2-C5 alkenyl groups used herein may be optionally independently substituted by one or more of the following substituents: deuterium, halo, hydroxy, -O(C1-C4 alkyl), cyano, -C(=O)$NR^aR^b$, -C(=O)$OR^c$, -S(=O)$_2$(C1-C4 alkyl), -$NR^aR^b$, -$NR^a$C(=O)$OR^c$ and -$NR^a$C(=O)(C1-C4 alkyl), wherein each of $R^a$, $R^b$, and $R^c$ is independently hydrogen, C3-C6 cycloalkyl, heterocycloalkyl or C1-C4 alkyl, or $R^a$/$R^b$ and the nitrogen atom they are attached to form a 5 or 6-membered ring optionally independently substituted by one or more substituents selected from halo, C1-C4 alkyl, cyano, hydroxy and -O(C1-C4 alkyl).

The term "heteroalkyl" refers to an alkyl group where 1, 2, or 3 of the carbon atoms is substituted by an O. In one embodiment, heteroalkyl is

$$—(CH_2)_{1\text{-}12}—$$ ,

wherein 1, 2, or 3 methylene groups are replaced by an oxygen. In another embodiment of the invention, heteroalkyl is selected from

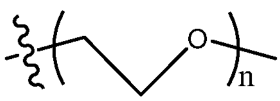 ,

wherein n is 1, 2, or 3.

The term "heterocycloalkyl" as used herein refers to a stable and not fully aromatic 3- to 18-membered ring (i.e., C3-C18 heterocycloalkyl) radical that comprises two to twelve ring carbon atoms and from one to six ring heteroatoms selected from nitrogen, oxygen and sulfur. Whenever it appears herein, a numerical range such as "3 to 18" refers to each integer in the given range; e.g., "3 to 18 ring atoms" means that the heterocycloalkyl group may consist of 3 ring atoms, 4 ring atoms, 5 ring atoms, etc., up to and including 18 ring atoms. In some embodiments, it is a C5-C10 heterocycloalkyl. In some embodiments, it is a C4-C10 heterocycloalkyl. In some embodiments, it is a C3-C10 heterocycloalkyl. The heterocycloalkyl radical may be a monocyclic, bicyclic, tricyclic or tetracyclic ring system, which may include fused or bridged ring systems. The heteroatoms, e.g. sulfur, in the heterocycloalkyl radical may be optionally oxidized. One or more nitrogen atoms, if present, are optionally quaternized. The heterocycloalkyl radical is partially or fully saturated. The heterocycloalkyl may be attached to the rest of a molecule through any atom of the ring(s). Examples of such heterocycloalkyl radicals include, but are not limited to, 6,7-dihydro-5H-cyclopenta[b]pyridine, dioxolanyl,

thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, I-oxo-thiomorpholinyl, and 1,1-dioxothiomorpholinyl. Heterocycloalkyl moieties, herein, may be optionally independently substituted by one or more substituents: halo, hydroxy, -O(C1-C4 alkyl), cyano, -C(=O)NR$^a$R$^b$, -C(=O)OR$^c$, -S(=O)z(C1-C4 alkyl), -NR$^a$R$^b$,-NR$^a$C(=O)OR$^c$ and -NR$^a$C(=O)(C1-C4 alkyl), wherein each R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

The term "phenyl" as used herein refers to a radical with the formula $C_6H_5$. One or more of the hydrogens in a phenyl moiety disclosed herein, may be optionally independently substituted by one or more of the following substituents: C1-C4 alkyl, halo, hydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

The term "heteroaryl" as used herein refers to 5-6 membered heteroaromatic rings having one or more heteroatoms selected from O, S and N. Heteroaryl moieties herein may be optionally independently substituted by one or more of the following substituents: C1-C4 alkyl, halo, hydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

The term "halogen" includes fluoro, chloro, bromo and iodo.

The term "oxy" means an oxygen (O) atom. The term "thio" means a sulfur (S) atom.

The term "oxo" means "=O". The term "carbonyl" means "C=O."

The term "substituted" as used herein refers to moieties having substituents replacing a hydrogen on one or more carbons or heteroatoms of the structure. It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc.

The term "optional" or "optionally" as used herein means that the subsequently described event of circumstances may or may not occur, and that the description includes instances where the event or circumstance occurs and instances in which it does not. For example, "optionally substituted aryl" means that the aryl group may or may not be substituted and that the description includes both substituted aryl groups and aryl groups having no substitution.

Compounds of the present disclosure also include crystalline and amorphous forms of those compounds and pharmaceutically acceptable salts.

It will be appreciated that certain compounds according to the invention may contain one or more centers of asymmetry and may therefore be prepared and isolated in a mixture of isomers such as a racemic mixture, or in an enantiomerically pure form. It is intended that all stereoisomeric forms of the compounds of the invention, including but not limited to, diastereomers, enantiomers and atropisomers, as well as mixtures thereof such as racemic mixtures, form part of the present invention.

In the structures shown herein, where the stereochemistry of any particular chiral atom is not specified, then all stereoisomers are contemplated and included as the compounds of the invention. Where stereochemistry is specified by a solid wedge or dashed line representing a particular configuration, then that stereoisomer is so specified and defined.

The invention is directed to compounds of Formulae I and II, including pharmaceutically acceptable salts thereof. In addition, the invention is directed to compounds of Formulae I and II and salts thereof, which are not necessarily pharmaceutically acceptable salts, and which may be useful as intermediates for preparing and/or purifying compounds of Formulae I and II and/or for separating enantiomers of compounds of Formulae I and II. Particular examples include hydrochloride and trifluoroacetate salts of compounds of Formulae I and II.

It will further be appreciated that the compounds of Formulae I and II or their salts may be isolated in the form of solvates, and accordingly that any such solvate is included within the scope of the present invention.

In the compounds of formulae I and II, the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds of formula I. For example, different isotopic forms of hydrogen (H) include protium ($^1$H) and deuterium ($^2$H or D). Protium is the predominant hydrogen isotope found in nature. Enriching for deuterium may afford certain therapeutic advantages, such as increasing *in vivo* half-life or reducing dosage

requirements, or may provide a compound useful as a standard for characterization of biological samples. Isotopically-enriched compounds within formula I can be prepared without undue experimentation by conventional techniques well known to those skilled in the art or by processes analogous to those described in the Schemes and Examples herein using appropriate isotopically-enriched reagents and/or intermediates.

Unless expressly stated to the contrary, all ranges cited herein are inclusive. For example, a heteroaryl ring described as containing from "1 to 3 heteroatoms" means the ring can contain 1, 2, or 3 heteroatoms. It is also to be understood that any range cited herein includes within its scope, all of the sub-ranges within that range. The oxidized forms of the heteroatoms N and S are also included within the scope of the present invention.

The term "salt" or "pharmaceutically acceptable salt" refers to salts derived from a variety of organic and inorganic counter ions well known in the art. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminum, and the like. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. In some embodiments, the pharmaceutically acceptable base addition salt is chosen from ammonium, potassium, sodium, calcium, and magnesium salts.

The term "pharmaceutically acceptable carrier, diluent or excipient" includes without limitation any adjuvant, carrier, excipient, glidant, sweetening agent, diluent, preservative, dye, colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

The term "effective amount" or "therapeutically effective amount" refers to that amount of a compound described herein that is sufficient to affect the intended application, including but not limited to disease treatment, as defined below. The therapeutically effective amount may vary depending upon the intended treatment application (in vivo), or the subject and disease condition being treated, e.g., the weight and age of the subject, the severity of the disease condition, the manner of administration and the like, which can readily be determined by one of ordinary skill in the art. The term also applies to a dose that will induce a particular response in target cells, e.g., reduction of platelet adhesion and/or cell migration. The specific dose will vary depending on the particular compounds chosen, the dosing regimen to be followed, whether it is administered in combination with other compounds, timing of administration, the tissue to which it is administered, and the physical delivery system in which it is carried.

As used herein, "treatment" or "treating" refers to an approach for obtaining beneficial or desired results with respect to a disease, disorder, or medical condition including but not limited to a therapeutic benefit and/or a prophylactic benefit. A therapeutic benefit can include, for example, the eradication or amelioration of the underlying disorder being treated. Also, a therapeutic benefit can include, for example, the eradication or amelioration of one or more of the physiological symptoms associated with the underlying disorder such that an improvement is observed in the subject, notwithstanding that the subject may still be afflicted with the underlying disorder. In certain embodiments, for prophylactic benefit, the compositions are administered to a subject at risk of developing a particular disease, or to a subject reporting one or more of the physiological symptoms of a disease, even though a diagnosis of this disease may not have been made.

A "therapeutic effect," as that term is used herein, encompasses a therapeutic benefit and/or a prophylactic benefit as described above. A prophylactic effect includes delaying or eliminating the appearance of a disease or condition, delaying or eliminating the onset of symptoms of a disease or condition, slowing, halting, or reversing the progression of a disease or condition, or any combination thereof.

The term "degrader" as used herein refers to a compound having the ability to induce the degradation of a target protein. For example, a degrader may induce the ubiquitination and subsequent proteasomal degradation of a target protein. Targeted protein degradation can be undertaken for the purposes of inhibiting the biological function of the target protein, meaning that a degrader can be a member of a specific sub-class of antagonist. Compounds that potentiate the formation of a complex between a target protein and any portion of an E3 ubiquitin ligase complex are specifically included within this definition.

The term "RBM39 degrader" as used herein refers to a compound, such as a compound of Formula I or II, having the ability to induce the degradation of RBM39 protein. For example, an RBM39 degrader may induce the ubiquitination and subsequent proteasomal degradation of RBM39 protein. Targeted protein degradation can be undertaken for the purposes of inhibiting the biological function of the target protein. Compounds that potentiate the formation of a complex between RBM39 protein and any portion of an E3 ubiquitin ligase complex are included within this definition.

The term "cell proliferation" refers to a phenomenon by which the cell number has changed as a result of division. This term also encompasses cell growth by which the cell morphology has changed (e.g., increased in size) consistent with a proliferative signal.

The term "subject" refers to an animal, such as a mammal, for example a human. The methods described herein can be useful in both human therapeutics and veterinary applications. In some embodiments, the subject is a mammal, and in some embodiments, the subject is human. "Mammal" includes humans and both domestic animals such as laboratory animals and household pets (*e.g.*, cats, dogs, swine, cattle, sheep, goats, horses, rabbits), and non-domestic animals such as wildlife and the like.

The term "in vivo" refers to an event that takes place in a subject's body.

The term "in vitro" refers to an event that takes places outside of a subject's body. For example, an *in vitro* assay encompasses any assay run outside of a subject. *In vitro* assays encompass cell-based assays in which cells alive or dead are employed. In vitro assays also encompass a cell-free assay in which no intact cells are employed.

The chemical naming protocol and structure diagrams used herein are a modified form of the I.U.P.A.C. nomenclature system, using ChemDraw Professional or OpenEye Scientific Software's mol2nam application. For complex chemical names employed herein, a substituent group is typically named before the group to which it attaches. For example, cyclopropylethyl comprises an ethyl backbone with a cyclopropyl substituent. Except as described below, all bonds are identified in the chemical structure diagrams herein, except for all bonds on some carbon atoms, which are assumed to be bonded to sufficient hydrogen atoms to complete the valency.

## Compounds

The chemical naming protocol and structure diagrams used herein are a modified form of the I.U.P.A.C. nomenclature

Referring to the $R_4$ substituent of Formula I, in certain embodiments, $R_4$ is unsubstituted or substituted C1-C6 alkyl, heteroalkyl, C1-C4 fluoroalkyl, C3-C6 cycloalkyl, -(C1-C4 alkyl)(C3-C6 cycloalkyl), -(C1-C4 alkyl)heterocycloalkyl, heterocycloalkyl -C1-C4alkylS(=O)$_2$(C1-C4 alkyl), S(=O)zphenyl, or S(=O)$_2$heteroaryl.

In certain other embodiments, $R_4$ is C1-C6 alkyl, heteroalkyl, and C1-C4 fluoroalkyl, substituted with 0, 1, 2, or 3 substituents independently selected from deuterium, halo, hydroxy, -(C0-C4alkyl)O(C1-C4 alkyl), -C1-C4 alkyl, -(C0-C4alkyl)cyano, -S(=O)$_2$(C1-C4 alkyl), - C0-C4 alkylC(=O)NR$^a$R$^b$, -C(=O)O(C1-C4 alkyl), C1-C4 fluoroalkyl, oxo, -(C1-C4 alkyl)OH, and -NH$_2$, wherein each of R$^a$ and R$^b$ is independently substituted with 0, 1, 2, or 3 hydrogen, C3-C6 cycloalkyl, heterocycloalkyl or C1-C4 alkyl. Particular examples of unsubstituted and substituted C1-C6 alkyl, heteroalkyl, and C1-C4 fluoroalkyl include, but not limited to, the following:

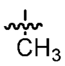 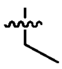 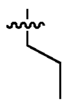 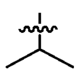 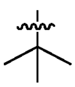 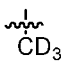 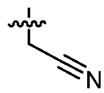 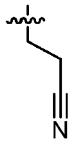 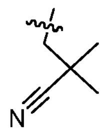 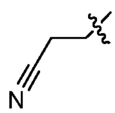

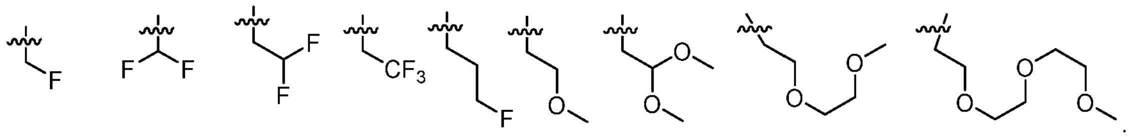

In certain embodiments of Formula I, $R_4$ is unsubstituted C3-C6 cycloalkyl or unsubstituted -(C1-C4 alkyl)(C3-C6 cycloalkyl). In certain other embodiments, $R_4$ is C3-C6 cycloalkyl or -(C1-C4 alkyl)(C3-C6 cycloalkyl) each independently substituted with 1, 2, or 3 substituents selected from deuterium, halo, hydroxy, -(C0-C4alkyl)O(C1-C4 alkyl), -C1-C4 alkyl, -(C0-C4alkyl)cyano, -S(=O)$_2$(C1-C4 alkyl), - C0-C4 alkylC(=O)NR$^a$R$^b$, -C(=O)O(C1-C4 alkyl), C1-C4 fluoroalkyl, oxo, -(C1-C4 alkyl)OH, and -NH$_2$, wherein each of R$^a$ and R$^b$ is independently substituted with 0, 1, 2, or 3 hydrogen, C3-C6 cycloalkyl, heterocycloalkyl or C1-C4 alkyl. Particular examples of -(C1-C4 alkyl)(C3-C6 cycloalkyl) and C3-C6 cycloalkyl include, but not limited to cyclopropyl, cyclobutyl, cyclopentyl, cyclohexyl, 3-bicyclo[1.1.1]pentanyl, cyclopropylmethyl, cyclobutylmethyl, cyclopentylmethyl, cyclohexylmethyl, cyclopropylethyl, cyclobutylethyl, cyclopentylethyl and cyclohexylmethyl, each independently substituted with 1, 2, or 3 substituents selected from deuterium, halo, hydroxy, -(C0-C4alkyl)O(C1-C4 alkyl), -C1-C4 alkyl, -(C0-C4alkyl)cyano, -S(=O)$_2$(C1-C4 alkyl), - C0-C4 alkylC(=O)NR$^a$R$^b$, -C(=O)O(C1-C4 alkyl), C1-C4 fluoroalkyl, oxo, -(C1-C4 alkyl)OH, and -NH$_2$, wherein each of R$^a$ and R$^b$ is independently substituted with 0, 1, 2, or 3 hydrogen, C3-C6 cycloalkyl, heterocycloalkyl or C1-C4 alkyl.

In certain embodiments of Formula I, $R_4$ is unsubstituted -(C1-C4 alkyl)heterocycloalkyl or heterocycloalkyl. In

certain other embodiments, $R_4$ is substituted -(C1-C4 alkyl)heterocycloalkyl or heterocycloalkyl wherein **$R_4$** is substituted with 0, 1, 2, or 3 substituents selected from deuterium, halo, hydroxy, -(C0-C4alkyl)O(C1-C4 alkyl), -C1-C4 alkyl, -(C0-C4alkyl)cyano, $-S(=O)_2$(C1-C4 alkyl), - C0-C4 alkylC(=O)$NR^aR^b$, -C(=O)O(C1-C4 alkyl), C1-C4 fluoroalkyl, oxo, -(C1-C4 alkyl)OH, and $-NH_2$, wherein each of $R^a$ and $R^b$ is independently substituted with 0, 1, 2, or 3 hydrogen, C3-C6 cycloalkyl, heterocycloalkyl or C1-C4 alkyl. Particular examples of unsubstituted and substituted -(C1-C4 alkyl)heterocycloalkyl and heterocycloalkyl include, but not limited to, the following:

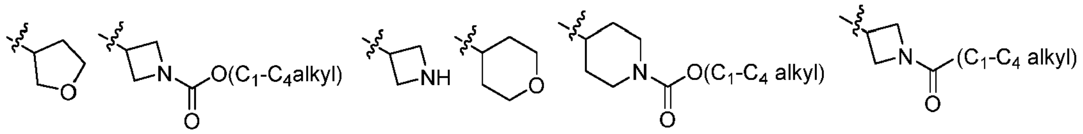

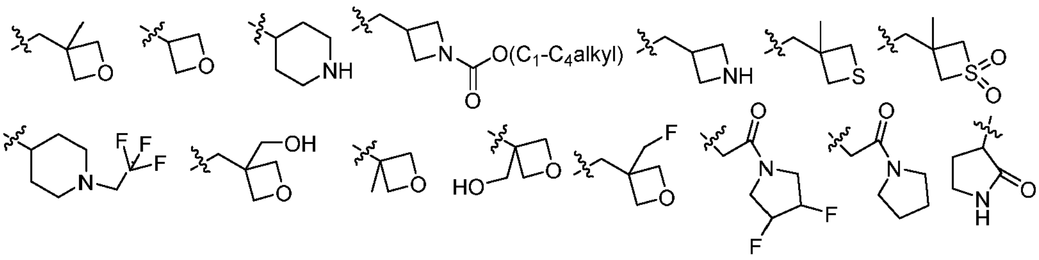

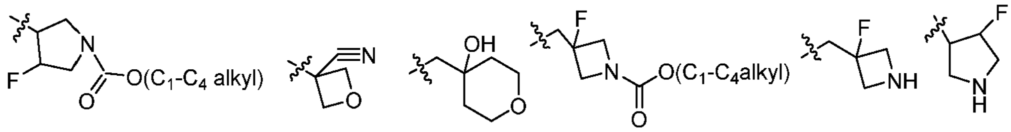

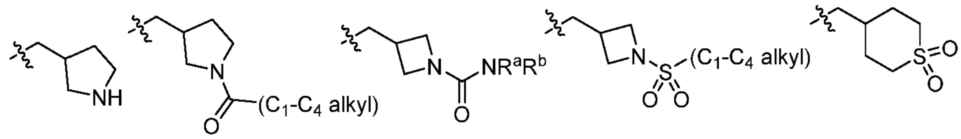

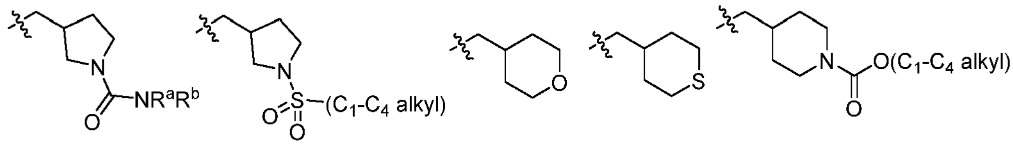

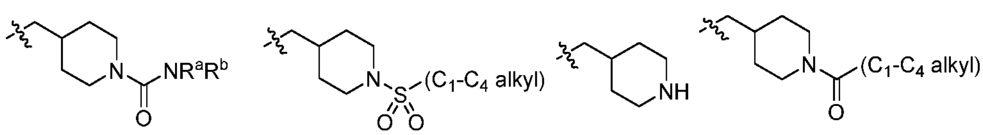

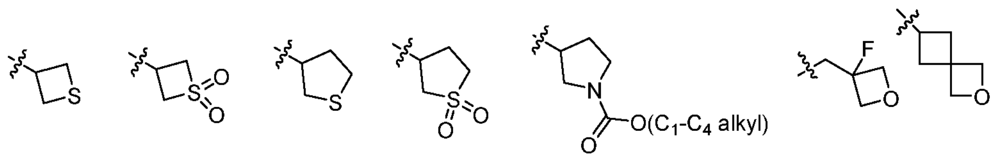

In certain embodiments of Formula I, $R_4$ is -C1-C4alkyl$S(=O)_2$(C1-C4 alkyl), $S(=O)_2$phenyl, or $S(=O)_2$heteroaryl, wherein **$R_4$** is substituted with 0, 1, 2, or 3 substituents selected from deuterium, halo, hydroxy, -O-(C0-C4alkyl)O(C1-C4 alkyl), -C1-C4 alkyl, -(C0-C4alkyl)cyano, $-S(=O)_2$(C1-C4 alkyl), - C0-C4 alkylC(=O)$NR^aR^b$, -C(=O)O(C1-C4 alkyl), C1-C4 fluoroalkyl, oxo, -(C1-C4 alkyl)OH, and $-NH_2$, wherein each of $R^a$ and $R^b$ is independently substituted with 0, 1, 2, or 3 hydrogen, C3-C6 cycloalkyl, heterocycloalkyl or C1-C4 alkyl. Particular examples of unsubstituted and substituted

-C1-C4alkylS(=O)$_2$(C1-C4 alkyl), S(=O)$_2$phenyl, and S(=O)$_2$heteroaryl include, but not limited to, the following:

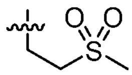
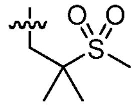
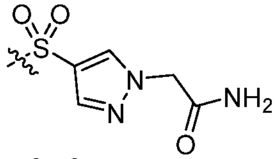
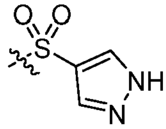
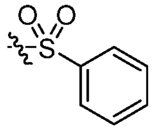
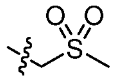
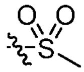
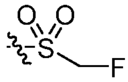

.

In certain embodiments of Formula I, $R_4$ is unsubstituted. Referring to the $R_4$ substituent of Formula I, in certain embodiments, $\mathbf{R_4}$ is -C0-C4 alkylC(=O)heterocycloalkyl, -(C0-C4 alkyl)C(=O)O(C1-C4 alkyl), -(C0-C4 alkyl)C(=O)C1-C4 alkyl, -(C0-C4 alkyl)C(=O)OH, -C0-C4 alkyl C(=O)NR$^a$R$^b$, or -(C1-C8 alkyl)OH, wherein $\mathbf{R_4}$ is substituted with 0, 1, 2, or 3 substituents selected from deuterium, halo, hydroxy, --(C0-C4alkyl)O(C1-C4 alkyl), -C1-C4 alkyl, -(C0-C4alkyl)cyano, -S(=O)$_2$(C1-C4 alkyl), -C0-C4 alkylC(=O)NR$^a$R$^b$, -C(=O)O(C1-C4 alkyl), C1-C4 fluoroalkyl, oxo, -(C1-C4 alkyl)OH, and -NH$_2$, wherein each of R$^a$ and R$^b$ is independently substituted with 0, 1, 2, or 3 hydrogen, C3-C6 cycloalkyl, heterocycloalkyl or C1-C4 alkyl. Particular examples of unsubstituted and substituted -C0-C4 alkylC(=O)heterocycloalkyl, -(C0-C4 alkyl)C(=O)O(C1-C4 alkyl), -(C0-C4 alkyl)C(=O)C1-C4 alkyl, -(C0-C4 alkyl)C(=O)OH or -(C1-C8 alkyl)OH, include, but not limited to, the following:

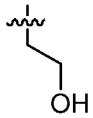
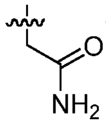
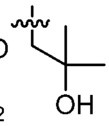
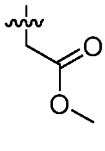
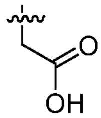
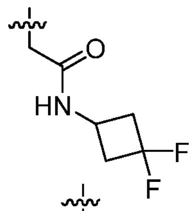
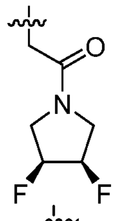
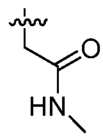
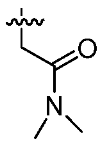
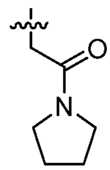
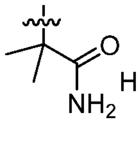
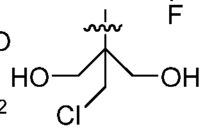
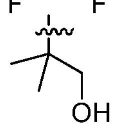
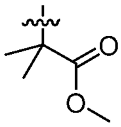
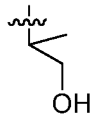
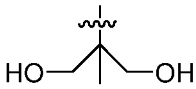
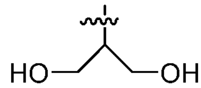
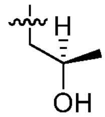
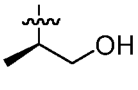
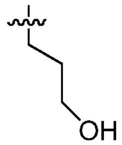
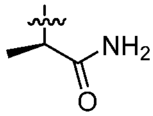
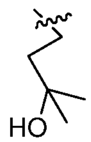
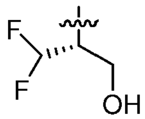
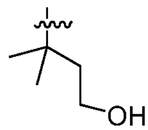
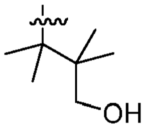
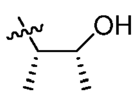
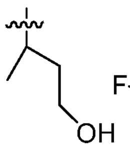
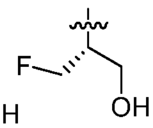
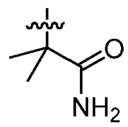
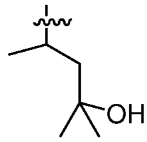

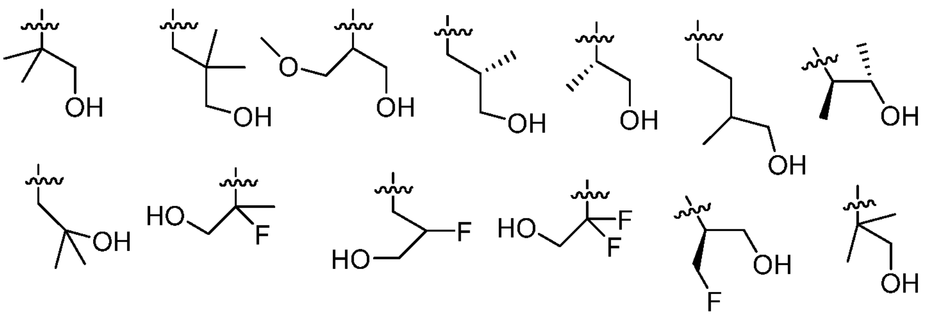

Referring to the $R_4$ substituent of Formula I, in certain embodiments, **$R_4$** is -(C1-C4 alkyl)phenyl, phenyl, heteroaryl, -(C1-C4 alkyl)heteroaryl, or C2-C5 alkenyl, wherein **$R_4$** is substituted with 0, 1, 2, or 3 substituents selected from deuterium, halo, hydroxy, -(C0-C4alkyl)O(C1-C4 alkyl), -C1-C4 alkyl, -(C0-C4alkyl)cyano, $-S(=O)_2$(C1-C4 alkyl), - C0-C4 alkylC(=O)$NR^aR^b$, -C(=O)O(C1-C4 alkyl), C1-C4 fluoroalkyl, oxo, -(C1-C4 alkyl)OH, and $-NH_2$, wherein each of $R^a$ and $R^b$ is independently substituted with 0, 1, 2, or 3 hydrogen, C3-C6 cycloalkyl, heterocycloalkyl or C1-C4 alkyl. Particular examples of unsubstituted and substituted is -(C1-C4 alkyl)phenyl, phenyl, heteroaryl, -(C1-C4 alkyl)heteroaryl, and C2-C5 alkenyl include, but are not limited to, the following:

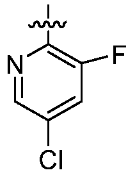 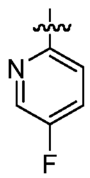 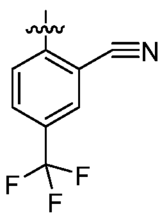 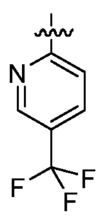 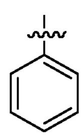 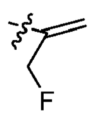.

Referring to the $R_5$ substituent of Formula I, in certain embodiments, $R_5$ is hydrogen. In certain other embodiments, $R_5$ is halo. In a further embodiment, $R_5$ is fluoro. In a further embodiment, $R_5$ is chloro. In certain other embodiments, $R_5$ is $NH_2$. In certain embodiments, $R_5$ is methyl.

Referring to the $R_6$ substituent of Formula I, in certain embodiments, $R_6$ is hydrogen. In certain other embodiments, $R_6$ is halo. In a further embodiment, $R_6$ is fluoro.

Referring to the $R_7$ substituent of Formula I, in certain embodiments, $R_7$ is hydrogen. In certain other embodiments, $R_7$ is heteroalkyl. In a further embodiment, $R_7$ is

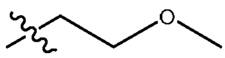.

In certain other embodiments, $R_7$ is -(C1-C4 alkyl)(C3-C6 cycloalkyl). In a further embodiment, $R_7$ is

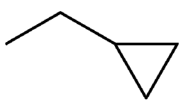.

Compounds of Formula I include compounds of Formula Ia, wherein $R_1$ is C1-C4 alkyl and $R_2$ is cyano; compounds of Formula Iaa, wherein $R_1$ is methyl and $R_2$ is cyano; compounds of Formula Ib, wherein $R_1$ is halo and $R_2$ is cyano; compounds of Formula Ibb, wherein $R_1$ is chloro or fluoro and $R_2$ is cyano; compounds of Formula Ic, wherein each of $R_1$ and $R_2$ is independently halo; compounds of Formula Icc, wherein each of $R_1$ and $R_2$ is chloro; compounds of Formula Id, wherein $R_1$ is hydrogen and $R_2$ is chloro; compounds of Formula If, wherein $R_1$ is -C1-C4 fluoroalkyl and $R_2$ is cyano; compounds of Formula Ifa, wherein $R_1$ is - $CF_3$ and $R_2$ is cyano; compounds of Formula Ifb, wherein $R_1$ is $-CF_3$ and $R_2$ is halo; compounds of Formula Ifc, compounds wherein $R_1$ is $-CF_3$ and $R_2$ is chloro or fluoro.

Referring to the $R_3$ substituent of Formulae Ia, Iaa, Ib, Ibb, Ic, Icc Id, If, Ifa, Ifb, and Ifc, in certain embodiments, $R_3$ is hydrogen.

For Formulae Ia, Iaa, Ib, Ibb, Ic, Icc Id, If, Ifa, Ifb, and Ifc, in certain embodiments, $R_3$ is halo. In a further

embodiment, $R_3$ is fluoro or chloro.

For Formulae Ia, Iaa, Ib, Ibb, Ic, Icc Id, If, Ifa, Ifb, and Ifc , in certain embodiments, $R_3$ is C1-C4 alkyl or C1-C4 fluoroalkyl. In a further embodiment, $R_3$ is methyl. In another further embodiment, $R_3$ is $CHF_2$.

For Formulae Ia, Iaa, Ib, Ibb, Ic, Icc Id, If, Ifa, Ifb, and Ifc, in certain embodiments, $R_3$ is $-NR^aR^b$. In a further embodiment, $R_3$ is $-NH_2$.

For Formulae Ia, Iaa, Ib, Ibb, Ic, Icc Id, If, Ifa, Ifb, and Ifc, in certain embodiments, $R_3$ is -(C1-C4 alkyl)OH. In a further embodiment, $R_3$ is $-CH_2CH_2OH$.

Referring to the $R_4$ substituent of the Formulae Ia, Iaa, Ib, Ibb, Ic, Icc Id, If, Ifa, Ifb, and Ifc, in certain embodiments, $R_4$ is hydrogen, C1-C6 alkyl, C1-C10heteroalkyl, C1-C4 fluoroalkyl, -(C1-C4 alkyl)$NH_2$, -(C1-C4 alkyl)phenyl, phenyl, heteroaryl, -(C1-C4 alkyl)heteroaryl, C2-C5 alkenyl, -C0-C4 alkylS(=O)$_2$(C1-C4 alkyl), -S(=O)$_2$phenyl, -S(=O)$_2$heteroaryl, -C0-C4 alkylC(=O)heterocycloalkyl, -C0-C4 alkyl C(=O)$NR^aR^b$, -(C0-C4 alkyl)C(=O)O(C1-C4 alkyl), -(C0-C4 alkyl)C(=O)C1-C4 alkyl, -(C0-C4 alkyl)C(=O)OH or -(C1-C8 alkyl)OH, wherein $\mathbf{R_4}$ is substituted with 0, 1, 2, or 3 substituents selected from deuterium, halo, hydroxy, -(C0-C4alkyl)O(C1-C4 alkyl), -C1-C4 alkyl, -(C0-C4alkyl)cyano, -S(=O)$_2$(C1-C4 alkyl), - C0-C4 alkylC(=O)$NR^aR^b$, -C(=O)O(C1-C4 alkyl), C1-C4 fluoroalkyl, oxo, -(C1-C4 alkyl)OH, and $-NH_2$, wherein each of $R^a$ and $R^b$ is independently substituted with 0, 1, 2, or 3 hydrogen, C3-C6 cycloalkyl, heterocycloalkyl or C1-C4 alkyl.

For the Formulae Ia, Iaa, Ib, Ibb, Ic, Icc Id, If, Ifa, Ifb, and Ifc, in certain embodiments, $R_4$ is C1-C6 alkyl, heteroalkyl, or C1-C4 fluoroalkyl, substituted with 0, 2, or 3 substituents independently selected from deuterium, halo, hydroxy, -(C0-C4alkyl)O(C1-C4 alkyl), -C1-C4 alkyl, -(C0-C4alkyl)cyano, -S(=O)$_2$(C1-C4 alkyl), - C0-C4 alkylC(=O)$NR^aR^b$, -C(=O)O(C1-C4 alkyl), C1-C4 fluoroalkyl, oxo, -(C1-C4 alkyl)OH, and $-NH_2$, wherein each of $R^a$ and $R^b$ is independently substituted with 0, 1, 2, or 3 hydrogen, C3-C6 cycloalkyl, heterocycloalkyl or C1-C4 alkyl. Particular examples of unsubstituted and substituted C1-C6 alkyl, heteroalkyl, and C1-C4 fluoroalkyl include, but not limited to, the following:

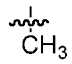 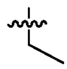 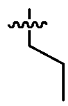 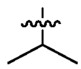 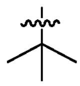 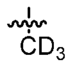 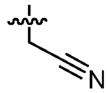 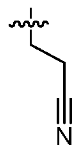 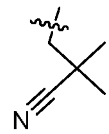 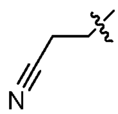

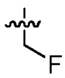 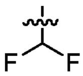 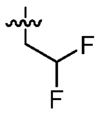 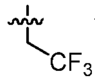 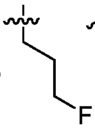 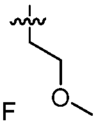 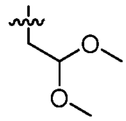 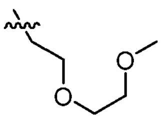 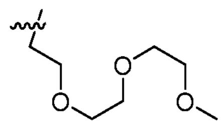

For Formulae Ia, Iaa, Ib, Ibb, Ic, Icc Id, If, Ifa, Ifb, and Ifc, in certain embodiments, $R_4$ is is C3-C6 cycloalkyl or -(C1-C4 alkyl)(C3-C6 cycloalkyl) each independently substituted 1, 2, or 3 substituents selectedfrom deuterium, halo, hydroxy, -(C0-C4alkyl)O(C1-C4 alkyl), -C1-C4 alkyl, -(C0-C4alkyl)cyano, -S(=O)$_2$(C1-C4 alkyl), - C0-C4 alkylC(=O)$NR^aR^b$, -C(=O)O(C1-C4 alkyl), C1-C4 fluoroalkyl, oxo, -(C1-C4 alkyl)OH, and $-NH_2$, wherein each of $R^a$ and $R^b$ is independently substituted with 0, 1, 2, or 3 hydrogen, C3-C6 cycloalkyl, heterocycloalkyl or C1-C4 alkyl. Particular examples of -(C1-C4 alkyl)(C3-C6 cycloalkyl) and C3-C6 cycloalkyl include, but not limited to cyclopropyl, cyclobutyl, cyclopentyl, cyclohexyl, 3-bicyclo[1.1.1]pentanyl, cyclopropylmethyl, cyclobutylmethyl, cyclopentylmethyl, cyclohexylmethyl, cyclopropylethyl, cyclobutylethyl, cyclopentylethyl and cyclohexylmethyl, each independently substituted 1, 2, or 3 substituents selected from deuterium, halo, hydroxy, -O(C1-C4 alkyl), -C1-C4 alkyl, -(C0-C4alkyl)cyano, -S(=O)$_2$(C1-C4 alkyl), - C0-C4 alkylC(=O)$NR^aR^b$, -C(=O)O(C1-C4 alkyl), C1-C4 fluoroalkyl, C1-C6heteroalkyl, oxo, -(C1-C4 alkyl)OH, and $-NH_2$, wherein each of $R^a$ and $R^b$ is independently substituted with 0, 1, 2, or 3 hydrogen, C3-C6 cycloalkyl, heterocycloalkyl or C1-C4 alkyl.

For Formulae Ia, Iaa, Ib, Ibb, Ic, Icc Id, If, Ifa, Ifb, and Ifc, in certain embodiments, $R_4$ is unsubstituted heterocycloalkyl. In certain other embodiments, $R_4$ is substituted heterocycloalkyl with one or more substituents independently selected from deuterium, C1-C4 alkyl, hydroxy, fluoro, -(C0-C4alkyl)O(C1-C4 alkyl), or cyano. In certain other embodiments, $R_4$ is substituted heterocycloalkyl with one or more substituents independently selected from deuterium, halo, hydroxy,-(C0-C4alkyl)O(C1-C4 alkyl), cyano, -C(=O)$NR^aR^b$, -C(=O)$OR^c$, -S(=O)$_2$(C1-C4 alkyl), $-NR^aR^b$, $-NR^aC$(=O)$OR^c$ or $-NR^aC$(=O)(C1-C4 alkyl), wherein each of $R^a$, $R^b$, and $R^c$ is independently hydrogen, C3-C6 cycloalkyl, heterocycloalkyl or C1-C4 alkyl, or $R^a/R^b$ and the nitrogen atom they are attached to form a 5 or 6-membered ring optionally independently substituted by one or more substituents selected from halo, C1-C4 alkyl, cyano, hydroxy and -O(C1-C4 alkyl). Particular examples of heterocycloalkyl include, but not limited to, the following:

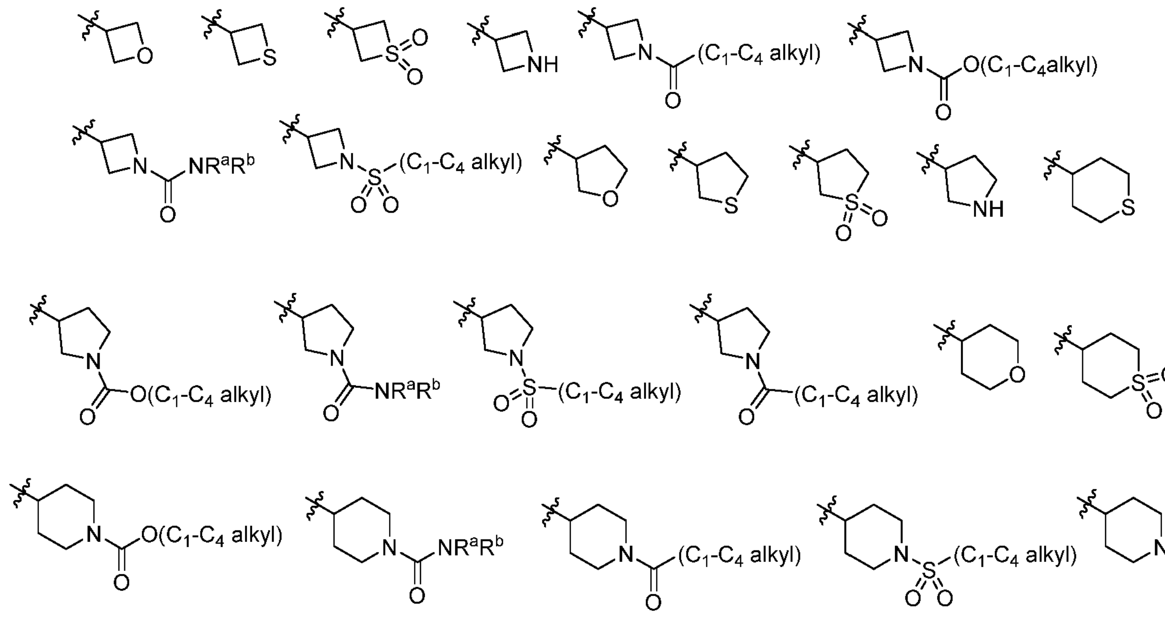

,

wherein each of the heterocycloalkyl is optionally substituted with one or more substituents independently selected from deuterium, halo, hydoxyhydroxyhydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

Referring to Formulae Ia, Iaa, Ib, Ibb, Ic, Icc Id, If, Ifa, Ifb, and Ifc, in certain embodiments, R$_4$ is -C0-C4 alkylC(=O)heterocycloalkyl, -(C0-C4 alkyl)C(=O)O(C1-C4 alkyl), -(C0-C4 alkyl)C(=O)C1-C4 alkyl, -(C0-C4 alkyl)C(=O)OH, -C0-C4 alkyl C(=O)NR$^a$R$^b$, or -(C1-C8 alkyl)OH, wherein **R$_4$** is substituted with 0, 1, 2, or 3 substituents selected from deuterium, halo, hydroxy, -(C0-C4alkyl)O(C1-C4 alkyl), -C1-C4 alkyl, -(C0-C4alkyl)cyano, -S(=O)$_2$(C1-C4 alkyl), -C0-C4 alkylC(=O)NR$^a$R$^b$, -C(=O)O(C1-C4 alkyl), C1-C4 fluoroalkyl, oxo, -(C1-C4 alkyl)OH, and -NH$_2$, wherein each of R$^a$ and R$^b$ is independently substituted with 0, 1, 2, or 3 hydrogen, C3-C6 cycloalkyl, heterocycloalkyl or C1-C4 alkyl. Particular examples of unsubstituted and substituted -C0-C4 alkylC(=O)heterocycloalkyl, -(C0-C4 alkyl)C(=O)O(C1-C4 alkyl), -(C0-C4 alkyl)C(=O)C1-C4 alkyl, -(C0-C4 alkyl)C(=O)OH or -(C1-C8 alkyl)OH, include, but not limited to, the following:

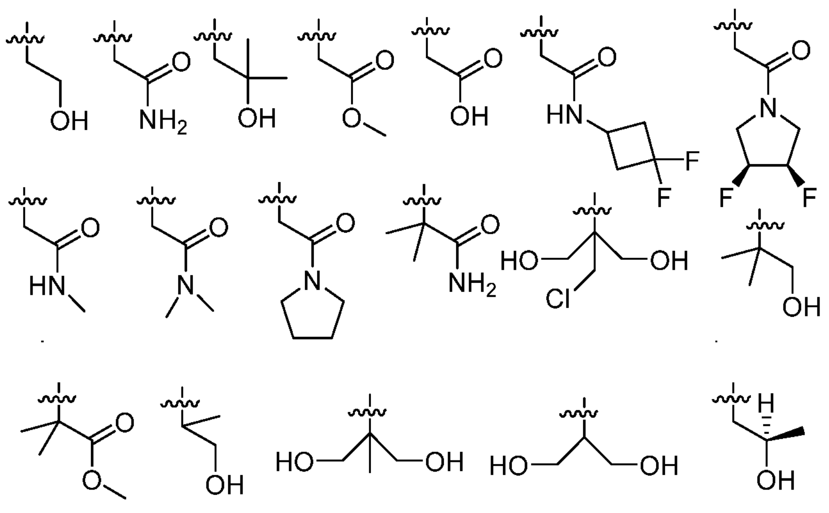

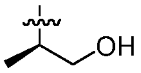 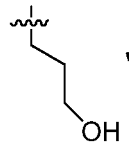 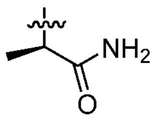 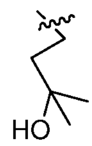 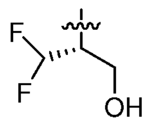 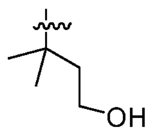

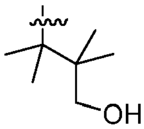 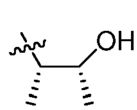 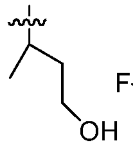 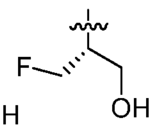 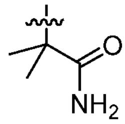 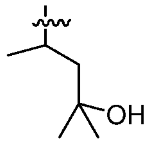

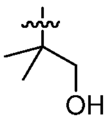 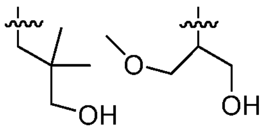 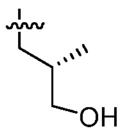 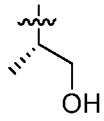 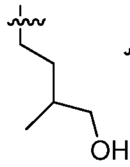 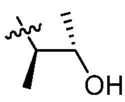

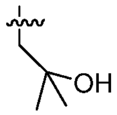 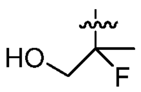 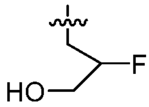 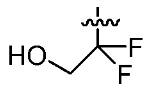 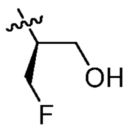 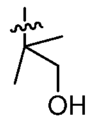.

Referring to the Formulae Ia, Iaa, Ib, Ibb, Ic, Icc Id, If, Ifa, Ifb, and Ifc, , in certain embodiments, $R_4$ is -(C1-C4 alkyl)phenyl, phenyl, heteroaryl, -(C1-C4 alkyl)heteroaryl, or C2-C5 alkenyl, wherein $\mathbf{R_4}$ is substituted with 0, 1, 2, or 3 substituents selected from deuterium, halo, hydroxy, -(C0-C4alkyl)O(C1-C4 alkyl), -C1-C4 alkyl, -(C0-C4alkyl)cyano, $-S(=O)_2$(C1-C4 alkyl), - C0-C4 alkylC(=O)$NR^aR^b$, -C(=O)O(C1-C4 alkyl), C1-C4 fluoroalkyl, oxo, -(C1-C4 alkyl)OH, and $-NH_2$, wherein each of $R^a$ and $R^b$ is independently substituted with 0, 1, 2, or 3 hydrogen, C3-C6 cycloalkyl, heterocycloalkyl or C1-C4 alkyl. Particular examples of unsubstituted and substituted is -(C1-C4 alkyl)phenyl, phenyl, heteroaryl, -(C1-C4 alkyl)heteroaryl, and C2-C5 alkenyl include, but are not limited to, the following:

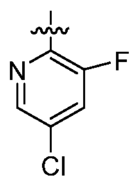 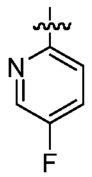 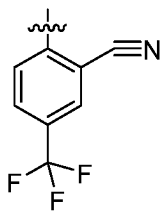 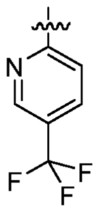 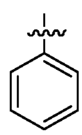 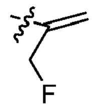.

Referring to the $R_5$ substituent of Formulae Ia, Iaa, Ib, Ibb, Ic, Icc Id, If, Ifa, Ifb, and Ifc, in certain embodiments, $R_5$ is hydrogen. In certain other embodiments, $R_5$ is halo. In a further embodiment, $R_5$ is fluoro or chloro. In certain other embodiments, $R_5$ is C1-C4 alkyl. In a further embodiment, $R_5$ is methyl. In certain other embodiments, $R_5$ is $NH_2$.

Referring to the Formulae Ia, Iaa, Ib, Ibb, Ic, Icc Id, If, Ifa, Ifb, and Ifc, in certain embodiments, $R_6$ is hydrogen. In certain other embodiments, $R_6$ is halo. In a further embodiment, $R_6$ is fluoro.

Referring to the Formulae Ia, Iaa, Ib, Ibb, Ic, Icc Id, If, Ifa, Ifb, and Ifc, in certain enbodiments, $R_7$ is hydrogen. In certain other embodiments, $R_7$ is heteroalkyl. In a further embodiment, $R_7$ is

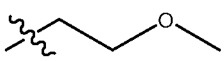.

In certain other embodiments, $R_7$ is -(C1-C4 alkyl)(C3-C6 cycloalkyl). In a further embodiment, $R_7$ is

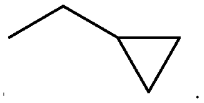

In one aspect, the present invention provides a compound of Formula II:

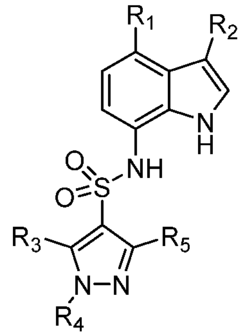

or a pharmaceutically acceptable salt thereof, wherein:

$R_1$ is hydrogen, C1-C4 alkyl or halo;
$R_2$ is halo or cyano;
$R_3$ is hydrogen, C1-C4 alkyl, halo, cyano or $-NR^aR^b$, wherein $R^a$ or $R^b$ is independently hydrogen, C3-C6 cycloalkyl, heterocycloalkyl or C1-C4 alkyl, or $R^a/R^b$ and the nitrogen atom they are attached to form a 5 or 6-membered ring optionally independently substituted by one or more substituents selected from halo, C1-C4 alkyl, cyano, hydroxy and -O(C1-C4 alkyl);
$R_4$ is hydrogen, C1-C6 alkyl, C3-C6 cycloalkyl, -(C1-C4 alkyl)(C3-C6 cycloalkyl), -(C1-C4 alkyl)phenyl, phenyl, heteroaryl, -(C1-C4 alkyl)heteroaryl, C2-C5 alkenyl, -(C1-C4 alkyl)heterocycloalkyl, heterocycloalkyl, $-S(=O)_2$(C1-C4 alkyl), $-S(=O)_2$phenyl or $-S(=O)_2$heteroaryl; and
$R_5$ is hydrogen, halo or C1-C4 alkyl.

Referring to the $R_1$ substituent of Formula II, in certain embodiments, $R_1$ is C1-C4 alkyl or halo. In a further embodiment, $R_1$ is methyl. In another further embodiment, $R_1$ is chloro. In another further embodiment, $R_1$ is fluoro.
In certain embodiments of Formula II, $R_1$ is hydrogen.
Referring to the $R_2$ substituent of Formula II, in certain embodiments, $R_2$ is chloro. In certain other embodiments, $R_2$ is cyano.
Referring to the $R_3$ substituent of Formula II, in certain embodiments, $R_3$ is hydrogen.
In certain embodiments of Formula II, $R_3$ is halo. In a further embodiment, $R_3$ is fluoro.
In certain embodiments of Formula II, $R_3$ is C1-C4 alkyl. In a further embodiment, $R_3$ is methyl. In another further embodiment, $R_3$ is $CHF_2$.
In certain embodiments of Formula II, $R_3$ is $-NR^aR^b$. In a further embodiment, $R_3$ is $-NH^2$.
In certain embodiments of Formula II, $R_3$ is cyano.
Referring to the $R_4$ substituent of Formula II, in certain embodiments, $R_4$ is C1-C6 alkyl, C3-C6 cycloalkyl, -(C1-C4 alkyl)(C3-C6 cycloalkyl), -(C1-C4 alkyl)heterocycloalkyl, heterocycloalkyl or -S(=O)2(C1-C4 alkyl).
In certain embodiments of Formula II, $R_4$ is unsubstituted C1-C6 alkyl. In certain other embodiments, $R_4$ is C1-C6 alkyl substituted with one or more substituents independently selected from deuterium, C1-C4 alkyl, hydroxyhydroxy, fluoro, -O(C1-C4 alkyl) and cyano. In certain other embodiments, $R_4$ is C1-C6 alkyl substituted with one or more substituents independently selected from deuterium, halo, hydroxyhydroxy, -O(C1-C4 alkyl), cyano, $-C(=O)NR^aR^b$, $-C(=O)OR^c$, $-S(=O)_2$(C1-C4 alkyl), $-NR^aR^b$, $-NR^aC(=O)OR^c$ and $-NR^aC(=O)$(C1-C4 alkyl), wherein each of $R^a$, $R^b$, and $R^c$ is independently hydrogen, C3-C6 cycloalkyl, heterocycloalkyl or C1-C4 alkyl, or $R^a/R^b$ and the nitrogen atom they are attached to form a 5 or 6-membered ring optionally independently substituted by one or more substituents selected from halo, C1-C4 alkyl, cyano, hydroxy and -O(C1-C4 alkyl). Particular examples of C1-C6 alkyl include, but not limited to, the following:

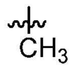 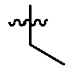 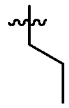 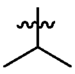 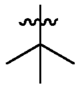 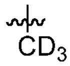 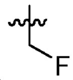 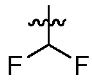 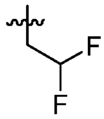 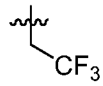

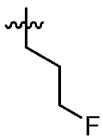 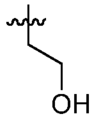 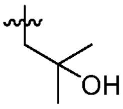 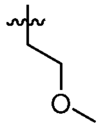 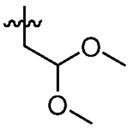 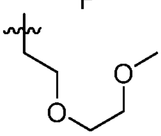 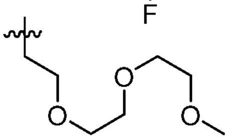

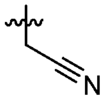 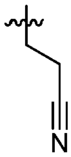 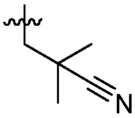 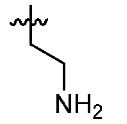 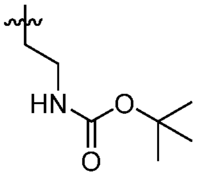 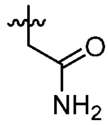 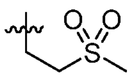 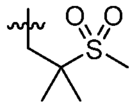

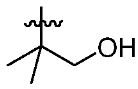 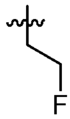 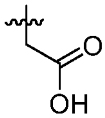 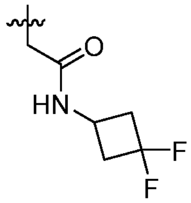 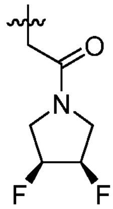 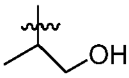 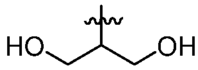

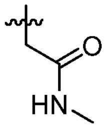 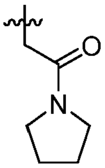 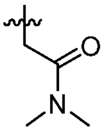 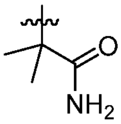 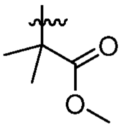 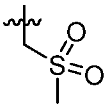 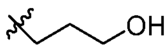 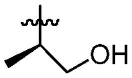 ,

wherein each of the C1-C6 alkyl is optionally substituted with one or more substituents independently selected from deuterium, halo, hydroxyhydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

In certain embodiments of Formula II, $R_4$ is unsubstituted C3-C6 cycloalkyl. In certain other embodiments, $R_4$ is C3-C6 cycloalkyl substituted with one or more substituents independently selected from deuterium, C1-C4 alkyl, hydroxy, fluoro, -O(C1-C4 alkyl) and cyano. In certain other embodiments, $R_4$ is C3-C6 cycloalkyl substituted with one or more substituents independently selected from deuterium, halo, hydroxyhydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl). Particular examples of C3-C6 cycloalkyl include cyclopropyl, cyclobutyl, cyclopentyl and cyclohexyl, each of which is optionally substituted with one or more substituents independently selected from deuterium, halo, hydroxyhydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

In certain embodiments of Formula II, $R_4$ is unsubstituted -(C1-C4 alkyl)(C3-C6 cycloalkyl). In certain other embodiments, $R_4$ is substituted -(C1-C4 alkyl)(C3-C6 cycloalkyl) with one or more substituents independently selected from deuterium, C1-C4 alkyl, hydroxy, fluoro, -O(C1-C4 alkyl) and cyano. In certain other embodiments, $R_4$ is substituted -(C1-C4 alkyl)(C3-C6 cycloalkyl) with one or more substituents independently selected from deuterium, halo, hydroxyhydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally

independently substituted by one or more substituents selected from halo, C1-C4 alkyl, cyano, hydroxy and -O(C1-C4 alkyl). Particular examples of -(C1-C4 alkyl)(C3-C6 cycloalkyl) include, but are not limited to, the following:

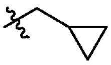 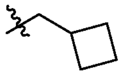 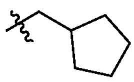 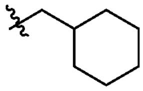 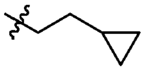 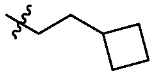

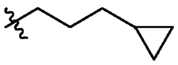 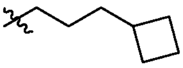 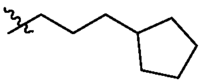 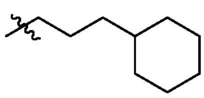 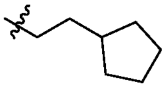 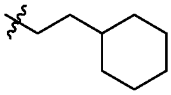 ,

wherein each of the -(C1-C4 alkyl)(C3-C6 cycloalkyl) is optionally substituted with one or more substituents independently selected from deuterium, halo, hydroxyhydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

In certain embodiments of Formula II, $R_4$ is unsubstituted -(C1-C4 alkyl)heterocycloalkyl. In certain other embodiments, $R_4$ is substituted -(C1-C4 alkyl)heterocycloalkyl with one or more substituents independently selected from deuterium, C1-C4 alkyl, hydroxy, fluoro, -O(C1-C4 alkyl) and cyano. In certain other embodiments, $R_4$ is substituted -(C1-C4 alkyl)heterocycloalkyl with one or more substituents independently selected from deuterium, halo, hydroxyhydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl). Particular examples of -(C1-C4 alkyl)heterocycloalkyl include, but are not limited to, the following:

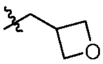 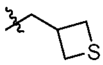 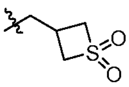 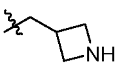 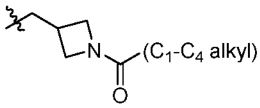 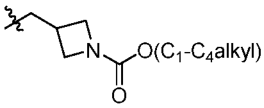

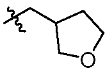 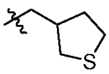 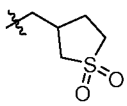 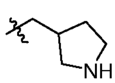 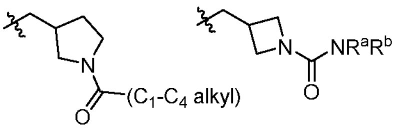 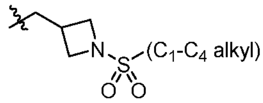

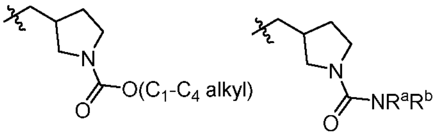 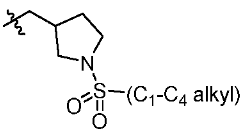 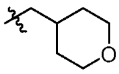 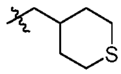 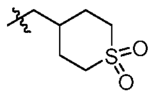

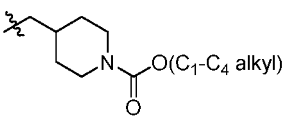 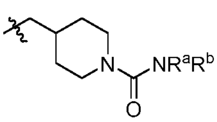 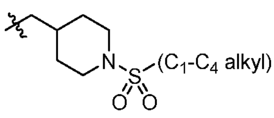 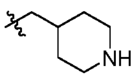 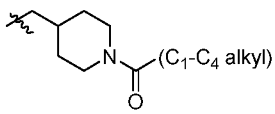 ,

wherein each of the -(C1-C4 alkyl)heterocycloalkyl is optionally substituted with one or more substituents independently selected from deuterium, halo, hydoxyhydroxyhydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

In certain embodiments of Formula II, $R_4$ is unsubstituted heterocycloalkyl. In certain other embodiments, $R_4$

is substituted heterocycloalkyl with one or more substituents independently selected from deuterium, C1-C4 alkyl, hydroxy, fluoro, -O(C1-C4 alkyl) and cyano. In certain other embodiments, $R_4$ is substituted heterocycloalkyl with one or more substituents independently selected from deuterium, halo, hydroxyhydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl). Particular examples of heterocycloalkyl include, but are not limited to, the following:

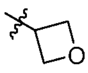 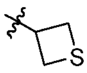 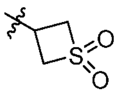 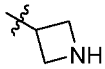 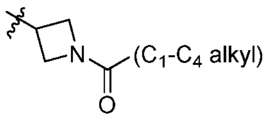 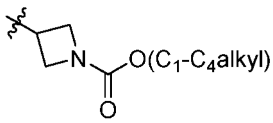

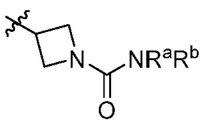 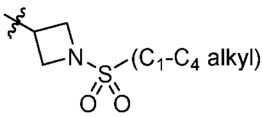 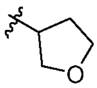 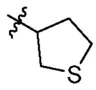 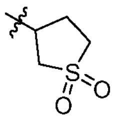 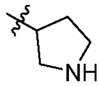 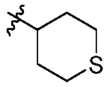

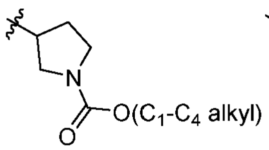 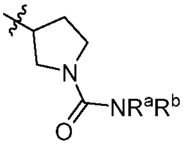 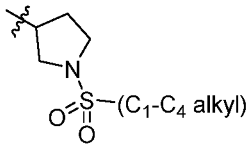 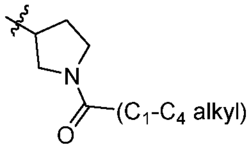 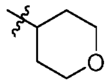 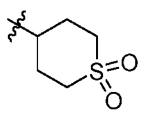

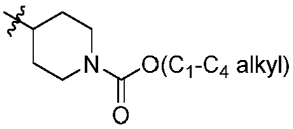 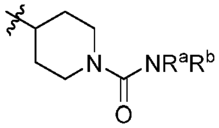 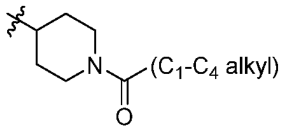 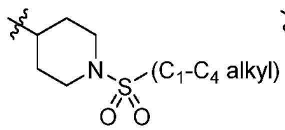 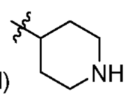

wherein each of the heterocycloalkyl is optionally substituted with one or more substituents independently selected from deuterium, halo, hydoxyhydroxyhydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

Referring to the $R_5$ substituent of Formula II, in certain embodiments, $R_5$ is hydrogen. In certain other embodiments, $R_5$ is halo. In a further embodiment, $R_5$ is fluoro.

Compounds of Formula II include compounds of Formula IIa, wherein $R_1$ is C1-C4 alkyl and $R_2$ is cyano; compounds of Formula IIaa, wherein $R_1$ is methyl and $R_2$ is cyano; compounds of Formula lib, wherein $R_1$ is halo and $R_2$ is cyano; compounds of Formula IIbb, wherein $R_1$ is chloro or fluoro and $R_2$ is cyano; compounds of Formula IIc, wherein each of $R_1$ and $R_2$ is independently halo; compounds of Formula IIcc, wherein each of $R_1$ and $R_2$ is chloro; and compounds of Formula IId, wherein $R_1$ is hydrogen and $R_2$ is chloro.

Referring to the $R_3$ substituent of Formulae IIa, IIaa, IIb, IIbb, IIc, IIcc and IId, in certain embodiments, $R_3$ is hydrogen.

For Formulae IIa, IIaa, IIb, IIbb, IIc, IIcc and IId, in certain embodiments, $R_3$ is halo. In a further embodiment, $R_3$ is fluoro.

For Formulae IIa, IIaa, IIb, IIbb, IIc, IIcc and IId, in certain embodiments, $R_3$ is C1-C4 alkyl. In a further embodiment, $R_3$ is methyl. In another further embodiment, $R_3$ is $CHF_2$.

For Formulae IIa, IIaa, IIb, IIbb, IIc, IIcc and IId, in certain embodiments, $R_3$ is -NR$^a$R$^b$. In a further embodiment, $R_3$ is -$NH_2$.

Referring to the $R_4$ substituent of the Formulae IIa, IIaa, IIb, IIbb, IIc, IIcc and IId, in certain embodiments, $R_4$ is C1-C6 alkyl, C3-C6 cycloalkyl, -(C1-C4 alkyl)(C3-C6 cycloalkyl), -(C1-C4 alkyl)heterocycloalkyl, heterocycloalkyl or -S(=O)$_2$(C1-C4 alkyl).

For the Formulae IIa, IIaa, IIb, IIbb, IIc, IIcc and IId, in certain embodiments, $R_4$ is unsubstituted C1-C6 alkyl. In certain other embodiments, $R_4$ is C1-C6 alkyl substituted with one or more substituents independently selected from deuterium, C1-C4 alkyl, hydroxyhydroxy, fluoro, -O(C1-C4 alkyl) and cyano. In certain other embodiments, $R_4$ is C1-C6

alkyl substituted with one or more substituents independently selected from deuterium, halo, hydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl). Particular examples of C1-C6 alkyl include, but not limited to, the following:

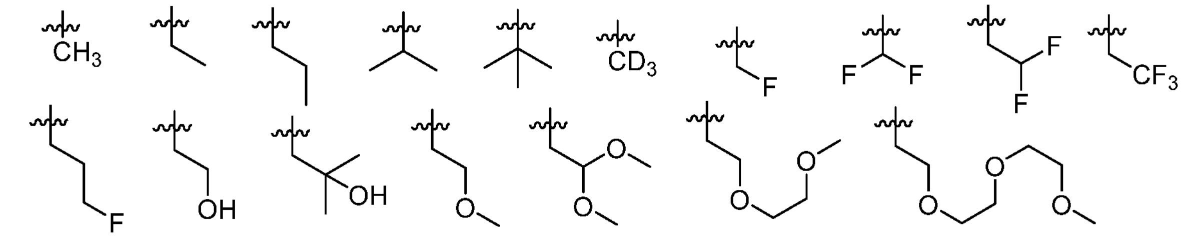

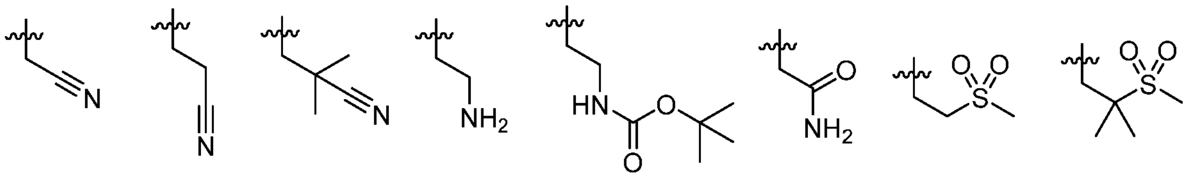

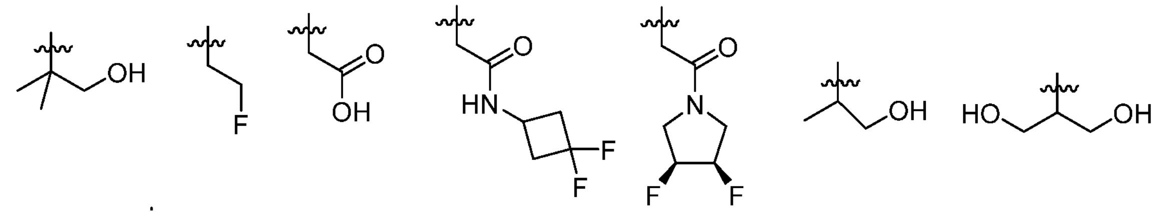

.

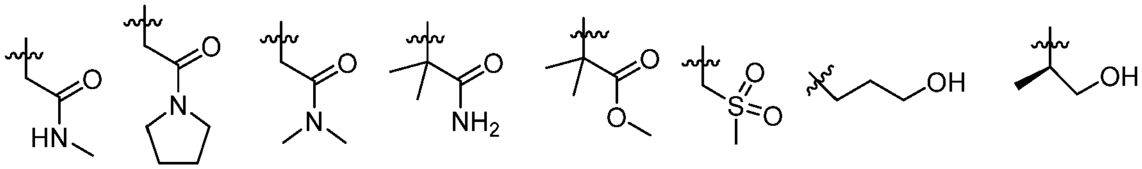

,

wherein each of the C1-C6 alkyl is optionally substituted with one or more substituents independently selected from deuterium, halo, hydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

For Formulae IIa, IIaa, IIb, IIbb, IIc, IIcc and IId, in certain embodiments, $R_4$ is unsubstituted C3-C6 cycloalkyl. In certain other embodiments, $R_4$ is C3-C6 cycloalkyl substituted with one or more substituents independently selected from deuterium, C1-C4 alkyl, hydroxy, fluoro, -O(C1-C4 alkyl) and cyano. In certain other embodiments, $R_4$ is C3-C6 cycloalkyl substituted with one or more substituents independently selected from deuterium, halo, hydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl). Particular examples of C3-C6 cycloalkyl include cyclopropyl, cyclobutyl, cyclopentyl and cyclohexyl, each of which is optionally substituted with one or more substituents independently selected from deuterium, halo, hydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

For Formulae IIa, IIaa, IIb, IIbb, IIc, IIcc and IId, in certain embodiments, $R_4$ is unsubstituted -(C1-C4 alkyl)(C3-C6 cycloalkyl). In certain other embodiments, $R_4$ is substituted -(C1-C4 alkyl)(C3-C6 cycloalkyl) with one or more substituents independently selected from deuterium, C1-C4 alkyl, hydroxy, fluoro, -O(C1-C4 alkyl) and cyano. In certain other embodiments, $R_4$ is substituted -(C1-C4 alkyl)(C3-C6 cycloalkyl) with one or more substituents independently selected from deuterium, halo, hydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl). Particular examples of -(C1-C4 alkyl)(C3-C6 cycloalkyl) include, but are not limited to, the following:

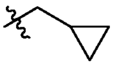 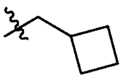 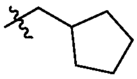 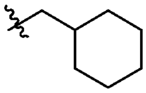 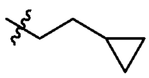 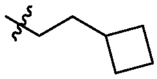

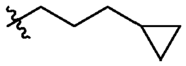 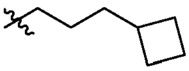 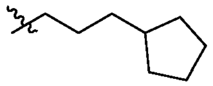 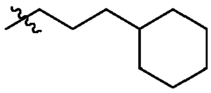 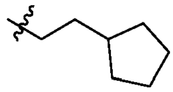 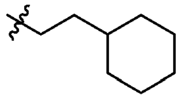

wherein each of the -(C1-C4 alkyl)(C3-C6 cycloalkyl) is optionally substituted with one or more substituents independently selected from deuterium, halo, hydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

For Formulae IIa, IIaa, IIb, IIbb, IIc, IIcc and IId, in certain embodiments, $R_4$ is unsubstituted -(C1-C4 alkyl)heterocycloalkyl. In certain other embodiments, $R_4$ is substituted -(C1-C4 alkyl)heterocycloalkyl with one or more substituents independently selected from deuterium, C1-C4 alkyl, hydroxy, fluoro, -O(C1-C4 alkyl) and cyano. In certain other embodiments, $R_4$ is substituted -(C1-C4 alkyl)heterocycloalkyl with one or more substituents independently selected from deuterium, halo, hydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl). Particular examples of -(C1-C4 alkyl)heterocycloalkyl include, but not limited to, the following:

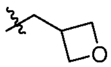 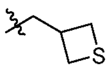 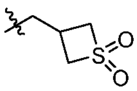 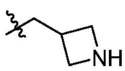 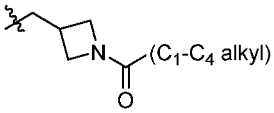 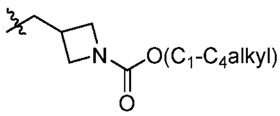

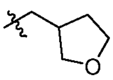 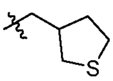 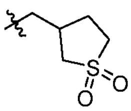 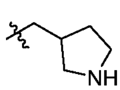 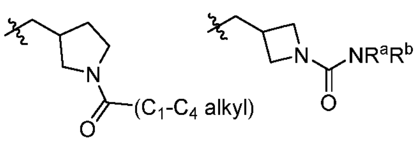 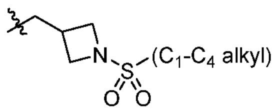

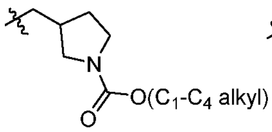 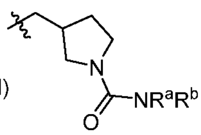 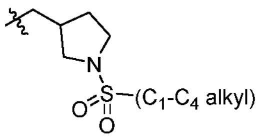 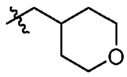 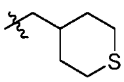 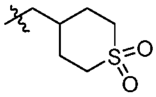

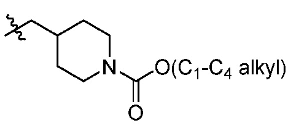 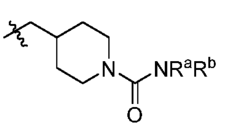 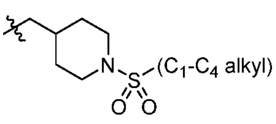 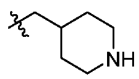 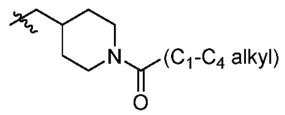

wherein each of the -(C1-C4 alkyl)heterocycloalkyl is optionally substituted with one or more substituents independently selected from deuterium, halo, hydoxyhydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

For Formula IIa, IIaa, IIb, IIbb, IIc, IIcc and IId, in certain embodiments, $R_4$ is unsubstituted heterocycloalkyl. In certain other embodiments, $R_4$ is substituted heterocycloalkyl with one or more substituents independently selected from deuterium, C1-C4 alkyl, hydroxy, fluoro, -O(C1-C4 alkyl) or cyano. In certain other embodiments, $R_4$ is substituted heterocycloalkyl with one or more substituents independently selected from deuterium, halo, hydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ or -NR$^a$C(=O)(C1-C4 alkyl), wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl). Particular examples of heterocycloalkyl include, but not limited to, the following:

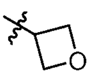 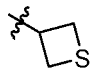 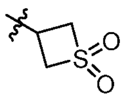 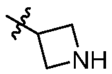 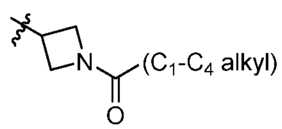 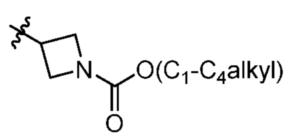

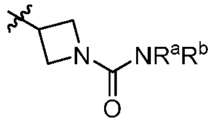 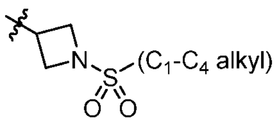 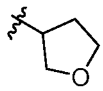 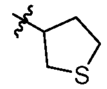 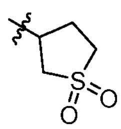 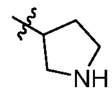 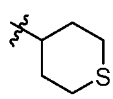

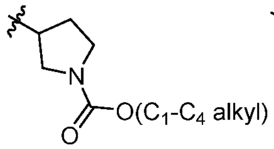 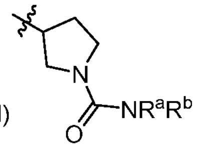 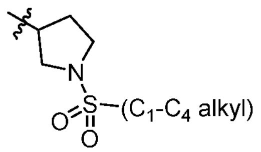 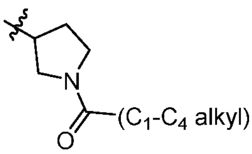 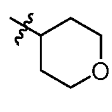 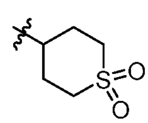

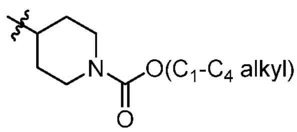 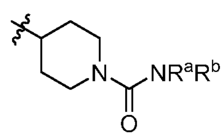 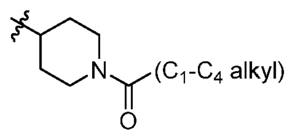 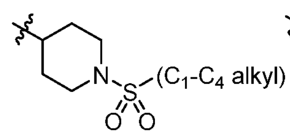 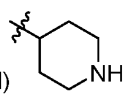

wherein each of the heterocycloalkyl is optionally substituted with one or more substituents independently selected from deuterium, halo, hydoxyhydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

Referring to the $R_5$ substituent of Formulae IIa, IIaa, IIb, IIbb, IIc, IIcc and IId, in certain embodiments, $R_5$ is hydrogen. In certain other embodiments, $R_5$ is halo. In a further embodiment, $R_5$ is fluoro.

Referring to the $R_3$ and $R_5$ substituents of Formulae IIa, IIaa, IIb, IIbb, IIc, IIcc and IId, in certain embodiments, each of $R_3$ and $R_5$ is hydrogen. In a further embodiment, $R_4$ is C1-C6 alkyl optionally substituted with one or more substituents independently selected from deuterium, C1-C4 alkyl, hydroxy, fluoro, -O(C1-C4 alkyl) and cyano. In a still further embodiment, $R_4$ is C1-C6 alkyl optionally substituted with one or more hydroxy. In certain other embodiments, $R_3$ is -$NH_2$ and $R_5$ is hydrogen. In a further embodiment, $R_4$ is C1-C6 alkyl optionally substituted with one or more substituents independently selected from deuterium, C1-C4 alkyl, hydroxy, fluoro, -O(C1-C4 alkyl) and cyano. Particular

examples of C1-C6 alkyl include, but not limited to, the following:

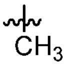 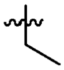 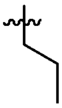 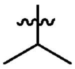 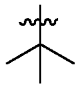 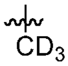 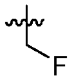 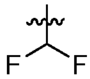 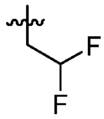 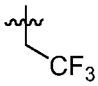

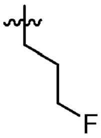 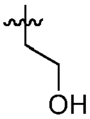 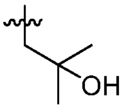 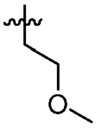 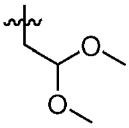 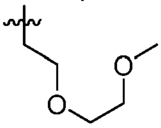 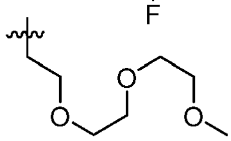

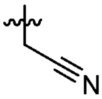 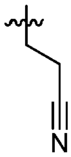 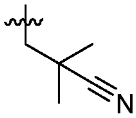 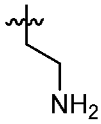 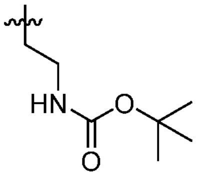 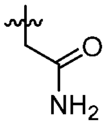 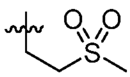 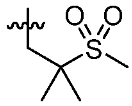

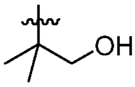 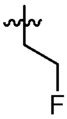 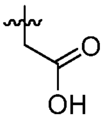 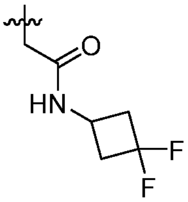 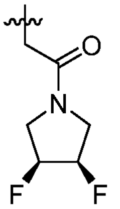 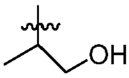 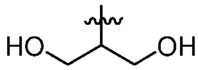

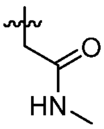 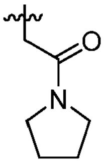 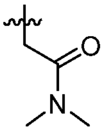 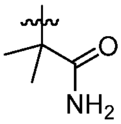 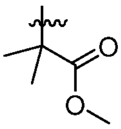 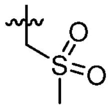 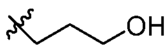 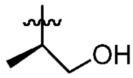

,

wherein each of the C1-C6 alkyl is optionally substituted with one or more substituents independently selected from deuterium, halo, hydroxy, -O(C1-C4 alkyl), cyano, $-C(=O)NR^aR^b$, $-C(=O)OR^c$, $-S(=O)_2$(C1-C4 alkyl), $-NR^aR^b$, $-NR^aC(=O)OR^c$ and $-NR^aC(=O)$(C1-C4 alkyl), and wherein each of $R^a$, $R^b$, and $R^c$ is independently hydrogen, C3-C6 cycloalkyl, heterocycloalkyl or C1-C4 alkyl, or $R^a/R^b$ and the nitrogen atom they are attached to form a 5 or 6-membered ring optionally independently substituted by one or more substituents selected from halo, C1-C4 alkyl, cyano, hydroxy and -O(C1-C4 alkyl).

For Formulae IIa, IIaa, IIb, IIbb, IIc, IIcc and IId, in certain embodiments, each of $R_3$ and $R_5$ is hydrogen, and $R_4$ is unsubstituted heterocycloalkyl. In certain other embodiments, each of $R_3$ and $R_5$ is hydrogen, and $R_4$ is substituted heterocycloalkyl with one or more substituents independently selected from deuterium, C1-C4 alkyl, hydroxy, fluoro, -O(C1-C4 alkyl) and cyano. In certain other embodiments, each of $R_3$ and $R_5$ is hydrogen, and $R_4$ is substituted heterocycloalkyl with one or more substituents independently selected from deuterium, halo, hydroxy, -O(C1-C4 alkyl), cyano, $-C(=O)NR^aR^b$, $-C(=O)OR^c$, $-S(=O)_2$(C1-C4 alkyl), $-NR^aR^b$, $-NR^aC(=O)OR^c$ and $-NR^aC(=O)$(C1-C4 alkyl), wherein each of $R^a$, $R^b$, and $R^c$ is independently hydrogen, C3-C6 cycloalkyl, heterocycloalkyl or C1-C4 alkyl, or $R^a/R^b$ and the nitrogen atom they are attached to form a 5 or 6-membered ring optionally independently substituted by one or more substituents selected from halo, C1-C4 alkyl, cyano, hydroxy and -O(C1-C4 alkyl). Particular examples of heterocycloalkyl include, but not limited to, the following:

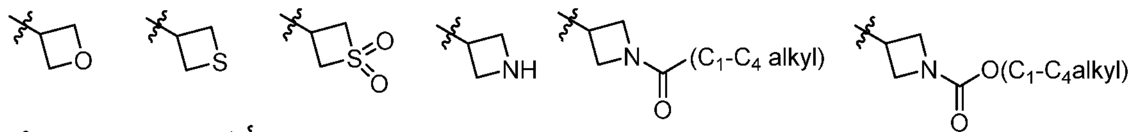

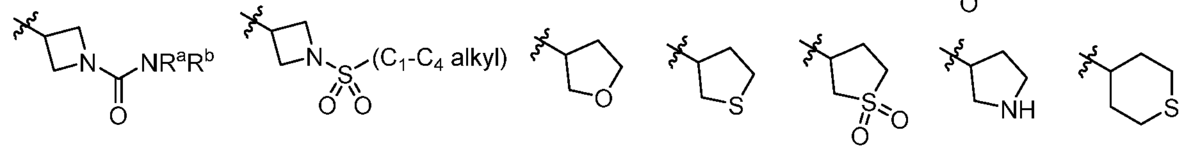

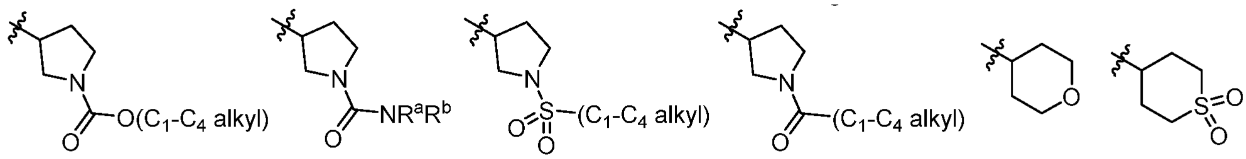

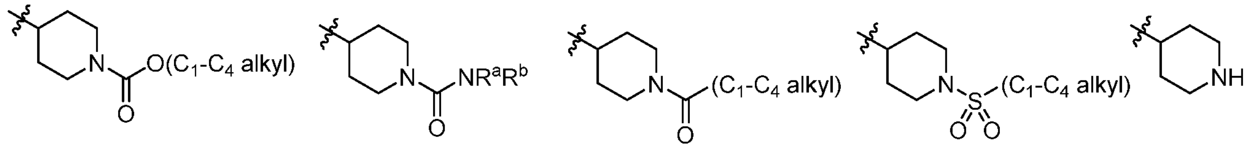

wherein each of the heterocycloalkyl is optionally substituted with one or more substituents independently selected from deuterium, halo, hydoxyhydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

For Formulae IIa, IIaa, IIb, IIbb, IIc, IIcc and IId, in certain embodiments, each of $R_3$ and $R_5$ is hydrogen, and $R_4$ is unsubstituted -(C1-C4 alkyl)heterocycloalkyl. In certain other embodiments, each of $R_3$ and $R_5$ is hydrogen, and $R_4$ is substituted -(C1-C4 alkyl)heterocycloalkyl with one or more substituents independently selected from deuterium, C1-C4 alkyl, hydroxy, fluoro, -O(C1-C4 alkyl) and cyano. In certain other embodiments, each of $R_3$ and $R_5$ is hydrogen, and $R_4$ is substituted -(C1-C4 alkyl)heterocycloalkyl with one or more substituents independently selected from deuterium, halo, hydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl). Particular examples of -(C1-C4 alkyl)heterocycloalkyl include, but not limited to, the following:

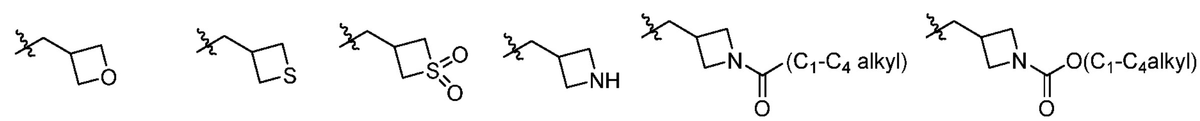

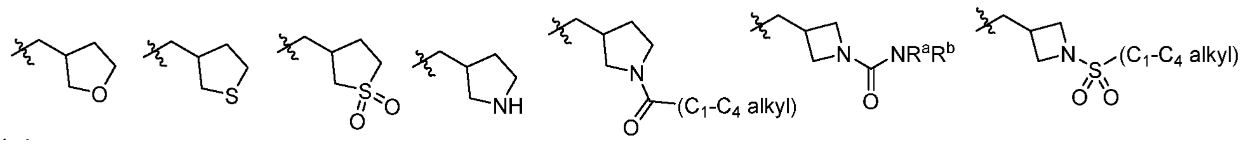

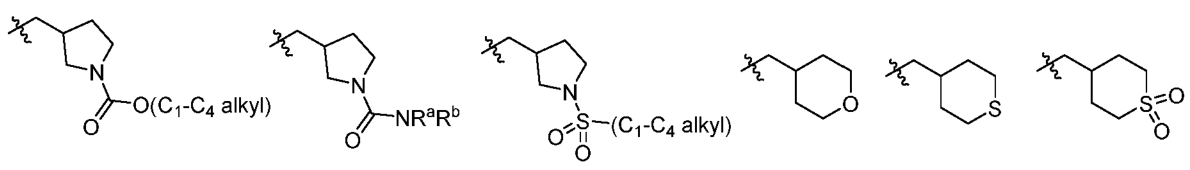

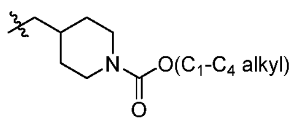 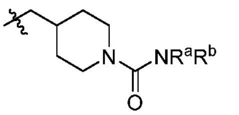 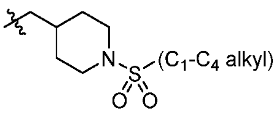 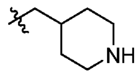 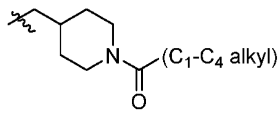

wherein each of the -(C1-C4 alkyl)heterocycloalkyl is optionally substituted with one or more substituents independently selected from deuterium, halo, hydoxyhydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

For Formulae IIa, IIaa, IIb, IIbb, IIc, IIcc and IId, in certain embodiments, each of $R_3$ and $R_5$ is hydrogen, and $R_4$ is unsubstituted -(C1-C4 alkyl)(C3-C6 cycloalkyl). In certain other embodiments, each of $R_3$ and $R_5$ is hydrogen, and $R_4$ is substituted -(C1-C4 alkyl)(C3-C6 cycloalkyl) with one or more substituents independently selected from deuterium, C1-C4 alkyl, hydroxy, fluoro, -O(C1-C4 alkyl) and cyano. In certain other embodiments, each of $R_3$ and $R_5$ is hydrogen, and $R_4$ is substituted -(C1-C4 alkyl)(C3-C6 cycloalkyl) with one or more substituents independently selected from deuterium, halo, hydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl). Particular examples of -(C1-C4 alkyl)(C3-C6 cycloalkyl) include, but not limited to, the following:

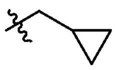 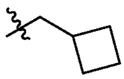 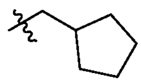 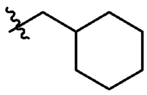 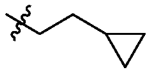 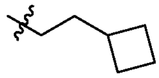

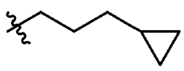 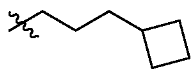 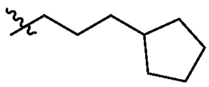 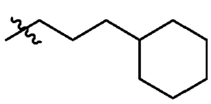 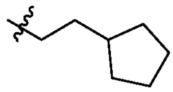 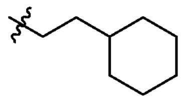

wherein each of the -(C1-C4 alkyl)(C3-C6 cycloalkyl) is optionally substituted with one or more substituents independently selected from deuterium, halo, hydroxy, -O(C1-C4 alkyl), cyano, -C(=O)NR$^a$R$^b$, -C(=O)OR$^c$, -S(=O)$_2$(C1-C4 alkyl), -NR$^a$R$^b$, -NR$^a$C(=O)OR$^c$ and -NR$^a$C(=O)(C1-C4 alkyl), and wherein each of R$^a$, R$^b$, and R$^c$ is independently hydrogen, C3-C6 cycloalkyl, heterocycloalkyl or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

One or more additional pharmacologically active agents may be administered in combination with a compound of Formula (I) or (II) (or a pharmaceutically acceptable salt thereof). An additional active agent (or agents) is intended to mean a pharmaceutically active agent (or agents) that is active in the body, including pro-drugs that convert to pharmaceutically active form after administration, which are different from the compounds of Formulae (I) or (II). The additional active agents also include free-acid, free-base and pharmaceutically acceptable salts of said additional active agents. Generally, any suitable additional active agent or agents, including chemotherapeutic agents or therapeutic antibodies may be used in any combination with a compound of Formula (I) or (II) in a single dosage formulation (a fixed dose drug combination), or in one or more separate dosage formulations which allows for concurrent or sequential administration of the active agents (co-administration of the separate active agents) to subjects. In addition, the compounds of Formulae (I) or (II) (or pharmaceutically acceptable salts thereof) can be administered in combination with radiation therapy, hormone therapy, surgery or immunotherapy.

The additional active agent may modulate other pathways, or other components of the same pathway, or even overlapping sets of target enzymes which are used in combination with a compound of Formula (I) or (II), or a pharmaceutically acceptable salt thereof. Such therapy includes but is not limited to the combination of one or more compounds of Formula (I) or (II) with chemotherapeutic agents, therapeutic antibodies, and radiation treatment, to provide a synergistic or additive therapeutic effect.

The combination therapies may comprise chemotherapeutic agents. Many such agents are presently known in the art and can be used in combination with the compounds of Formula (I) or Formula (II). The chemotherapeutic agent may be selected from the group consisting of mitotic inhibitors, alkylating agents, anti-metabolites, intercalating

antibiotics, growth factor inhibitors, cell cycle inhibitors, enzymes, topoisomerase inhibitors, biological response modifiers, anti- hormones, angiogenesis inhibitors, and anti-androgens. Non-limiting examples are cytotoxic agents, and non-peptide small molecules such as Gleevec® (Imatinib Mesylate), Kyprolis® (carfilzomib), Velcade® (bortezomib), Casodex (bicalutamide), Iressa® (gefitinib), and Adriamycin as well. Non-limiting examples of chemotherapeutic agents include alkylating agents such as thiotepa and cyclosphosphamide (CYTOXANTM™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, Casodex™, chromomycins, dactinomycin, daunorubicin, detorubicin, 6-diazo-5-oxo- L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifiuridine, enocitabine, floxuridine, androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfomithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2- ethylhydrazide; procarbazine; PSK; razoxane; sizofiran; spirogermanium; tenuazonic acid; triaziquone; 2,2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxanes, e.g. paclitaxel and docetaxel; retinoic acid; esperamicins; capecitabine; and pharmaceutically acceptable salts, acids or derivatives of any of the above.

Suitable chemotherapeutic cell conditioners may also be included and are anti-hormonal agents that act to regulate or inhibit hormone action on tumors such as anti-estrogens including for example tamoxifen, (Nolvadex™), raloxifene, aromatase inhibiting 4(5)-imidazoles, 4- hydroxy tamoxifen, trioxifene, keoxifene, LY 117018, onapristone, and toremifene; and anti-androgens such as flutamide, nilutamide, bicalutamide, leuprolide, and goserelin; chlorambucil; gemcitabine; 6-thioguanine; mercaptopurine; methotrexate; platinum analogs such as cisplatin and carboplatin; vinblastine; platinum; etoposide (VP- 16); ifosfamide; mitomycin C; mitoxantrone; vincristine; vinorelbine; navelbine; novantrone; teniposide; daunomycin; aminopterin; xeloda; ibandronate; camptothecin-11 (CPT-11); and topoisomerase inhibitor RFS 2000.

Where desired, the compounds of Formula (I) or (II) or pharmaceutical compositions containing such compounds can be used in combination with commonly prescribed anti-cancer drugs such as Herceptin®, Avastin®, Erbitux®, Rituxan®, Taxol®, Arimidex®, Taxotere®, ABVD, AVICINE, abagovomab, acridine carboxamide, adecatumumab, 17-N-allylamino-17- demethoxygeldanamycin, alpharadin, alvocidib, 3-aminopyridine-2-carboxaldehyde thiosemicarbazone, amonafide, anthracenedione, anti-CD22 immunotoxins, Antineoplastic, antitumorigenic herbs, apaziquone, atiprimod, azathioprine, belotecan, bendamustine, BIBW 2992, biricodar, brostallicin, bryostatin, buthionine sulfoximine, calyculin, cell-cycle nonspecific antineoplastic agents, dichloroacetic acid, discodermolide, elsamitrucin, enocitabine, epothilone, eribulin, everolimus, exatecan, exisulind, ferruginol, forodesine, fosfestrol, ICE chemotherapy regimen, IT-101, imexon, imiquimod, indolocarbazole, irofulven, laniquidar, larotaxel, lenalidomide, lucanthone, lurtotecan, mafosfamide, mitozolomide, nafoxidine, nedaplatin, olaparib, ortataxel, PAC- 1, pixantrone, proteasome inhibitor, rebeccamycin, resiquimod, rubitecan, SN-38, salinosporamide a, sapacitabine, swainsonine, talaporfin, tariquidar, tegafur- uracil, temozolimide, tesetaxel, triplatin tetranitrate, tris(2-chloroethyl)amine, troxacitabine, Vadimezan, Vinflunine, ZD6126 or Zosuquidar.

The compounds of Formula (I) or (II) or pharmaceutical compositions provided herein may be used in combination with radiation therapy for inhibiting abnormal cell growth or treating the hyperproliferative disorder in the mammal. Techniques for administering radiation therapy are known in the art, and these techniques can be used in the combination therapy described herein. The administration of the compound of Formula (I) or (II) in this combination therapy can be determined as described herein.

Radiation therapy can be administered through one of several methods, or a combination of methods, including without limitation external -beam therapy, internal radiation therapy, implant radiation, stereotactic radiosurgery, systemic radiation therapy, radiotherapy and permanent or temporary interstitial brachy therapy. The term "brachytherapy," as used herein, refers to radiation therapy delivered by a spatially confined radioactive material inserted into the body at or near a tumor or other proliferative tissue disease site. The term is intended without limitation to include exposure to radioactive isotopes (e.g., At-211, I-131, I-125, Y-90, Re-186, Re-188, Sm- 153, Bi-212, P-32, and radioactive isotopes

of Lu). Suitable radiation sources for use as a cell conditioner of the present disclosure include both solids and liquids. By way of non-limiting example, the radiation source can be a radionuclide, such as I-125, 1-131, Yb-169, Ir-192 as a solid source, 1-125 as a solid source, or other radionuclides that emit photons, beta particles, gamma radiation, or other therapeutic rays. The radioactive material can also be a fluid made from any solution of radionuclide(s), e.g., a solution of I-125 or 1-131, or a radioactive fluid can be produced using a slurry of a suitable fluid containing small particles of solid radionuclides, such as Au-198, Y-90. Moreover, the radionuclide(s) can be embodied in a gel or radioactive microspheres.

The compounds of Formula (I) or (II) or pharmaceutical compositions containing such compounds can be used in combination with an amount of one or more substances selected from anti-angiogenesis agents, signal transduction inhibitors, antiproliferative agents, glycolysis inhibitors, or autophagy inhibitors.

Anti-angiogenesis agents, such as MMP -2 (matrix-metalloproteinase 2) inhibitors and MMP-9 (matrix-metalloproteinase 9) inhibitors, can be used in conjunction with a compound of the disclosure and pharmaceutical compositions described herein. Anti-angiogenesis agents include, for example, rapamycin, temsirolimus (CCI-779), everolimus (RAD001), sorafenib, sunitinib, and bevacizumab. Examples of useful matrix metalloproteinase inhibitors are described in WO 96/33172, WO 96/27583 European Patent Publication No. EP0818442, European Patent Publication No. EP1004578, WO 98/07697, WO 98/03516, WO 98/34918, WO 98/34915, WO 98/33768, WO 98/30566, European Patent Publication No. 606046, European Patent Publication No. 931788, WO 90/05719, WO 99/52910, WO 99/52889, WO 99/29667, WO 1999007675 , European Patent Publication No. EP1786785, European Patent Publication No. EP1181017, U.S. Publication No. US20090012085 , U.S. Patent No. 5,863,949, U.S. Patent No. 5,861,510, and European Patent Publication No. EP0780386. Preferred MMP-2 and MMP-9 inhibitors are those that have little or no activity inhibiting MMP-1. More preferred, are those that selectively inhibit MMP-2 and/or AMP-9 relative to the other matrix-metalloproteinases (i.e., MAP-1, MMP-3, MMP-4, MMP-5, MMP-6, MMP- 7, MMP- 8, MMP-10, MMP-11, MMP-12, and MMP-13). Some specific examples of MMP inhibitors useful in the combinations are AG-3340, RO 32-3555, and RS 13-0830.

The compounds of Formula (I) or (II) may also be used in co-therapies with other anti-neoplastic agents, such as acemannan, aclarubicin, aldesleukin, alemtuzumab, alitretinoin, altretamine, amifostine, aminolevulinic acid, amrubicin, amsacrine, anagrelide, anastrozole, ANCER, ancestim, ARGLABIN, arsenic trioxide, BAM 002 (Novelos), bexarotene, bicalutamide, broxuridine, capecitabine, celmoleukin, cetrorelix, cladribine, clotrimazole, cytarabine ocfosfate, DA 3030 (Dong-A), daclizumab, denileukin diftitox, deslorelin, dexrazoxane, dilazep, docetaxel, docosanol, doxercalciferol, doxifluridine, doxorubicin, bromocriptine, carmustine, cytarabine, fluorouracil, HIT diclofenac, interferon alfa, daunorubicin, doxorubicin, tretinoin, edelfosine, edrecolomab, eflornithine, emitefur, epirubicin, epoetin beta, etoposide phosphate, exemestane, exisulind, fadrozole, filgrastim, finasteride, fludarabine phosphate, formestane, fotemustine, gallium nitrate, gemcitabine, gemtuzumab zogamicin, gimeracil/oteracil/tegafur combination, glycopine, goserelin, heptaplatin, human chorionic gonadotropin, human fetal alpha fetoprotein, ibandronic acid, idarubicin, (imiquimod, interferon alfa, interferon alfa, natural, interferon alfa-2, interferon alfa-2a, interferon alfa-2b, interferon alfa-NI, interferon alfa-n3, interferon alfacon-1, interferon alpha, natural, interferon beta, interferon beta- la, interferon beta- lb, interferon gamma, natural interferon gamma- la, interferon gamma-lb, interleukin-1 beta, iobenguane, irinotecan, irsogladine, lanreotide, LC 9018 (Yakult), leflunomide, lenograstim, lentinan sulfate, letrozole, leukocyte alpha interferon, leuprorelin, levamisole + fluorouracil, liarozole, lobaplatin, lonidamine, lovastatin, masoprocol, melarsoprol, metoclopramide, mifepristone, miltefosine, mirimostim, mismatched double stranded RNA, mitoguazone, mitolactol, mitoxantrone, molgramostim, nafarelin, naloxone + pentazocine, nartograstim, nedaplatin, nilutamide, noscapine, novel erythropoiesis stimulating protein, NSC 631570 octreotide, oprelvekin, osaterone, oxaliplatin, paclitaxel, pamidronic acid, pegaspargase, peginterferon alfa-2b, pentosan polysulfate sodium, pentostatin, picibanil, pirarubicin, rabbit antithymocyte polyclonal antibody, polyethylene glycol interferon alfa-2a, porfimer sodium, raloxifene, raltitrexed, rasburiembodiment, rhenium Re 186 etidronate, RII retinamide, rituximab, romurtide, samarium (153 Sm) lexidronam, sargramostim, sizofiran, sobuzoxane, sonermin, strontium-89 chloride, suramin, tasonermin, tazarotene, tegafur, temoporfin, temozolomide, teniposide, tetrachlorodecaoxide, thalidomide, thymalfasin, thyrotropin alfa, topotecan, toremifene, tositumomab-iodine 131, trastuzumab, treosulfan, tretinoin, trilostane, trimetrexate, triptorelin, tumor necrosis factor alpha, natural, ubenimex, bladder cancer vaccine, Maruyama vaccine, melanoma lysate vaccine, valrubicin, verteporfin, vinorelbine, VIRULIZIN, zinostatin stimalamer, or zoledronic acid; abarelix; AE 941 (Aeterna), ambamustine, bcl-2 (Genta), APC 8015 (Dendreon), cetuximab, decitabine, dexaminoglutethimide, diaziquone, EL 532 (Elan), EM 800 (Endorecherche), eniluracil, etanidazole, fenretinide, filgrastim SD01 (Amgen), fulvestrant, galocitabine, gastrin 17 immunogen, HLA-B7 gene therapy (Vical), granulocyte macrophage colony stimulating factor, histamine dihydrochloride, ibritumomab tiuxetan, ilomastat, IM 862 (Cytran), interleukin-2, iproxifene, LDI 200 (Milkhaus), leridistim, lintuzumab, CA 125 MAb (Biomira), cancer MAb (Japan Pharmaceutical Development), HER- 2 and Fc MAb (Medarex), idiotypic 105AD7 MAb (CRC Technology), idiotypic CEA MAb (Trilex), LYM-1 -iodine 131 MAb (Techni clone), polymorphic epithelial mucin-yttrium 90 MAb (Antisoma), marimastat, menogaril, mitumomab, motexafin gadolinium, MX 6 (Galderma), nelarabine, nolatrexed, P 30 protein, pegvisomant, pemetrexed, porfiromycin, prinomastat, RL 0903 (Shire), rubitecan, satraplatin, sodium phenyl acetate, sparfosic acid, SRL 172 (SR

Pharma), SU 5416 (SUGEN), TA 077 (Tanabe), tetrathiomolybdate, thaliblastine, thrombopoietin, tin ethyl etiopurpurin, tirapazamine, cancer vaccine (Biomira), melanoma vaccine (New York University), melanoma vaccine (Sloan Kettering Institute), melanoma oncolysate vaccine (New York Medical College), viral melanoma cell lysates vaccine (Royal Newcastle Hospital), or valspodar.

The compounds of Formula (I) or (II) may further be used with VEGFR inhibitors.

The combination may comprise a composition of the present invention in combination with at least one anti-angiogenic agent. An agent can be an agonist, antagonist, allosteric modulator, toxin or, more generally, may act to inhibit or stimulate its target (e.g., receptor or enzyme activation or inhibition), and thereby promote cell death or arrest cell growth.

Exemplary anti-angiogenic agents include ERBITUX™, KDR (kinase domain receptor) inhibitory agents (e.g., antibodies and antigen binding regions that specifically bind to the kinase domain receptor), anti-VEGF agents (e.g., antibodies or antigen binding regions that specifically bind VEGF, or soluble VEGF receptors or a ligand binding region thereof) such as AVASTIN™ or VEGF-TRAP™, and anti-VEGF receptor agents (e.g., antibodies or antigen binding regions that specifically bind thereto), EGFR inhibitory agents (e.g., antibodies or antigen binding regions that specifically bind thereto) such as Vectibix (panitumumab), IRES S A™ (gefitinib), TARCEVA™ (erlotinib), anti-Angl and anti-Ang2 agents (e.g., antibodies or antigen binding regions specifically binding thereto or to their receptors, e.g., Tie2/Tek), and anti-Tie2 kinase inhibitory agents (e.g., antibodies or antigen binding regions that specifically bind thereto). The pharmaceutical compositions of the present invention can also include one or more agents (e.g., antibodies, antigen binding regions, or soluble receptors) that specifically bind and inhibit the activity of growth factors, such as antagonists of hepatocyte growth factor (HGF, also known as Scatter Factor), and antibodies or antigen binding regions that specifically bind its receptor "c-met". Other anti-angiogenic agents include Campath, IL-8, B-FGF, Tek antagonists (Ceretti et al, U.S. Publication No. 2003/0162712; U.S. Patent No. 6,413,932), anti-TWEAK agents (e.g., specifically binding antibodies or antigen binding regions, or soluble TWEAK receptor antagonists; see, Wiley, U.S. Patent No. 6,727,225), ADAM distintegrin domain to antagonize the binding of integrin to its ligands (Fanslow et al., U.S. Publication No. 2002/0042368), specifically binding anti-eph receptor and/or anti-ephrin antibodies or antigen binding regions (U.S. Patent Nos. 5,981,245; 5,728,813; 5,969,110; 6,596,852; 6,232,447; and 6,057,124), and anti-PDGF-BB antagonists (e.g., specifically binding antibodies or antigen binding regions) as well as antibodies or antigen binding regions specifically binding to PDGF-BB ligands, and PDGFR kinase inhibitory agents (e.g., antibodies or antigen binding regions that specifically bind thereto).

Additional anti-angiogenic/anti -tumor agents include: SD-7784 (Pfizer, USA); cilengitide (Merck KGaA, Germany); pegaptanib octasodium, (Gilead Sciences, USA); alphastatin (BioActa, UK); M-PGA, ilomastat, (Arriva, USA); emaxanib, (Pfizer, USA); vatalanib (Novartis, Switzerland); 2-methoxyestradiol; TLC ELL-12 (Elan, Ireland); anecortave acetate (Alcon, USA); alpha-D148 Mab, (Amgen, USA); CEP-7055 (Cephalon, USA); anti-Vn Mab (Crucell, Netherlands) angiocidin (InKine Pharmaceutical, USA); KM-2550 (Kyowa Hakko, Japan); SU-0879 (Pfizer, USA); CGP-79787 (Novartis, Switzerland, EP 970070); fibrinogen-E fragment (BioActa, UK); TBC-1635 (Encysive Pharmaceuticals, USA); SC-236 (Pfizer, USA); metastatin (EntreMed, USA); maspin (Sosei, Japan); ER-68203-00 (IVAX, USA); benefin (Lane Labs, USA); Tz-93 (Tsumura, Japan); TAN-1120 (Takeda, Japan); FR-111142 (Fujisawa, Japan); platelet factor 4; vascular endothelial growth factor antagonist, (Borean, Denmark); bevacizumab (pINN), (Genentech, USA); angiogenesis inhibitors, (SUGEN, USA); XL 784, (Exelixis, USA); XL 647, (Exelixis, USA); MAb, alpha5beta3 integrin, second generation, (Applied Molecular Evolution, USA and MedImmune, USA); enzastaurin hydrochloride (USAN), (Lilly, USA); CEP 7055, (Cephalon, USA and Sanofi-Synthelabo, France); BC 1, (Genoa Institute of Cancer Research, Italy); rBPI 21 and BPI-derived antiangiogenic (XOMA, USA); PI 88 (Progen, Australia); cetuximab, (Aventis, France); AVE 8062 (Ajinomoto, Japan); AS 1404, (Cancer Research Laboratory, New Zealand); SG 292, (Telios, USA); endostatin, (Boston Childrens Hospital, USA); ANGIOSTATIN (Boston Childrens Hospital, USA); AZD 6474, (AstraZeneca, UK); ZD 6126 (Angiogene Pharmaceuticals, UK); PPI 2458, (Praecis, USA); AZD 9935 (AstraZeneca, UK); AZD 2171 (AstraZeneca, UK); vatalanib (Novartis, Switzerland and Schering AG, Germany); tissue factor pathway inhibitors, (EntreMed, USA); pegaptanib (Pinn), (Gilead Sciences, USA); xanthorrhizol, (Yonsei University, South Korea); SDX 103, (University of California at San Diego, USA); PX 478, (ProlX, USA); METASTATIN (EntreMed, USA); troponin I, (Harvard University, USA); SU 6668, (SUGEN, USA); OXI 4503 (OXiGENE, USA); motuporamine C, (British Columbia University, Canada); CDP 791 (Celltech Group, UK); atiprimod (GlaxoSmithKline, UK); E 7820 (Eisai, Japan); CYC 381 (Harvard University, USA); AE 941 (Aeterna, Canada); urokinase plasminogen activator inhibitors; HIF-1 alfa inhibitors; angiocidin (InKine, USA); GW 2286 (GlaxoSmithKline, UK); EHT 0101 (ExonHit, France); CP 868596 (Pfizer, USA); CP 564959 (OSI, USA); CP 547632 (Pfizer, USA); 786034, (GlaxoSmithKline, UK); KRN 633 (Kirin Brewery, Japan); tumor necrosis factor-alpha inhibitors; KDR kinase inhibitors; combretastatin A4 prodrug (Arizona State University, USA); chondroitinase AC (IBEX, Canada); BAY RES 2690 (Bayer, Germany); tetrathiomolybdate (University of Michigan, USA); GCS 100 (Wayne State University, USA) CV 247 (Ivy Medical, UK); CKD 732, (Chong Kun Dang, South Korea); MAb, vascular endothelium growth factor, (Xenova, UK); irsogladine (Nippon Shinyaku, Japan); RG 13577 (Aventis, France); VE-cadherin-2 antagonists; vasostatin, (National Institutes of Health, USA); Flk-1, (ImClone Systems, USA); TZ 93 (Tsumura, Japan); TumStatin (Beth Israel Hospital, USA); forms of FLT 1 (vascular endothelial growth factor receptor 1); Tie-2 ligands (Regeneron, USA); and thrombospondin 1 inhibitor (Allegheny Health, USA).

Additional active compounds/agents that can be used in the treatment of cancers and that can be used in combination with one or more compounds of Formula (I) or (II) include: epoetin alfa; darbepoetin alfa; panitumumab; pegfilgrastim; palifermin; filgrastim; denosumab; ancestim or a pharmaceutically acceptable salt thereof.

The compounds of the present invention may also be used in combination with an additional pharmaceutically active compound that disrupts or inhibits RAS-RAF-ERK or PI3K-AKT-TOR signaling pathways. In other such combinations, the additional pharmaceutically active compound is a PD-1 and PD-L1 antagonist. The compounds or pharmaceutical compositions of the disclosure can also be used in combination with an amount of one or more substances selected from EGFR inhibitors, MEK inhibitors, ERK inhibitors, PI3K inhibitors, AKT inhibitors, TOR inhibitors, Mcl-1 inhibitors, BCL-2 inhibitors, SHP2 inhibitors, proteasome inhibitors, and immune therapies, including monoclonal antibodies, immunomodulatory imides (IMiDs), anti-PD-1, anti-PDL-1, anti-CTLA4, anti-LAGl, and anti-OX40 agents, GITR agonists, CAR-T cells, and BiTEs.

EGFR inhibitors include, but are not limited to, small molecule antagonists, antibody inhibitors, or specific antisense nucleotide or siRNA. Useful antibody inhibitors of EGFR include cetuximab (Erbitux), panitumumab (Vectibix), zalutumumab, nimotuzumab, and matuzumab. Small molecule antagonists of EGFR include gefitinib, erlotinib, and lapatinib.

Antibody-based EGFR inhibitors include any anti-EGFR antibody or antibody fragment that can partially or completely block EGFR activation by its natural ligand. Non-limiting examples of antibody-based EGFR inhibitors include those described in Modjtahedi, H., et al., 1993, Br. J. Cancer 67:247-253; Teramoto, T., et al., 1996, Cancer 77:639-645; Goldstein et al, 1995, Clin. Cancer Res. 1 : 1311-1318; Huang, S. M., et al., 1999, Cancer Res. 15:59(8): 1935-40; and Yang, X., et al., 1999, Cancer Res. 59: 1236-1243. The EGFR inhibitor can be monoclonal antibody Mab E7.6.3 (Yang, 1999 supra), or Mab C225 (ATCC Accession No. HB-8508), or an antibody or antibody fragment having the binding specificity thereof.

MEK inhibitors include, but are not limited to, CI-1040, AZD6244, PD318088, PD98059, PD334581, RDEA119, ARRY-142886, ARRY-438162, and PD-325901.

PI3K inhibitors include, but are not limited to, wortmannin, 17-hydroxywortmannin analogs described in WO 06/044453, 4-[2-(lH-Indazol-4-yl)-6-[[4-(methylsulfonyl)piperazin-1-yl]methyl]thieno[3,2-d]pyrimidin-4-yl]morpholine (also known as GDC 0941 and described in PCT Publication Nos. WO 09/036,082 and WO 09/055,730), 2-Methyl-2-[4-[3-methyl-2-oxo-8-(quinolin-3-yl)-2,3-dihydroimidazo[4,5-c]quinolin-1-yl]phenyl]propionitrile (also known as BEZ 235 or NVP-BEZ 235, and described in PCT Publication No. WO 06/122806), LY294002 (2-(4-Morpholinyl)-8-phenyl-4H-1-benzopyran-4-one available from Axon Medchem), PI 103 hydrochloride (3-[4-(4-morpholinylpyrido-[3',2':4,5]furo[3,2-d]pyrimidin-2-yl] phenol hydrochloride available from Axon Medchem), PIK 75 (N'-[(1E)-(6-bromoinddazo[1,2-a]pyridin-3-yl)methylene]-N,2-dimethyl-5-nitrobenzenesulfono-hydrazide hydrochloride available from Axon Medchem), PIK 90 (N-(7,8-dimethoxy-2,3-dihydro-imidazo[1,2-c]quinazolin-5-yl)-nicotinamide available from Axon Medchem), GDC-0941 bismesylate (2-(1H-Indazol-4-yl)-6-(4-methanesulfonyl-piperazin-1-ylmethyl)-4-mo holin- 4-yl-thieno[3,2-d]pyrimidine bismesylate available from Axon Medchem), AS-252424 (5-[1- [5-(4-fluoro-2-hydroxy-phenyl)-furan-2-yl]-meth-(Z)-ylidene]-thiazolidine-2,4-dione available from Axon Medchem), and TGX-221 (7-Methyl-2-(4-morpholinyl)-9-[1- (phenylamino)ethyl]-4H-pyrido-[1,2-a]pyrirnidin-4-one available from Axon Medchem), XL-765, and XL-147. Other PI3K inhibitors include demethoxyviridin, perifosine, CAL101, PX- 866, BEZ235, SF1126, INK1117, IPI-145, BKM120, XL147, XI,765, Palomid 529, GSK1059615, ZSTK474, PWT33597, IC87114, TGI 00-115, CAL263, PI-103, GNE-477, CUDC-907, and AEZS-136.

AKT inhibitors include, but are not limited to, Akt-1-1 (inhibits Aktl) (Barnett et al. (2005) Biochem. J., 385 (Pt. 2), 399-408); Akt-1-1,2 (Barnett et al. (2005) Biochem. J. 385 (Pt. 2), 399-408); API-59CJ-Ome (e.g., Jin et al. (2004) Br. J. Cancer 91, 1808-12); 1-H-imidazo[4,5-c]pyridinyl compounds (e.g., WO05011700); indole-3-carbinol and derivatives thereof (*e.g.*, U.S. Patent No. 6,656,963; Sarkar and Li (2004) J Nutr. 134(12 Suppl), 3493S-3498S); perifosine; Dasmahapatra et al. (2004) Clin. Cancer Res. 10(15), 5242-52, 2004); phosphatidylinositol ether lipid analogues (e.g., Gills and Dennis (2004) Expert. Opin. Investig. Drugs 13, 787-97); and triciribine (TCN or API-2 or NCI identifier: NSC 154020; Yang et al. (2004) Cancer Res. 64, 4394-9).

TOR inhibitors include, but are not limited to, inhibitors include AP -23573, CCI-779, everolimus, RAD-001, rapamycin, temsirolimus, ATP-competitive TORC1/TORC2 inhibitors, including PI-103, PP242, PP30 and Torin 1. Other TOR inhibitors in FKBP12 enhancer; rapamycins and derivatives thereof, including: CCI-779 (temsirolimus), RAD001 (Everolimus; WO 9409010) and AP23573; rapalogs, e.g. as disclosed in WO 98/02441 and WO 01/14387, e.g. AP23573, AP23464, or AP23841; 40-(2-hydroxyethyl)rapamycin, 40-[3-hydroxy(hydroxymethyl)methylpropanoate] -rapamycin , 40-epi-(tetrazolyt)-rapamycin (also called ABT578), 32-deoxorapamycin, 16-pentynyloxy-32(S)- dihydrorapanycin, and other derivatives disclosed in WO 05005434; derivatives disclosed in U.S. Pat. No. 5,258,389, WO 94/090101, WO 92/05179, U.S. Pat. No. 5,118,677, U.S. Pat. No. 5,118,678, U.S. Pat. No. 5,100,883, U.S. Pat. No. 5,151,413, U.S. Pat. No. 5,120,842, WO 93/111130, WO 94/02136, WO 94/02485, WO 95/14023, WO 94/02136, WO 95/16691, WO 96/41807, WO 96/41807 and U.S. Pat. No. 5,256,790; and phosphorus-containing rapamycin derivatives (e.g., WO

05016252).

MCl-1 inhibitors include, but are not limited to, AMG-176, MIK665, and S63845.

Proteasome inhibitors include, but are not limited to, Kyprolis® (carfilzomib), Velcade® (bortezomib), and oprozomib.

Immune therapies include, but are not limited to, anti-PD-1 agents, anti-PD-L1 agents, anti-CTLA-4 agents, anti-LAGI agents, and anti-OX40 agents.

Monoclonal antibodies include, but are not limited to, Darzalex® (daratumumab), Herceptin® (trastuzumab), Avastin® (bevacizumab), Rituxan® (rituximab), Lucentis® (ranibizumab), and Eylea® (aflibercept).

The compounds of Formula (I) and (I) may be used in combination with an anti-PD-1 antibody. The anti-PD-1 antibody may be pembrolizumab, cemiplimab, or nivolumab, preferably pembrolizumab.

The compounds of Formula (I) and (II) may be used in combination with an anti-PD-L1 antibody, such as atezolizumab, durvalumab, or avelumab.

The compounds of Formula (I) and (II) may be used in combination with an anti-CTLA-4 antibody, e.g., ipilumumab.

The compounds of the invention can be used in combination with the agents disclosed herein or other suitable agents, depending on the condition being treated. Hence, the one or more compounds of the invention may be co-administered with other agents as described above. When used in combination therapy, the compounds described herein are administered with the second agent simultaneously or separately. This administration in combination can include simultaneous administration of the two agents in the same dosage form, simultaneous administration in separate dosage forms, and separate administration. That is, a compound of Formula (I) or (II) and any of the agents described above can be formulated together in the same dosage form and administered simultaneously. Alternatively, a compound of Formula (I) or (II) and any of the agents described above can be simultaneously administered, wherein both the agents are present in separate formulations. In another alternative, a compound of Formula (I) or (II) can be administered just followed by and any of the agents described above, or vice versa. In some embodiments of the separate administration protocol, a compound of Formula (I) or (II) and any of the agents described above are administered a few minutes apart, or a few hours apart, or a few days apart.

As the treatment of the disease/conditions may involve a combination of pharmaceutically active compounds that may be administered separately, the separate pharmaceutical compositions may be combined in kit form. The kit may comprise two separate pharmaceutical compositions: a compound of Formula (I) or (II), and a second pharmaceutical compound. The kit may comprise a container for containing the separate compositions such as a divided bottle or a divided foil packet. Additional examples of containers include syringes, boxes, and bags. The kit may comprise directions for the use of the separate components. The kit form is particularly advantageous when the separate components are preferably administered in different dosage forms (e.g., oral and parenteral), are administered at different dosage intervals, or when titration of the individual components of the combination is desired by the prescribing health care professional.

In another aspect, the present invention provides a process for preparing a compound of Formula I:

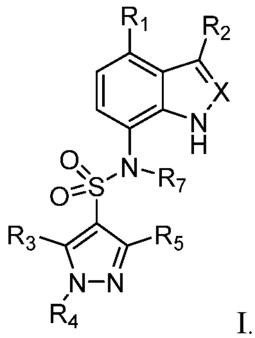

I.

the process comprising reacting an intermediate of Formula A:

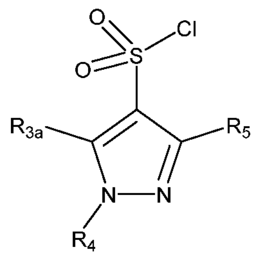

with an intermediate of Formula B:

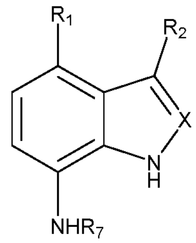

in the presence of a base to give a compound of Formula I, optionally adding or removing any protecting groups if desired, and optionally forming any salt if desired, wherein:
or a pharmaceutically acceptable salt thereof, wherein:

X is N or $CR_6$;
**$R_1$** is hydrogen, C1-C4 alkyl, C1-C4 fluoroalkyl, or halo;
**$R_2$** is halo or cyano;
$R_3$ is hydrogen, C1-C4 alkyl, halo, cyano, C1-C4 fluoroalkyl, -(C1-C4 alkyl)OH, or -$NR^aR^b$, wherein $R^a$ or $R^b$ is independently hydrogen, C3-C6 cycloalkyl, heterocycloalkyl or C1-C4 alkyl, or $R^a/R^b$ and the nitrogen atom they are attached to form a 5 or 6-membered ring optionally independently substituted by one or more substituents selected from halo, C1-C4 alkyl, cyano, hydroxy and -O(C1-C4 alkyl);
**$R_{3a}$** is hydrogen, C1-C4 alkyl, halo, cyano, C1-C4 fluoroalkyl, -(C1-C4 alkyl)OH, or -$NR^aR^b$, wherein $R^a$ or $R^b$ is independently hydrogen, C3-C6 cycloalkyl, heterocycloalkyl, -C(=O)O(C1-C4 alkyl) or C1-C4 alkyl, or $R^a/R^b$ and the nitrogen atom they are attached to form a 5 or 6-membered ring optionally independently substituted by one or more substituents selected from halo, C1-C4 alkyl, cyano, hydroxy and -O(C1-C4 alkyl);
**$R_4$** is hydrogen, C1-C6 alkyl, C1-C10heteroalkyl, C1-C4 fluoroalkyl, -(C1-C4 alkyl)$NH_2$, C3-C6 cycloalkyl, -(C1-C4 alkyl)(C3-C6 cycloalkyl), -(C1-C4 alkyl)phenyl, phenyl, heteroaryl, -(C1-C4 alkyl)heteroaryl, C2-C5 alkenyl, -(C1-C4 alkyl)heterocycloalkyl, heterocycloalkyl, -C0-C4 alkyl$S(=O)_2$(C1-C4 alkyl), -$S(=O)_2$phenyl, -$S(=O)_2$heteroaryl, -C0-C4 alkylC(=O)heterocycloalkyl, -C0-C4 alkyl C(=O)$NR^aR^b$, -(C0-C4 alkyl)C(=O)O(C1-C4 alkyl), -(C0-C4 alkyl)C(=O)C1-C4 alkyl, -(CO-C4 alkyl)C(=O)OH or -(C1-C8 alkyl)OH, wherein **$R_4$** is substituted with 0, 1, 2, or 3 substituents selected from deuterium, halo, hydroxy, -(C0-C4alkyl)O(C1-C4 alkyl), -C1-C4 alkyl, -(C0-C4alkyl)cyano, -$S(=O)_2$(C1-C4 alkyl), - C0-C4 alkylC(=O)$NR^aR^b$, -C(=O)O(C1-C4 alkyl), C1-C4 fluoroalkyl, oxo, -(C1-C4 alkyl)OH, and -$NH_2$, wherein each of $R^a$ and $R^b$ is independently substituted with 0, 1, 2, or 3 hydrogen, C3-C6 cycloalkyl, heterocycloalkyl or C1-C4 alkyl;
**$R_5$** is hydrogen, halo, -$NH_2$ or C1-C4 alkyl;
**$R_6$** is hydrogen, halo or C1-C4 alkyl; and
**$R_7$** is hydrogen, halo, heteroalkyl, C1-C4 alkyl, -(C0-C4 alkyl)O( C1-C4 alkyl), -(C1-C4 alkyl)(C3-C6 cycloalkyl), C3-C6 cycloalkyl, -(C1-C4 alkyl)heterocycloalkyl, or heterocycloalkyl.

The present invention also provides a process for preparing a compound of Formula II:

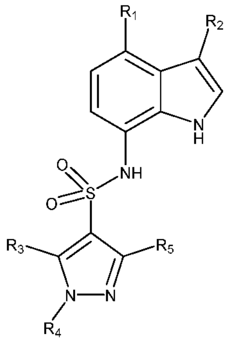

II.

The process comprises reacting an intermediate of Formula A:

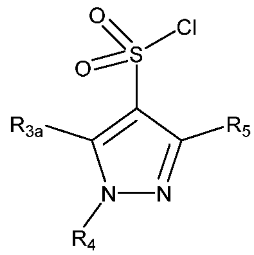

with an intermediate of Formula B:

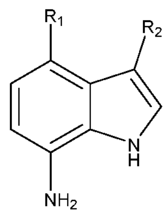

in the presence of a base to give a compound of Formula II, optionally adding or removing any protecting groups if desired, and optionally forming any salt if desired, wherein:

$R_1$ is hydrogen, C1-C4 alkyl or halo;
$R_2$ is halo or cyano;
$R_3$ is hydrogen, C1-C4 alkyl, halo, cyano or $-NR^aR^b$, wherein $R^a$ or $R^b$ is independently hydrogen, C3-C6 cycloalkyl, heterocycloalkyl or C1-C4 alkyl, or $R^a/R^b$ and the nitrogen atom they are attached to form a 5 or 6-membered ring optionally independently substituted by one or more substituents selected from halo, C1-C4 alkyl, cyano, hydroxy and -O(C1-C4 alkyl);
$R_{3a}$ is hydrogen, C1-C4 alkyl, halo, cyano or $-NR^aR^b$, wherein $R^a$ or $R^b$ is independently hydrogen, C3-C6 cycloalkyl, heterocycloalkyl, -C(=O)O(C1-C4 alkyl) or C1-C4 alkyl, or $R^a/R^b$ and the nitrogen atom they are attached to form a 5 or 6-membered ring optionally independently substituted by one or more substituents selected from halo, C1-C4 alkyl, cyano, hydroxy and -O(C1-C4 alkyl);
$R_4$ is hydrogen, C1-C6 alkyl, C3-C6 cycloalkyl, -(C1-C4 alkyl)(C3-C6 cycloalkyl), -(C1-C4 alkyl)phenyl, phenyl, heteroaryl, -(C1-C4 alkyl)heteroaryl, C2-C5 alkenyl, -(C1-C4 alkyl)heterocycloalkyl, heterocycloalkyl, $-S(=O)_2$(C1-C4 alkyl), $-S(=O)_2$phenyl or $-S(=O)_2$heteroaryl; and $R_5$ is hydrogen, halo or C1-C4 alkyl.

Examples of bases include inorganic bases (e.g., sodium bicarbonate, sodium carbonate, potassium bicarbonate, monopotassium phosphate, dipotassium phosphate, and tripotassium phosphate) and organic bases (e.g., triethylamine, diisopropylethylamine, and 2,6-lutidine). Suitable solvent include pyridine, DMF, THF and dichloromethane. Unless specified to the contrary, the reactions described herein take place at atmospheric pressure, generally within a temperature range from -10 °C to 200 °C. Further, except as otherwise specified, reaction times and conditions are intended to be approximate, e.g., taking place at about atmospheric pressure within a temperature range of about -10

°C to about 110 °C over a period of about 1 to about 24 hours; reactions left to run overnight average a period of about 16 hours.

In some embodiments, a compound of the present disclosure, for example a compound in **Table 1**, is synthesized according to the process for compounds of Formulae I and II outlined above, **Examples 1-36** or by methods generally known in the art.

**Table 1**

| Compound No. | M+H | M-H | Structure | Name |
|---|---|---|---|---|
| 1 | | | 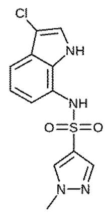 | N-(3-chloro-1H-indol-7-yl)-1-methyl-pyrazole-4-sulfonamide |
| 2 | 339 | | 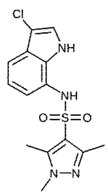 | N-(3-chloro-1H-indol-7-yl)-1,3,5-trimethyl-pyrazole-4-sulfonamide |
| 3 | | | 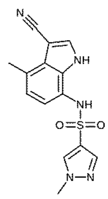 | N-(3-cyano-4-methyl-1H-indol-7-yl)-1-methyl-pyrazole-4-sulfonamide |
| 4 | 325 | | 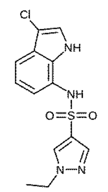 | N-(3-chloro-1H-indol-7-yl)-1-ethyl-pyrazole-4-sulfonamide |
| 5 | 325 | | 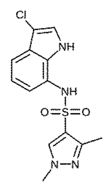 | N-(3-chloro-1H-indol-7-yl)-1,3-dimethylpyrazole-4-sulfonamide |
| 6 | | | 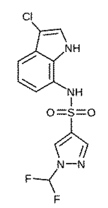 | N-(3-chloro-1H-indol-7-yl)-1-(difluoromethyl)pyrazole-4-sulfonamide |

(continued)

| Compound No. | M+H | M-H | Structure | Name |
|---|---|---|---|---|
| 7 | | | 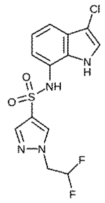 | N-(3-chloro-1H-indol-7-yl)-1-(2,2-difluoroethyl) pyrazole-4-sulfonamide |
| 8 | 367 | | 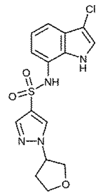 | N-(3-chloro-1H-indol-7-yl)-1-tetrahydrofuran-3-yl-pyrazole-4-sulfonamide |
| 9 | 403 | | 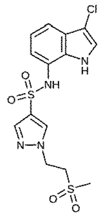 | N-(3-chloro-1H-indol-7-yl)-1-(2-methylsulfonylethyl) pyrazole-4-sulfonamide |
| 10 | 297 | | 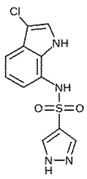 | N-(3-chloro-1H-indol-7-yl)-1H-pyrazole-4-sulfonamide |
| 11 | | | 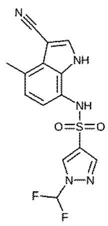 | N-(3-cyano-4-methyl-1H-indol-7-yl)-1-(difluoromethyl) pyrazole-4-sulfonamide |
| 12 | 450 | | 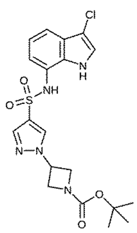 | tert-butyl 3-[4-[(3-chloro-1H-indol-7-yl)sulfamoyl] pyrazol-1-yl]azetidine-1- carboxylate |
| 13 | 341 | | 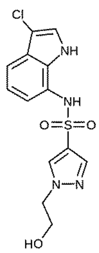 | N-(3-chloro-1H-indol-7-yl)-1-(2-hydroxyethyl)pyrazole-4-sulfonamide |

(continued)

| Compound No. | M+H | M-H | Structure | Name |
|---|---|---|---|---|
| 14 | 336 | | 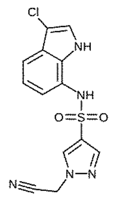 | N-(3-chloro-1H-indol-7-yl)-1-(cyanomethyl)pyrazole-4-sulfonamide |
| 15 | 426 | | 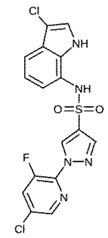 | 1-(5-chloro-3-fluoro-2-pyridyl)-N-(3-chloro-1H-indol-7-yl)pyrazole-4-sulfonamide |
| 16 | 354 | | 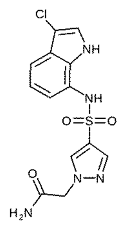 | 2-[4-[(3-chloro-1H-indol-7-yl)sulfamoyl]pyrazol-1-yl] acetamide |
| 17 | 484 | | 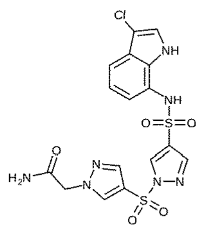 | 2-[4-[4-[(3-chloro-1H-indol-7-yl)sulfamoyl]pyrazol-1-yl] sulfonylpyrazol-1-yl]acetamide |
| 18 | 352 | | 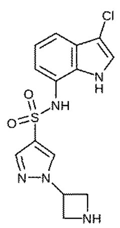 | 1-(azetidin-3-yl)-N-(3-chloro-1H-indol-7-yl)pyrazole-4-sulfonamide |
| 19 | 427 | | 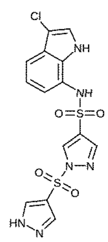 | N-(3-chloro-1H-indol-7-yl)-1-(1H-pyrazol-4-ylsulfonyl) pyrazole-4-sulfonamide |

(continued)

| Compound No. | M+H | M-H | Structure | Name |
|---|---|---|---|---|
| 20 | 381 | | 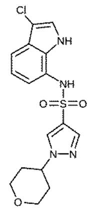 | N-(3-chloro-1H-indol-7-yl)-1-tetrahydropyran-4-yl-pyrazole-4-sulfonamide |
| 21 | 392 | | 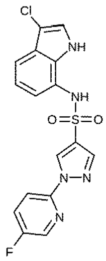 | N-(3-chloro-1H-indol-7-yl)-1-(5-fluoro-2-pyridyl) pyrazole-4-sulfonamide |
| 22 | 466 | | 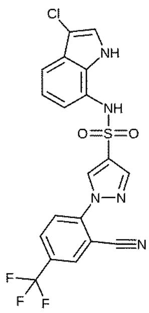 | N-(3-chloro-1H-indol-7-yl)-1-[2-cyano-4-(trifluoromethyl)phenyl]pyrazole-4-sulfonamide |
| 23 | 399 | | 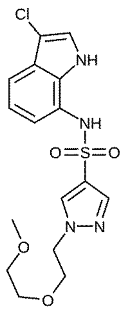 | N-(3-chloro-1H-indol-7-yl)-1-[2-(2-methoxyethoxy)ethyl] pyrazole-4-sulfonamide |
| 24 | 443 | | 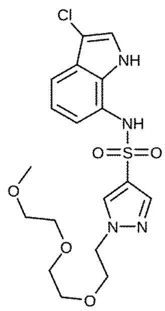 | N-(3-chloro-1H-indol-7-yl)-1-[2-[2-(2-methoxyethoxy) ethoxy]ethyl]pyrazole-4-sulfonamide |

(continued)

| Compound No. | M+H | M-H | Structure | Name |
|---|---|---|---|---|
| 25 | | | 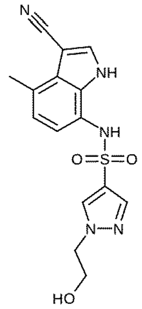 | N-(3-cyano-4-methyl-1H-indol-7-yl)-1-(2-hydroxyethyl) pyrazole-4-sulfonamide |
| 26 | | 478 | 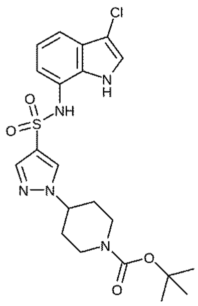 | tert-butyl 4-[4-[(3-chloro-1H-indol-7-yl)sulfamoyl] pyrazol-1-yl]piperidine-1-carboxylate |
| 27 | | 392 | 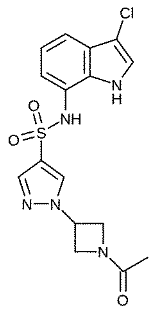 | 1-(1-acetylazetidin-3-yl)-N-(3-chloro-1H-indol-7-yl) pyrazole-4-sulfonamide |
| 28 | 381 | | 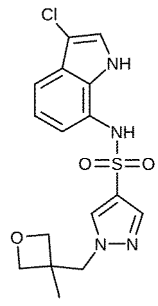 | N-(3-chloro-1H-indol-7-yl)-1-[(3-methyloxetan-3-yl) methyl]pyrazole-4-sulfonamide |
| 29 | | | 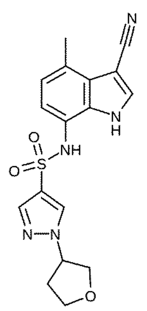 | N-(3-cyano-4-methyl-1H-indol-7-yl)-1-tetrahydrofuran-3-yl-pyrazole-4-sulfonamide |

(continued)

| Compound No. | M+H | M-H | Structure | Name |
|---|---|---|---|---|
| 30 | 442 | | 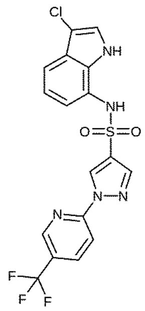 | N-(3-chloro-1H-indol-7-yl)-1-[5-(trifluoromethyl)-2-pyridyl]pyrazole-4-sulfonamide |
| 31 | 447 | | 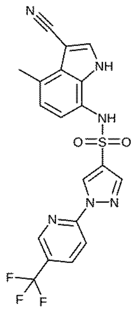 | N-(3-cyano-4-methyl-1H-indol-7-yl)-1-[5-(trifluoromethyl)-2-pyridyl]pyrazole-4-sulfonamide |
| 32 | 385 | | 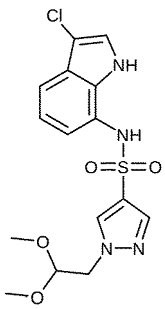 | N-(3-chloro-1H-indol-7-yl)-1-(2,2-dimethoxyethyl) pyrazole-4-sulfonamide |
| 33 | | 438 | 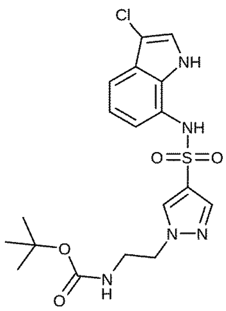 | tert-butyl N-[2-[4-[(3-chloro-1H-indol-7-yl)sulfamoyl] pyrazol-1-yl]ethyl]carbamate |
| 34 | 340 | | 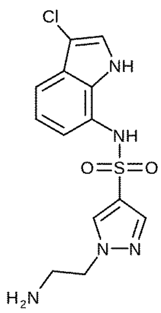 | 1-(2-aminoethyl)-N-(3-chloro-1H-indol-7-yl)pyrazole-4-sulfonamide |

(continued)

| Compound No. | M+H | M-H | Structure | Name |
|---|---|---|---|---|
| 35 | 343 | | 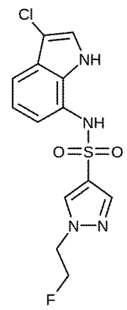 | N-(3-chloro-1H-indol-7-yl)-1-(2-fluoroethyl)pyrazole-4-sulfonamide |
| 36 | | | 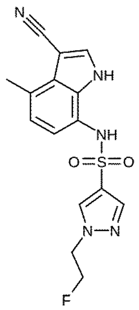 | N-(3-cyano-4-methyl-1H-indol-7-yl)-1-(2-fluoroethyl) pyrazole-4-sulfonamide |
| 37 | 373 | | 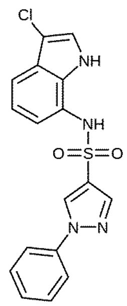 | N-(3-chloro-1H-indol-7-yl)-1-phenyl-pyrazole-4-sulfonamide |
| 38 | | | 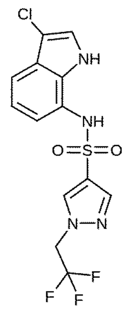 | N-(3-chloro-1H-indol-7-yl)-1-(2,2,2-trifluoroethyl) pyrazole-4-sulfonamide |
| 39 | | | 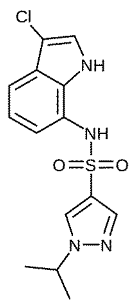 | N-(3-chloro-1H-indol-7-yl)-1-isopropyl-pyrazole-4-sulfonamide |

(continued)

| Compound No. | M+H | M-H | Structure | Name |
|---|---|---|---|---|
| 40 | | | 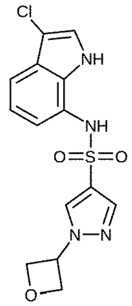 | N-(3-chloro-1H-indol-7-yl)-1-(oxetan-3-yl)pyrazole-4-sulfonamide |
| 41 | 380 | | 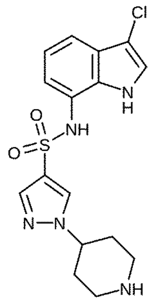 | N-(3-chloro-1H-indol-7-yl)-1-(4-piperidyl)pyrazole-4-sulfonamide |
| 42 | | 464 | 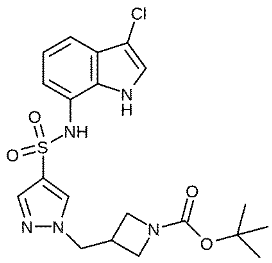 | tert-butyl 3-[[4-[(3-chloro-1H-indol-7-yl)sulfamoyl] pyrazol-1-yl]methyl]azetidine-1-carboxylate |
| 43 | 366 | | 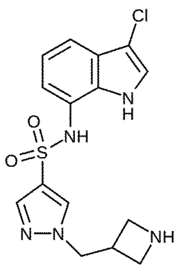 | 1-(azetidin-3-ylmethyl)-N-(3-chloro-1H-indol-7-yl) pyrazole-4-sulfonamide |
| 44 | 397 | | 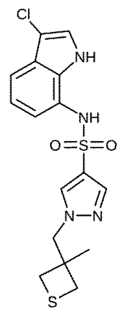 | N-(3-chloro-1H-indol-7-yl)-1-[(3-methylthietan-3-yl) methyl]pyrazole-4-sulfonamide |

(continued)

| Compound No. | M+H | M-H | Structure | Name |
|---|---|---|---|---|
| 45 | 429 | | 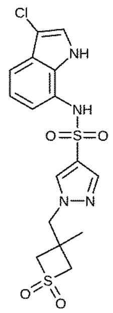 | N-(3-chloro-1H-indol-7-yl)-1-[(3-methyl-1,1-dioxo-thietan-3-yl)methyl]pyrazole-4-sulfonamide |
| 46 | 302 | | 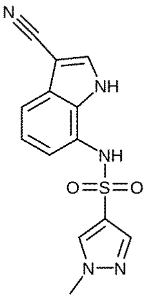 | N-(3-cyano-1H-indol-7-yl)-1-methylpyrazole-4-sulfonamide |
| 47 | 351 | | 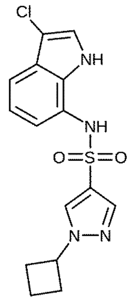 | N-(3-chloro-1H-indol-7-yl)-1-cyclobutyl-pyrazole-4-sulfonamide |
| 48 | | | 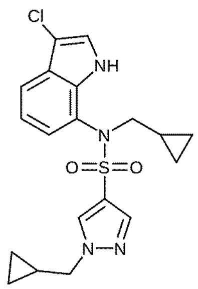 | N-(3-chloro-1H-indol-7-yl)-N,1-bis(cyclopropylmethyl) pyrazole-4-sulfonamide |
| 49 | 355 | | 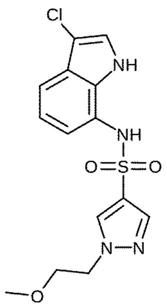 | N-(3-chloro-1H-indol-7-yl)-1-(2-methoxyethyl)pyrazole-4-sulfonamide |

(continued)

| Compound No. | M+H | M-H | Structure | Name |
|---|---|---|---|---|
| 50 | | 411 | 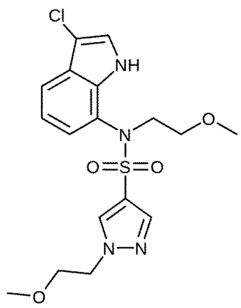 | N-(3-chloro-1H-indol-7-yl)-N,1-bis(2-methoxyethyl) pyrazole-4-sulfonamide |
| 51 | 351 | | 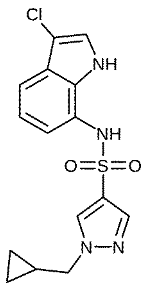 | N-(3-chloro-1H-indol-7-yl)-1-(cyclopropylmethyl) pyrazole-4-sulfonamide |
| 52 | | 374 | 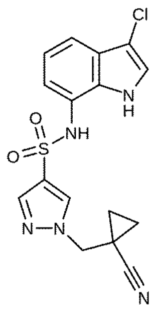 | N-(3-chloro-1H-indol-7-yl)-1-[(1-cyanocyclopropyl) methyl]pyrazole-4-sulfonamide |
| 53 | 401 | | 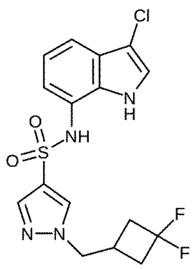 | N-(3-chloro-1H-indol-7-yl)-1-[(3,3-difluorocyclobutyl) methyl]pyrazole-4-sulfonamide |
| 54 | 329 | | 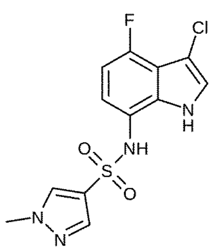 | N-(3-chloro-4-fluoro-1H-indol-7-yl)-1-methyl-pyrazole-4-sulfonamide |

(continued)

| Compound No. | M+H | M-H | Structure | Name |
|---|---|---|---|---|
| 56 | | | 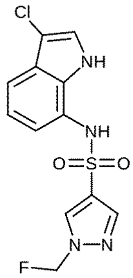 | N-(3-chloro-1H-indol-7-yl)-1-(fluoromethyl)pyrazole-4-sulfonamide |
| 57 | 357 | | 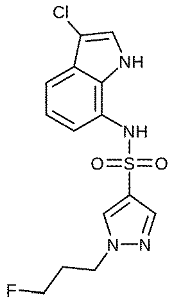 | N-(3-chloro-1H-indol-7-yl)-1-(3-fluoropropyl)pyrazole-4-sulfonamide |
| 58 | 401 | | 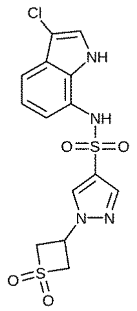 | N-(3-chloro-1H-indol-7-yl)-1-(1,1-dioxothietan-3-yl) pyrazole-4-sulfonamide |
| 59 | | | 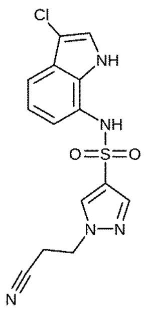 | N-(3-chloro-1H-indol-7-yl)-1-(2-cyanoethyl)pyrazole-4-sulfonamide |
| 60 | | | 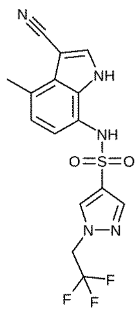 | N-(3-cyano-4-methyl-1H-indol-7-yl)-1-(2,2,2-trifluoroethyl)pyrazole-4-sulfonamide |

(continued)

| Compound No. | M+H | M-H | Structure | Name |
|---|---|---|---|---|
| 61 | | | 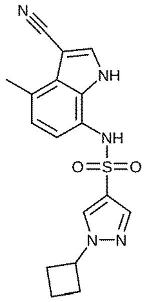 | N-(3-cyano-4-methyl-1H-indol-7-yl)-1-cyclobutyl-pyrazole-4-sulfonamide |
| 62 | 302 | | 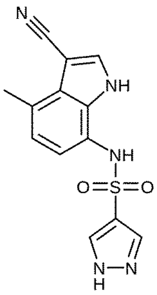 | N-(3-cyano-4-methyl-1H-indol-7-yl)-1H-pyrazole-4-sulfonamide |
| 63 | | | 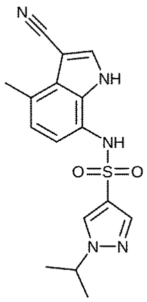 | N-(3-cyano-4-methyl-1H-indol-7-yl)-1-isopropyl-pyrazole-4-sulfonamide |
| 64 | | | 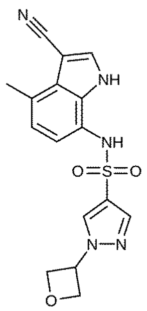 | N-(3-cyano-4-methyl-1H-indol-7-yl)-1-(oxetan-3-yl) pyrazole-4-sulfonamide |
| 65 | | | 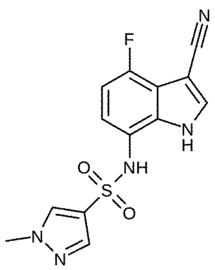 | N-(3-cyano-4-fluoro-1H-indol-7-yl)-1-methyl-pyrazole-4-sulfonamide |

(continued)

| Compound No. | M+H | M-H | Structure | Name |
|---|---|---|---|---|
| 66 | | | 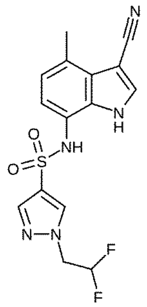 | N-(3-cyano-4-methyl-1H-indol-7-yl)-1-(2,2-difluoroethyl)pyrazole-4-sulfonamide |
| 67 | | 406 | 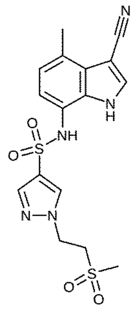 | N-(3-cyano-4-methyl-1H-indol-7-yl)-1-(2-methylsulfonylethyl)pyrazole-4-sulfonamide |
| 68 | | | 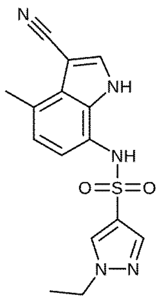 | N-(3-cyano-4-methyl-1H-indol-7-yl)-1-ethyl-pyrazole-4-sulfonamide |
| 69 | | | 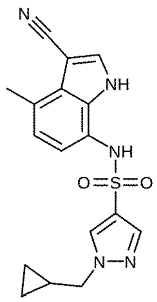 | N-(3-cyano-4-methyl-1H-indol-7-yl)-1-(cyclopropylmethyl)pyrazole-4-sulfonamide |
| 70 | | | 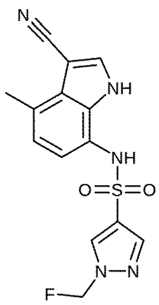 | N-(3-cyano-4-methyl-1H-indol-7-yl)-1-(fluoromethyl) pyrazole-4-sulfonamide |

(continued)

| Compound No. | M+H | M-H | Structure | Name |
|---|---|---|---|---|
| 71 | | | 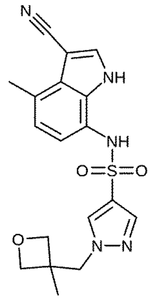 | N-(3-cyano-4-methyl-1H-indol-7-yl)-1-[(3-methyloxetan-3-yl)methyl]pyrazole-4-sulfonamide |
| 72 | | | 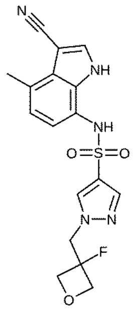 | N-(3-cyano-4-methyl-1H-indol-7-yl)-1-[(3-fluorooxetan-3-yl)methyl]pyrazole-4-sulfonamide |
| 73 | | | 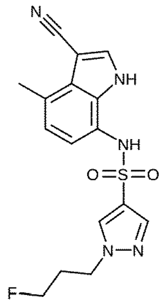 | N-(3-cyano-4-methyl-1H-indol-7-yl)-1-(3-fluoropropyl) pyrazole-4-sulfonamide |
| 75 | | | 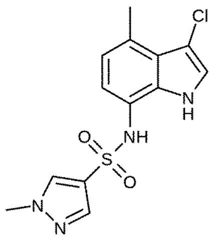 | N-(3-chloro-4-methyl-1H-indol-7-yl)-1-methyl-pyrazole-4-sulfonamide |
| 76 | 383 | | 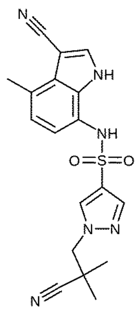 | N-(3-cyano-4-methyl-1H-indol-7-yl)-1-(2-cyano-2-methyl-propyl)pyrazole-4-sulfonamide |

(continued)

| Compound No. | M+H | M-H | Structure | Name |
|---|---|---|---|---|
| 77 | | | 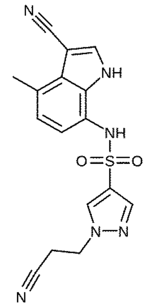 | 1-(2-cyanoethyl)-N-(3-cyano-4-methyl-1H-indol-7-yl) pyrazole-4-sulfonamide |
| 78 | 370 | | 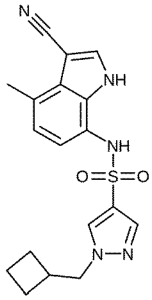 | N-(3-cyano-4-methyl-1H-indol-7-yl)-1-(cyclobutylmethyl)pyrazole-4-sulfonamide |
| 79 | | | 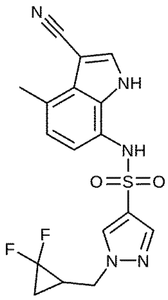 | N-(3-cyano-4-methyl-1H-indol-7-yl)-1-[(2,2-difluorocyclopropyl)methyl]pyrazole-4-sulfonamide |
| 80 | | | 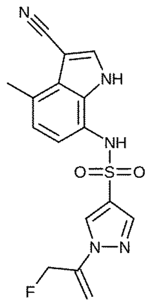 | N-(3-cyano-4-methyl-1H-indol-7-yl)-1-[1-(fluoromethyl) vinyl]pyrazole-4-sulfonamide |
| 81 | | | 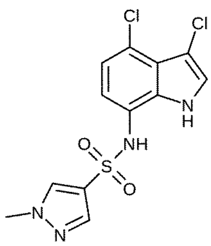 | N-(3,4-dichloro-1H-indol-7-yl)-1-methylpyrazole-4-sulfonamide |

(continued)

| Compound No. | M+H | M-H | Structure | Name |
|---|---|---|---|---|
| 82 | | | 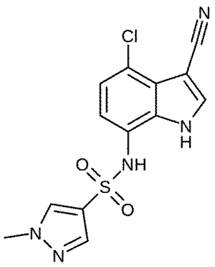 | N-(4-chloro-3-cyano-1H-indol-7-yl)-1-methyl-pyrazole-4-sulfonamide |
| 83 | 367 | | 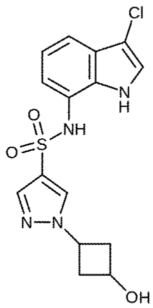 | N-(3-chloro-1H-indol-7-yl)-1-(3-hydroxycyclobutyl) pyrazole-4-sulfonamide |
| 84 | 436 | | 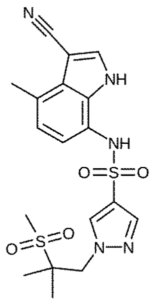 | N-(3-cyano-4-methyl-1H-indol-7-yl)-1-(2-methyl-2-methylsulfonyl-propyl)pyrazole-4-sulfonamide |
| 85 | 398 | | 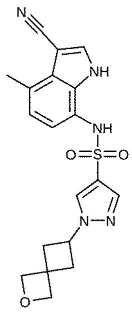 | N-(3-cyano-4-methyl-1H-indol-7-yl)-1-(2-oxaspiro[3.3] heptan-6-yl)pyrazole-4-sulfonamide |
| 86 | 420 | | 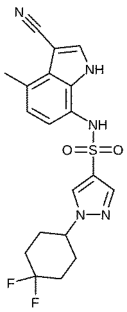 | N-(3-cyano-4-methyl-1H-indol-7-yl)-1-(4,4-difluorocyclohexyl)pyrazole-4-sulfonamide |

(continued)

| Compound No. | M+H | M-H | Structure | Name |
|---|---|---|---|---|
| 87 | 467 | | 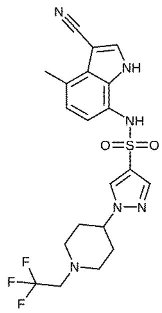 | N-(3-cyano-4-methyl-1H-indol-7-yl)-1-[1-(2,2,2-trifluoroethyl)-4-piperidyl]pyrazole-4-sulfonamide |
| 88 | | | 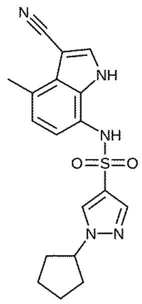 | N-(3-cyano-4-methyl-1H-indol-7-yl)-1-cyclopentyl-pyrazole-4-sulfonamide |
| 89 | 392 | | 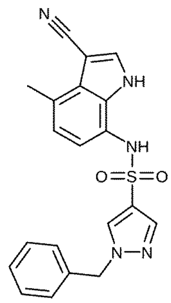 | 1-benzyl-N-(3-cyano-4-methyl-1H-indol-7-yl)pyrazole-4-sulfonamide |
| 90 | | | 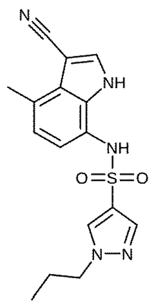 | N-(3-cyano-4-methyl-1H-indol-7-yl)-1-propyl-pyrazole-4-sulfonamide |
| 91 | | | 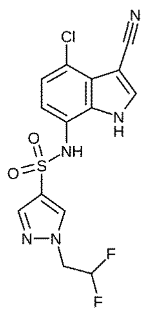 | N-(4-chloro-3-cyano-1H-indol-7-yl)-1-(2,2-difluoroethyl) pyrazole-4-sulfonamide |

(continued)

| Compound No. | M+H | M-H | Structure | Name |
|---|---|---|---|---|
| 92 | | 368 | 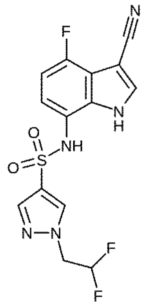 | N-(3-cyano-4-fluoro-1H-indol-7-yl)-1-(2,2-difluoroethyl) pyrazole-4-sulfonamide |
| 93 | 380 | | 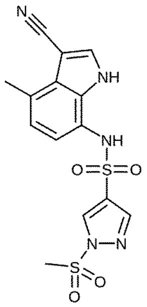 | N-(3-cyano-4-methyl-1H-indol-7-yl)-1-methylsulfonyl-pyrazole-4-sulfonamide |
| 94 | | | 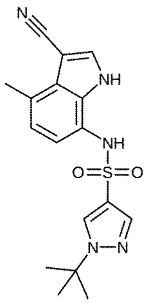 | 1-tert-butyl-N-(3-cyano-4-methyl-1H-indol-7-yl) pyrazole-4-sulfonamide |
| 95 | 353 | | 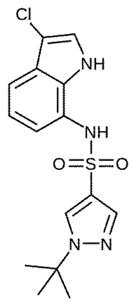 | 1-tert-butyl-N-(3-chloro-1H-indol-7-yl)pyrazole-4-sulfonamide |
| 97 | 379 | | 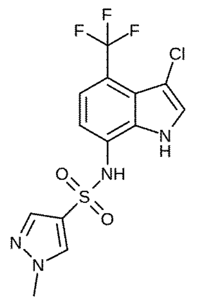 | N-[3-chloro-4-(trifluoromethyl)-1H-indol-7-yl]-1-methyl-pyrazole-4-sulfonamide |

(continued)

| Compound No. | M+H | M-H | Structure | Name |
|---|---|---|---|---|
| 98 | | | 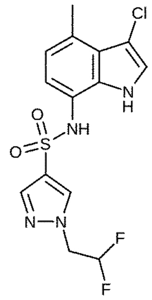 | N-(3-chloro-4-methyl-1H-indol-7-yl)-1-(2,2-difluoroethyl)pyrazole-4-sulfonamide |
| 99 | 424 | | 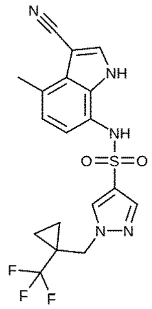 | N-(3-cyano-4-methyl-1H-indol-7-yl)-1-[[1-(trifluoromethyl)cyclopropyl]methyl]pyrazole -4-sulfonamide |
| 100 | 398 | | 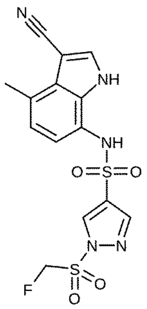 | N-(3-cyano-4-methyl-1H-indol-7-yl)-1-(fluoromethylsulfonyl)pyrazole-4-sulfonamide |
| 101 | 442 | | 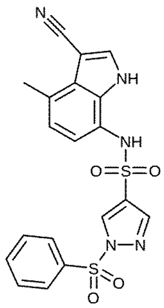 | 1-(benzenesulfonyl)-N-(3-cyano-4-methyl-1H-indol-7-yl)pyrazole-4-sulfonamide |
| 102 | | | 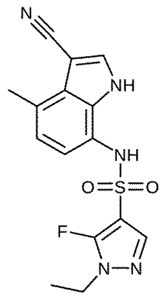 | N-(3-cyano-4-methyl-1H-indol-7-yl)-1-ethyl-5-fluoro-pyrazole-4-sulfonamide |

(continued)

| Compound No. | M+H | M-H | Structure | Name |
|---|---|---|---|---|
| 103 | | 427 | 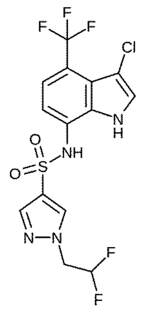 | N-[3-chloro-4-(trifluoromethyl)-1H-indol-7-yl]-1-(2,2-difluoroethyl)pyrazole-4-sulfonamide |
| 104 | | 418 | 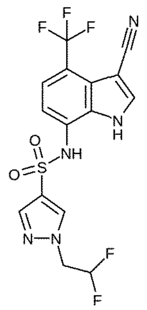 | N-[3-cyano-4-(trifluoromethyl)-1H-indol-7-yl]-1-(2,2-difluoroethyl)pyrazole-4-sulfonamide |
| 105 | 370 | | 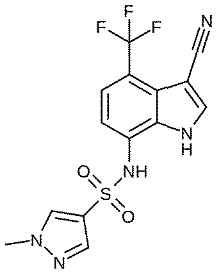 | N-[3-cyano-4-(trifluoromethyl)-1H-indol-7-yl]-1-methyl-pyrazole-4-sulfonamide |
| 106 | 402 | | 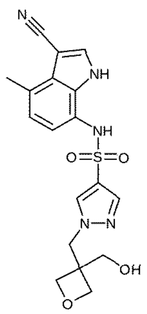 | N-(3-cyano-4-methyl-1H-indol-7-yl)-1-[[3-(hydroxymethyl)oxetan-3-yl]methyl]pyrazole-4-sulfonamide |
| 107 | | | 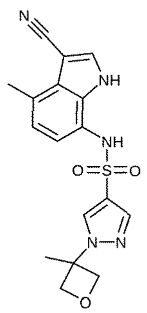 | N-(3-cyano-4-methyl-1H-indol-7-yl)-1-(3-methyloxetan-3-yl)pyrazole-4-sulfonamide |

(continued)

| Compound No. | M+H | M-H | Structure | Name |
|---|---|---|---|---|
| 108 | | | 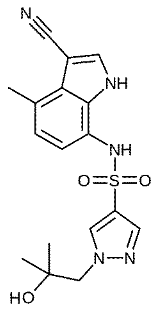 | N-(3-cyano-4-methyl-1H-indol-7-yl)-1-(2-hydroxy-2-methyl-propyl)pyrazole-4-sulfonamide |
| 109 | 392 | | 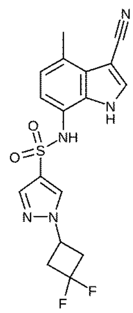 | N-(3-cyano-4-methyl-1H-indol-7-yl)-1-(3,3-difluorocyclobutyl)pyrazole-4-sulfonamide |
| 110 | | | 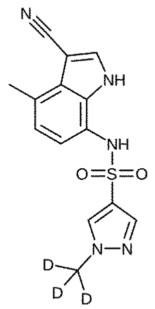 | N-(3-cyano-4-methyl-1H-indol-7-yl)-1-(trideuteriomethyl)pyrazole-4-sulfonamide |
| 111 | | 372 | 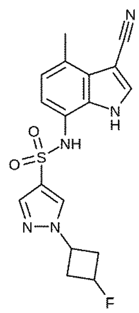 | N-(3-cyano-4-methyl-1H-indol-7-yl)-1-(3-fluorocyclobutyl)pyrazole-4-sulfonamide |
| 112 | | | 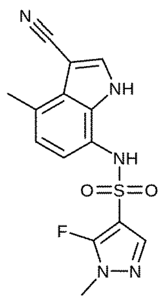 | N-(3-cyano-4-methyl-1H-indol-7-yl)-5-fluoro-1-methyl-pyrazole-4-sulfonamide |

(continued)

| Compound No. | M+H | M-H | Structure | Name |
|---|---|---|---|---|
| 113 | | | 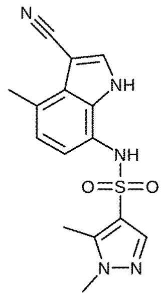 | N-(3-cyano-4-methyl-1H-indol-7-yl)-1,5-dimethyl-pyrazole-4-sulfonamide |
| 114 | 330 | | 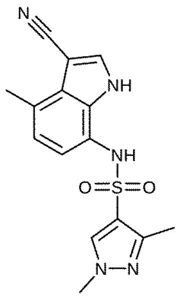 | N-(3-cyano-4-methyl-1H-indol-7-yl)-1,3-dimethyl-pyrazole-4-sulfonamide |
| 115 | | | 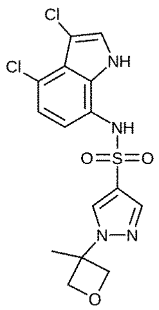 | N-(3,4-dichloro-1H-indol-7-yl)-1-(3-methyloxetan-3-yl) pyrazole-4-sulfonamide |
| 116 | 388 | | 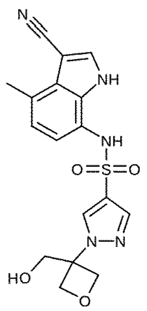 | N-(3-cyano-4-methyl-1H-indol-7-yl)-1-[3-(hydroxymethyl)oxetan-3-yl]pyrazole-4-sulfonamide |
| 117 | | | 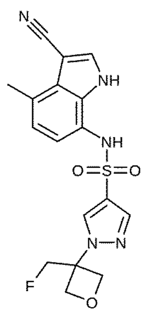 | N-(3-cyano-4-methyl-1H-indol-7-yl)-1-[3-(fluoromethyl) oxetan-3-yl]pyrazole-4-sulfonamide |

(continued)

| Compound No. | M+H | M-H | Structure | Name |
|---|---|---|---|---|
| 118 | | | 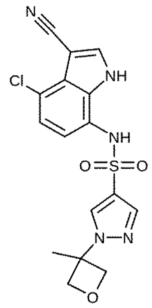 | N-(4-chloro-3-cyano-1H-indol-7-yl)-1-(3-methyloxetan-3-yl)pyrazole-4-sulfonamide |
| 119 | | | 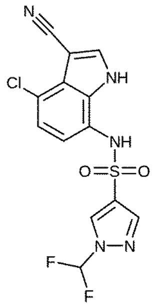 | N-(4-chloro-3-cyano-1H-indol-7-yl)-1-(difluoromethyl) pyrazole-4-sulfonamide |
| 120 | | | 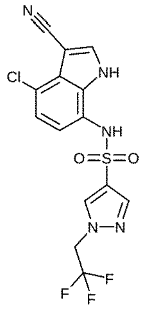 | N-(4-chloro-3-cyano-1H-indol-7-yl)-1-(2,2,2-trifluoroethyl)pyrazole-4-sulfonamide |
| 121 | | | 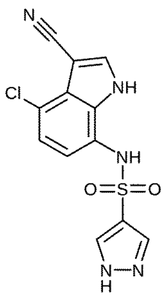 | N-(4-chloro-3-cyano-1H-indol-7-yl)-1H-pyrazole-4-sulfonamide |
| 122 | | | 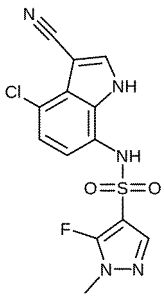 | N-(4-chloro-3-cyano-1H-indol-7-yl)-5-fluoro-1-methyl-pyrazole-4-sulfonamide |

(continued)

| Compound No. | M+H | M-H | Structure | Name |
|---|---|---|---|---|
| 123 | | | 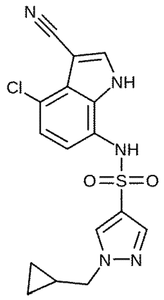 | N-(4-chloro-3-cyano-1H-indol-7-yl)-1-(cyclopropylmethyl)pyrazole-4-sulfonamide |
| 124 | | | 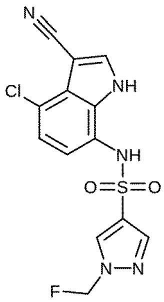 | N-(4-chloro-3-cyano-1H-indol-7-yl)-1-(fluoromethyl) pyrazole-4-sulfonamide |
| 125 | | | 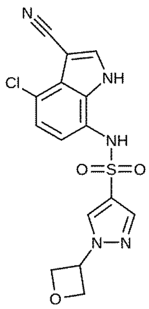 | N-(4-chloro-3-cyano-1H-indol-7-yl)-1-(oxetan-3-yl) pyrazole-4-sulfonamide |
| 126 | | | 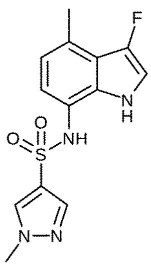 | N-(3-fluoro-4-methyl-1H-indol-7-yl)-1-methyl-pyrazole-4-sulfonamide |
| 127 | | | 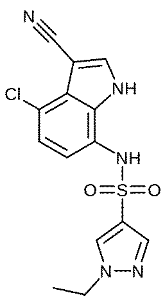 | N-(4-chloro-3-cyano-1H-indol-7-yl)-1-ethyl-pyrazole-4-sulfonamide |

(continued)

| Compound No. | M+H | M-H | Structure | Name |
|---|---|---|---|---|
| 128 | | | 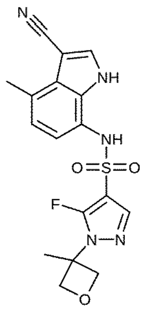 | N-(3-cyano-4-methyl-1H-indol-7-yl)-5-fluoro-1-(3-methyloxetan-3-yl)pyrazole-4-sulfonamide |
| 129 | | | 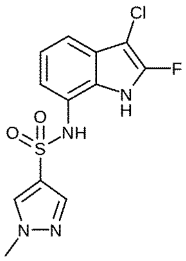 | N-(3-chloro-2-fluoro-1H-indol-7-yl)-1-methyl-pyrazole-4-sulfonamide |
| 130 | | | 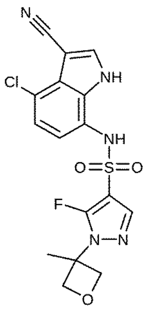 | N-(4-chloro-3-cyano-1H-indol-7-yl)-5-fluoro-1-(3-methyloxetan-3-yl)pyrazole-4-sulfonamide |
| 131 | 341 | | 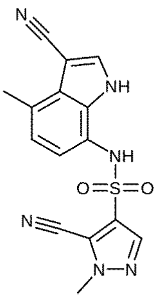 | 5-cyano-N-(3-cyano-4-methyl-1H-indol-7-yl)-1-methyl-pyrazole-4-sulfonamide |
| 132 | 361 | | 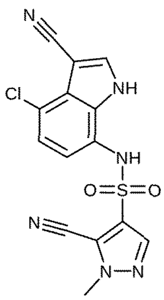 | N-(4-chloro-3-cyano-1H-indol-7-yl)-5-cyano-1-methyl-pyrazole-4-sulfonamide |

(continued)

| Compound No. | M+H | M-H | Structure | Name |
|---|---|---|---|---|
| 133 | | | 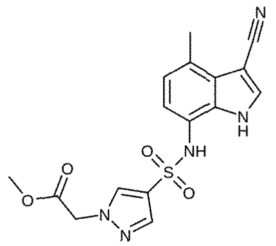 | methyl 2-[4-[(3-cyano-4-methyl-1H-indol-7-yl)sulfamoyl] pyrazol-1-yl]acetate |
| 134 | | | 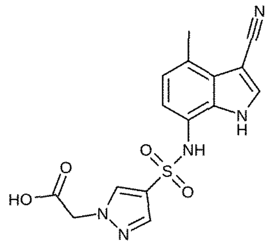 | 2-[4-[(3-cyano-4-methyl-1H-indol-7-yl)sulfamoyl] pyrazol-1-yl]acetic acid |
| 135 | | | 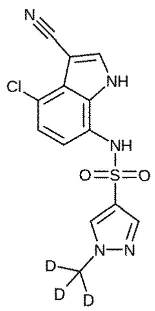 | N-(4-chloro-3-cyano-1H-indol-7-yl)-1-(trideuteriomethyl)pyrazole-4-sulfonamide |
| 136 | | | 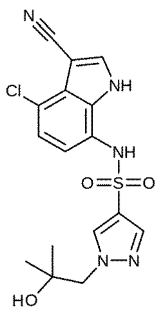 | N-(4-chloro-3-cyano-1H-indol-7-yl)-1-(2-hydroxy-2-methyl-propyl)pyrazole-4-sulfonamide |
| 137 | | | 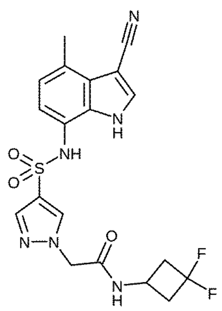 | 2-[4-[(3-cyano-4-methyl-1H-indol-7-yl)sulfamoyl] pyrazol-1-yl]-N-(3,3-difluorocyclobutyl)acetamide |

(continued)

| Compound No. | M+H | M-H | Structure | Name |
|---|---|---|---|---|
| 138 | | | 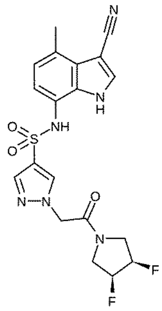 | N-(3-cyano-4-methyl-1H-indol-7-yl)-1-[2-[(3R,4S)-3,4-difluoropyrrolidin-1-yl]-2-oxoethyl]pyrazole-4-sulfonamide |
| 139 | | | 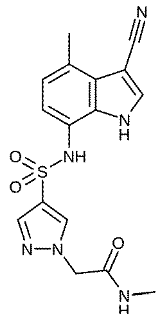 | 2-[4-[(3-cyano-4-methyl-1H-indol-7-yl)sulfamoyl] pyrazol-1-yl]-N-methylacetamide |
| 140 | | | 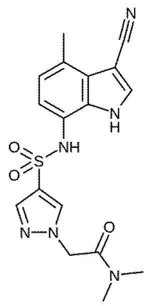 | 2-[4-[(3-cyano-4-methyl-1H-indol-7-yl)sulfamoyl] pyrazol-1-yl]-N,N-dimethylacetamide |
| 141 | | | 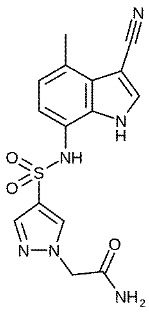 | 2-[4-[(3-cyano-4-methyl-1H-indol-7-yl)sulfamoyl] pyrazol-1-yl]acetamide |
| 142 | | | 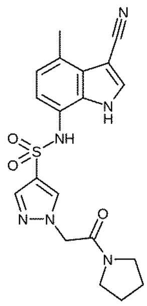 | N-(3-cyano-4-methyl-1H-indol-7-yl)-1-(2-oxo-2-pyrrolidin-1-yl-ethyl)pyrazole-4-sulfonamide |

(continued)

| Compoun d No. | M+ H | M- H | Structure | Name |
|---|---|---|---|---|
| 143 | | | 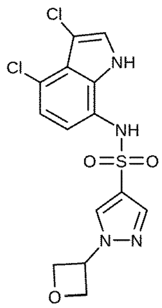 | N-(3,4-dichloro-1H-indol-7-yl)-1-(oxetan-3-yl)pyrazole-4-sulfonamide |
| 144 | | | 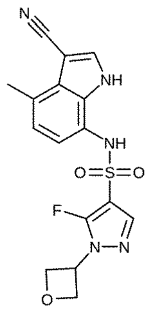 | N-(3-cyano-4-methyl-1H-indol-7-yl)-5-fluoro-1-(oxetan-3-yl)pyrazole-4-sulfonamide |
| 145 | | 37 9 | 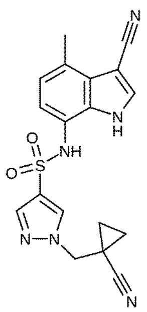 | 1-[(1-cyanocyclopropyl)methyl]-N-(3-cyano-4-methyl-1H-indol-7-yl)pyrazole-4-sulfonamide |
| 146 | 384 | | 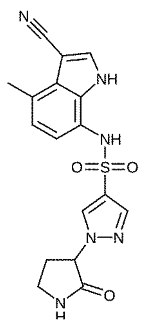 | N-(3-cyano-4-methyl-1H-indol-7-yl)-1-(2-oxopyrrolidin-3-yl)pyrazole-4-sulfonamide |
| 147 | 405 | | 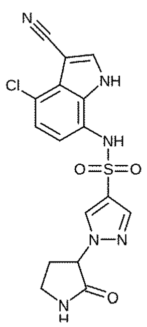 | N-(4-chloro-3-cyano-1H-indol-7-yl)-1-(2-oxopyrrolidin-3-yl)pyrazole-4-sulfonamide |

(continued)

| Compound No. | M+H | M-H | Structure | Name |
|---|---|---|---|---|
| 148 | 334 | | 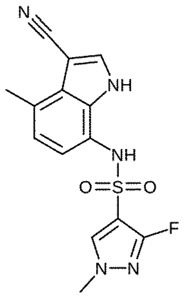 | N-(3-cyano-4-methyl-1H-indol-7-yl)-3-fluoro-1-methyl-pyrazole-4-sulfonamide |
| 149 | 366 | | 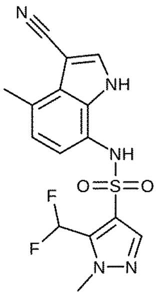 | N-(3-cyano-4-methyl-1H-indol-7-yl)-5-(difluoromethyl)-1-methyl-pyrazole-4-sulfonamide |
| 150 | 386 | | 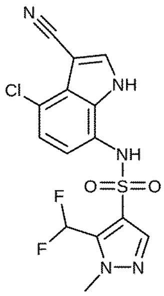 | N-(4-chloro-3-cyano-1H-indol-7-yl)-5-(difluoromethyl)-1-methyl-pyrazole-4-sulfonamide |
| 151 | | | 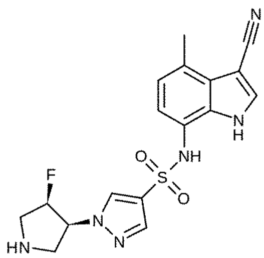 | N-(3-cyano-4-methyl-1H-indol-7-yl)-1-[(3S,4R)-4-fluoropyrrolidin-3-yl]pyrazole-4-sulfonamide |
| 152 | | | 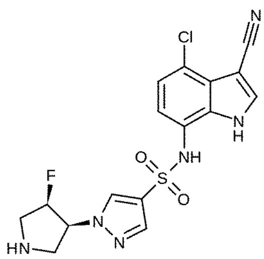 | N-(4-chloro-3-cyano-1H-indol-7-yl)-1-[(3S,4R)-4-fluoropyrrolidin-3-yl]pyrazole-4-sulfonamide |

(continued)

| Compound No. | M+H | M-H | Structure | Name |
|---|---|---|---|---|
| 153 | | | 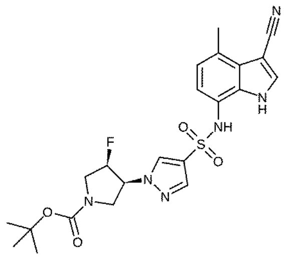 | tert-butyl (3S,4R)-3-[4-[(3-cyano-4-methyl-1H-indol-7-yl)sulfamoyl]pyrazol-1-yl]-4-fluoro-pyrrolidine-1-carboxylate |
| 154 | | 405 | 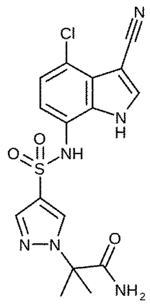 | 2-[4-[(4-chloro-3-cyano-1H-indol-7-yl)sulfamoyl] pyrazol-1-yl]-2-methyl-propanamide |
| 155 | | | 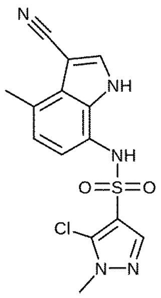 | 5-chloro-N-(3-cyano-4-methyl-1H-indol-7-yl)-1-methyl-pyrazole-4-sulfonamide |
| 156 | | | 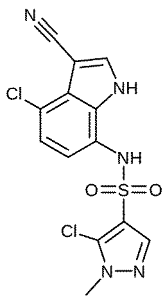 | 5-chloro-N-(4-chloro-3-cyano-1H-indol-7-yl)-1-methyl-pyrazole-4-sulfonamide |
| 157 | | | 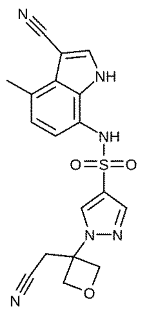 | N-(3-cyano-4-methyl-1H-indol-7-yl)-1-[3-(cyanomethyl) oxetan-3-yl]pyrazole-4-sulfonamide |

(continued)

| Compound No. | M+H | M-H | Structure | Name |
|---|---|---|---|---|
| 158 | | | 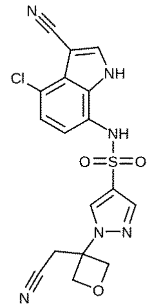 | N-(4-chloro-3-cyano-1H-indol-7-yl)-1-[3- (cyanomethyl) oxetan-3-yl]pyrazole-4-sulfonamide |
| 159 | | | 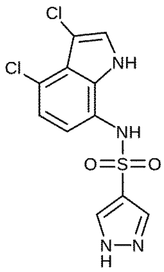 | N-(3,4-dichloro-1H-indol-7-yl)-1H-pyrazole-4-sulfonamide |
| 160 | | | 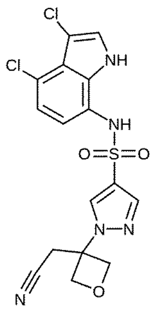 | 1-[3-(cyanomethyl)oxetan-3-yl]-N-(3,4-dichloro-1H-indol-7-yl)pyrazole-4-sulfonamide |
| 161 | | | 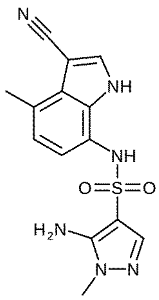 | 5-amino-N-(3-cyano-4-methyl-1H-indol-7-yl)-1-methyl-pyrazole-4-sulfonamide |
| 162 | | | 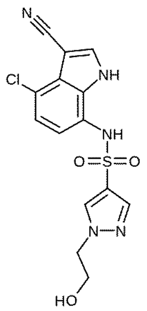 | N-(4-chloro-3-cyano-1H-indol-7-yl)-1-(2-hydroxyethyl) pyrazole-4-sulfonamide |

(continued)

| Compound No. | M+H | M-H | Structure | Name |
|---|---|---|---|---|
| 163 | | | 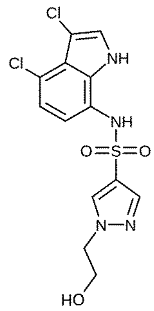 | N-(3,4-dichloro-1H-indol-7-yl)-1-(2-hydroxyethyl) pyrazole-4-sulfonamide |
| 164 | | | 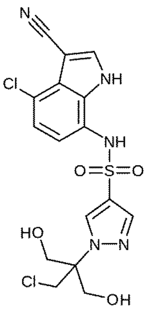 | N-(4-chloro-3-cyano-1H-indol-7-yl)-1-[1-(chloromethyl)-2-hydroxy-1-(hydroxymethyl)ethyl]pyrazole-4-sulfonamide |
| 165 | | | 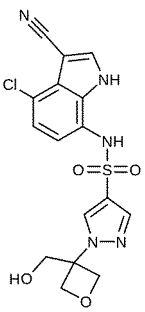 | N-(4-chloro-3-cyano-1H-indol-7-yl)-1-[3-(hydroxymethyl)oxetan-3-yl]pyrazole-4-sulfonamide |
| 166 | | | 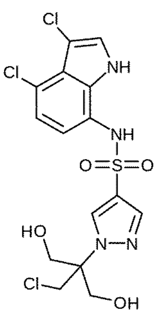 | 1-[1-(chloromethyl)-2-hydroxy-1-(hydroxymethyl) ethyl]-N-(3,4-dichloro-1H-indol-7-yl)pyrazole-4-sulfonamide |
| 167 | | | 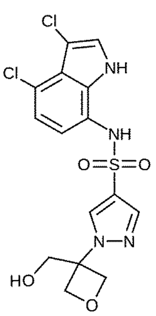 | N-(3,4-dichloro-1H-indol-7-yl)-1-[3-(hydroxymethyl) oxetan-3-yl]pyrazole-4-sulfonamide |

(continued)

| Compound No. | M+H | M-H | Structure | Name |
|---|---|---|---|---|
| 168 | | | 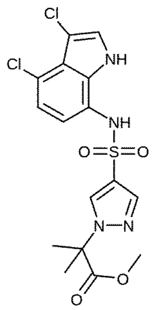 | methyl 2-[4-[(3,4-dichloro-1H-indol-7-yl)sulfamoyl] pyrazol-1-yl]-2-methylpropanoate |
| 169 | | | 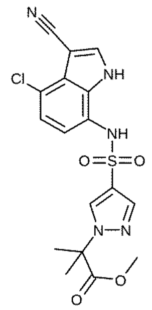 | methyl 2-[4-[(4-chloro-3-cyano-1H-indol-7-yl)sulfamoyl] pyrazol-1-yl]-2-methylpropanoate |
| 170 | | | 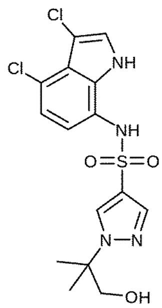 | N-(3,4-dichloro-1H-indol-7-yl)-1-(2-hydroxy-1,1-dimethyl-ethyl)pyrazole-4-sulfonamide |
| 171 | | | 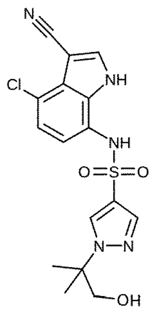 | N-(4-chloro-3-cyano-1H-indol-7-yl)-1-(2-hydroxy-1,1-dimethyl-ethyl)pyrazole-4-sulfonamide |
| 172 | | | 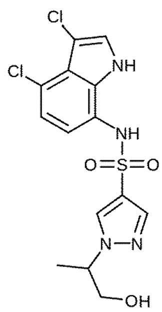 | N-(3,4-dichloro-1H-indol-7-yl)-1-(2-hydroxy-1-methyl-ethyl)pyrazole-4-sulfonamide |

(continued)

| Compound No. | M+H | M-H | Structure | Name |
|---|---|---|---|---|
| 173 | | | 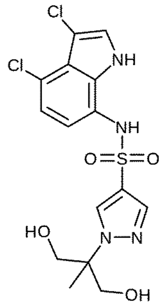 | N-(3,4-dichloro-1H-indol-7-yl)-1-[2-hydroxy-1-(hydroxymethyl)-1-methylethyl]pyrazole-4-sulfonamide |
| 174 | | 390 | 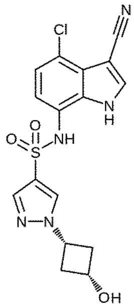 | N-(4-chloro-3-cyano-1H-indol-7-yl)-1-(3-hydroxycyclobutyl)pyrazole-4-sulfonamide |
| 175 | | 390 | 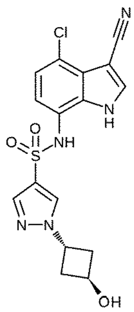 | N-(4-chloro-3-cyano-1H-indol-7-yl)-1-(3-hydroxycyclobutyl)pyrazole-4-sulfonamide |
| 176 | | | 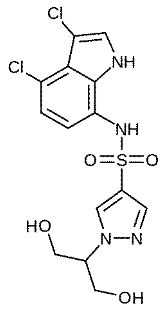 | N-(3,4-dichloro-1H-indol-7-yl)-1-[2-hydroxy-1-(hydroxymethyl)ethyl]pyrazole-4-sulfonamide |
| 177 | | | 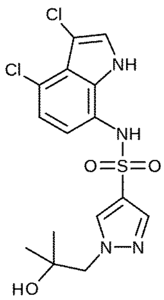 | N-(3,4-dichloro-1H-indol-7-yl)-1-(2-hydroxy-2-methyl-propyl)pyrazole-4-sulfonamide |

(continued)

| Compound No. | M+H | M-H | Structure | Name |
|---|---|---|---|---|
| 178 | | | 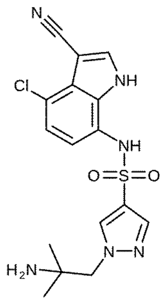 | 1-(2-amino-2-methyl-propyl)-N-(4-chloro-3-cyano-1H-indol-7-yl)pyrazole-4-sulfonamide |
| 179 | | | 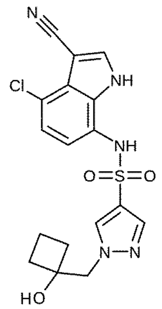 | N-(4-chloro-3-cyano-1H-indol-7-yl)-1-[(1-hydroxycyclobutyl)methyl]pyrazole-4-sulfonamide |
| 180 | | | 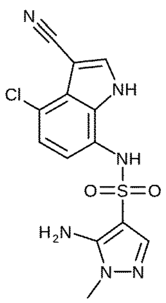 | 5-amino-N-(4-chloro-3-cyano-1H-indol-7-yl)-1-methyl-pyrazole-4-sulfonamide |
| 181 | | | 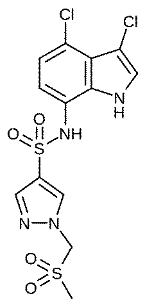 | N-(3,4-dichloro-1H-indol-7-yl)-1-(methylsulfonylmethyl) pyrazole-4-sulfonamide |
| 182 | | | 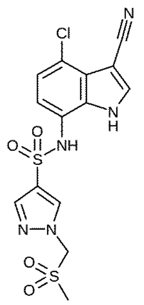 | N-(4-chloro-3-cyano-1H-indol-7-yl)-1-(methylsulfonylmethyl)pyrazole-4-sulfonamide |

(continued)

| Compound No. | M+H | M-H | Structure | Name |
|---|---|---|---|---|
| 183 | | 399 | 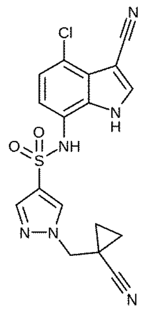 | N-(4-chloro-3-cyano-1H-indol-7-yl)-1-[(1-cyanocyclopropyl)methyl]pyrazole-4-sulfonamide |
| 184 | | | 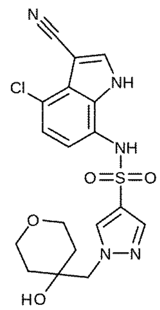 | N-(4-chloro-3-cyano-1H-indol-7-yl)-1-[(4-hydroxytetrahydropyran-4-yl)methyl]pyrazole-4-sulfonamide |
| 188 | 412 | | 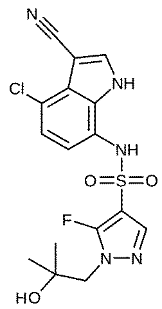 | N-(4-chloro-3-cyano-1H-indol-7-yl)-5-fluoro-1-(2-hydroxy-2-methyl-propyl)pyrazole-4-sulfonamide |
| 189 | | 404 | 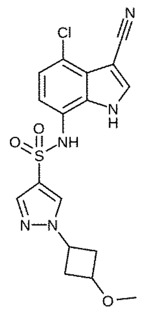 | N-(4-chloro-3-cyano-1H-indol-7-yl)-1-(3-methoxycyclobutyl)pyrazole-4-sulfonamide |
| 190 | | 379 | 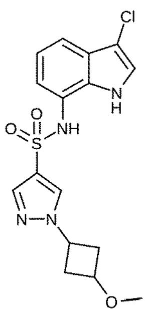 | N-(3-chloro-1H-indol-7-yl)-1-(3-methoxycyclobutyl) pyrazole-4-sulfonamide |

(continued)

| Compound No. | M+H | M-H | Structure | Name |
|---|---|---|---|---|
| 191 | 406 | | 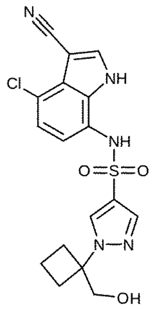 | N-(4-chloro-3-cyano-1H-indol-7-yl)-1-[1-(hydroxymethyl)cyclobutyl]pyrazole-4-sulfonamide |
| 192 | 378 | | 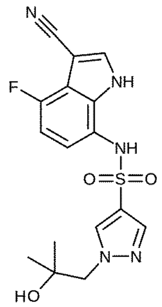 | N-(3-cyano-4-fluoro-1H-indol-7-yl)-1-(2-hydroxy-2-methyl-propyl)pyrazole-4-sulfonamide |
| 193 | 372 | | 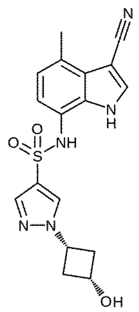 | N-(3-cyano-4-methyl-1H-indol-7-yl)-1-(3-hydroxycyclobutyl)pyrazole-4-sulfonamide |
| 194 | 367 | | 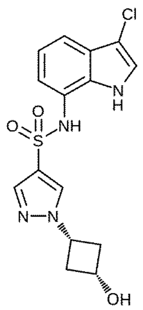 | N-(3-chloro-1H-indol-7-yl)-1-(3-hydroxycyclobutyl) pyrazole-4-sulfonamide |
| 195 | 406 | | 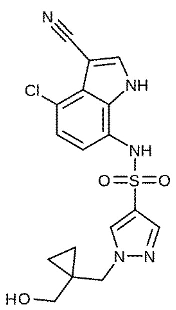 | N-(4-chloro-3-cyano-1H-indol-7-yl)-1-[[1-(hydroxymethyl)cyclopropyl]methyl]pyrazole -4-sulfonamide |

(continued)

| Compound No. | M+H | M-H | Structure | Name |
|---|---|---|---|---|
| 196 | 380 | | 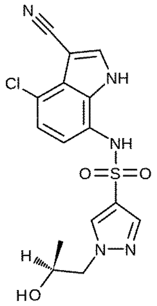 | N-(4-chloro-3-cyano-1H-indol-7-yl)-1-[(2S)-2-hydroxypropyl]pyrazole-4-sulfonamide |
| 197 | 380 | | 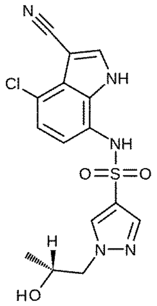 | N-(4-chloro-3-cyano-1H-indol-7-yl)-1-[(2R)-2-hydroxypropyl]pyrazole-4-sulfonamide |
| 198 | 374 | | 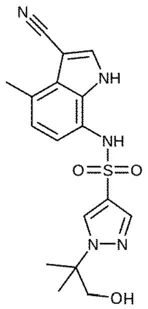 | N-(3-cyano-4-methyl-1H-indol-7-yl)-1-(2-hydroxy-1,1-dimethyl-ethyl)pyrazole-4-sulfonamide |
| 204 | 412 | | 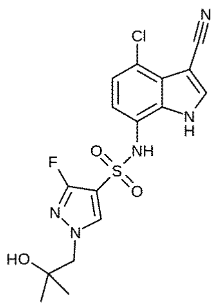 | N-(4-chloro-3-cyano-1H-indol-7-yl)-3-fluoro-1-(2-hydroxy-2-methyl-propyl)pyrazole-4-sulfonamide |
| 205 | 392 | | 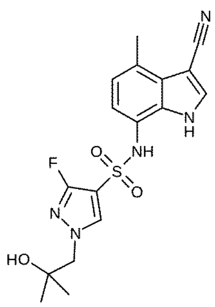 | N-(3-cyano-4-methyl-1H-indol-7-yl)-3-fluoro-1-(2-hydroxy-2-methylpropyl)pyrazole-4-sulfonamide |

(continued)

| Compound No. | M+H | M-H | Structure | Name |
|---|---|---|---|---|
| 206 | 396 | | 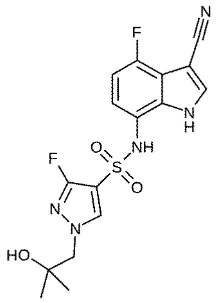 | N-(3-cyano-4-fluoro-1H-indol-7-yl)-3-fluoro-1-(2-hydroxy-2-methyl-propyl)pyrazole-4-sulfonamide |
| 207 | 360 | | 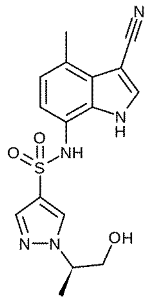 | N-(3-cyano-4-methyl-1H-indol-7-yl)-1-[(1R)-2-hydroxy-1-methyl-ethyl]pyrazole-4-sulfonamide |
| 208 | 380 | | 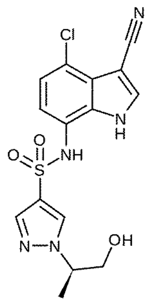 | N-(4-chloro-3-cyano-1H-indol-7-yl)-1-[(1R)-2-hydroxy-1-methyl-ethyl]pyrazole-4-sulfonamide |
| 209 | 380 | | 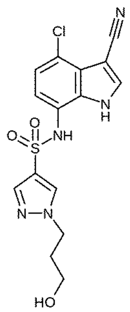 | N-(4-chloro-3-cyano-1H-indol-7-yl)-1-(3-hydroxypropyl) pyrazole-4-sulfonamide |
| 210 | 360 | | 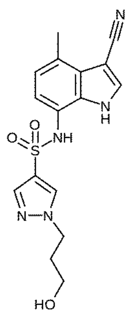 | N-(3-cyano-4-methyl-1H-indol-7-yl)-1-(3-hydroxypropyl)pyrazole-4-sulfonamide |

(continued)

| Compound No. | M+H | M-H | Structure | Name |
|---|---|---|---|---|
| 211 | 364 | | 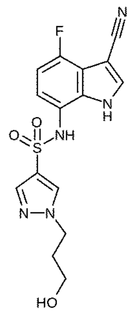 | N-(3-cyano-4-fluoro-1H-indol-7-yl)-1-(3-hydroxypropyl) pyrazole-4-sulfonamide |
| 212 | 364 | | 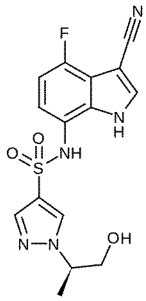 | N-(3-cyano-4-fluoro-1H-indol-7-yl)-1-[(1R)-2-hydroxy-1-methyl-ethyl]pyrazole-4-sulfonamide |
| 213 | | 391 | 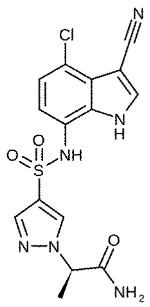 | (2R)-2-[4-[(4-chloro-3-cyano-1H-indol-7-yl)sulfamoyl] pyrazol-1-yl]propanamide |
| 214 | 306 | | 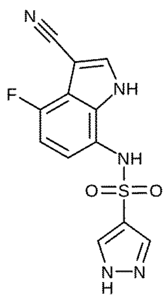 | N-(3-cyano-4-fluoro-1H-indol-7-yl)-1H-pyrazole-4-sulfonamide |
| 215 | 356 | | 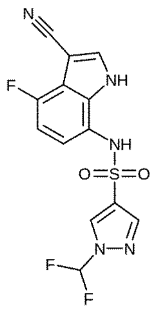 | N-(3-cyano-4-fluoro-1H-indol-7-yl)-1-(difluoromethyl) pyrazole-4-sulfonamide |

(continued)

| Compound No. | M+H | M-H | Structure | Name |
|---|---|---|---|---|
| 216 | 388 | | 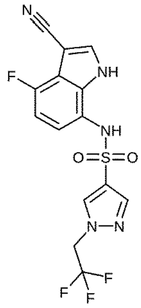 | N-(3-cyano-4-fluoro-1H-indol-7-yl)-1-(2,2,2-trifluoroethyl)pyrazole-4-sulfonamide |
| 217 | 334 | | 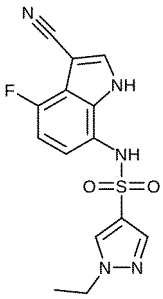 | N-(3-cyano-4-fluoro-1H-indol-7-yl)-1-ethyl-pyrazole-4-sulfonamide |
| 218 | 368 | | 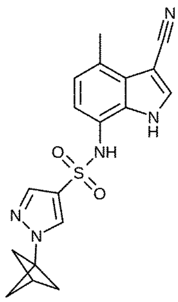 | 1-(3-bicyclo[1.1.1]pentanyl)-N-(3-cyano-4-methyl-1H-indol-7-yl)pyrazole-4-sulfonamide |
| 219 | 372 | | 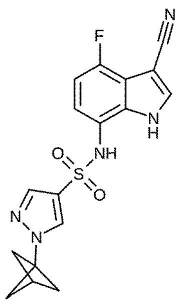 | 1-(3-bicyclo[1.1.1]pentanyl)-N-(3-cyano-4-fluoro-1H-indol-7-yl)pyrazole-4-sulfonamide |
| 220 | 408 | | 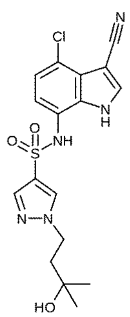 | N-(4-chloro-3-cyano-1H-indol-7-yl)-1-(3-hydroxy-3-methyl-butyl)pyrazole-4-sulfonamide |

(continued)

| Compound No. | M+H | M-H | Structure | Name |
|---|---|---|---|---|
| 221 | 388 | | 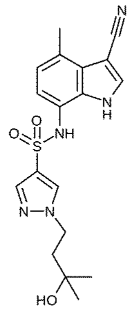 | N-(3-cyano-4-methyl-1H-indol-7-yl)-1-(3-hydroxy-3-methyl-butyl)pyrazole-4-sulfonamide |
| 222 | 392 | | 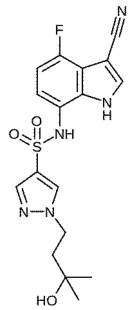 | N-(3-cyano-4-fluoro-1H-indol-7-yl)-1-(3-hydroxy-3-methyl-butyl)pyrazole-4-sulfonamide |
| 223 | 388 | | 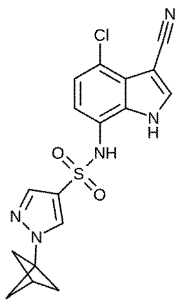 | 1-(3-bicyclo[1.1.1]pentanyl)-N-(4-chloro-3-cyano-1H-indol-7-yl)pyrazole-4-sulfonamide |
| 224 | 378 | | 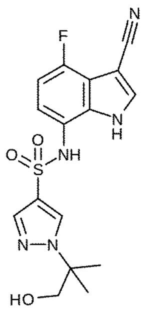 | N-(3-cyano-4-fluoro-1H-indol-7-yl)-1-(2-hydroxy-1,1-dimethyl-ethyl)pyrazole-4-sulfonamide |
| 225 | 366 | | 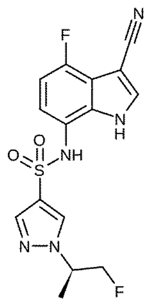 | N-(3-cyano-4-fluoro-1H-indol-7-yl)-1-[(1R)-2-fluoro-1-methyl-ethyl]pyrazole-4-sulfonamide |

(continued)

| Compound No. | M+H | M-H | Structure | Name |
|---|---|---|---|---|
| 226 | 416 | | 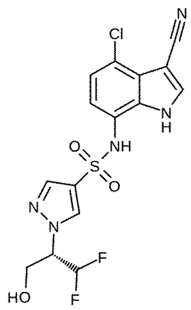 | N-(4-chloro-3-cyano-1H-indol-7-yl)-1-[(1S)-2,2-difluoro-1-(hydroxymethyl)ethyl]pyrazole-4-sulfonamide |
| 227 | 396 | | 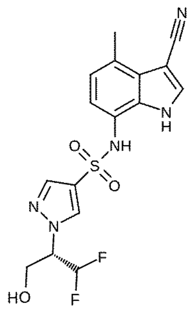 | N-(3-cyano-4-methyl-1H-indol-7-yl)-1-[(1S)-2,2-difluoro-1-(hydroxymethyl)ethyl]pyrazole-4-sulfonamide |
| 228 | 400 | | 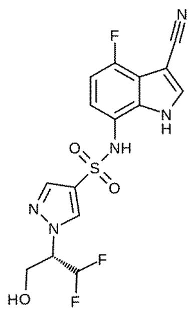 | N-(3-cyano-4-fluoro-1H-indol-7-yl)-1-[(1S)-2,2-difluoro-1-(hydroxymethyl)ethyl]pyrazole-4-sulfonamide |
| 229 | 388 | | 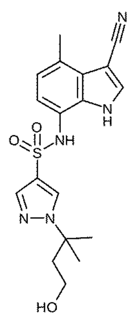 | N-(3-cyano-4-methyl-1H-indol-7-yl)-1-(3-hydroxy-1,1-dimethyl-propyl)pyrazole-4-sulfonamide |
| 230 | 408 | | 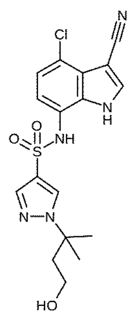 | N-(4-chloro-3-cyano-1H-indol-7-yl)-1-(3-hydroxy-1,1-dimethyl-propyl)pyrazole-4-sulfonamide |

(continued)

| Compound No. | M+H | M-H | Structure | Name |
|---|---|---|---|---|
| 231 | 416 | | 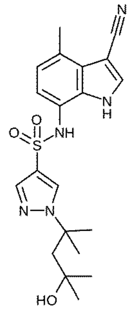 | N-(3-cyano-4-methyl-1H-indol-7-yl)-1-(3-hydroxy-1,1,3-trimethyl-butyl)pyrazole-4-sulfonamide |
| 232 | 394 | | 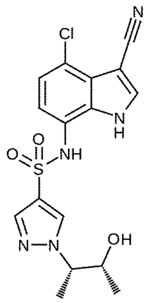 | N-(4-chloro-3-cyano-1H-indol-7-yl)-1-[(1S,2R)-2-hydroxy-1-methylpropyl]pyrazole-4-sulfonamide |
| 233 | 378 | | 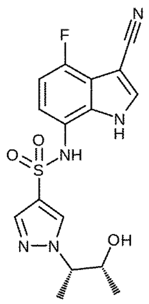 | N-(3-cyano-4-fluoro-1H-indol-7-yl)-1-[(1S,2R)-2-hydroxy-1-methylpropyl]pyrazole-4-sulfonamide |
| 234 | 374 | | 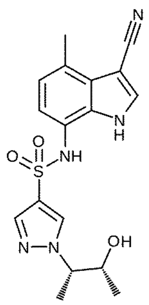 | N-(3-cyano-4-methyl-1H-indol-7-yl)-1-[(1S,2R)-2-hydroxy-1-methylpropyl]pyrazole-4-sulfonamide |
| 235 | 360 | | 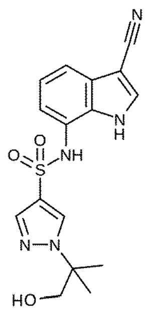 | N-(3-cyano-1H-indol-7-yl)-1-(2-hydroxy-1,1-dimethyl-ethyl)pyrazole-4-sulfonamide |

(continued)

| Compound No. | M+H | M-H | Structure | Name |
|---|---|---|---|---|
| 236 | 374 | | 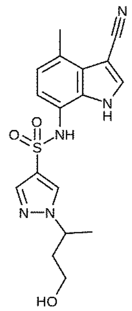 | N-(3-cyano-4-methyl-1H-indol-7-yl)-1-(3-hydroxy-1-methyl-propyl)pyrazole-4-sulfonamide |
| 237 | 394 | | 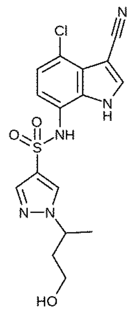 | N-(4-chloro-3-cyano-1H-indol-7-yl)-1-(3-hydroxy-1-methyl-propyl)pyrazole-4-sulfonamide |
| 238 | 398 | | 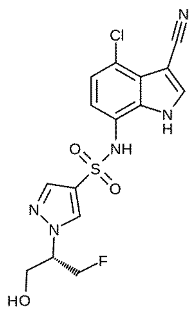 | N-(4-chloro-3-cyano-1H-indol-7-yl)-1-[(1S)-1-(fluoromethyl)-2-hydroxy-ethyl]pyrazole-4-sulfonamide |
| 239 | | 358 | 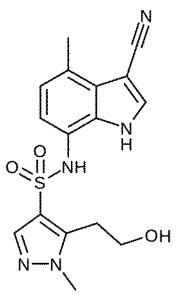 | N-(3-cyano-4-methyl-1H-indol-7-yl)-5-(2-hydroxyethyl)-1-methyl-pyrazole-4-sulfonamide |
| 240 | | 390 | 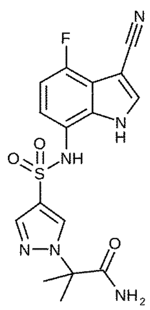 | 2-[4-[(3-cyano-4-fluoro-1H-indol-7-yl)sulfamoyl]pyrazol-1-yl]-2-methyl-propanamide |

(continued)

| Compound No. | M+H | M-H | Structure | Name |
|---|---|---|---|---|
| 241 | | 385 | 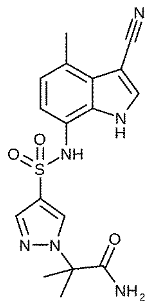 | 2-[4-[(3-cyano-4-methyl-1H-indol-7-yl)sulfamoyl] pyrazol-1-yl]-2-methyl-propanamide |
| 242 | 402 | | 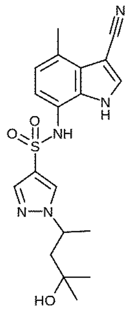 | N-(3-cyano-4-methyl-1H-indol-7-yl)-1-(3-hydroxy-1,3-dimethyl-butyl)pyrazole-4-sulfonamide |
| 243 | 422 | | 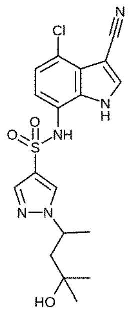 | N-(4-chloro-3-cyano-1H-indol-7-yl)-1-(3-hydroxy-1,3-dimethyl-butyl)pyrazole-4-sulfonamide |
| 244 | 408 | | 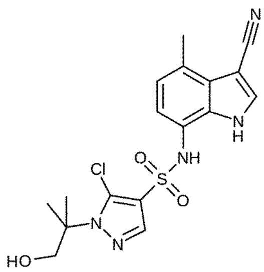 | 5-chloro-N-(3-cyano-4-methyl-1H-indol-7-yl)-1-(2-hydroxy-1,1-dimethylethyl)pyrazole-4-sulfonamide |
| 245 | 414 | | 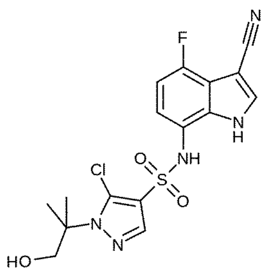 | 5-chloro-N-(3-cyano-4-fluoro-1H-indol-7-yl)-1-(2-hydroxy-1,1-dimethyl-ethyl)pyrazole-4-sulfonamide |

(continued)

| Compound No. | M+H | M-H | Structure | Name |
|---|---|---|---|---|
| 246 | 378 | | 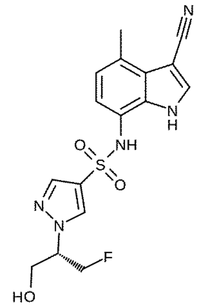 | N-(3-cyano-4-methyl-1H-indol-7-yl)-1-[(1S)-1-(fluoromethyl)-2-hydroxy-ethyl]pyrazole-4-sulfonamide |
| 247 | 382 | | 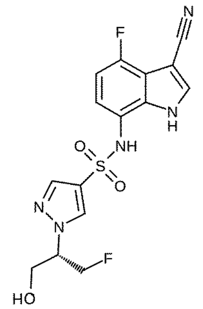 | N-(3-cyano-4-fluoro-1H-indol-7-yl)-1-[(1S)-1-(fluoromethyl)-2-hydroxy-ethyl]pyrazole-4-sulfonamide |
| 248 | 433 | | 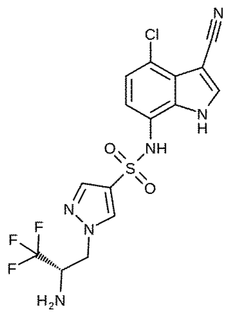 | 1-[(2S)-2-amino-3,3,3-trifluoro-propyl]-N-(4-chloro-3-cyano-1H-indol-7-yl)pyrazole-4-sulfonamide |
| 249 | 413 | | 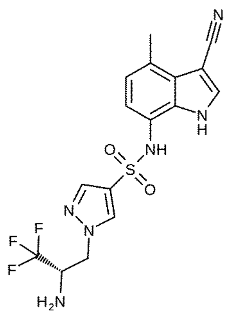 | 1-[(2S)-2-amino-3,3,3-trifluoro-propyl]-N-(3-cyano-4-methyl-1H-indol-7-yl)pyrazole-4-sulfonamide |
| 250 | | 426 | 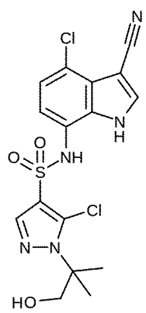 | 5-chloro-N-(4-chloro-3-cyano-1H-indol-7-yl)-1-(2-hydroxy-1,1-dimethylethyl)pyrazole-4-sulfonamide |

(continued)

| Compound No. | M+H | M-H | Structure | Name |
|---|---|---|---|---|
| 251 | 394 | | 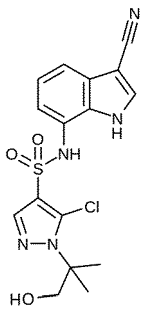 | 5-chloro-N-(3-cyano-1H-indol-7-yl)-1-(2-hydroxy-1,1-dimethyl-ethyl)pyrazole-4-sulfonamide |
| 252 | | | 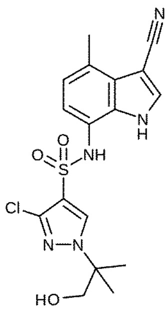 | 3-chloro-N-(3-cyano-4-methyl-1H-indol-7-yl)-1-(2-hydroxy-1,1-dimethylethyl)pyrazole-4-sulfonamide |
| 253 | 392 | | 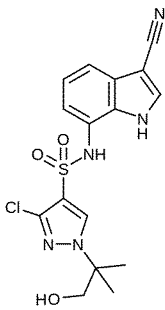 | 3-chloro-N-(3-cyano-1H-indol-7-yl)-1-(2-hydroxy-1,1-dimethyl-ethyl)pyrazole-4-sulfonamide |
| 254 | 509 | | 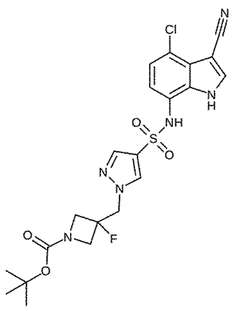 | tert-butyl3-[[4-[(4-chloro-3-cyano-1H-indol-7-yl) sulfamoyl]pyrazol-1-yl]methyl]-3-fluoro-azetidine-1-carboxylate |
| 255 | 489 | | 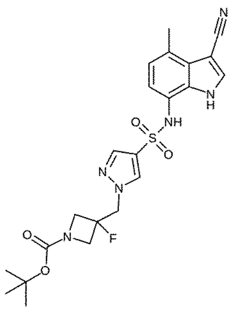 | tert-butyl3-[[4-[(3-cyano-4-methyl-1H-indol-7-yl) sulfamoyl]pyrazol-1-yl]methyl]-3-fluoro-azetidine-1-carboxylate |

(continued)

| Compound No. | M+H | M-H | Structure | Name |
|---|---|---|---|---|
| 256 | 409 | | 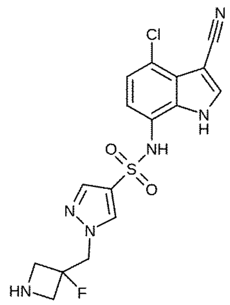 | N-(4-chloro-3-cyano-1H-indol-7-yl)-1-[(3-fluoroazetidin-3-yl)methyl]pyrazole-4-sulfonamide |
| 257 | 389 | | 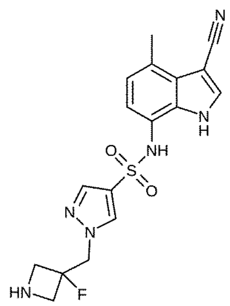 | N-(3-cyano-4-methyl-1H-indol-7-yl)-1-[(3-fluoroazetidin-3-yl)methyl]pyrazole-4-sulfonamide |
| 258 | 388 | | 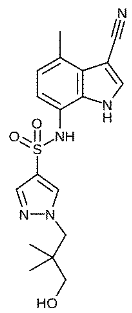 | N-(3-cyano-4-methyl-1H-indol-7-yl)-1-(3-hydroxy-2,2-dimethyl-propyl)pyrazole-4-sulfonamide |
| 259 | 408 | | 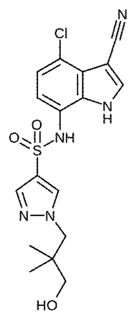 | N-(4-chloro-3-cyano-1H-indol-7-yl)-1-(3-hydroxy-2,2-dimethyl-propyl)pyrazole-4-sulfonamide |
| 260 | 428 | | 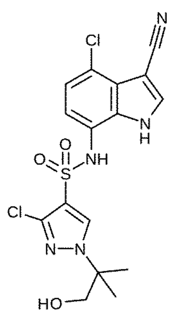 | 3-chloro-N-(4-chloro-3-cyano-1H-indol-7-yl)-1-(2-hydroxy-1,1-dimethylethyl)pyrazole-4-sulfonamide |

(continued)

| Compound No. | M+H | M-H | Structure | Name |
|---|---|---|---|---|
| 261 | | 410 | 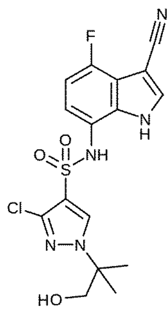 | 3-chloro-N-(3-cyano-4-fluoro-1H-indol-7-yl)-1-(2-hydroxy-1,1-dimethyl-ethyl)pyrazole-4-sulfonamide |
| 262 | 410 | | 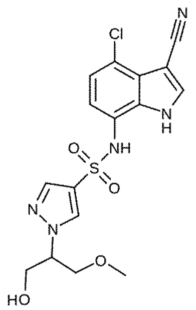 | N-(4-chloro-3-cyano-1H-indol-7-yl)-1-[1-(hydroxymethyl)-2-methoxy-ethyl]pyrazole-4-sulfonamide |
| 263 | 390 | | 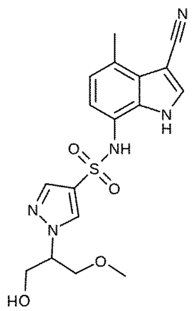 | N-(3-cyano-4-methyl-1H-indol-7-yl)-1-[1-(hydroxymethyl)-2-methoxy-ethyl]pyrazole-4-sulfonamide |
| 264 | 374 | | 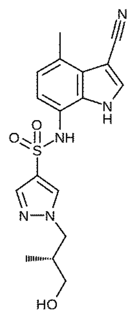 | N-(3-cyano-4-methyl-1H-indol-7-yl)-1-[(2R)-3-hydroxy-2-methyl-propyl]pyrazole-4-sulfonamide |
| 265 | 394 | | 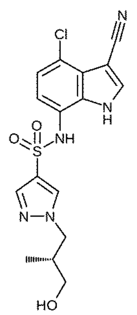 | N-(4-chloro-3-cyano-1H-indol-7-yl)-1-[(2R)-3-hydroxy-2-methyl-propyl]pyrazole-4-sulfonamide |

(continued)

| Compound No. | M+H | M-H | Structure | Name |
|---|---|---|---|---|
| 266 | | 378 | 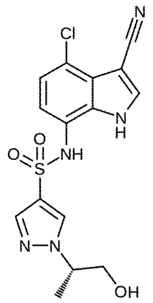 | N-(4-chloro-3-cyano-1H-indol-7-yl)-1-[(1S)-2-hydroxy-1-methyl-ethyl]pyrazole-4-sulfonamide |
| 267 | 364 | | 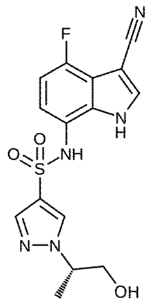 | N-(3-cyano-4-fluoro-1H-indol-7-yl)-1-[(1S)-2-hydroxy-1-methyl-ethyl]pyrazole-4-sulfonamide |
| 268 | 372 | | 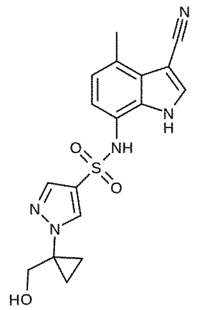 | N-(3-cyano-4-methyl-1H-indol-7-yl)-1-[1-(hydroxymethyl)cyclopropyl]pyrazole-4-sulfonamide |
| 269 | 392 | | 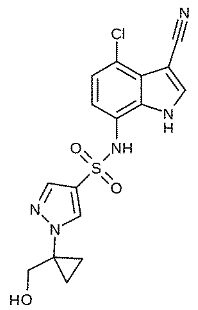 | N-(4-chloro-3-cyano-1H-indol-7-yl)-1-[1-(hydroxymethyl)cyclopropyl]pyrazole-4-sulfonamide |
| 270 | 374 | | 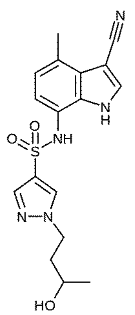 | N-(3-cyano-4-methyl-1H-indol-7-yl)-1-(3-hydroxybutyl) pyrazole-4-sulfonamide |

(continued)

| Compound No. | M+H | M-H | Structure | Name |
|---|---|---|---|---|
| 271 | 345 | | 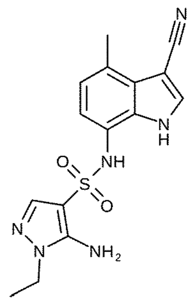 | 5-amino-N-(3-cyano-4-methyl-1H-indol-7-yl)-1-ethyl-pyrazole-4-sulfonamide |
| 272 | 349 | | 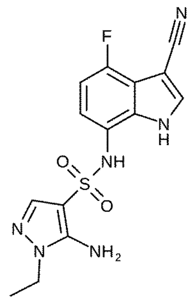 | 5-amino-N-(3-cyano-4-fluoro-1H-indol-7-yl)-1-ethyl-pyrazole-4-sulfonamide |
| 273 | 365 | | 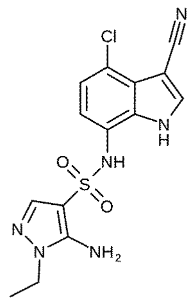 | 5-amino-N-(4-chloro-3-cyano-1H-indol-7-yl)-1-ethyl-pyrazole-4-sulfonamide |
| 274 | | 372 | 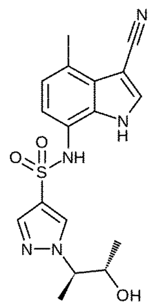 | N-(3-cyano-4-methyl-1H-indol-7-yl)-1-[(1R,2S)-2-hydroxy-1-methylpropyl]pyrazole-4-sulfonamide |
| 275 | | 392 | 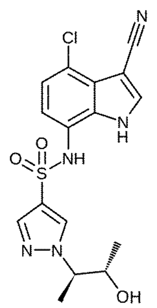 | N-(4-chloro-3-cyano-1H-indol-7-yl)-1-[(1R,2S)-2-hydroxy-1-methylpropyl]pyrazole-4-sulfonamide |

(continued)

| Compound No. | M+H | M-H | Structure | Name |
|---|---|---|---|---|
| 276 | 406 | | 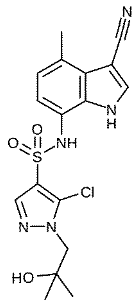 | 5-chloro-N-(3-cyano-4-methyl-1H-indol-7-yl)-1-(2-hydroxy-2-methyl-propyl)pyrazole-4-sulfonamide |
| 277 | 381 | | 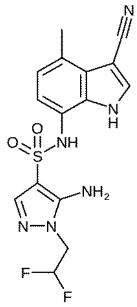 | 5-amino-N-(3-cyano-4-methyl-1H-indol-7-yl)-1-(2,2-difluoroethyl)pyrazole-4-sulfonamide |
| 278 | 401 | | 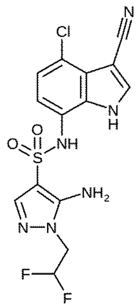 | 5-amino-N-(4-chloro-3-cyano-1H-indol-7-yl)-1-(2,2-difluoroethyl)pyrazole-4-sulfonamide |
| 279 | 385 | | 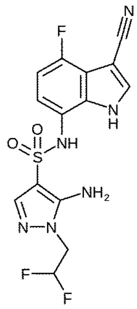 | 5-amino-N-(3-cyano-4-fluoro-1H-indol-7-yl)-1-(2,2-difluoroethyl)pyrazole-4-sulfonamide |
| 280 | | 410 | 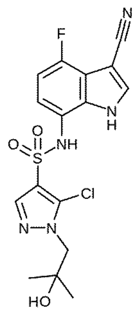 | 5-chloro-N-(3-cyano-4-fluoro-1H-indol-7-yl)-1-(2-hydroxy-2-methyl-propyl)pyrazole-4-sulfonamide |

(continued)

| Compound No. | M+H | M-H | Structure | Name |
|---|---|---|---|---|
| 281 | 362 | | 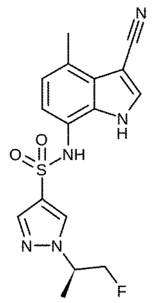 | N-(3-cyano-4-methyl-1H-indol-7-yl)-1-[(1R)-2-fluoro-1-methyl-ethyl]pyrazole-4-sulfonamide |
| 282 | 335 | | 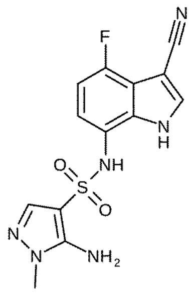 | 5-amino-N-(3-cyano-4-fluoro-1H-indol-7-yl)-1-methyl-pyrazole-4-sulfonamide |
| 283 | 389 | | 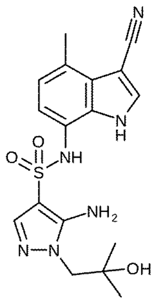 | 5-amino-N-(3-cyano-4-methyl-1H-indol-7-yl)-1-(2-hydroxy-2-methyl-propyl)pyrazole-4-sulfonamide |
| 284 | 367 | | 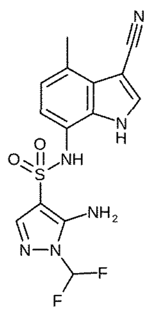 | 5-amino-N-(3-cyano-4-methyl-1H-indol-7-yl)-1-(difluoromethyl)pyrazole-4-sulfonamide |
| 285 | 371 | | 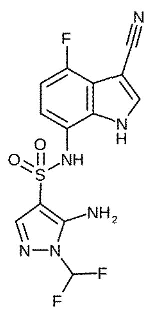 | 5-amino-N-(3-cyano-4-fluoro-1H-indol-7-yl)-1-(difluoromethyl)pyrazole-4-sulfonamide |

(continued)

| Compound No. | M+H | M-H | Structure | Name |
|---|---|---|---|---|
| 286 | | 392 | 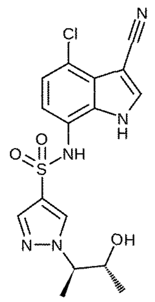 | N-(4-chloro-3-cyano-1H-indol-7-yl)-1-[(1R,2R)-2-hydroxy-1-methylpropyl]pyrazole-4-sulfonamide |
| 287 | 374 | | 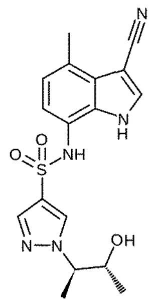 | N-(3-cyano-4-methyl-1H-indol-7-yl)-1-[(1R,2R)-2-hydroxy-1-methylpropyl]pyrazole-4-sulfonamide |
| 288 | 378 | | 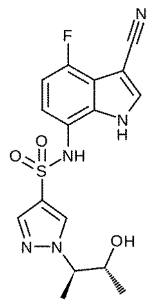 | N-(3-cyano-4-fluoro-1H-indol-7-yl)-1-[(1R,2R)-2-hydroxy-1-methylpropyl]pyrazole-4-sulfonamide |
| 289 | | 407 | 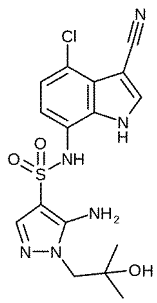 | 5-amino-N-(4-chloro-3-cyano-1H-indol-7-yl)-1-(2-hydroxy-2-methyl-propyl)pyrazole-4-sulfonamide |
| 290 | 345 | | 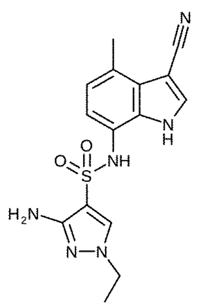 | 3-amino-N-(3-cyano-4-methyl-1H-indol-7-yl)-1-ethyl-pyrazole-4-sulfonamide |

(continued)

| Compound No. | M+H | M-H | Structure | Name |
|---|---|---|---|---|
| 291 | 349 | | 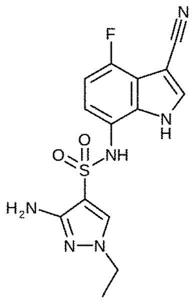 | 3-amino-N-(3-cyano-4-fluoro-1H-indol-7-yl)-1-ethyl-pyrazole-4-sulfonamide |
| 292 | 353 | | 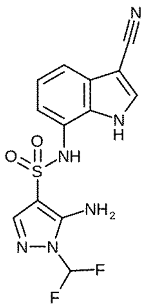 | 5-amino-N-(3-cyano-1H-indol-7-yl)-1-(difluoromethyl) pyrazole-4-sulfonamide |
| 293 | 378 | | 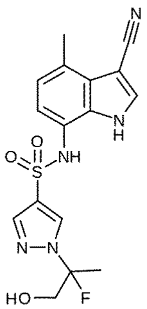 | N-(3-cyano-4-methyl-1H-indol-7-yl)-1-(1-fluoro-2-hydroxy-1-methyl-ethyl)pyrazole-4-sulfonamide |
| 294 | 382 | | 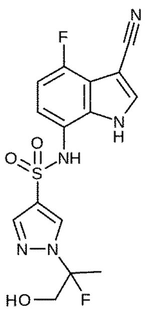 | N-(3-cyano-4-fluoro-1H-indol-7-yl)-1-(1-fluoro-2-hydroxy-1-methyl-ethyl)pyrazole-4-sulfonamide |
| 295 | 317 | | 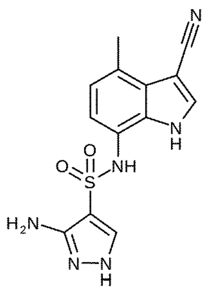 | 3-amino-N-(3-cyano-4-methyl-1H-indol-7-yl)-1H-pyrazole-4-sulfonamide |

(continued)

| Compound No. | M+H | M-H | Structure | Name |
|---|---|---|---|---|
| 296 | | 335 | 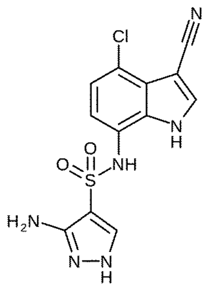 | 3-amino-N-(4-chloro-3-cyano-1H-indol-7-yl)-1H-pyrazole-4-sulfonamide |
| 297 | 364 | | 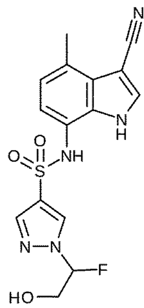 | N-(3-cyano-4-methyl-1H-indol-7-yl)-1-(1-fluoro-2-hydroxy-ethyl)pyrazole-4-sulfonamide |
| 298 | 368 | | 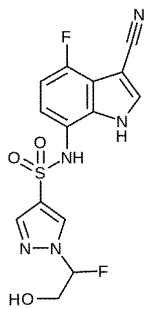 | N-(3-cyano-4-fluoro-1H-indol-7-yl)-1-(1-fluoro-2-hydroxy-ethyl)pyrazole-4-sulfonamide |
| 299 | 317 | | 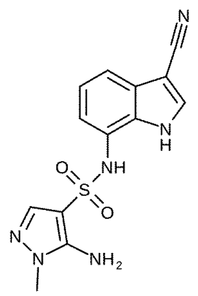 | 5-amino-N-(3-cyano-1H-indol-7-yl)-1-methyl-pyrazole-4-sulfonamide |
| 300 | 321 | | 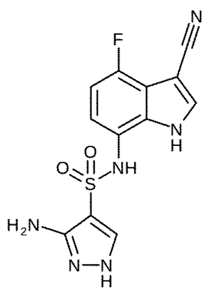 | 3-amino-N-(3-cyano-4-fluoro-1H-indol-7-yl)-1H-pyrazole-4-sulfonamide |

(continued)

| Compound No. | M+H | M-H | Structure | Name |
|---|---|---|---|---|
| 301 | 382 | | 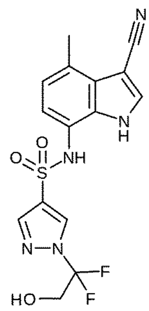 | N-(3-cyano-4-methyl-1H-indol-7-yl)-1-(1,1-difluoro-2-hydroxy-ethyl)pyrazole-4-sulfonamide |
| 302 | 338 | | 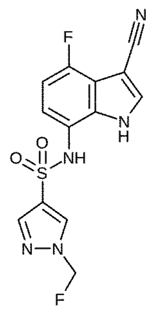 | N-(3-cyano-4-fluoro-1H-indol-7-yl)-1-(fluoromethyl) pyrazole-4-sulfonamide |
| 303 | | 373 | 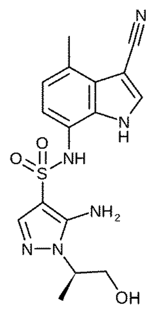 | 5-amino-N-(3-cyano-4-methyl-1H-indol-7-yl)-1-[(1R)-2-hydroxy-1-methylethyl]pyrazole-4-sulfonamide |
| 304 | 332 | | 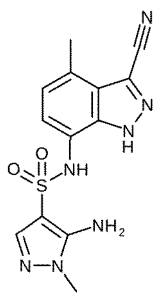 | 5-amino-N-(3-cyano-4-methyl-1H-indazol-7-yl)-1-methyl-pyrazole-4-sulfonamide |
| 305 | 335 | | 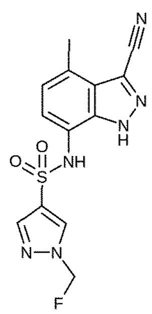 | N-(3-cyano-4-methyl-1H-indazol-7-yl)-1-(fluoromethyl) pyrazole-4-sulfonamide |

(continued)

| Compound No. | M+H | M-H | Structure | Name |
|---|---|---|---|---|
| 306 | | 393 | 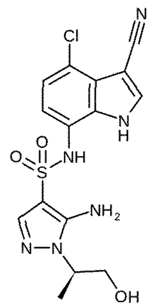 | 5-amino-N-(4-chloro-3-cyano-1H-indol-7-yl)-1-[(1R)-2-hydroxy-1-methylethyl]pyrazole-4-sulfonamide |
| 307 | | 350 | 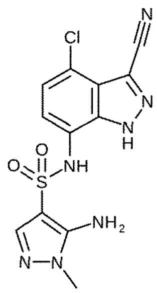 | 5-amino-N-(4-chloro-3-cyano-1H-indazol-7-yl)-1-methyl-pyrazole-4-sulfonamide |
| 308 | 349 | | 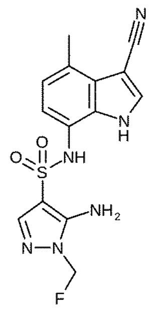 | 5-amino-N-(3-cyano-4-methyl-1H-indol-7-yl)-1-(fluoromethyl)pyrazole-4-sulfonamide |
| 309 | 353 | | 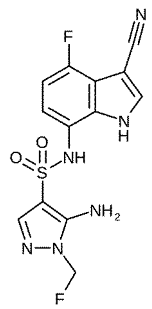 | 5-amino-N-(3-cyano-4-fluoro-1H-indol-7-yl)-1-(fluoromethyl)pyrazole-4-sulfonamide |
| 310 | | 397 | 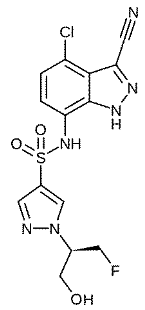 | N-(4-chloro-3-cyano-1H-indazol-7-yl)-1-[(1R)-1-(fluoromethyl)-2-hydroxyethyl]pyrazole-4-sulfonamide |

(continued)

| Compound No. | M+H | M-H | Structure | Name |
|---|---|---|---|---|
| 311 | 398 | | 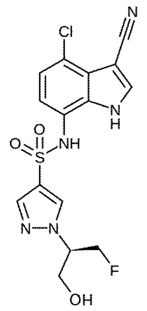 | N-(4-chloro-3-cyano-1H-indol-7-yl)-1-[(1R)-1-(fluoromethyl)-2-hydroxy-ethyl]pyrazole-4-sulfonamide |
| 312 | 335 | | 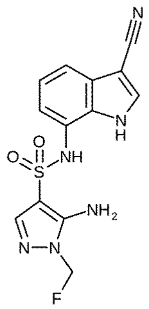 | 5-amino-N-(3-cyano-1H-indol-7-yl)-1-(fluoromethyl) pyrazole-4-sulfonamide |
| 313 | 378 | | 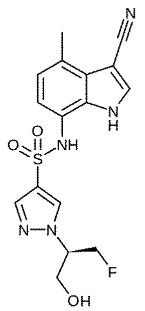 | N-(3-cyano-4-methyl-1H-indol-7-yl)-1-[(1R)-1-(fluoromethyl)-2-hydroxy-ethyl]pyrazole-4-sulfonamide |
| 314 | 379 | | 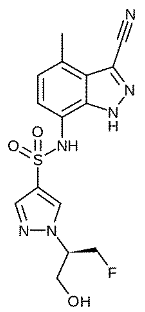 | N-(3-cyano-4-methyl-1H-indazol-7-yl)-1-[(1R)-1-(fluoromethyl)-2-hydroxyethyl]pyrazole-4-sulfonamide |
| 315 | 389 | | 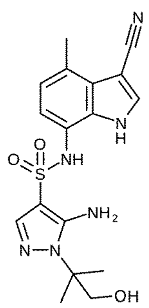 | 5-amino-N-(3-cyano-4-methyl-1H-indol-7-yl)-1-(2-hydroxy-1,1-dimethylethyl)pyrazole-4-sulfonamide |

(continued)

| Compound No. | M+H | M-H | Structure | Name |
|---|---|---|---|---|
| 316 | | 407 | 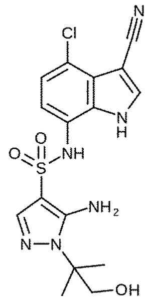 | 5-amino-N-(4-chloro-3-cyano-1H-indol-7-yl)-1-(2-hydroxy-1,1-dimethylethyl)pyrazole-4-sulfonamide |

**Method of Use**

An RBM39 degrader can have therapeutic benefits, for example, for the treatment of proliferative disorders. Though RBM39 protein may lack enzymatic active sites that can be targeted by inhibitors, small molecules can selectively target RBM39 protein for ubiquitination and subsequent proteasomal degradation. For example, the small molecule aromatic sulfonamides indisulam and E7820 potentiate the productive formation of a complex between RBM39 protein and DCAF15, the substrate recognition subunit of an E3 ubiquitin ligase complex CUL4-DDB 1-DDA1-RBX1-DCAF15 that does not otherwise ubiquitinate RBM39 protein *in vivo* (Han, et al. Science. 2017 Apr 28;356(6336); Uehara, et al. Nat Chem Biol. 2017 Jun; 13(6):675-680).

With RBM39 protein established as the primary molecular target of the aromatic sulfonamides indisulam and E7820, the need to develop safe, selective, efficacious, and orally bioavailable alternatives to these sulfonamides becomes paramount. Clinical studies with these sulfonamides were obscured by the lack of knowledge of molecular target. Since the introduction of indisulam and E7820, at least 4 different proteins/pathways have been proposed as potential molecular targets, including carbonic anhydrase, anti-angiogenesis mediated by suppression of integrin a2 expression, regulation of mitochondria, and cytostasis induced by retinoblastoma hypophosphorylation. A lack of mechanistic insight and selectivity for these agents likely explains their toxicity and limited impact in the clinic. In addition, there were significant developmental obstacles for both indisulam and E7820. Indisulam has poor oral bioavailability and was restricted to a cycle of IV dosing followed by drug holiday (700 mg/m$^2$, 1 hour IV infusion, every 3 weeks). The on/off dosing cycle was necessitated by a treatment-induced myelosuppressive effect. The most frequent myelosuppressive adverse events with toxicity grade ≥ 3 were neutropenia and thrombocytopenia. On the other hand, despite achieving good oral bioavailability, E7820 faced many of the same challenges as indisulam. Although it was dosed orally and for longer periods than indisulam, treatment with E7820 resulted in the same myelosuppressive effects and required a similar on/off dosing cycle to mitigate (1 cycle = 28 days at 100 mg q.d. followed by 7 days holiday). E7820 also suffers from greater than 99% plasma protein binding; according to the free drug principle, this means that less than 1% of the measured E7820 drug concentration is available to mediate RBM39 protein degradation. To achieve effective target coverage, the free drug principle implies that higher dosing will be necessary for highly protein-bound compounds such as E7820. The development of RBM39 degraders with good oral bioavailability, selectivity and reduced plasma protein binding can enable a favorable circumstance for evaluating the therapeutic potential of small molecule-mediated RBM39 degradation.

The present disclosure provides compounds that are capable of inducing degradation of RBM39 protein. Without wishing to be bound by any particular theory, the inventors believe that the compounds induce degradation of RBM39 protein (i.e., they are RBM39 degraders) by potentiating the productive formation of a complex between RBM39 protein and DCAF15, the substrate recognition subunit of an E3 ubiquitin ligase complex. This leads to subsequent ubiquitination and protosomal degradation of RBM39 protein.

In one aspect, the present disclosure provides a compound of Formula I for use in a method for treating cancer in a subject in need thereof, comprising administering to said subject a therapeutically effective amount of a compound of Formula I, as disclosed herein. In some embodiments, the method for treating cancer comprises administering to said subject an RBM39 degrader. In some embodiments, the compound of Formula I is an RBM39 degrader.. In some embodiments, said cancer condition is a cancer selected from the group consisting of leukemia, melanoma, lymphoma, lung cancer, colon cancer and ovarian cancer. In a further embodiment, said cancer condisiton is colon cancer.

As used herein, a therapeutically effective amount refers to an amount sufficient to effect the intended application, including but not limited to, disease treatment, as defined herein. Also contemplated is the use of a sub-therapeutic

amount of a compound of Formula I for treating an intended disease condition.

The amount of a compound of Formula I administered may vary depending upon the intended application (*in vitro* or *in vivo*), or the subject and disease condition being treated, e.g., the weight and age of the subject, the severity of the disease condition, the manner of administration and the like, which can readily be determined by one of ordinary skill in the art.

In some other embodiments, the disease condition that is to be treated is associated with RBM39 protein, wherein the disease condition is cancer. Aberrant pre-mRNA splicing is emerging as a common theme in diverse human cancers; consequently, agents that target these pathways, for example, RBM39 protein, have therapeutic value. The data presented in the Examples herein below demonstrate the anti-cancer effects of compounds of Formula I. As such, the compound is particularly useful for treating a proliferative disorder, such as leukemia such as for example, Acute Myeliod Leukemia (AML), lymphoma, including EZH2 mutant limphoma, lung cancer, melanoma, colon cancer and ovarian cancer.

Compounds of the invention may be administered by any convenient route, e.g. into the gastrointestinal tract (e.g. rectally or orally), the nose, lungs, musculature or vasculature, or transdermally or dermally. Compounds may be administered in any convenient administrative form, e.g. tablets, powders, capsules, solutions, dispersions, suspensions, syrups, sprays, suppositories, gels, emulsions, patches etc. Such compositions may contain components conventional in pharmaceutical preparations, e.g. diluents, carriers, pH modifiers, sweeteners, bulking agents, and further active agents. If parenteral administration is desired, the compositions will be sterile and in a solution or suspension form suitable for injection or infusion. Such compositions form a further aspect of the invention.

A composition of the present disclosure may be formulated in any suitable pharmaceutical formulation. A pharmaceutical composition of the present disclosure typically contains an active ingredient (i.e., a compound of Formula I or a pharmaceutically acceptable salt thereof), and one or more pharmaceutically acceptable excipients or carriers, including but not limited to: inert solid diluents and fillers, diluents, sterile aqueous solution and various organic solvents, permeation enhancers, solubilizers, and adjuvants. A composition of the present disclosure may be formulated in any suitable pharmaceutical formulation for intended route of delivery. In some embodiments, the pharmaceutical acceptable carriers or excipients are selected from water, alcohol, glycerol, chitosan, alginate, chondroitin, Vitamin E, mineral oil, and dimethyl sulfoxide (DMSO).

Pharmaceutical formulations may be provided in any suitable form, which may depend on the route of administration. In some embodiments, the pharmaceutical composition disclosed herein can be formulated in dosage form for administration to a subject. In some embodiments, the pharmaceutical composition is formulated for oral, intravenous, intraarterial, aerosol, parenteral, buccal, topical, transdermal, rectal, intramuscular, subcutaneous, intraosseous, intranasal, intrapulmonary, transmucosal, inhalation, and/or intraperitoneal administration. In some embodiments, the dosage form is formulated for oral administration. For example, the pharmaceutical composition can be formulated in the form of a pill, a tablet, a capsule, an inhaler, a liquid suspension, a liquid emulsion, a gel, or a powder. In some embodiments, the pharmaceutical composition can be formulated as a unit dosage in liquid, gel, semi-liquid, semi-solid, or solid form.

In some embodiments, the invention provides a solid pharmaceutical composition for oral administration containing: (i) a compound having Formula I or Formula II; and (ii) a pharmaceutical excipient suitable for oral administration. The composition may further contain: (iii) a third agent or even a fourth agent. Each compound or agent may be present in a therapeutically effective amount. One or more compounds or agents may also be present in a sub-therapeutic amount, and the compounds or agents act synergistically to provide a therapeutically effective pharmaceutical composition.

In some embodiments, the present invention provides a compound of Formulae I or II or a pharmaceutically acceptable salt thereof for use as a medicament for the treatment of a condition that can be treated with an RBM39 degrader, i.e., cancer.

## EXAMPLES

Preparative thin layer chromatography (PTLC) separations described herein were typically performed on 20 × 20 cm plates (500 micron thick silica gel).

Chromatographic purifications were typically performed using Biotage Isolera One automated systems running Biotage Isolera One 2.0.6 software (Biotage LLC, Charlotte, NC). Flow rates were the default values specified for the particular column in use. Reverse phase chromatography was performed using elution gradients of water and acetonitrile on KP-C18-HS Flash+ columns (Biotage LLC) of various sizes. Typical loading was between 1:50 and 1:1000 crude sample : RP $SiO_2$ by weight. Normal phase chromatography was performed using elution gradients of various solvents (e.g. hexane, ethyl acetate, methylene chloride, methanol, acetone, chloroform, MTBE, etc.). The columns were SNAP Cartridges containing KP-SIL or SNAP Ultra (25 μm spherical particles) of various sizes (Biotage LLC). Typical loading was between 1:10 to 1: 150 crude sample : $SiO_2$ by weight. Alternatively, silica gel chromatography was performed on a Biotage Horizon flash chromatography system.

$^1$H NMR analyses of intermediates and exemplified compounds were typically performed on an Agilent Technologies 400/54 or Bruker Ascend TM 400 spectrometer (operating at 400 MHz) at 298 °K following standard operating procedure suggested by manufacturer. Reference frequency was set using TMS as an internal standard. Typical deuterated solvents were utilized as indicated in the individual examples.

LCMS analyses were typically performed using one of the two conditions listed below:

(1) LCMS spectra were taken on an Agilent Technologies 1260 Infinity coupled to 6120 Quadrupole spectrometer. The mobile phase for the LC was acetontrile (A) and water (B) with 0.01% formic acid, and the eluent gradient was from 5-95% A in 6.0 min, 60-95% A in 5.0 min, 80-100% A in 5.0 min and 85-100% A in 10 min using a SBC18 50 mm×4.6 mm× 2.7 μm capillary column. Mass spectra (MS) were measured by electrospray ion-mass spectroscopy (ESI). All temperatures are in degrees Celsius unless otherwise noted.

(2) LCMS analysis of intermediates and exemplified compounds was performed on an Agilent Technologies 1200 Series HPLC system coupled to an Agilent Technologies 6150 Quadrapole LC/MS detector. Analytes were detected by UV absorbance at 220 and 254 nm. Analyte ions were detected by mass spectrometry in both negative and positive modes (110 - 800 amu scan range, API-ES ionization). A long HPLC method was run on a Phenomenex® Kinetex 2.6 μ C18 100Å, 30 × 3.00 mm column. The column temperature was set at 40 °C. UV absorptions were detected at 220 and 254 nm. Samples were prepared as a solution in about 1:1 (v/v) acetonitrile:water mixture. Flow rate was about 0.80 mL/minute. Elution solvents were acetonitrile and water each containing 0.1% formic acid. In a typical run, a linear gradient starting with 5% acetonitrile and 95% water and ending with 95% acetonitrile and 5% water over 12 minutes was carried out. At the end of each run, the column was washed with 95% acetonitrile and 5% water for 2 minutes.

Typically, analytical HPLC mass spectrometry conditions were as follows:

LC1: Column: SB-C18 50 mm×4.6 mm× 2.7 μm; Temperature: 50 °C; Eluent: 5:95 v/v acetonitrile/water + 0.01% formic acid in 6 min; Flow Rate: 1.5 mL/min; Injection 5 μL; Detection: PDA, 200-600 nm; MS: mass range 150-750 amu; positive ion electrospray ionization.

LC2: Column: SB-C18 50 mm×4.6 mm× 2.7 μm; Temperature: 50 °C; Eluent: 5:95 to 95:5 v/v acetonitrile/water + 0.05% TFA over 3.00 min; Flow Rate: 1.5 mL/min; Injection 5μL; Detection: PDA, 200-600 nm; MS: mass range 150-750 amu; positive ion electrospray ionization.

LC3: Column: SB-C18 50 mm×4.6 mm× 2.7 μm; Temperature: 50 °C; Eluent: 10:90 to 98:2 v acetonitrile/water + 0.05% TFA over 3.75 min; Flow Rate: 1.0 mL/min; Injection 10 μL; Detection: PDA, 200-600 nm; MS: mass range 150-750 amu; positive ion electrospray ionization.

Preparative HPLC were carried out with one of the two conditions listed below:

Condition 1: GILSON Preparative HPLC System; Column: SHISEIDO CAPCELL PAK, MG; C18, 20mm×250mm, 5μm; Mobile phase: Water + 0.1% trifluoroacetic acid; ACN + 0.1% trifluoroacetic acid; Method: 15 minutes gradient elution; Initial organic : 10%; Final organic: 80%; UV1: 240; UV2: 230; Flow: 15ml/min.

Condition 2: GILSON Preparative HPLC System; Column: SunFire® Prep C18 OBD™ 5 uM, 19mm×150mm; Mobile phase: Water + 0.1% trifluoroacetic acid; ACN + 0.1% trifluoroacetic acid; Method: 20 minutes gradient elution; Initial organic: 10%; Final organic: 80%; UV1: 220; UV2: 254; Flow: 15 ml/min.

Compound names were generated with ChemDraw Professional 15.1 or OpenEye Scientific Software's mol2nam application.

**Example 1:** Synthesis of N-(3-chloro-1H-indol-7-yl)-1-methyl-1H-pyrazole-4-sulfonamide **(Compound 1**).

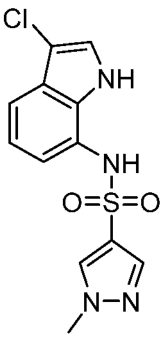

A vial was charged with 3-chloro-1H-indol-7-amine (64.6 mg, 0.39 mmol) and 1-methylpyrazole-4-sulfonyl chloride (70.0 mg, 0.39 mmol). Pyridine (1.5 mL) was added and the sealed vial was stirred at ambient temperature for 30 min. The reaction mixture was concentrated and partitioned between EtOAc and water. The EtOAc was washed with brine, dried over $MgSO_4$, filtered, and evaporated. The residue was chromatographed on a Biotage 10 g ultra SNAP column with a 10% to 80% EtOAc:DCM gradient. Product-containing fractions were evaporated to afford N-(3-chloro-1H-indol-7-yl)-1-methyl-1H-pyrazole-4-sulfonamide (98.9 mg, 0.302 mmol, 78 % yield) as a beige solid. $^1H$ NMR (400 MHz, $CDCl_3$): δ 9.20 (br s, 1H), 7.59 (s, 1H), 7.54 (d, 1H), 7.44 (s, 1H), 7.00 (d, 1H), 6.56 (d, 1H), 6.52 (s, 1H), 1.57 (s, 3H). *m/z* (ES-API-pos) [M+1] 311, 313.

**Example 2:** Synthesis of N-(3-cyano-4-methyl-1H-indol-7-yl)-1-methylpyrazole-4-sulfonamide (**Compound 3**).

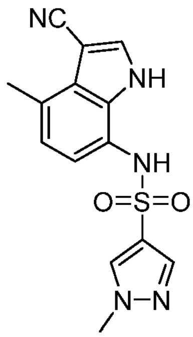

N-(3-cyano-4-methyl-1H-indol-7-yl)-1-methyl-pyrazole-4-sulfonamide was prepared analogously to the procedure for Example 1, substituting 7-amino-4-methyl-1H-indole-3-carbonitrile for 3-chloro-1H-indol-7-amine. $^1H$ NMR (400 MHz, $CD_3OD$): δ 7.92 (s, 1H), 7.80 (s, 1H), 7.56 (s, 1H), 6.83 (d, 1H), 6.63 (d, 1H), 3.84 (s, 3H), 2.67 (s, 3H). *m/z* (ES-API-pos) [M+1] 316.

**Example 3:** Synthesis of N-(3-cyano-4-methyl-1H-indol-7-yl)-1-(2-hydroxyethyl)-1H-pyrazole-4-sulfonamide (**Compound 25**).

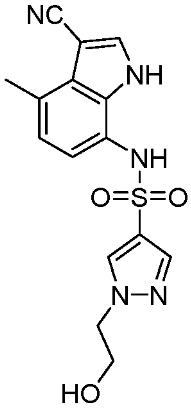

Step A: Preparation of 2-(4-benzylsulfanylpyrazol-1-yl)ethanol: A vial containing a solution of 2-hydrazinylethanol (0.02 mL, 0.31 mmol) and 2-benzylsulfanylpropanedial (60 mg, 0.31 mmol) in methanol (1 mL) was heated at 65 °C. After 20 min, the reaction mixture was evaporated to afford 2-(4-benzylsulfanylpyrazol-1-yl)ethanol. The crude product was used as is in the next step. *m/z* (ES-API-pos) [M+1] 235.

Step B: Preparation of 1-(2-hydroxyethyl)pyrazole-4-sulfonyl chloride: A solution of crude 2-(4-benzylsulfanylpyrazol-1-yl)ethanol (72 mg theoretical, 0.31mmol) in acetic acid (1mL) and water (0.1 mL) was treated with N-chlorosuccinimide (102.6 mg, 0.77 mmol) at room temperature. A slight exotherm was observed. After 5 min, the reaction mixture was partitioned between EtOAc and water. The EtOAc was washed with brine, dried over $MgSO_4$, filtered, and evaporated. The residue was chromatographed on a Biotage 10 g KP-sil column with a 60% to 100% EtOAc:hexane gradient. Product-containing fractions were evaporated to afford 1-(2-hydroxyethyl)pyrazole-4-sulfonyl chloride (17.2 mg, 0.0817 mmol, 26.6% yield over 2 steps). *m/z* (ES-API-pos) [M+1] 211.

Step C: Preparation of N-(3-cyano-4-methyl-1H-indol-7-yl)-1-(2-hydroxyethyl)pyrazole-4-sulfonamide: A flask was charged with 7-amino-4-methyl-1H-indole-3-carbonitrile (53.64 mg, 0.31 mmol) and 1-(2-hydroxyethyl)pyrazole-4-sulfonyl chloride (66 mg, 0.31 mmol). Pyridine (2 mL) was added and the mixture was stirred at ambient temperature overnight. The reaction mixture was concentrated and partitioned between EtOAc and water. The EtOAc was washed with brine, dried over $MgSO_4$, filtered, and evaporated. The residue was absorbed on silica gel and chromatographed on a Biotage 25 g ultra SNAP column with a 5% to 80% (3:1 EtOAc:EtOH):DCM gradient. Product-containing fractions were evaporated to afford N-(3-cyano-4-methyl-1H-indol-7-yl)-1-(2-hydroxyethyl)pyrazole-4-sulfonamide (60.3 mg, 0.166 mmol, 52.9 % yield) as a beige solid. $^1$H NMR (400 MHz, $CD_3OD$): δ 7.92 (s, 1H), 7.86 (s, 1H), 7.58 (s, 1H), 6.84 (d, 1H), 6.65 (d, 1H), 4.18 (t, 2H), 3.82 (t, 2H), 2.67 (s, 3H). *m/z* (ES-API-pos) [M+1] 346.

**Example 4:** Synthesis of N-(3-chloro-1H-indol-7-yl)-1-(oxetan-3-yl)pyrazole-4-sulfonamide **(Compound 40).**

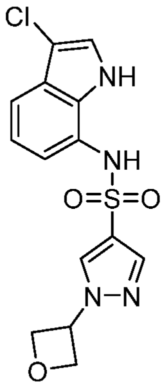

Cesium carbonate (76.9 mg, 0.24 mmol) was added to a microwave vial containing a mixture of N-(3-chloro-1H-indol-7-yl)-1H-pyrazole-4-sulfonamide (35.0 mg, 0.12 mmol) in DMF (1 mL). 3-Bromooxetane (0.0098 mL, 0.12 mmol) was added to this mixture and the vial was heated in a microwave at 150 °C for 30 min. The reaction mixture was partitioned between EtOAc and water. The EtOAc was washed with brine, dried over $MgSO_4$, filtered, and evaporated.

The residue was chromatographed on a Biotage 10 g ultra SNAP column with a 10% to 100% EtOAc:hexane gradient. Product-containing fractions were evaporated to afford N-(3-chloro-1H-indol-7-yl)-1-(oxetan-3-yl)pyrazole-4-sulfonamide (13.4 mg, 0.036 mmol, 31 % yield) as a solid. $^1$H NMR (400 MHz, $CD_3OD$): δ 7.91 (s, 1H), 7.73 (s, 1H), 7.38 (d, 1H), 7.25 (s, 1H), 6.98 (t, 1H), 6.79 (d, 1H), 5.49-5.42 (m, 1H), 4.96-4.89 (m, 4H). *m/z* (ES-API-pos) [M+1] 353, 355.

**Example 5:** Synthesis of N-(3-chloro-1H-indol-7-yl)-1-(2-cyanoethyl)pyrazole-4-sulfonamide **(Compound 59).**

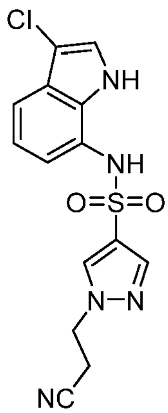

A vial containing 3-bromopropanenitrile (0.0084 mL, 0.1 mmol), N-(3-chloro-1H-indol-7-yl)-1H-pyrazole-4-sulfonamide (30 mg, 0.1 mmol), and cesium carbonate (65.9 mg, 0.2 mmol) in DMF (1 mL) was stirred at 60 °C. After 8 h, the reaction mixture was partitioned between EtOAc and water. The EtOAc was washed with brine, dried over $MgSO_4$, filtered, and evaporated. The residue was chromatographed on a Biotage 10 g ultra SNAP column with a 10% to 100% EtOAc:hexane gradient. Product-containing fractions were evaporated to afford N-(3-chloro-1H-indol-7-yl)-1-(2-cyanoethyl)pyrazole-4-sulfonamide (31.8 mg, 0.086 mmol, 85% yield). $^1$H NMR (400 MHz, $CD_3OD$): δ 7.95 (s, 1H), 7.62 (s, 1H), 7.39 (d, 1H), 7.25 (s, 1H), 6.99 (t, 1H), 6.76 (d, 1H), 4.28 (t, 2H), 2.96 (t, 2H). *m/z* (ES-API-pos) [M+1] 350, 352.

**Example 6:** Synthesis of N-(3-cyano-4-methyl-1H-indol-7-yl)-1-cyclobutyl-pyrazole-4-sulfonamide **(Compound 61).**

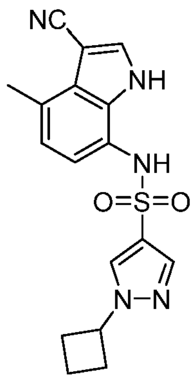

N-(3-cyano-4-methyl-1H-indol-7-yl)-1-cyclobutyl-pyrazole-4-sulfonamide was prepared as described in Example 1, substituting 1-cyclobutylpyrazole-4-sulfonyl chloride for 1-methylpyrazole-4-sulfonyl chloride and 7-amino-4-methyl-1H-indole-3-carbonitrile for 3-chloro-1H-indol-7-amine. $^1$H NMR (400 MHz, $CD_3OD$): δ 7.92 (s, 1H), 7.82 (s, 1H), 7.61 (s, 1H), 6.83 (d, 1H), 6.61 (d, 1H), 4.77 (p, 1H), 2.67 (s, 3H), 2.50-2.38 (m, 4H), 1.89-1.80 (m, 2H). *m/z* (ES-API-pos) [M+1] 356.

**Example 7:** Synthesis of N-(3-cyano-4-fluoro-1H-indol-7-yl)-1-methyl-pyrazole-4-sulfonamide (**Compound 65**).

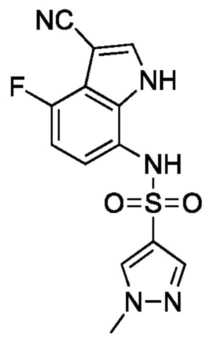

Step A: Preparation of 4-fluoro-7-nitro-1H-indole-3-carbaldehyde: A mixture of phosphorus (V) oxychloride (0.52 mL, 5.6 mmol) and DMF (15 mL) was stirred at 0 °C for 1 hour. A solution of 4-fluoro-7-nitro-1H-indole (1.00 g, 5.55 mmol) in DMF (10 mL) was added in one portion. The reaction mixture was stirred at ambient temperature for 16 hours. The reaction mixture was poured into ice. Aqueous NaOH solution (3 N, 7.4 mL, 22 mmol) was added, and the mixture was heated at 80 °C for 10 min. The resulting solid was collected by filtration, washed with water and dried to give 4-fluoro-7-nitro-1H-indole-3-carbaldehyde. The crude product was used in the next step without further purification. *m*/*z* (ES-API-pos) [M+1] 209.

Step B: Preparation of 4-fluoro-7-nitro-1H-indole-3-carbaldehyde oxime: A mixture of 4-fluoro-7-nitro-1H-indole-3-carbaldehyde (1.15 g, 5.52 mmol), sodium acetate (0.906 g, 11.0 mmol) and hydroxylamine hydrochloride (0.576 g, 8.29 mmol) in ethanol (28 mL) was stirred at ambient temperature for 3 hours. The reaction mixture was filtered and concentrated to give crude 4-fluoro-7-nitro-1H-indole-3-carbaldehyde oxime, which was used in the next step without further purification. *m*/*z* (ES-API-pos) [M+1] 224.

Step C: Preparation of 4-fluoro-7-nitro-1H-indole-3-carbonitrile: 4-fluoro-7-nitro-1H-indole-3-carbaldehyde oxime (1.23 g, 5.51 mmol) was dissolved in THF (18 mL) and treated with 1;1'-thiocarbonyldiimidazole (1.67 g, 9.37 mmol) for 3 hours at ambient temperature. The solvent was removed and the residue was partitioned between EtOAc and water. The organic layer was washed with water and brine, dried and concentrated under reduced pressure. The residue was purified by flash column chromatography on silica gel (10-50% EtOAc/hexanes) to give 4-fluoro-7-nitro-1H-indole-3-carbonitrile (140 mg, 12% yield). *m*/*z* (ES-API-pos) [M+1] 206.

Step D: Preparation of 7-amino-4-fluoro-1H-indole-3-carbonitrile: To a solution of 4-fluoro-7-nitro-1H-indole-3-carbonitrile (0.14 g, 0.68 mmol) in ethanol (6 mL) and water (0.6 mL) was added iron (0.19 g, 3.4 mmol) and $NH_4Cl$ (0.37 g, 6.8 mmol). The mixture was stirred at 80 °C for 2 hours. After cooling, the mixture was concentrated and diluted with ethyl acetate. The mixture was then filtered. The filtrate was washed with brine, dried over anhydrous $Na_2SO_4$, filtered, concentrated and purified by flash column chromatography on silica gel (20-80% EtOAc/hexanes) to give 7-amino-4-fluoro-1H-indole-3-carbonitrile (0.080 g, 67% yield). *m*/*z* (ES-API-pos) [M+1] 176.

Step E: Preparation of N-(3-cyano-4-fluoro-1H-indol-7-yl)-1-methyl-pyrazole-4-sulfonamide: A mixture of 7-amino-4-fluoro-1H-indole-3-carbonitrile (0.015 g, 0.086 mmol) and 1-methyl-1H-pyrazole-4-sulfonyl chloride (0.016 g, 0.086 mmol) in pyridine (0.8 mL) was stirred at ambient temperature for 30 min. The mixture was concentrated under reduced pressure. The residue was purified by flash column chromatography on silica gel (1-5% MeOH/DCM) to give N-(3-cyano-4-fluoro-1H-indol-7-yl)-1-methyl-pyrazole-4-sulfonamide (25 mg, 91% yield). $^1$H NMR (400 MHz, $(CD_3)_2CO$): δ 8.17 (s, 1H), 7.89 (s, 1H), 7.56 (s, 1H), 6.88-6.89 (m, 1H), 6.86-6.88 (m, 1H), 3.87 (s, 3H). *m*/*z* (ES-API-pos) [M+1] 320.

**Example 8:** Synthesis of N-(3-cyano-4-methyl-1H-indol-7-yl)-1-(cyclopropylmethyl)pyrazole-4-sulfonamide **(Compound 69)**.

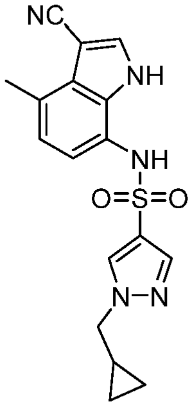

A vial containing (bromomethyl)cyclopropane (0.008 mL, 0.083 mmol), N-(3-cyano-4-methyl-1H-indol-7-yl)-1H-pyrazole-4-sulfonamide (25.mg, 0.08mmol), and cesium carbonate (54.1 mg, 0.17 mmol) in DMF (1 mL) was stirred at 50 °C. After 2.75 h, the reaction mixture was partitioned between EtOAc and water. The EtOAc was washed with brine, dried over $MgSO_4$, filtered, and evaporated. The residue was chromatographed on a Biotage 10 g ultra SNAP column with a 20% to 100% EtOAc:hexane gradient. Product-containing fractions were evaporated to afford N-(3-cyano-4-methyl-1H-indol-7-yl)-1-(cyclopropylmethyl)pyrazole-4-sulfonamide (14.7 mg, 0.039 mmol, 47% yield) as a white solid. $^1$H NMR (400 MHz, $CD_3OD$): δ 7.92 (s, 1H), 7.80 (s, 1H), 7.61 (s, 1H), 6.83 (d, 1H), 6.62 (d, 1H), 3.93 (d, 2H), 2.67 (s, 3H), 1.23-1.09 (m, 1H), 0.58-0.52 (m, 2H), 0.30-0.26 (m, 2H). *m/z* (ES-API-pos) [M+1] 356.

**Example 9:** Synthesis of N-(3-cyano-4-methyl-1H-indol-7-yl)-1-[(3-methyloxetan-3-yl)methyl]pyrazole-4-sulfonamide **(Compound 71).**

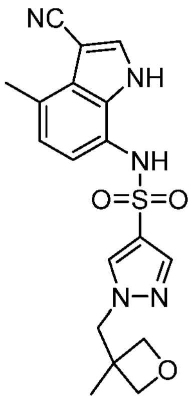

N-(3-cyano-4-methyl-1H-indol-7-yl)-1-[(3-methyloxetan-3-yl)methyl]pyrazole-4-sulfonamide was prepared in a similar way as Example 8, substituting 3-(bromomethyl)-3-methyloxetane for (bromomethyl)cyclopropane. $^1$H NMR (400 MHz, $CD_3OD$): δ 7.91 (s, 1H), 7.76 (s, 1H), 7.64 (s, 1H), 6.81 (d, 1H), 6.60 (d, 1H), 4.59 (d, 2H), 4.29-4.26 (m, 4H), 2.66 (s, 3H), 1.04 (s, 3H). *m/z* (ES-API-pos) [M+1] 386.

**Example 10:** Synthesis of N-(3-chloro-4-methyl-1H-indol-7-yl)-1-methylpyrazole-4-sulfonamide **(Compound 75).**

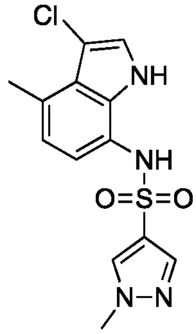

Step A: Preparation of 4-methyl-1H-indol-7-amine: To a microwave reactor tube was added CuI (21 mg, 0.11 mmol), L-proline (26 mg, 0.22 mmol), potassium carbonate (233 mg, 1.69 mmol), DMSO (2.8 mL) and 7-bromo-4-methyl-1H-Indole (118 mg, 0.560 mmol) under nitrogen, followed by the addition of 28-30% ammonia hydroxide aqueous solution (1.4 mL). The resulting suspension was heated under microwave at 155 °C for 1 hour. The reaction mixture was then cooled to ambient temperature, suspended in water and EtOAc, and further extracted with EtOAc. Combined organic layers were washed with brine, dried over $Na_2SO_4$, and concentrated. The crude oil was then purified by flash column chromatography on silica gel (20-100% EtOAc/hexanes) to give 4-methyl-1H-indol-7-amine (41 mg, 50% yield). *m/z* (ES-API-pos) [M+1] 147.

Step B: Preparation of 1-methyl-N-(4-methyl-1H-indol-7-yl)pyrazole-4-sulfonamide: A mixture of 4-methyl-1H-indol-7-amine (0.010 g, 0.068 mmol) and 1-methyl-1H-pyrazole-4-sulfonyl chloride (0.012 g, 0.068 mmol) in pyridine (0.7 mL) was stirred at ambient temperature for 30 min. The mixture was concentrated under reduced pressure. The residue was purified by flash column chromatography on silica gel (1-5% MeOH/DCM) to give 1-methyl-N-(4-methyl-1H-indol-7-yl)pyrazole-4-sulfonamide (10 mg, 50% yield). *m/z* (ES-API-pos) [M+1] 291.

Step C: Preparation of N-(3-chloro-4-methyl-1H-indol-7-yl)-1-methyl-pyrazole-4-sulfonamide: To a stirred solution of 1-methyl-N-(4-methyl-1H-indol-7-yl)pyrazole-4-sulfonamide (5 mg, 0.02 mmol) in THF (0.3 mL) was added 1N HCl (10 □L) followed by N-chlorosuccinamide (3 mg, 0.02 mmol). The reaction mixture was stirred at ambient temperature for 1 hour. The reaction was partitioned between EtOAc and water. The organic layer was washed with saturated aqueous NaHCOs solution and brine, dried and concentrated. The residue was purified by flash column chromatography (1-5% MeOH/DCM) to give N-(3-chloro-4-methyl-1H-indol-7-yl)-1-methyl-pyrazole-4-sulfonamide (3 mg, 54% yield). $^1H$ NMR (400 MHz, $(CD_3)_2CO$): δ 10.24 (s, 1H), 8.53 (s, 1H), 7.87 (s, 1H), 7.54 (s, 1H), 7.37 (d, 1H), 6.74 (d, 1H), 6.71 (d, 1H), 3.86 (s, 3H), 2.69 (s, 3H). *m/z* (ES-API-pos) [M+1] 325.

**Example 11:** Synthesis of 1-(2-Cyanoethyl)-N-(3-cyano-4-methyl-1H-indol-7-yl)pyrazole-4-sulfonamide **(Compound 77).**

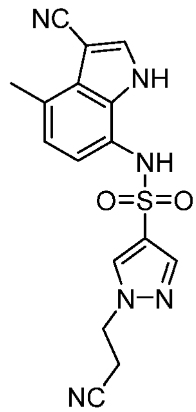

1-(2-Cyanoethyl)-N-(3-cyano-4-methyl-1H-indol-7-yl)pyrazole-4-sulfonamide was prepared in a similar manner as Example 8, substituting 3-bromopropanenitrile for (bromomethyl)cyclopropane. $^1H$ NMR (400 MHz, $CD_3OD$): δ 7.95 (s, 1H), 7.92 (s, 1H), 7.64 (s, 1H), 6.84 (d, 1H), 6.62 (d, 1H), 4.39 (t, 2H), 3.00 (t, 2H), 2.67 (s, 3H). *m/z* (ES-API-pos) [M+1] 355.

**Example 12:** Synthesis of N-(3-cyano-4-methyl-1H-indol-7-yl)-1-[(2,2-difluorocyclopropyl)methyl]pyrazole-4-sulfonamide **(Compound 79).**

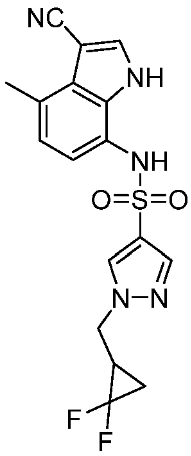

Step A: Preparation of (2,2-difluorocyclopropyl)methyl methanesulfonate: Methanesulfonyl chloride (0.018 mL, 0.24 mmol) was added to an ice-cold solution of triethylamine (0.055 mL, 0.40 mmol) and 2,2-difluoro-cyclopropanemethanol, (0.019 mL, 0.16 mmol) in dichloromethane (1 mL). The mixture was stirred in ice. After 40min the reaction mixture was diluted with DCM, washed with saturated aqueous $NaHCO_3$, dried over $MgSO_4$, filtered, and evaporated to afford (2,2-difluorocyclopropyl)methyl methanesulfonate (25.5 mg, 0.137 mmol, 87% yield) as a colorless oil. Used as is in the next step.

Step B: Preparation of N-(3-cyano-4-methyl-1H-indol-7-yl)-1-[(2,2-difluorocyclopropyl)methyl]pyrazole-4-sulfonamide: A vial containing (2,2-difluorocyclopropyl)methyl methanesulfonate (25.3 mg, 0.14 mmol), N-(3-cyano-4-methyl-1H-indol-7-yl)-1H-pyrazole-4-sulfonamide (Compound 99, 41.0 mg, 0.14 mmol), and cesium carbonate (88.7 mg, 0.27 mmol) in DMF (1 mL) was heated at 100 °C. After 45 min, the reaction mixture was partitioned between EtOAc and water. The EtOAc was washed with brine, dried over $MgSO_4$, filtered, and evaporated. The residue was chromatographed on a Biotage 10 g ultra SNAP column with a 20% to 100% EtOAc:hexane gradient. Product-containing fractions were evaporated to afford impure desired product. This was chromatographed on a Biotage 12+M reverse phase column with a 20% to 100% ACN:water gradient. Product-containing fractions were evaporated to afford N-(3-cyano-4-methyl-1H-indol-7-yl)-1-[(2,2-difluorocyclopropyl)methyl]pyrazole-4-sulfonamide (14.6 mg, 0.035 mmol, 26% yield). $^1H$ NMR (400 MHz, $CD_3OD$): $\delta$ 7.92 (s, 1H), 7.82 (s, 1H), 7.63 (s, 1H), 6.83 (d, 1H), 6.61 (d, 1H), 4.25-4.20 (m, 2H), 2.67 (s, 3H), 2.17-2.05 (m, 1H), 1.60-1.51 (m, 1H), 1.34-1.26 (m, 1H). *m/z* (ES-API-pos) [M+1] 392.

**Example 13:** Synthesis ofN-(3-cyano-4-methyl-1H-indol-7-yl)-1-[1-(fluoromethyl)vinyl]pyrazole-4-sulfonamide **(Compound 80).**

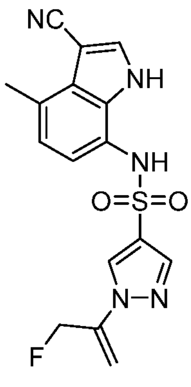

Step A: Preparation of [2-fluoro-1-(fluoromethyl)ethyl] methanesulfonate: Methanesulfonyl chloride (0.018 mL,

0.24 mmol) was added to an ice-cold solution of triethylamine (0.55 mL, 0.40 mmol) and 1,3-difluoro-2-propanol (0.012 mL, 0.16 mmol) in dichloromethane (1 mL). The mixture was stirred in ice. After 45 min the reaction mixture was diluted with DCM, washed with saturated aqueous $NaHCO_3$, dried over $MgSO_4$, filtered, and evaporated to afford [2-fluoro-1-(fluoromethyl)ethyl] methanesulfonate (20.8 mg,0.12 mmol, 72% yield). Used as is in the next step.

Step B: Preparation of N-(3-cyano-4-methyl-1H-indol-7-yl)-1-[1-(fluoromethyl)vinyl]pyrazole-4-sulfonamide: A vial containing [2-fluoro-1-(fluoromethyl)ethyl] methanesulfonate (20.8 mg, 0.12 mmol), N-(3-cyano-4-methyl-1H-indol-7-yl)-1H-pyrazole-4-sulfonamide (36.0 mg, 0.12 mmol), and cesium carbonate (77.9 mg, 0.24 mmol) in DMF (1 mL) was heated in a microwave at 130 °C for 30min. The reaction mixture was partitioned between EtOAc and water. The EtOAc was washed with brine, dried over $MgSO_4$, filtered, and evaporated. The residue was chromatographed on a Biotage 10 g ultra SNAP column with a 20% to 100% EtOAc:hexane gradient. Product-containing fractions were evaporated to afford N-(3-cyano-4-methyl-1H-indol-7-yl)-1-[1-(fluoromethyl)vinyl]pyrazole-4-sulfonamide (4.0 mg, 0.011 mmol, 9% yield) as a solid. $^1H$ NMR (400 MHz, $CD_3OD$): δ 8.16 (s, 1H), 7.93 (s, 1H), 7.70 (s, 1H), 6.84 (d, 1H), 6.63 (d, 1H), 5.74-5.72 (m, 1H), 5.31-5.29 (m, 1H), 5.29 (d, 2H), 2.68 (s, 3H). *m/z* (ES-API-pos) [M+1] 360.

**Example 14:** Synthesis of N-(3,4-dichloro-1H-indol-7-yl)-1-methyl-pyrazole-4-sulfonamide **(Compound 81).**

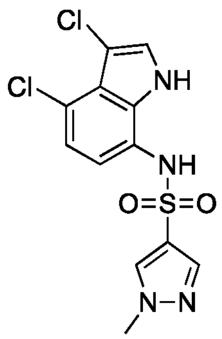

Step A: Preparation of *tert*-Butyl N-(4-chloro-1H-indol-7-yl)carbamate: To a stirred solution of 4-chloro-1H-indole-7-carboxylic acid (721 mg, 3.69 mmol) in toluene (18 mL) were added triethylamine (2.06 mL, 14.7 mmol) and diphenylphosphoryl azide (0.91 mL, 4.8 mmol). The reaction mixture was stirred at ambient temperature for 30 min. *tert*-Butanol (0.78 mL, 8.1 mmol) was added to the reaction. The mixture was heated at 100 °C for 3 hours. After cooling, the reaction was concentrated under reduced pressure. The residue was partitioned between EtOAc and water. The organic layer was washed with water and brine, dried and concentrated. The residue was purified by flash column chromatography on silica gel (10-40% EtOAc/hexanes) to give *tert*-butyl N-(4-chloro-1H-indol-7-yl)carbamate (470 mg, 48% yield). *m/z* (ES-API-pos) [M+1] 267.

Step B: Preparation of *tert*-Butyl N-(3,4-dichloro-1H-indol-7-yl)carbamate: To a stirred solution of *tert*-butyl N-(4-chloro-1H-indol-7-yl)carbamate (0.043 g, 0.16 mmol) in THF (1.6 mL) was added 1N HCl (50 □L) followed by N-chlorosuccinamide (0.024 g, 0.18 mmol). The reaction mixture was stirred at ambient temperature for 1 hour. The reaction was partitioned between EtOAc and water. The organic layer was washed with saturated aqueous $NaHCO_3$ solution and brine, dried and concentrated. The residue was purified by flash column chromatography on silica gel (10-30% EtOAc/hexanes) to give *tert*-butyl N-(3,4-dichloro-1H-indol-7-yl)carbamate (0.038 g, 78% yield). *m/z* (ES-API-pos) [M+1] 301.

Step C: Preparation of 3,4-dichloro-1H-indol-7-amine: To a stirred solution of *tert*-butyl N-(3,4-dichloro-1H-indol-7-yl)carbamate (0.038 g, 0.13mmol) in DCM (0.4687mL) was added trifluoroacetic acid (0.097 mL, 1.3 mmol). The reaction mixture was stirred at ambient temperature for 1 hour. The reaction was concentrated under reduced pressure. The residue was dissolved in EtOAc, washed with saturated aqueous NaHCOs solution, water and brine, dried and concentrated to afford 3,4-dichloro-1H-indol-7-amine, which was used in the next step without further purification. *m/z* (ES-API-pos) [M+1] = 201.

Step D: Preparation of N-(3,4-dichloro-1H-indol-7-yl)-1-methyl-pyrazole-4-sulfonamide: A mixture of 3,4-dichloro-1H-indol-7-amine (0.010 g, 0.05 mmol) and 1-methyl-1H-pyrazole-4-sulfonyl chloride (0.009 g, 0.05 mmol) in pyridine (0.5 mL) was stirred at ambient temperature for 30 min. The mixture was concentrated under reduced pressure. The residue was purified by flash column chromatography on silica gel (1-5% MeOH/DCM) to give N-(3,4-dichloro-1H-indol-7-yl)-1-methyl-pyrazole-4-sulfonamide (0.010 g, 58% yield). $^1H$ NMR (400 MHz, $(CD_3)_2CO$): δ 10.64 (s, 1H), 8.74 (s, 1H), 7.92 (s, 1H), 7.58 (s, 1H), 7.51 (s, 1H), 6.99 (d, 1H), 6.85 (d, 1H), 3.86 (s, 3H). *m/z* (ES-API-pos) [M+1] 345.

**Example 15:** Synthesis of N-(3-Cyano-4-methyl-1H-indol-7-yl)-1-ethyl-5-fluoro-pyrazole-4-sulfonamide **(Compound 102).**

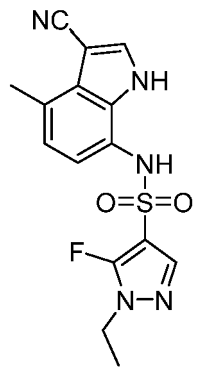

N-(3-Cyano-4-methyl-1H-indol-7-yl)-1-ethyl-5-fluoro-pyrazole-4-sulfonamide was prepared as described in Example 1, substituting 1-ethyl-5-fluoro-pyrazole-4-sulfonyl chloride for 1-methylpyrazole-4-sulfonyl chloride and 7-amino-4-methyl-1H-indole-3-carbonitrile for 3-chloro-1H-indol-7-amine. $^1H$ NMR (400 MHz, $CD_3OD$): δ 7.94 (s, 1H), 7.49 (br s, 1H), 6.88 (d, 1H), 6.68 (d, 1H), 4.03 (q, 2H), 2.68 (s, 3H), 1.33 (t, 3H). *m/z* (ES-API-pos) [M+1] 348.

**Example 16:** Synthesis of N-(3-Cyano-4-methyl-1H-indol-7-yl)-1-(3-methyloxetan-3-yl)pyrazole-4-sulfonamide (Compound 38).

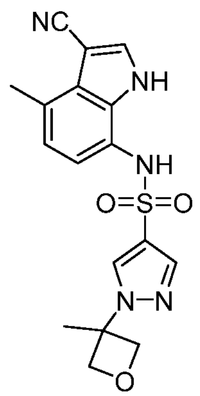

Step A: Preparation of tert-Butyl N-[(3-methyloxetan-3-yl)amino]carbamate: tert-Butyl 3-(4-cyanophenyl)oxaziridine-2-carboxylate (148.4 mg, 0.60 mmol) was added to a solution of 3-methyloxetan-3-amine (50.0 mg, 0.57 mmol) in diethyl ether (2 mL). The mixture was stirred at room temperature for 4h. The reaction mixture was evaporated and chromatographed on a Biotage 10 g ultra SNAP column with a 5% to 100% EtOAc:hexane gradient. Product-containing fractions were evaporated to afford tert-butyl N-[(3-methyloxetan-3-yl)amino]carbamate (47.7 mg, 0.21 mmol, 37% yield).

Step B: Preparation of [(3-Methyloxetan-3-yl)amino]ammonium; 2,2,2-trifluoroacetate: Trifluoroacetic acid (1 mL, 13.5 mmol) was added to a solution of tert-butyl N-[(3-methyloxetan-3-yl)amino]carbamate (15.9 mg, 0.079 mmol) in dichloromethane (1 mL). The mixture was stirred at room temperature and monitored by TLC (silica gel; 2:1 hexane:EtOAc; iodine development). After 1.25 h, the reaction mixture was evaporated without heat to afford [(3-methyloxetan-3-yl)amino]ammonium; 2,2,2-trifluoroacetate (16 mg, 0.074 mmol, 94% yield). Used as is in the next reaction.

Step C: Preparation of 4-Benzylsulfanyl-1-(3-methyloxetan-3-yl)pyrazole: A vial containing [(3-methyloxetan-3-yl)amino]ammonium; 2,2,2-trifluoroacetate (16.0 mg, 0.074 mmol) and 2-benzylsulfanylpropanedial (14.4 mg, 0.074 mmol) in methanol (1 mL) was heated at 70 °C overnight. The reaction mixture was partitioned between EtOAc and water. The EtOAc was washed with brine, dried over MgSO4, filtered, and evaporated to afford 4-benzylsulfanyl-1-(3-methyloxetan-3-yl)pyrazole (18.4 mg, 0.0707 mmol, 96% yield). m/z (ES-API-pos) [M+1] 261.

Step D: Preparation of 1-(3-Methyloxetan-3-yl)pyrazole-4-sulfonyl chloride: A solution of crude 4-benzylsulfanyl-1-(3-methyloxetan-3-yl)pyrazole (18.4 mg, 0.0707 mmol) in acetic acid (1 mL) and water (0.1 mL) was treated with N-chlorosuccinimide (23.6 mg, 0.18 mmol). After 15 min, the reaction mixture was concentrated and the residue was chromatographed on a Biotage 10 g ultra SNAP column with a 5% to 100% EtOAc:hexane gradient. Product-containing fractions were evaporated to afford 1-(3-methyloxetan-3-yl)pyrazole-4-sulfonyl chloride (10.0 mg, 0.0423 mmol, 60% yield). m/z (ES-API-pos) [M+1] 237, 239.

Step E: Preparation of N-(3-cyano-4-methyl-1H-indol-7-yl)-1-(3-methyloxetan-3-yl)pyrazole-4-sulfonamide: A flask was charged with 7-amino-4-methyl-1H-indole-3-carbonitrile (7.0 mg, 0.041 mmol) and 1-(3-methyloxetan-3-yl)pyrazole-4-sulfonyl chloride (9.68 mg, 0.041 mmol). Pyridine (1 mL) was added and the mixture was stirred at ambient temperature. After 30 min, the reaction mixture was concentrated and partitioned between EtOAc and water. The EtOAc was washed with brine, dried over MgSO4, filtered, and evaporated. The residue was absorbed on silica gel and chromatographed on a Biotage 10 g ultra SNAP column with a 20% to 100% EtOAc:hexane gradient. Product-containing fractions were evaporated to afford N-(3-cyano-4-methyl-1H-indol-7-yl)-1-(3-methyloxetan-3-yl)pyrazole-4-sulfonamide (13 mg, 0.033 mmol, 81% yield). 1HNMR (400 MHz, CD3OD): δ 7.93 (s, 1H), 7.91 (s, 1H), 7.70 (s, 1H), 6.84 (d, 1H), 6.61 (d, 1H), 4.98 (d, 2H), 4.61 (d, 2H), 2.68 (s, 3H), 1.79 (s, 3H). m/z (ES-API-pos) [M+1] 372.

**Example 17:** Synthesis of N-(3-Cyano-4-methyl-1H-indol-7-yl)-1-(2-hydroxy-2-methyl-propyl)pyrazole-4-sulfonamide **(Compound 108).**

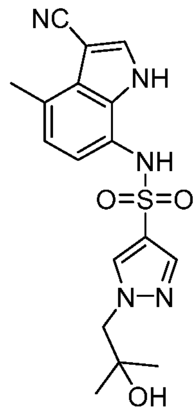

N-(3-Cyano-4-methyl-1H-indol-7-yl)-1-(2-hydroxy-2-methyl-propyl)pyrazole-4-sulfonamide was prepared as described in Example 16, substituting 1-amino-2-methyl-2-propanol for 3-methyloxetan-3-amine in Step A. $^1$H NMR (400 MHz, $CD_3OD$): δ 7.91 (s, 1H), 7.81 (s, 1H), 7.56 (s, 1H), 6.82 (d, 1H), 6.65 (d, 1H), 4.03 (d, 2H), 3.32-3.29 (m, 1H), 2.66 (s, 3H), 1.06 (s, 6H). *m/z* (ES-API-pos) [M+1] 374.

**Example 18:** Synthesis of N-(3-Cyano-4-methyl-1H-indol-7-yl)-1-(trideuteriomethyl)pyrazole-4-sulfonamide **(Compound 110).**

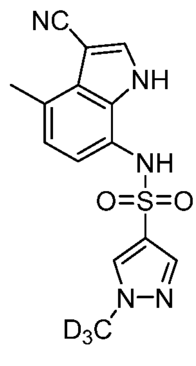

N-(3-Cyano-4-methyl-1H-indol-7-yl)-1-(trideuteriomethyl)pyrazole-4-sulfonamide was prepared similarly to Example 3, substituting 2-(methyl-d3)hydrazin-1-ium hydrogen sulfate for 2-hydrazinylethanol in Step A. $^1$H NMR (400 MHz, $CD_3OD$): δ 7.93 (s, 1H), 7.81 (s, 1H), 7.57 (s, 1H), 6.83 (d, 1H), 6.63 (d, 1H), 2.67 (s, 3H). *m/z* (ES-API-pos) [M+1] 319.

**Example 19:** Synthesis of N-(3,4-Dichloro-1H-indol-7-yl)-1-(3-methyloxetan-3-yl)pyrazole-4-sulfonamide **(Compound 115).**

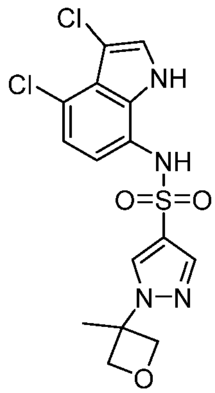

Step A: Preparation of Diethyl 2-(4-iodopyrazol-1-yl)-2-methyl-propanedioate: Potassium carbonate (1425 mg, 10.3 mmol) was added to a vial containing a solution of 4-iodo-1H-pyrazole (1000 mg, 5.16 mmol) in DMF (4 mL). The mixture was stirred at room temperature for 2 min, then treated with 2-bromo-2-methyl-propanedioic acid- 1,3-diethyl ester (1.18 mL, 6.19 mmol). The sealed vial was heated at 100 °C. After 25 min, the reaction mixture was partitioned between EtOAc and water. The EtOAc was washed with water, brine, dried over $MgSO_4$, filtered, and evaporated. The residue was chromatographed on a Biotage 50 g ultra SNAP column with a 0% to 40% EtOAc:hexane gradient. Product-containing fractions were evaporated to afford diethyl 2-(4-iodopyrazol-1-yl)-2-methyl-propanedioate (1773 mg, 4.60 mmol, 89% yield). *m/z* (ES-API-pos) [M+1] 367.

Step B: Preparation of 2-(4-Iodopyrazol-1-yl)-2-methyl-propane-1,3-diol: Sodium borohydride (365.8 mg, 9.67 mmol) was added portionwise over 5 min to a solution of diethyl 2-(4-iodopyrazol-1-yl)-2-methyl-propanedioate (1770 mg, 4.83 mmol) in methanol (20 mL) cooled in an ice-bath. The mixture was stirred at ambient temperature for 30 min. The reaction mixture was concentrated and partitioned between EtOAc and water. The EtOAc was washed with brine, dried over MgSO4, filtered, and evaporated to afford 2-(4-iodopyrazol-1-yl)-2-methyl-propane-1,3-diol (1070 mg, 3.79 mmol, 79% yield). Used as is. *m/z* (ES-API-pos) [M+1] 283.

Step C: Preparation of [3-Hydroxy-2-(4-iodopyrazol-1-yl)-2-methyl-propyl] 4-methylbenzenesulfonate: p-Toluenesulfonyl chloride (723 mg, 3.79 mmol) was added to a vial containing a solution of 2-(4-iodopyrazol-1-yl)-2-methyl-propane-1,3-diol (1070 mg, 3.79 mmol) in pyridine (12 mL). The sealed vial was stirred at RT for 1.25 h. The reaction mixture was concentrated and chromatographed on a Biotage 50 g ultra SNAP column with a 10% to 100% EtOAc:hexane gradient. Product-containing fractions were evaporated to afford [3-hydroxy-2-(4-iodopyrazol-1-yl)-2-methyl-propyl] 4-methylbenzenesulfonate (569 mg, 1.24 mmol, 32.7% yield). *m/z* (ES-API-pos) [M+1] 437; [M+23] 459.

Step D: Preparation of 4-Iodo-1-(3-methyloxetan-3-yl)pyrazole: 60% Sodium hydride (62.5 mg, 1.56 mmol) was added portion-wise to an ice-cold solution of [3-hydroxy-2-(4-iodopyrazol-1-yl)-2-methyl-propyl] 4-methylbenzenesulfonate (568 mg, 1.3 mmol) in THF (5 mL). The vial was stirred at room temperature. After gas evolution ceased, the sealed vial was vented, and then heated at 65 °C 1.5 h. The reaction mixture was treated with ~2 mL water and concentrated. The residue was chromatographed on a Biotage 25 g ultra SNAP column with a 10% to 100% EtOAc:hexane gradient. Product-containing fractions were evaporated to afford 4-iodo-1-(3-methyloxetan-3-yl)pyrazole (246 mg, 0.932 mmol, 72% yield). *m/z* (ES-API-pos) [M+1] 265.

Step E: Preparation of 4-Benzylsulfanyl-1-(3-methyloxetan-3-yl)pyrazole: A vial was charged with 4-iodo-1-(3-methyloxetan-3-yl)pyrazole (100 mg, 0.38 mmol), THF (2 mL), benzyl mercaptan (0.044 mL, 0.38 mmol), and triethylamine (0.10 mL, 0.76 mmol). The mixture was sparged with nitrogen and treated with palladium, [2'-(amino-.kappa.N)[1,1'-biphenyl]-2-yl-.kappa.C][[5-(diphenylphosphino)-9,9-dimethyl-9H-xanthen-4-yl]diphenylphosphine-.kappa.P](methanesulfonato-.kappa.O)- (Xantphos Palladacycle Gen. 3, 26.9 mg, 0.0284 mmol). The sealed vial was heated at 70 °C overnight. The reaction mixture was partitioned between EtOAc and water. The EtOAc was washed with brine, dried over $MgSO_4$, filtered, and evaporated. The residue was chromatographed on a Biotage 10 g ultra SNAP column with a 10% to 80% EtOAc:hexane gradient. Product-containing fractions were evaporated to afford 4-benzylsulfanyl-1-(3-methyloxetan-3-yl)pyrazole (53.7 mg, 0.196 mmol, 52% yield). *m/z* (ES-API-pos) [M+1] 261.

Step F: Preparation of N-(3,4-dichloro-1H-indol-7-yl)-1-(3-methyloxetan-3-yl)pyrazole-4-sulfonamide: A solution of 4-benzylsulfanyl-1-(3-methyloxetan-3-yl)pyrazole (53.7mg, 0.196 mmol) in acetic acid (1.5 mL) and water (0.15 mL) was treated with N-chlorosuccinimide (68.9 mg, 0.52 mmol). After 7 h, the reaction mixture was concentrated and the residue was chromatographed on a Biotage 10 g ultra SNAP column with a 5% to 100% EtOAc:hexane gradient. Product-containing fraction were evaporated to afford 1-(3-methyloxetan-3-yl)pyrazole-4-sulfonyl chloride (11.3 mg, 0.0358 mmol, 17% yield. *m/z* (ES-API-pos) [M+1] 237, 239.

Step G: Preparation of N-(3,4-dichloro-1H-indol-7-yl)-1-(3-methyloxetan-3-yl)pyrazole-4-sulfonamide: A flask was charged with 3,4-dichloro-1H-indol-7-amine (7.0 mg, 0.035 mmol) and 1-(3-methyloxetan-3-yl)pyrazole-4-sulfonyl chloride (8.24 mg, 0.035 mmol). Pyridine (1 mL) was added and the mixture was stirred at room temperature for 10 min. The reaction mixture was concentrated and partitioned between EtOAc and water. The EtOAc was washed with brine, dried over $MgSO_4$, filtered, and evaporated. The residue was chromatographed on a Biotage 10 g ultraSNAP column with a 20% to 100% EtOAc:hexane gradient. Product-containing fractions were evaporated to afford an impure product. This was rechromatographed on a Biotage 10 g ultra SNAP column with a 50% to 100% EtOAc:hexane gradient. Product-containing fractions were evaporated to afford N-(3,4-dichloro-1H-indol-7-yl)-1-(3-methyloxetan-3-yl)pyrazole-4-sulfonamide (3.7 mg, 0.0088 mmol, 25% yield). $^1H$ NMR (400 MHz, $CD_3OD$): δ 7.94 (s, 1H), 7.73 (s, 1H), 7.29 (s, 1H), 6.92 (d, 1H), 6.66 (d, 1H), 4.98 (d, 2H), 4.60 (d, 2H), 1.78 (s, 3H). *m/z* (ES-API-pos) [M+1] 401, 403.

**Example 20:** Synthesis of N-(4-chloro-3-cyano-1H-indol-7-yl)-1-(oxetan-3-yl)pyrazole-4-sulfonamide **(Compound 125).**

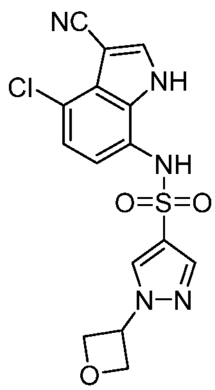

N-(4-chloro-3-cyano-1H-indol-7-yl)-1-(oxetan-3-yl)pyrazole-4-sulfonamide was prepared as described for Example 5, substituting N-(4-chloro-3-cyano-1H-indol-7-yl)-1H-pyrazole-4-sulfonamide for N-(3-chloro-1H-indol-7-yl)-1H-pyrazole-4-sulfonamide. $^1H$ NMR (400 MHz, $CD_3OD$): δ 8.04 (s, 1H), 7.90 (s, 1H), 7.77 (s, 1H), 7.07 (d, 1H), 6.73 (d, 1H), 5.54-5.46 (m, 1H), 5.00-4.91 (m, 4H). *m/z* (ES-API-pos) [M+1] 378, 380.

**Example 21:** Synthesis of N-(4-chloro-3-cyano-1H-indol-7-yl)-1-(2-hydroxy-2-methyl-propyl)pyrazole-4-sulfonamide **(Compound 136)**

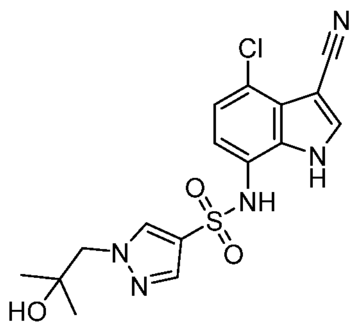

Step A: 1-(4-benzylsulfanylpyrazol-1-yl)-2-methyl-propan-2-ol: A vial 2-(2-hydroxy-2-methylpropyl)hydrazin-1-ium chloride (60 mg, 0.43 mmol) and 2-benzylsulfanylpropanedial (82.9 mg, 0.43 mmol) in methanol (2 mL) was heated at 70 °C overnight. The reaction mixture was partitioned between EtOAc and water. The EtOAc was washed with brine, dried over MgSO4, filtered, and evaporated to afford 1-(4-benzylsulfanylpyrazol-1-yl)-2-methyl-propan-2-ol (95 mg, 0.362 mmol, 84.85% yield) as a beige oil. Used without purification in the next step. m/z (ES-API-pos) [M+1] 263.

Step B: 1-(2-hydroxy-2-methyl-propyl)pyrazole-4-sulfonyl chloride: A solution of crude 1-(4-benzylsulfanylpyrazol-1-yl)-2-methyl-propan-2-ol (95 mg, 0.362 mmol) in acetic acid (1.5 mL) and water (0.15 mL) was treated with N-chlorosuccinimide (120.9 mg, 0.91 mmol). After 10min, the reaction mixture was concentrated and the residue was chromatographed on a Biotage 10 g ultra SNAP column, eluting with a 10% to 100% EtOAc:hexane gradient. Product-containing fractions were evaporated to afford 1-(2-hydroxy-2-methyl-propyl)pyrazole-4-sulfonyl chloride (59.5 mg, 0.249 mmol, 68.85% yield). m/z (ES-API-pos) [M+1] 239, 241.

Step C: N-(4-chloro-3-cyano-1H-indol-7-yl)-1-(2-hydroxy-2-methylpropyl)pyrazole-4-sulfonamide: A flask was charged with 7-amino-4-chloro-1H-indole-3-carbonitrile (47.8 mg, 0.25 mmol) and 1-(2-hydroxy-2-methyl-propyl)pyrazole-4-sulfonyl chloride (59.5 mg, 0.25 mmol). Pyridine (1.5 mL) was added and the mixture was stirred at RT for 20 min. The reaction mixture was concentrated and partitioned between EtOAc and water. The EtOAc was washed with brine, dried over MgSO4, filtered, and evaporated. The residue was chromatographed on a Biotage 10 g ultra SNAP column with a 40% to 100% EtOAc:hexane gradient. Product-containing fractions were evaporated to afford N-(4-chloro-3-cyano-1H-indol-7-yl)-1-(2-hydroxy-2-methyl-propyl)pyrazole-4-sulfonamide (55.3 mg,0.133 mmol, 53.5 % yield). $^1$H NMR (400 MHz, $CD_3OD$): $\delta$ 8.03 (s, 1H), 7.84 (s, 1H), 7.61 (s, 1H), 7.07 (d, 1H), 6.73 (d, 1H), 4.87 (s, 2H), 1.07 (s, 6H). m/z (ES-API-pos) [M+1] 394, 396.

Alternative synthesis of Step A intermediate 1-(4-benzylsulfanylpyrazol-1-yl)-2-methyl-propan-2-ol:

Step A: 1-(4-Iodopyrazol-1-yl)-2-methyl-propan-2-ol: 1H-Pyrazole, 4-iodo- (10 g, 51.55 mmol), 1-chloro-2-methyl-propan-2-ol (11.2 g, 103.1 mmol) and $Cs_2CO_3$ (33.59 g, 103.1 mmol) were combined in DMF (100 mL). After stirring at 110 °C for 48h, the reaction mixture was diluted with EtOAc/hexanes (1:1) and washed with $H_2O$/brine, with back-extraction. The organic layer was dried over $MgSO_4$, concentrated and purified by silica gel chromatography using a 0-20% EtOAc in hexanes gradient to give the title compound 1-(4-iodopyrazol-1-yl)-2-methyl-propan-2-ol (10.1 g, 38 mmol, 73.6% yield).

Step B: 1-(4-Benzylsulfanylpyrazol-1-yl)-2-methyl-propan-2-ol: A mixture of 1-(4-iodopyrazol-1-yl)-2-methyl-propan-2-ol (10.56 g, 39.67 mmol), benzyl mercaptan (5.17 g, 41.66 mmol), $Pd_2(dba)_3$ (572.1 mg, 0.99 mmol), xantphos (908.2 mg, 0.99 mmol) and DIEA (5.12 g, 39.7 mmol) in 1,4-dioxane (10 mL) was stirred for 16 h at 100 °C under $N_2$. The mixture was filtered through a celite pad and the filtrate was concentrated to give a residue. The residue was taken up in DCM (300 ml) and the organics washed with water, then saturated brine solution. The organics were then separated and dried (MgSO4) before concentration to dryness. The crude was then purified by column chromatography. The desired fractions were concentrated to dryness in vacuo to afford 1-(4-benzylsulfanylpyrazol-1-yl)-2-methyl-propan-2-ol (6g, 22.9 mmol, 57.6% yield).

**Example 22:** Synthesis of N-(3-cyano-4-fluoro-1H-indol-7-yl)-1-ethyl-pyrazole-4-sulfonamide **(Compound 217)**

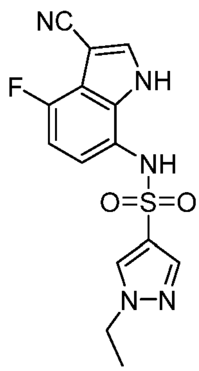

A flask was charged with 7-amino-4-fluoro-1H-indole-3-carbonitrile (35.5 mg, 0.203 mmol) and 1H-pyrazole-4-sulfonyl chloride, 1-ethyl- (39.4 mg, 0.203 mmol). Pyridine (1 mL) was added and the mixture was stirred at RT. After 15 min, the reaction mixture was evaporated and treated with EtOAc and water. A ppt formed and was collected by vacuum filtration and dried to afford N-(3-cyano-4-fluoro-1H-indol-7-yl)-1-ethyl-pyrazole-4-sulfonamide (18.5 mg, 0.0527 mmol, 26% yield). $^1$H NMR (400 MHz, $CD_3OD$): $\delta$ 7.98 (s, 1H), 7.82 (s, 1H), 7.61 (s, 1H), 6.79 (dd, 1H), 6.66 (dd, 1H),

4.13 (q, 2H), 1.38 (t, 3H). m/z (ES-API-pos) [M+1] = 334.

**Example 23:** Synthesis of N-(3-cyano-4-methyl-1H-indol-7-yl)-1-(2-hydroxy-1,1-dimethyl-ethyl)pyrazole-4-sulfonamide **(Compound 198)**

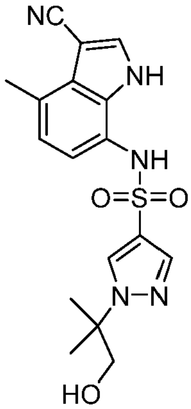

Step A: Preparation of 3 methyl 2-[4-[(3-cyano-4-methyl-1H-indol-7-yl)sulfamoyl]pyrazol-1-yl]-2-methyl-propanoate: Cesium carbonate (307.1 mg, 0.94 mmol) was added to a vial containing a solution of propanoic acid, 2-bromo-2-methyl-, methyl ester (0.064 mL, 0.49 mmol) and N-(3-cyano-4-methyl-1H-indol-7-yl)-1H-pyrazole-4-sulfonamide (142 mg, 0.47 mmol) in DMF (3 mL). The sealed vial was heated at 70 °C. After 1.5h, the reaction mixture was partitioned between EtOAc and water. The EtOAc was washed with brine, dried over $MgSO_4$, filtered, and evaporated. The residue was chromatographed on a Biotage 10 g ultra SNAP column with a 30% to 100% EtOAc:hexane gradient. Product-containing fractions were evaporated to afford methyl 2-[4-[(3-cyano-4-methyl-1H-indol-7-yl)sulfamoyl]pyrazol-1-yl]-2-methyl-propanoate (126 mg,0.314 mmol, 66.6% yield). *m/z* (ES-API-pos) [M+1] = 401.9.

Step B: Preparation of N-(3-cyano-4-methyl-1H-indol-7-yl)-1-(2-hydroxy-1,1-dimethyl-ethyl)pyrazole-4-sulfonamide: Sodium borohydride (83.1 mg, 2.2 mmol) was added portion-wise over 30min to an ice-cold suspension of methyl 2-[4-[(3-cyano-4-methyl-1H-indol-7-yl)sulfamoyl]pyrazol-1-yl]-2-methyl-propanoate (126 mg, 0.314 mmol) in methanol (4 mL). Considerable gas evolution occured. Additional portions of $NaBH_4$ were added until the starting material was consumed. The suspension slowly became a solution. The reaction mixture was evaporated and treated with water. The mixture was neutralized with 1M HCl and partitioned between EtOAc and water. The EtOAc was washed with brine, dried over $MgSO_4$, filtered, and evaporated. The residue was chromatographed on a Biotage 10 g ultra SNAP column with a 40% to 100% EtOAc:hexane gradient. Product-containing fractions were evaporated to afford N-(3-cyano-4-methyl-1H-indol-7-yl)-1-(2-hydroxy-1,1-dimethylethyl)pyrazole-4-sulfonamide (93.4 mg,0.238 mmol, 75.7% yield). $^1H$ NMR (400 MHz, $CD_3OD$): δ 8.03 (s, 1H), 7.84 (s, 1H), 7.61 (s, 1H), 7.07 (d, 1H), 6.73 (d, 1H), 4.04 (s, 2H), 1.07 (s, 6H). *m/z* (ES-API-pos) [M+1] = 374.

**Example 24:** Synthesis of N-(3-cyano-4-methyl-1H-indol-7-yl)-1-[(1S)-1-(fluoromethyl)-2-hydroxy-ethyl]pyrazole-4-sulfonamide **(Compound 246)**

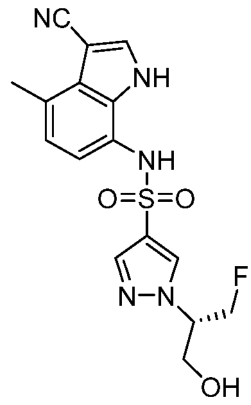

Step A: Preparation of (2R)-1-[tert-butyl(dimethyl)silyl]oxy-3-fluoro-propan-2-ol: Tert-Butyldimethylsily chloride (1.60 g, 10.6 mmol) was added to a solution of (2R)-3-fluoropropane-1,2-diol (1.00 g, 10.6 mmol), triethylamine (1.93 mL, 13.8 mmol) and 4-(dimethylamino)pyridine (0.060 g, 0.53 mmol) in dichloromethane (35 mL) at 0 °C and the reaction mixture was allowed to warm to room temperature and stirred at room temperature for 16 hours. The reaction mixture was diluted with water and extracted with dichloromethane. The combined organic fractions were washed with brine, dried over sodium sulfate, filtered and concentrated. The residue was purified by column chromatography on silica gel (2-20% EtOAc/hexanes) to give (2R)-1-[tert-butyl(dimethyl)silyl]oxy-3-fluoro-propan-2-ol (1.50 g, 68% yield). $^1$H NMR (400 MHz, $CDCl_3$): δ 4.46-4.53 (m, 1H), 4.34-4.42 (m, 1H), 3.84-3.95 (m, 1H), 3.65-3.72 (m, 2H), 2.39 (d, 1H), 0.96 (s, 6H), 0.08 (s, 9H).

Step B: Preparation of [(1R)-1-[[tert-butyl(dimethyl)silyl]oxymethyl]-2-fluoroethyl] methanesulfonate: Methanesulfonyl chloride (0.26 mL, 3.33 mmol) was added dropwise to a solution of (2R)-1-[tert-butyl(dimethyl)silyl]oxy-3-fluoro-propan-2-ol (578 mg, 2.77 mmol) and triethylamine (0.58 mL, 4.2 mmol) in dichloromethane (14 mL) at 0 °C under nitrogen. The reaction mixture stirred at 0 °C for 1 hour. The resultant mixture was diluted with DCM, washed with 0.5 N aqueous HCl and brine, dried over sodium sulfate and evaporated in vacuo. The crude was used in the next step directly.

Step C: Preparation of (2S)-3-fluoro-2-(4-iodopyrazol-1-yl)propan-1-ol: A mixture of 4-iodo-1H-pyrazole (538 mg, 2.78 mmol), [(1R)-1-[[tert-butyl(dimethyl)silyl]oxymethyl]-2-fluoro-ethyl] methanesulfonate (795 mg, 2.78 mmol) and cesium carbonate (1.36 g, 4.16 mmol) in DMA (9 mL) was heated at 90 °C for 3 hours. After cooling, the reaction mixture was partitioned between EtOAc and water. The aqueous layer was extracted with EtOAc. The combined organic layers were washed with water and brine, dried and concentrated. The residue was purified by column chromatography on silica gel (20-60% EtOAc/hexanes) to give (2S)-3-fluoro-2-(4-iodopyrazol-1-yl)propan-1-ol (298 mg, 40% yield). *m/z* (ES-API-pos) [M+1] = 271.

Step D: Preparation of (2S)-2-(4-benzylsulfanylpyrazol-1-yl)-3-fluoro-propan-1-ol: To a mixture of (2S)-3-fluoro-2-(4-iodopyrazol-1-yl)propan-1-ol (0.298 g, 1.10 mmol) and benzyl mercaptan (0.17 mL, 0.43 mmol) in 1,4-Dioxane (4 mL) were added N,N-diisopropylethylamine (0.58 mL, 3.3 mmol), tris(dibenzylideneacetone)dipalladium(0) (50 mg, 0.055 mmol) and 9,9-dimethyl-4;5-bis(diphenyl-phosphino)xanthene (64 mg, 0.11 mmol) under nitrogen. The reaction mixture was heated at reflux for 2 hours. After cooling, the reaction mixture was partitioned between EtOAc and water. The aqueous layer was acidified and extracted with EtOAc. The combined organic layers were washed with water and brine, dried and concentrated. The residue was purified by column chromatography on silica gel (20-60% EtOAc/hexanes) to give (2S)-2-(4-benzylsulfanylpyrazol-1-yl)-3-fluoro-propan-1-ol (0.213 g, 72% yield). *m/z* (ES-API-pos) [M+1] = 267.

Step E: Preparation of 1-[(1S)-1-(fluoromethyl)-2-hydroxy-ethyl]pyrazole-4-sulfonyl chloride: To a stirred solution of (2S)-2-(4-benzylsulfanylpyrazol-1-yl)-3-fluoro-propan-1-ol (213 mg, 0.800 mmol) in acetic acid (6 mL) and water (1 mL) was added N-chlorosuccinimide (342 mg, 2.56 mmol). The reaction mixture was stirred at ambient temperature for 1 hour. The reaction mixture was diluted with water and extracted with ethyl acetate. The combined organics were dried, concentrated and used directly in the next step. *m/z* (ES-API-pos) [M+1] = 243.

Step F: Preparation of N-(3-cyano-4-methyl-1H-indol-7-yl)-1-[(1S)-1-(fluoromethyl)-2-hydroxy-ethyl]pyrazole-4-sulfonamide: A mixture of 7-amino-4-methyl-1H-indole-3-carbonitrile (32 mg, 0.19 mmol) and 1-[(1S)-1-(fluoromethyl)-2-hydroxyethyl]pyrazole-4-sulfonyl chloride (45 mg, 0.19 mmol) in pyridine (1.8 mL) was stirred at ambient temperature for 30 min. The mixture was concentrated. The residue was purified by column chromatography on silica gel (1-5% DCM/MeOH) to give a crude product, which was further purified by flash chromatography on reverse phase Biotage C18 column (5-95% MeCN/water) to give N-(3-cyano-4-methyl-1H-indol-7-yl)-1-[(1S)-1-(fluoromethyl)-2-hydroxyethyl]pyrazole-4-sulfonamide (45 mg, 64% yield). $^1$H NMR (400 MHz, $(CD_3)_2CO$): δ 8.09 (s, 1H), 7.92 (s, 1H), 7.55 (s, 1H), 6.88 (d, 1H), 6.81 (d, 1H), 4.09-4.48 (m, 5H), 2.68 (s, 3H). *m/z* (ES-API-pos) [M+1] = 378.

**Example 25:** Synthesis of N-(3-cyano-4-methyl-1H-indol-7-yl)-1-[1-(hydroxymethyl)cyclopropyl]pyrazole-4-sulfonamide **(Compound 268)**

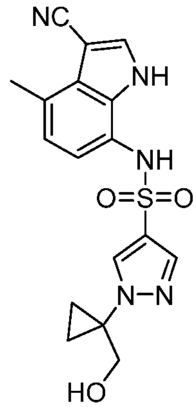

Step A: Preparation of methyl 1-(4-iodopyrazol-1-yl)cyclopropanecarboxylate: To a stirred suspension of sodium hydride (60 % dispersion in mineral oil, 0.619 g, 15.5 mmol) in DMF (20 mL) was added dropwise a solution of 4-iodo-1H-Pyrazole (2.00 g, 10.3 mmol) in DMF (10 mL) at 0 °C under nitrogen. The reaction mixture was stirred at 0 °C for 30 min. A solution of methyl 2,3-dibromopropanoate (2.54 g, 10.3 mmol) in DMF (5 mL) was added dropwise. The reaction mixture was allowed to warm to ambient temperature and stirred overnight. The reaction was quenched by water and extracted with EtOAc. The combined organic layers were washed with water and brine, dried and concentrated. The residue was purified by column chromatography on silica gel (10-50% EtOAc/hexanes) to give methyl 1-(4-iodopyrazol-1-yl)cyclopropanecarboxylate (0.64 g, 21% yield). *m/z* (ES-API-pos) [M+1] = 293.

Step B: Preparation of methyl 1-(4-benzylsulfanylpyrazol-1-yl)cyclopropanecarboxylate: To a mixture of methyl 1-(4-iodopyrazol-1-yl)cyclopropanecarboxylate (0.76 g, 2.6 mmol) and benzyl mercaptan (0.34 mL, 2.9 mmol) in 1,4-dioxane (8.7 mL) were added N,N-diisopropylethylamine (1.36 mL, 7.81 mmol), tris(dibenzylideneacetone)dipalladium(0) (119 mg, 0.130 mmol) and 9,9-dimethyl-4;5-bis(diphenyl-phosphino)xanthene (151 mg, 0.260 mmol) under nitrogen. The reaction mixture was heated at reflux for 3 hours. After cooling, the reaction mixture was paritioned between EtOAc and water. The aqueous layer was acidified and extracted with EtOAc. The combined organic layers were washed with water and brine, dried and concentrated. The residue was purified by column chromatography on silica gel (10-50% EtOAc/hexanes) to give methyl 1-(4-benzylsulfanylpyrazol-1-yl)cyclopropanecarboxylate (0.672 g, 90% yield). *m/z* (ES-API-pos) [M+1] = 289.

Step C: Preparation of [1-(4-benzylsulfanylpyrazol-1-yl)cyclopropyl]methanol: To a stirred solution of methyl 1-(4-benzylsulfanylpyrazol-1-yl)cyclopropanecarboxylate (460 mg, 1.60 mmol) in DCM (8 mL) was added dropwise a 1N solution of diisobutylalumane in hexanes (4.79 mL, 4.79 mmol) at 0 °C under nitrogen. The reaction mixture was stirred at 0 °C for 30 min, and then allowed to warm to ambient temperature and stir for 1 hour. The reaction was cooled in an ice-water bath and quenched with saturated aqueous Rochelle's salt. After stirring for 30 min, the organic layer was separated, washed with water and brine, dried and concentrated. The crude was used in the next step without further purification. *m/z* (ES-API-pos) [M+1] = 261.

Step D: Preparation of N-(3-cyano-4-methyl-1H-indol-7-yl)-1-[1-(hydroxymethyl)cyclopropyl]pyrazole-4-sulfonamide: N-(3-cyano-4-methyl-1H-indol-7-yl)-1-[1-(hydroxymethyl)cyclopropyl]pyrazole-4-sulfonamide was prepared as described in Example 246, Step E to Step F, substituting [1-(4-benzylsulfanylpyrazol-1-yl)cyclopropyl]methanol for (2S)-2-(4-benzylsulfanylpyrazol-1-yl)-3-fluoro-propan-1-ol in Step E. $^1$H NMR (400 MHz, $(CD_3)_2CO$): δ 11.36 (s, 1H), 8.79 (s, 1H), 8.22 (s, 1H), 7.95 (s, 1H), 7.56 (s, 1H), 7.15 (d, 1H), 6.89 (d, 1H), 4.14 (br s, 1H), 3.72 (br s, 2H), 2.68 (s, 3H), 1.13-1.20 (m, 2H), 1.03-1.09 (m, 2H). *m/z* (ES-API-pos) [M+1] = 372.

**Example 26:** Synthesis of 5-amino-N-(3-cyano-4-methyl-1H-indol-7-yl)-1-ethyl-pyrazole-4-sulfonamide **(Compound 271).**

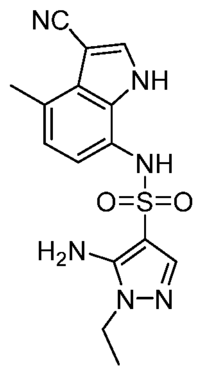

5-amino-N-(3-cyano-4-methyl-1H-indol-7-yl)-1-ethyl-pyrazole-4-sulfonamide was prepared as described in Example 1, substituting 5-amino-1-ethyl-pyrazole-4-sulfonyl chloride for 1-methylpyrazole-4-sulfonyl chloride and 7-amino-4-methyl-1H-indole-3-carbonitrile for 3-chloro-1H-indol-7-amine. $^1$H NMR (400 MHz, $(CD_3)_2CO$): δ 11.01 (s, 1H), 8.50 (s, 1H), 8.09 (s, 1H), 7.22 (s, 1H), 6.72-6.93 (m, 2H), 5.31 (s, 2H), 3.81-3.95 (m, 2H), 2.68 (s, 3H), 1.21 (t, 3H). m/z (ES-API-pos) [M+1] 345.

**Example 27:** Synthesis of 5-amino-N-(3-cyano-4-fluoro-1H-indol-7-yl)-1-ethyl-pyrazole-4-sulfonamide **(Compound 272).**

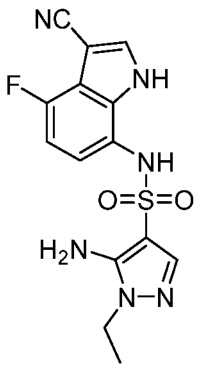

5-amino-N-(3-cyano-4-fluoro-1H-indol-7-yl)-1-ethyl-pyrazole-4-sulfonamide was prepared as described in Example 1, substituting 5-amino-1-ethyl-pyrazole-4-sulfonyl chloride for 1-methylpyrazole-4-sulfonyl chloride and 7-amino-4-fluoro-1H-indole-3-carbonitrile for 3-chloro-1H-indol-7-amine. $^1$H NMR (400 MHz, $(CD_3)_2CO$): δ 11.30 (s, 1H), 8.54 (s, 1H), 8.16 (s, 1H), 7.25 (s, 1H), 6.85-6.91 (m, 2H), 5.28 (s, 2H), 3.90 (q, 2H), 1.21 (t, 3H). m/z (ES-API-pos) [M+1] 349.

**Example 28:** Synthesis of 5-amino-N-(3-cyano-4-fluoro-1H-indol-7-yl)-1-methyl-pyrazole-4-sulfonamide **(Compound 282).**

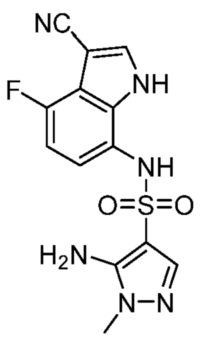

5-amino-N-(3-cyano-4-fluoro-1H-indol-7-yl)-1-methyl-pyrazole-4-sulfonamide was prepared as described in Example 1, substituting 5-amino-1-methyl-pyrazole-4-sulfonyl chloride for 1-methylpyrazole-4-sulfonyl chloride and 7-amino-4-fluoro-1H-indole-3-carbonitrile for 3-chloro-1H-indol-7-amine. $^1$H NMR (400 MHz, $(CD_3)_2CO$): δ 11.30 (s, 1H), 8.57 (s, 1H), 8.17 (s, 1H), 7.21 (s, 1H), 6.85-6.93 (m, 2H), 5.31 (s, 2H), 2.80 (s, 3H). m/z (ES-API-pos) [M+1] 335.

Example 29: Synthesis of *N*-(3-cyano-4-methyl-1*H*-indol-7-yl)-1-((1-cyanocyclopropyl)methyl)-1*H*-pyrazole-4-sulfonamide **(Compound 145)**

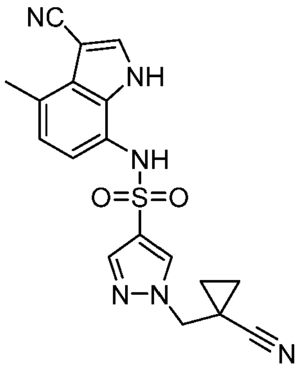

*N*-(3-cyano-4-methyl-1*H*-indol-7-yl)-1-((1-cyanocyclopropyl)methyl)-1*H*-pyrazole-4-sulfonamide was prepared similarly to **Example 12,** substituting *p*-Toluenesulfonyl chloride for methanesulfonyl chloride and 1-(Hydroxymethyl)cyclopropanecarbonitrile for 2,2-difluoro-cyclopropanemethanol in Step A. $^1$H NMR (400 MHz, $CD_3OD$) δ 7.90 (d, 2H), 7.65 (s, 1H), 6.83 (d, 1H), 6.64 (d, 1H), 4.22 (s, 2H), 2.66 (s, 3H), 1.34 - 1.27 (m, 3H), 1.21 - 1.16 (m, 2H). *m/z* (ES-API-neg) [M-1] = 379.

Example 30: Synthesis of (*R*)-*N*-(3-cyano-4-methyl-1*H*-indol-7-yl)-1-(1-hydroxypropan-2-yl)-1*H*-pyrazole-4-sulfonamide **(Compound 207)**

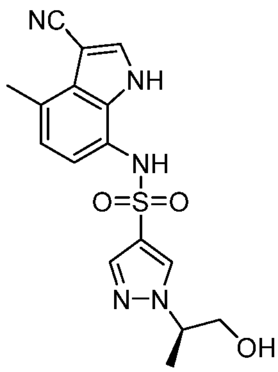

Step A: To a flame dried flask equipped with a stir bar was added tetrahydrofuran (54.36 mL), (S)-2-hydroxypropanoic acid methyl ester (0.62 mL, 6.52 mmol), 4-iodo-1H-pyrazole (1.08 mL, 5.44 mmol), and triphenylphosphine (2.85 g, 10.87 mmol). The reaction was cooled in an ice bath and azodicarboxylic acid diisopropyl ester (2.39 mL, 10.87 mmol) was added dropwise. Once that addition was complete, the reaction was warmed to 60 °C for 3 hours. The resulting solution was allowed to cool to ambient temperature and the solvent was removed under reduced pressure. The residue was chromatographed on a Biotage 10 g ultra SNAP column with a 5% to 25% EtOAc:hexane gradient. Product-containing fractions were evaporated to afford methyl (*R*)-2-(4-iodo-1*H*-pyrazol-1-yl)propanoate (933 mg, 3.33 mmol, 61% yield). *m/z* (ES-API-pos) [M+1] = 281.

Step B: Sodium borohydride (144 mg, 3.8 mmol) was added portionwise over five minutes to a solution of methyl (*R*)-2-(4-iodo-1*H*-pyrazol-1-yl)propanoate (820 mg, 2.93 mmol) in methanol (26 mL) cooling in an ice-bath. The mixture was allowed to warm to ambient temperature for 30 minutes and then the reaction was quenched with saturated

$NH_4Cl$. The residual MeOH was removed under reduced pressure and the resulting aqueous was then extracted 3x's with EtOAc. The combined organics were then dried over sodium sulfate, filtered and concentrated under reduced pressure to afford a crude product. The residue was chromatographed on a Biotage 10 g ultra SNAP column with a 25% to 85% EtOAc:hexane gradient. Product-containing fractions were evaporated to afford (*R*)-2-(4-iodo-1*H*-pyrazol-1-yl)propan-1-ol (529 mg, 2.1 mmol, 72% yield). *m*/*z* (ES-API-pos) [M+1] = 253.

Step C: A vial was charged with (*R*)-2-(4-iodo-1*H*-pyrazol-1-yl)propan-1-ol (266 mg, 0.1.1 mmol), dioxane (6 mL), benzyl mercaptan (0.117 mL, 1.0 mmol), and triethylamine (0.44 mL, 3.26 mmol) The mixture was sparged with nitrogen and treated with palladium, [2'-(amino-.kappa.N)[1,1'-biphenyl]-2-yl-.kappa.C][[5-(diphenylphosphino)-9,9-dimethyl-9H-xanthen-4-yl]diphenylphosphine-.kappa.P](methanesulfonato-.kappa.O)- (Xantphos Palladacycle Gen. 3, 50.0 mg, 0.053 mmol). The sealed vial was heated at 70 °C for 5 hours. The reaction mixture was then cooled to ambient temperature and concentrated under reduced pressure. The residue was chromatographed on a Biotage 10 g ultra SNAP column with a 10% to 75% EtOAc:DCM gradient. Product-containing fractions were evaporated to afford (*R*)-2-(4-(benzylthio)-1*H*-pyrazol-1-yl)propan-1-ol (189 mg, 0.76 mmol, 72% yield). *m*/*z* (ES-API-pos) [M+1] = 249.

Step D: A solution of (*R*)-2-(4-(benzylthio)-1*H*-pyrazol-1-yl)propan-1-ol (190 mg, 0.77 mmol) in acetic acid (5.1 mL) and water (2.6 mL) was treated with N-chlorosuccinimide (408.6 mg, 3.06 mmol). After 15 minutes the reaction mixture was concentrated under reduced pressure. The residue was dissolved in ether and washed with water 3xs. The organic phase was then dried over MgSO4, filtered and then concentrated to afford (*R*)-1-(1-hydroxypropan-2-yl)-1*H*-pyrazole-4-sulfonyl chloride. Crude was used without further purification. A flask was charged with (*R*)-1-(1-hydroxypropan-2-yl)-1*H*-pyrazole-4-sulfonyl chloride (43.4 mg, 0.19 mmol) and 7-amino-4-methyl-1*H*-indole-3-carbonitrile (25.9mg, 0.135 mmol). Pyridine (0.64 mL) was added and the mixture was stirred at room temperature for 30 minutes. The reaction mixture was concentrated under reduced pressure. The residue was chromatographed on a Biotage 10 g ultra SNAP column with a 20% to 100% EtOAc:hexane gradient. Product-containing fractions were evaporated to afford impure product. This was chromatographed on a Biotage 10 g ultra SNAP column with a 15% to 95% EtOAc:DCM gradient. Product-containing fractions were evaporated to afford (*R*)-*N*-(3-cyano-4-methyl-1*H*-indol-7-yl)-1-(1-hydroxypropan-2-yl)-1*H*-pyrazole-4-sulfonamide (30.6 mg, 0.080 mmol, 25.2% yield). $^1$H NMR (400 MHz, $CD_3OD$) $\delta$ 7.91 (s, 1H), 7.83 (s, 1H), 7.58 (s, 1H), 6.84 (d, 1H), 6.63 (d, 1H), 4.42 - 4.31 (m, 1H), 3.73 - 3.69 (m, 2H), 2.67 (s, 3H), 1.39 (d, 3H). *m*/*z* (ES-API-pos) [M+1] = 360.

Example 31: Synthesis of (*R*)-*N*-(4-chloro-3-cyano-1*H*-indol-7-yl)-1-(1-hydroxypropan-2-yl)-1*H*-pyrazole-4-sulfonamide **(Compound 208)**

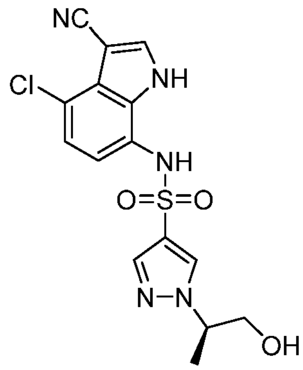

(*R*)-*N*-(4-chloro-3-cyano-1*H*-indol-7-yl)-1-(1-hydroxypropan-2-yl)-1*H*-pyrazole-4-sulfonamide was prepared similarly to **Example 30 (above example),** substituting 7-amino-4-chloro-1*H*-indole-3-carbonitrile for 7-amino-4-methyl-1*H*-indole-3-carbonitrile in Step D. $^1$H NMR (400 MHz, $CD_3OD$) $\delta$ 8.03 (s, 1H), 7.87 (s, 1H), 7.63 (s, 1H), 7.08 (d, 1H), 6.70 (d, 1H), 4.42 - 4.32 (m, 2H), 3.72 (td, 2H), 1.39 (d, 3H). *m*/*z* (ES-API-pos) [M+1] = 380.

Example 32: Synthesis of (*R*)-*N*-(3-cyano-4-fluoro-1*H*-indol-7-yl)-1-(1-hydroxypropan-2-yl)-1*H*-pyrazole-4-sulfonamide **(Compound 212)**

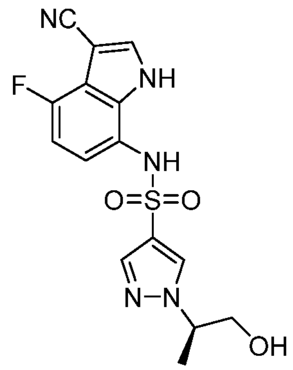

(*R*)-*N*-(3-cyano-4-fluoro-1*H*-indol-7-yl)-1-(1-hydroxypropan-2-yl)-1H-pyrazole-4-sulfonamide was prepared similarly to **Example 30 (above),** substituting 7-amino-4-fluoro-1*H*-indole-3-carbonitrile for 7-amino-4-methyl-1*H*-indole-3-carbonitrile in Step D. $^1$H NMR (400 MHz, CD3OD) δ 7.96 (s, 1H), 7.85 (s, 1H), 7.61 (s, 1H), 6.83 - 6.76 (m, 1H), 6.66 (dd, 1H), 4.43 - 4.32 (m, 2H), 3.72 (d, 2H), 1.40 (d, 3H). *m*/*z* (ES-API-pos) [M+1] = 364.

**Example 33:** Synthesis of N-(4-chloro-3-cyano-1H-indol-7-yl)-1-(3-hydroxypropyl)-1H-pyrazole-4-sulfonamide **(Compound 209)**

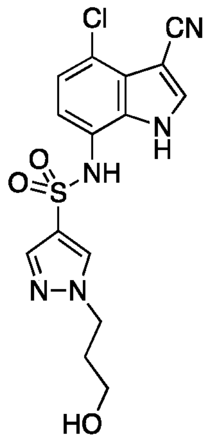

The sulfonyl chloride was prepared as described for the sequence of steps in Example 21 (alternate procedure, Synthesis of N-(4-chloro-3-cyano-1H-indol-7-yl)-1-(2-hydroxy-2-methyl-propyl)pyrazole-4-sulfonamide (Compound 136)) substituting 1-propanol, 3-bromo- for 1-chloro-2-methyl-propan-2-ol.

A mixture of 1-(3-hydroxypropyl)pyrazole-4-sulfonyl chloride (45.8 mg, 0.20 mmol) and 7-amino-4-chloro-1H-indole-3-carbonitrile (35.2 mg, 0.18 mmol) was dissolved in Pyridine (0.9 mL) at 25 C. After 1h, LCMS reveals complete reaction. The reaction mixture was poured into 20 mL of aqueous 10% $KHSO_4$ and extracted with 3 x 20 mL EtOAc. The combined organics were rinsed with 10 mL of brine, dried with $MgSO_4$, filtered, and concentrated to dryness. Purification was achieved by flash chromatography on a reverse phase Biotage C18 column (0-65% MeCN/water) to afford N-(4-chloro-3-cyano-1H-indol-7-yl)-1-(3-hydroxypropyl)-1H-pyrazole-4-sulfonamide (20.4 mg, 0.054 mmol, 26.3% yield). $^1$H NMR (400 MHz, Acetone-$d_6$) δ 8.23 (s, 1H), 7.93 (s, 1H), 7.60 (s, 1H), 7.16 (d, J = 8.2 Hz, 1H), 6.91 (d, J = 8.2 Hz, 1H), 4.24 (t, J = 6.8 Hz, 2H), 3.44 (t, J = 6.0 Hz, 2H), 1.96 (p, J = 6.4 Hz, 2H). *m*/*z* (ES-API-pos) [M+1] = 380/382.

**Example 34:** Synthesis of N-(3-cyano-4-methyl-1H-indol-7-yl)-1-(4-hydroxy-2-methylbutan-2-yl)-1H-pyrazole-4-sulfonamide **(Compound 229)**

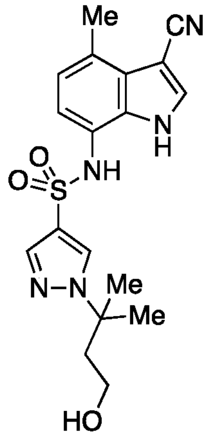

Step A: A solution of 1H-Pyrazole, 4-iodo- (2.0 g, 10.3 mol), 2-butenoic acid, 3-methyl-, methyl ester (3.9 mL, 30.9 mol) and DBU (1,8-diazabicyclo[5.4.0]undec-7-ene, 6.2 mL, 41.2 mol) in ACN (27 mL) was heated at reflux for 3.5 hours. The solvent was removed in vacuo. The residue was diluted with 80 mL of aqueous 10% $KHSO_4$, extracted with 3 x 30 mL of ethyl acetate, and the combined organic extracts were washed with saturated ammonium chloride, dried over $MgSO_4$, and concentrated. The crude residue was purified by flash column chromatography (ethyl acetate/hexanes) to yield methyl 4-(4-iodo-1H-pyrazol-1-yl)-4-methylpentanoate (1416 mg, 44.6%). *m/z* (ES-API-pos) [M+1] = 309.

Step B: A mixture of Methanesulfonato[9,9-dimethyl-4,5-bis(diphenylphosphino)xanthene][2'-amino-1,1'-biphenyl]palladium(II) dichloromethane adduct, min. 98% [Xantphos Palladacycle Gen. 3] (217.9 mg, 0.23 mmol) and methyl 3-(4-iodopyrazol-1-yl)-3-methyl-butanoate (1416 mg, 4.6 mmol) was suspended in 1,4-Dioxane (30 mL) and sparged with nitrogen for 3 mins. The reaction mixture was then treated sequentially with benzyl mercaptan (0.51 mL, 4.37 mmol) and N,N-Diisopropylethylamine (1.6 mL, 9.19 mmol) under continuous nitrogen stream. The vessel was sealed and heated to 100 C for 2 h. Volatiles were removed by concentration under reduced pressure. Purification was achieved by chromatography on silica using a gradient of 5-20% EtOAc/hexane to afford methyl 4-(4-(benzylthio)-1H-pyrazol-1-yl)-4-methylpentanoate (404 mg, 28.9%). *m/z* (ES-API-pos) [M+1] = 305.

Step C: A solution of methyl 3-(4-benzylsulfanylpyrazol-1-yl)-3-methylbutanoate (302 mg, 0.99 mmol) in Tetrahydrofuran (5.0 mL) at 0 C was treated with lithium aluminum hydride, ~ 1 M in THF (1.2 mL, 1.2 mmol). The reaction was allowed to slowly warm to room temperature. Once complete by LCMS, the reaction mixture was diluted with ether (5 mL) and cooled to 0 C. The reaction was quenched by the sequential addition of 45 μl water. Then 45 μl uL of 15% aqueous NaOH. And last, 135 μl of water. The resulting mixture was vigorously stirred for 15 min, treated with $MgSO_4$, stirred for another 15 min. The solid precipitates that formed were filtered through a pad of celilte using EtOAc as eluent. The filrate was concentrated and the resulting product used without further purification. *m/z* (ES-API-pos) [M+1] = 277.

Step D: A solution of the unpurified 3-(4-benzylsulfanylpyrazol-1-yl)-3-methyl-butan-1-ol (308 mg, ~1.1 mmol) in a mixture of acetic acid (8 mL) and water (0.8 mL) at 25 C was treated with N-chlorosuccinimide (416.6 mg, 3.12 mmol). After 1h, volatiles were removed by concentration under reduced pressure. The residue was reconcentrated from toluene (2x) to remove residual water and AcOH that remained and kept under high vac for 15 min. The sulfonyl chloride intermediate was purified by chromatography on silica using a gradient of 10-60% EtOAc/hexane to afford 1-(4-hydroxy-2-methylbutan-2-yl)-1H-pyrazole-4-sulfonyl chloride (155 mg, 55.0%). *m/z* (ES-API-pos) [M+1] = 253/255.

Step E: A mixture of 1-(3-hydroxy-1,1-dimethyl-propyl)pyrazole-4-sulfonyl chloride (77.6 mg, 0.31 mmol) and 7-amino-4-methyl-1H-indole-3-carbonitrile (49.9 mg, 0.29 mmol) was dissolved in pyridine (1.1 mL) at 25 C. After 1h, LCMS reveals complete reaction. The reaction mixture was poured into 20 mL of aqueous 10% $KHSO_4$ and extracted with 3 x 20 mL EtOAc. The combined organics were rinsed with 10 mL of brine, dried with $MgSO_4$, filtered, and concentrated to dryness. Purification was achieved by flash chromatography on a reverse phase Biotage C18 column (0-70% MeCN/water) to afford N-(3-cyano-4-methyl-1H-indol-7-yl)-1-(4-hydroxy-2-methylbutan-2-yl)-1H-pyrazole-4-sulfonamide (99.2 mg, 0.256 mmol, 83.4% yield). $^1$H NMR (400 MHz, Acetone-$d_6$) δ 8.10 (s, 1H), 7.80 (s, 1H), 7.57 (s, 1H), 6.90 (d, *J* = 7.7 Hz, 1H), 6.75 (d, *J* = 7.6 Hz, 1H), 3.29 (t, *J* = 6.8 Hz, 2H), 2.68 (s, 3H), 2.08 (m, 2H), 1.55 (s, 6H). *m/z* (ES-API-pos) [M+1] = 388.

**Example 35:** Synthesis of N-(3-cyano-4-methyl-1H-indol-7-yl)-1-(3-hydroxy-2,2-dimethylpropyl)-1H-pyrazole-4-sulfonamide **(Compound 258)**

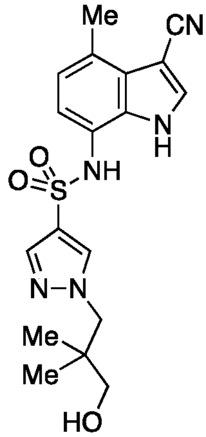

A mixture of 1-(3-hydroxy-2,2-dimethyl-propyl)pyrazole-4-sulfonyl chloride (75.1 mg, 0.30 mmol) and 7-amino-4-methyl-1H-indole-3-carbonitrile (48.3 mg, 0.28 mmol) was dissolved in Pyridine (1.1 mL) at 25 C. After 1h, LCMS reveals complete reaction. The reaction mixture was poured into 20 mL of aqueous 10% $KHSO_4$ and extracted with 3 x 20 mL EtOAc. The combined organics were rinsed with 10 mL of brine, dried with $MgSO_4$, filtered, and concentrated to dryness. Purification was achieved by flash chromatography on a reverse phase Biotage C18 column (0-70% MeCN/water) to afford N-(3-cyano-4-methyl-1H-indol-7-yl)-1-(3-hydroxy-2,2-dimethylpropyl)-1H-pyrazole-4-sulfonamide (99.1 mg, 0.256 mmol, 86.1% yield). $^1$H NMR (400 MHz, Acetone-$d_6$) $\delta$ 11.01 (br s, 1H), 8.64 (br s, 1H), 8.10 (s, 1H), 7.75 (s, 1H), 7.57 (s, 1H), 6.88 (d, $J$ = 7.7 Hz, 1H), 6.80 (d, $J$ = 7.7 Hz, 1H), 4.01 (s, 2H), 3.87 (br s, 1H), 3.09 (s, 2H), 2.67 (s, 3H), 0.75 (s, 6H). *m/z* (ES-API-pos) [M+1] = 388.

**Example 36:** Synthesis of N-(3-cyano-1H-indol-7-yl)-1-(difluoromethyl)-1H-pyrazole-4-sulfonamide **(Compound 292)**

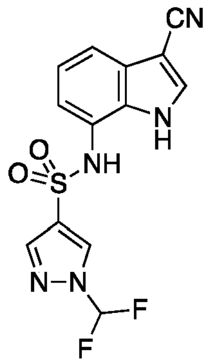

Step A: A solution of 1H-Pyrazol-5-amine, 1-(difluoromethyl)- (250.mg, 1.88mmol) at -78 C was treated with chlorosulfonic acid (1.0 mL, 15.0 mmol) in one and the ice bath was removed. The reaction was stirred while being allowed to warm to room temperature. Then it was heated at 100 C for 3 h. The reaction mixture was cooled to room temperature, poured onto ice, and extracted with 3 x 20 mL $CH_2Cl_2$. The combined organics were rinsed with 10 mL of brine, dried with MgSO4, filtered, and concentrated to dryness. The resulting product was used without further purification (225 mg, 51.7%). *m/z* (ES-API-pos) [M+1] = 232/234.

Step B: A mixture of 5-amino-1-(difluoromethyl)pyrazole-4-sulfonyl chloride (42.0 mg, 0.18 mmol) and 7-amino-1H-indole-3-carbonitrile (27.1 mg, 0.17 mmol) was dissolved in pyridine (0.65 mL) at 25 C. After 1h, LCMS reveals complete reaction. The reaction mixture was poured into 20 mL of aqueous 10% $KHSO_4$ and extracted with 3 x 20 mL EtOAc. The combined organics were rinsed with 10 mL of brine, dried with $MgSO_4$, filtered, and concentrated to dryness. Purification was achieved by flash chromatography on a reverse phase Biotage C18 column (0-55% MeCN/water) to afford N-(3-cyano-1H-indol-7-yl)-1-(difluoromethyl)-1H-pyrazole-4-sulfonamide (47.7 mg, 0.135 mmol, 77.7% yield). $^1$H NMR (400 MHz, Acetone-$d_6$) $\delta$ 11.12 (br s, 1H), 8.86 (br s, 1H), 8.14 (s, 1H), 7.59 (dd, $J$ = 8.0 Hz, 1H), 7.43 (s, 1H), 7.42 (t, $J_{H,F}$ = 57.9 Hz, 1H), 7.20 (t, $J$ = 7.8 Hz, 1H), 7.02 (dd, $J$ = 7.6, 0.8 Hz, 1H), 6.01 (s, 2H). *m/z* (ES-API-pos) [M+1] = 353.

**Example 37:** DCAF15 RBM39 Complex Formation Assay.

LANCE Eu-W1024 Anti-6xHis and SureLight Allophycocyanin-anti-FLAG antibodies were purchased from Perkin Elmer. Human DDB1DB and His-tagged DCAF15 were co-expressed in baculovirus/*sf9* system. While full length DDB1 contains three 7-bladed propellers termed A, B, and C, DDB1DB has only propeller A (amino acids 1 to 395) and C (amino acids 709-1140). The His-tagged DCAF15 contains six histidine residues directly preceding the DCAF15 sequence without any linker. An RBM39 fragment containing RRM1 and RRM2 was expressed in *E. coli.* Amino acids 150 to 331 of RBM39 were cloned into pGEX4T3 with an N-terminal TEV cleavage site and a C-terminal FLAG tag.

A compound of the present disclosure in DMSO (2 µL) was transferred to a 384-well assay plate. Compound was added in 14 points, 3-fold serial dilution (1 µM to 0.6 pico molar) in DMSO. 48 mL FRET assay buffer (25 mM HEPES, pH 7.5, 100 mM NaCl, 0.1 M BSA, 0.5 mM TCEP, 0.005% Tween-20, 0.2 nM Eu-W1024 Anti-6xHis, 50 nM SureLight Allophycocyanin-anti-FLAG, 20 nM DDB1DB /DCAF15, 50 nM RBM39 R1R2) was added and the resulting solution was incubated overnight at 4 °C. The plate was placed in a plate reader (Spark 10) with excitation at l = 340 nm and emissions monitored at l = 665 nm and l = 615 nm, and the FRET ratio (Em 665/Em 615) was determined. Low signal controls containing no compound and high signal controls containing 10 mM indisulam were measured for each plate.

$EC_{50}$ values were calculated using Dotmatics template equation for 4-parameter fit. Results are expressed as % formation using the following equation:

$$\%\, formation = \left(\frac{high\ control - sample}{high\ control - low\ control}\right) x\ 100$$

**Example 38:** CellTiter-Glo Assay.

HCT116 human colorectal carcinoma cells were used in these experiments. HCT116 cells were obtained from ATCC. CellTiter-Glo® Luminescent Cell Viability Assay Reagent was obtained from Promega.

About 3,000 cells were seeded into 96-well plate with 100 µL of media (DMEM supplemented with 10% FBS, 100 units penicillin, and 100 mg streptomycin per mL) the day before the experiment. For compound treatment, the master plate was prepared as follows: 100x compound stocks were prepared in a 96-well plate by 1 to 3 serial dilutions of 0.5 mM DMSO stocks. Each master plate contained serial dilution of 8 compounds, including the control compound E7820. 1 mL of 100x compound stock was added to each well of the assay plate to give final concentrations of 5, 1.67, 0.56, 0.19, 0.06, 0.02, 0.0069, 0.002, 0.0007, 0.0002, and 0 mM. Each concentration was tested in triplicate. After 3 days, cell viability was determined using CellTiter-Glo Luminescent Cell Viability Assay Reagent following the manufacturer's recommended protocol. Briefly, 50 mL of CellTiter-Glo reagent was added to each well of the assay plate and the contents were mixed for 3 minutes at 600 rpm on a shaker (Thermomixer R). The plate was immediately placed in a plate reader (Synergy 2) and the luminescence signal (0.5 second per well integration time) was determined. $IC_{50}$ values were calculated using Dotmatics software.

**Tables 2** shows biological activities of selected compounds described herein in the complex formation and cell-based assays, respectively. Compound numbers correspond to the numbers and structures provided in **Table 1** and **Examples 1-36.**

**Table 2**

| Assay | Less than 250 nM (+++) | 250 nM to 1000 nM (++) | Greater than 1000 nM (+) |
|---|---|---|---|
| CellTiter-Glo Assay $EC_{50}$ (uM) | 231, 301, 120, 274, 277, 36, 135, 229, 181, 221, 264, 161, | 189, 226, 212, 232, 266, 95, 116, 235, 62, 72, 217, 166, 148, 233, 223, 298, | 140, 133, 22, 178, 194, 137, 247, 147, 307, |

| | | | |
|---|---|---|---|
| | 171, 198, 60, 94, 230, 173, 163, 73, 191, 234, 75, 107, 113, 98, 118, 278, 128, 69, 115, 66, 284, 303, 122, 80, 160, 180, 258, 237, 244, 271, 82, 304, 77, 68, 90, 259, 292, 70, 64, 59, 268, 155, 143, 250, 81, 297, 281, 242, 236, 117, 159, 207, 124, 119, 273, 287, 102, 290 | 252, 311, 243, 227, 89, 158, 25, 210, 40, 220, 105, 170, 293, 164, 109, 28, 47, 114, 302, 291, 209, 305, 12, 263, 9, 108, 188, 165, 175, 276, 17, 112, 46, 213, 6, 76, 172, 145, 211, 83, 110, 93, 85, 270, 182, 248, 150, 238, 285, 32, 125, 179, 154, 306, 91, 3, 265, 279, 208, 254, 299, 205, 283, 100, 92, 38, 88, 149, 126, 106, 86, 130, 35, 99, 251, 308, 123, 63, 255, 215, 241, 78, 51, 7, 79, 176, 195, 289, 275, 49, 257, 197, 39, 249, 245, 183, 288, 219, 1, 168, 29, 190, 45, 67, 56, 65, 246, 127, 52, 262, 71, 121, 16, 157, 193, 11, 111, 144, 174, 19, 216, 61, 224, 136, 225, 269, 101, 169, 272, 222, 162, 196, 282, 104, 267, 177, 286, 295, 54, 156, 13, 167, 218 | 261, 204, 214, 30, 14, 26, 34, 312, 309, 138, 103, 24, 21, 296, 23, 253, 42, 142, 44, 53, 139, 151, 153, 129, 57, 141, 31, 97, 146, 228, 18, 43, 239, 192, 260, 87, 256, 41, 184, 206, 300, 152, 27, 134, 37, 20, 15, 84, 280, 33, 240, 294 |
| Complex Formation Assay $IC_{50}$ (uM) | 189, 36, 135, 226, 229, 171, 212, 232, 266, 314, 95, 116, 173, 204, 62, 191, | 42, 140, 131, 41, 24, 126, 21, 132, 20, 5, 18, 33 | 30, 48, 22, 2, 50, 58 |

| | | | |
|---|---|---|---|
| | 235, 72, 217, 166, 75, 113, 148, 223, 233, 298, 252, 118, 278, 311, 243, 271, 244, 227, 296, 89, 77, 158, 25, 259, 210, 40, 220, 105, 146, 155, 310, 43, 192, 170, 117, 256, 300, 159, 206, 293, 152, 164, 289, 109, 28, 240, 231, 47, 120, 114, 302, 291, 8, 137, 12, 209, 263, 305, 161, 261, 198, 9, 94, 108, 188, 163, 73, 165, 175, 276, 17, 34, 112, 46, 309, 98, 312, 213, 103, 6, 76, 172, 145, 211, 122, 80, 180, 258, 83, 110, 93, 23, 253, 85, 68, 270, 44, 292, 182, 248, 150, 64, 228, 238, 285, 315, 239, 297, 32, 125, 179, 154, 184, 91, 207, 306, 3, 265, 279, 208, 254, 299, 205, 301, 274, 283, 100, 92, 38, 88, 149, 194, 307, 221, 264, 313, 106, 60, 86, | | |

| | | | |
|---|---|---|---|
| | 214, 130, 35, 14, 107, 99, 251, 308, 138, 123, 63, 255, 115, 215, 66, 241, 160, 237, 4, 82, 78, 304, 51, 90, 7, 139, 79, 70, 57, 129, 153, 141, 176, 59, 49, 97, 195, 257, 197, 250, 39, 275, 249, 245, 183, 260, 288, 219, 1, 27, 29, 134, 168, 124, 45, 67, 190, 15, 273, 287, 102, 294, 56, 65, 133, 178, 247, 246, 277, 147, 127, 181, 52, 262, 10, 230, 280, 71, 234, 121, 16, 26, 157, 193, 11, 111, 144, 174, 19, 128, 216, 61, 69, 224, 136, 284, 303, 225, 269, 101, 142, 169, 272, 53, 151, 222, 162, 196, 31, 143, 104, 268, 267, 282, 81, 281, 177, 242, 286, 295, 87, 236, 54, 156, 13, 316, 119, 37, 84, 167, 218, 290 | | |

**Example 39:** In Vivo Efficacy Studies

**Compound 3** were formulated with 10% absolute ethanol, 30% PEG400, 60% water containing 0.5% methyl cellulose and 0.5% Tween80®. 2x106 HCT116 colorectal carcinoma cells (ATCC® CCL-247) in PBS and Matrigel (1:1 in volume) were inoculated subcutaneously in the right flank of Athymic Nude mice at 6-7 weeks of age for tumor development. When the xenografts reached about 150 $mm^3$ in size, the tumor bearing mice were randomly grouped into six groups (n=6) and treated by oral gavage with vehicle (10% absolute ethanol, 30% PEG400, 60% water containing 0.5% methyl cellulose and 0.5% Tween80®, BID) or **compound 3** at 100 mg/kg BID for 12 days. Tumor sizes were measured twice weekly in two dimensions using a caliper and the volume were expressed in $mm^3$ using the formula $V=0.5 \times a \times b^2$ wherein a and b were the long and short diameters of the tumor, respectively. The efficacy study for **compound 136** was carried out in the same manner as that of **compound 3** except **compound 136** was dosed at 30 mg/kg BID for 12 days. As shown in Tables 3 and 4, **Compound 3** or **136** treatment led to a statistically significant reduction of tumor volume and tumor weight in the HCT116 mouse model (all data displayed as mean with the standard error of the mean (SEM)) while there was no significant difference between the body weight of the vehicle and **compounds 3** and **136**-treated groups.

**Table 3**

| Treatment groups | Vehicle | Compound 3 (100 mg/kg) |
|---|---|---|
| Tumor Volume ($mm^3$) Mean ± SEM | 431.9 ± 116.3 | 122.9 ± 33.9 |
| Tumor Weight (g) Mean ± SD | 0.76 ± 0.39 | .013 ± 0.005 |
| Body Weight (g) Mean ± SEM | 22.5 ± 0.13 | 22.44 ± 0.16 |

**Table 4**

| Treatment groups | Vehicle | Compound 136 (30 mg/kg) |
|---|---|---|
| | | |
| Tumor Volume ($mm^3$) Mean ± SEM | 413.3 ± 113.8 | 175.7 ± 12.3 |
| Tumor Weight (g) Mean ± SD | 0.92 ± 0.29 | .083 ± 0.06 |
| Body Weight (g) Mean ± SEM | 21.31 ± 0.37 | 21.4 ± 0.14 |

## Claims

1. A compound of Formula I:

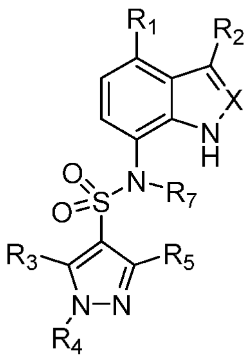

I

or a pharmaceutically acceptable salt thereof, wherein
**X** is N or **$CR_6$**;
**$R_1$** is hydrogen, C1-C4 alkyl, C1-C4 fluoroalkyl, or halo;
**$R_2$** is halo or cyano;
**$R_3$** is hydrogen, C1-C4 alkyl, halo, cyano, C1-C4 fluoroalkyl, -(C1-C4 alkyl)OH, or $-NR^aR^b$, wherein $R^a$ or $R^b$ is

independently hydrogen, C3-C6 cycloalkyl, heterocycloalkyl or C1-C4 alkyl, or $R^a/R^b$ and the nitrogen atom they are attached to form a 5 or 6-membered ring optionally independently substituted by one or more substituents selected from halo, C1-C4 alkyl, cyano, hydroxy and -O(C1-C4 alkyl);

**$R_4$** is hydrogen, C1-C6 alkyl, C1-C10heteroalkyl, C1-C4 fluoroalkyl, -(C1-C4 alkyl)$NH_2$, C3-C6 cycloalkyl, -(C1-C4 alkyl)(C3-C6 cycloalkyl), -(C1-C4 alkyl)phenyl, phenyl, heteroaryl, -(C1-C4 alkyl)heteroaryl, C2-C5 alkenyl, -(C1-C4 alkyl)heterocycloalkyl, heterocycloalkyl, -C0-C4 alkylS(=O)$_2$(C1-C4 alkyl), -S(=O)$_2$phenyl, -S(=O)$_2$heteroaryl, -C0-C4 alkylC(=O)heterocycloalkyl, -C0-C4 alkyl C(=O)$NR^aR^b$, -(C0-C4 alkyl)C(=O)O(C1-C4 alkyl), -(C0-C4 alkyl)C(=O)C1-C4 alkyl, -(C0-C4 alkyl)C(=O)OH or -(C1-C8 alkyl)OH, wherein **$R_4$** is substituted with 0, 1, 2, or 3 substituents selected from deuterium, halo, hydroxy, -(C0-C4alkyl)O(C1-C4 alkyl), -C1-C4 alkyl, -(C0-C4alkyl)cyano, -S(=O)$_2$(C1-C4 alkyl), - C0-C4 alkylC(=O)$NR^aR^b$, -C(=O)O(C1-C4 alkyl), C1-C4 fluoroalkyl oxo, -(C1-C4 alkyl)OH, and -$NH_2$, wherein each of $R^a$ and $R^b$ is independently substituted with 0, 1, 2, or 3 hydrogen, C3-C6 cycloalkyl, heterocycloalkyl or C1-C4 alkyl;

**$R_5$** is hydrogen, halo, -$NH_2$ or C1-C4 alkyl;

**$R_6$** is hydrogen, halo or C1-C4 alkyl; and

**$R_7$** is hydrogen, halo, heteroalkyl, C1-C4 alkyl, -(C0-C4 alkyl)O( C1-C4 alkyl), -(C1-C4 alkyl)(C3-C6 cycloalkyl), C3-C6 cycloalkyl, -(C1-C4 alkyl)heterocycloalkyl, or heterocycloalkyl.

**2.** A compound of claim 1 or a pharmaceutically acceptable salt thereof, wherein $R_1$ is C1-C4 alkyl or halo.

**3.** A compound of claim 2 or a pharmaceutically acceptable salt thereof, wherein $R_1$ is methyl, chloro or fluoro.

**4.** A compound of claim 1 or a pharmaceutically acceptable salt thereof, wherein $R_1$ is hydrogen.

**5.** A compound of any one of claims 1 to 4, or a pharmaceutically acceptable salt thereof, wherein $R_2$ is chloro, fluoro, or cyano.

**6.** A compound of any one of claims 1 to 5, or a pharmaceutically acceptable salt thereof, wherein $R_3$ is hydrogen, methyl, fluoro, chloro, difluoromethyl, -$NH_2$, or -$C_2H_4OH$.

**7.** A compound of any one of claims 1 to 6 or a pharmaceutically acceptable salt thereof,, wherein $R_4$ is is hydrogen, methyl, ethyl, propyl, isopropyl, tert-butyl, fluoromethyl, difluoromethyl, difluoroethyl, trifluoroethyl, fluoropropyl, methoxyethyl, methoxyethoxyethyl, cyclopropyl, cyclobutyl, cyclopentyl, cyclohexyl, 3-bicyclo[1.1.1]pentanyl, cyclopropylmethyl, cyclobutylmethyl, cyclopentylmethyl, cyclohexylmethyl, cyclopropylethyl, cyclobutylethyl, cyclopentylethyl and cyclohexylmethyl, tetrahydrofuranyl, azetidinyl, tetrahydropyranyl, piperidinyl, oxetanyl, thietanyl, pyrrolidinylethyl, pyrrolidinyl, pyrrolidinylmethyl, azetidinylmethyl, tetrahydrothiopyranylmethyl, piperidinylme-thyl, tetrahydropyranylmethyl, tetrahydrothiophenyl, oxetanylethyl, oxaspiro[3.3]heptyl, (methylsulfonyl)ethyl, 2-methyl-2-(methylsulfonyl)propyl, pyrazolyl(methylsulfonyl), methylsulfonyl, (methylsulfonyl)methyl, phenylsulfonyl, hydroxyethyl, aminocarbonyl-methyl, carboxymethyl, cyclobutylaminocarbonylmethyl, pyrrolidinylcarbonylmethyl, methylaminocarbonylmethyl, (dimethylamino)carbonylmethyl, aminocarbonyl(C1-C4alkyl), methoxycarbonyl(C1-C4alkyl), (aminocarbonyl)C1-C4alkyl, phenyl, pyridinyl, propenyl,

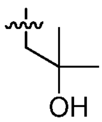 , 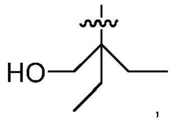 , 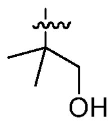 , 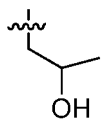 , 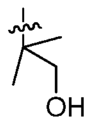 ,

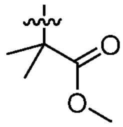 , 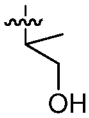 , 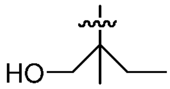 , 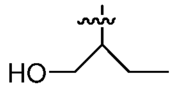 ,

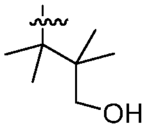, 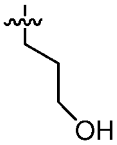, 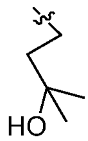, 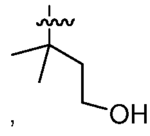, 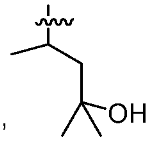, 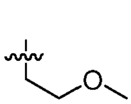,

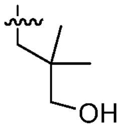, 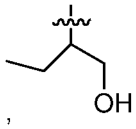, 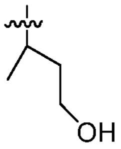, 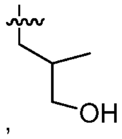, 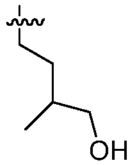,

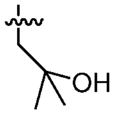, 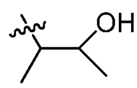, 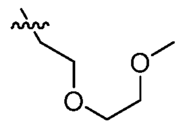 or 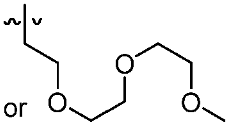,

and wherein **$R_4$** is substituted with 0, 1, 2, or 3 substituents selected from deuterium, halo, hydroxy, -(C0-C4alkyl)O(C1-C4 alkyl), -C1-C4 alkyl, -(C0-C4alkyl)cyano, - C0-C4 alkylC(=O)$NR^aR^b$, -C(=O)O(C1-C4 alkyl), C1-C4 fluoroalkyl, oxo, -(C1-C4 alkyl)OH, and $-NH_2$, wherein each of $R^a$ and $R^b$ is independently substituted with 0, 1, 2, or 3 hydrogen, C3-C6 cycloalkyl, heterocycloalkyl or C1-C4 alkyl.

**8.** A compound of any one of claims 1 to 7, or a pharmaceutically acceptable salt thereof, wherein $R_5$ is hydrogen methyl, fluoro, chloro, or $-NH_2$.

**9.** A compound of any one of claims 1 to 8, or a pharmaceutically acceptable salt thereof, wherein $R_2$ is cyano.

**10.** A compound of claim 1 which is selected from the group consisting of

N-(3-chloro-1H-indol-7-yl)-1-methyl-pyrazole-4-sulfonamide;
N-(3-chloro-1H-indol-7-yl)-1,3,5-trimethyl-pyrazole-4-sulfonamide;
N-(3-cyano-4-methyl-1H-indol-7-yl)-1-methyl-pyrazole-4-sulfonamide;
N-(3-chloro-1H-indol-7-yl)-1-ethyl-pyrazole-4-sulfonamide;
N-(3-chloro-1H-indol-7-yl)-1,3-dimethyl-pyrazole-4-sulfonamide;
N-(3-chloro-1H-indol-7-yl)-1-(difluoromethyl)pyrazole-4-sulfonamide;
N-(3-chloro-1H-indol-7-yl)-1-(2,2-difluoroethyl)pyrazole-4-sulfonamide;
N-(3-chloro-1H-indol-7-yl)-1-tetrahydrofuran-3-yl-pyrazole-4-sulfonamide;
N-(3-chloro-1H-indol-7-yl)-1-(2-methylsulfonylethyl)pyrazole-4-sulfonamide;
N-(3-chloro-1H-indol-7-yl)-1H-pyrazole-4-sulfonamide;
N-(3-cyano-4-methyl-1H-indol-7-yl)-1-(difluoromethyl)pyrazole-4-sulfonamide;
*tert-butyl* 3-[4-[(3-chloro-1H-indol-7-yl)sulfamoyl]pyrazol-1-yl]azetidine-1-carboxylate;
N-(3-chloro-1H-indol-7-yl)-1-(2-hydroxyethyl)pyrazole-4-sulfonamide;
N-(3-chloro-1H-indol-7-yl)-1-(cyanomethyl)pyrazole-4-sulfonamide;
1-(5-chloro-3-fluoro-2-pyridyl)-N-(3-chloro-1H-indol-7-yl)pyrazole-4-sulfonamide;
2-[4-[(3-chloro-1H-indol-7-yl)sulfamoyl]pyrazol-1-yl]acetamide;
2-[4-[4-[(3-chloro-1H-indol-7-yl)sulfamoyl]pyrazol-1-yl]sulfonylpyrazol-1-yl]acetamide;
1-(azetidin-3-yl)-N-(3-chloro-1H-indol-7-yl)pyrazole-4-sulfonamide;
N-(3-chloro-1H-indol-7-yl)-1-(1H-pyrazol-4-ylsulfonyl)pyrazole-4-sulfonamide;
N-(3-chloro-1H-indol-7-yl)-1-tetrahydropyran-4-yl-pyrazole-4-sulfonamide;
N-(3-chloro-1H-indol-7-yl)-1-(5-fluoro-2-pyridyl)pyrazole-4-sulfonamide;
N-(3-chloro-1H-indol-7-yl)-1-[2-cyano-4-(trifluoromethyl)phenyl]pyrazole-4-sulfonamide;
N-(3-chloro-1H-indol-7-yl)-1-[2-(2-methoxyethoxy)ethyl]pyrazole-4-sulfonamide;
N-(3-chloro-1H-indol-7-yl)-1-[2-[2-(2-methoxyethoxy)ethoxy]ethyl]pyrazole-4-sulfonamide;
N-(3-cyano-4-methyl-1H-indol-7-yl)-1-(2-hydroxyethyl)pyrazole-4-sulfonamide;
*tert*-butyl 4-[4-[(3-chloro-1H-indol-7-yl)sulfamoyl]pyrazol-1-yl]piperidine-1-carboxylate;
1-(1-acetylazetidin-3-yl)-N-(3-chloro-1H-indol-7-yl)pyrazole-4-sulfonamide;

N-(3-chloro-1H-indol-7-yl)-1-[(3 -methyloxetan-3-yl)methyl]pyrazole-4-sulfonamide;
N-(3-cyano-4-methyl-1H-indol-7-yl)-1-tetrahydrofuran-3-yl-pyrazole-4-sulfonamide;
N-(3-chloro-1H-indol-7-yl)-1-[5-(trifluoromethyl)-2-pyridyl]pyrazole-4-sulfonamide;
N-(3-cyano-4-methyl-1H-indol-7-yl)-1-[5-(trifluoromethyl)-2-pyridyl]pyrazole-4-sulfonamide;
N-(3-chloro-1H-indol-7-yl)-1-(2,2-dimethoxyethyl)pyrazole-4-sulfonamide;
*tert*-butyl N-[2-[4-[(3-chloro-1H-indol-7-yl)sulfamoyl]pyrazol-1-yl]ethyl]carbamate;
1-(2-aminoethyl)-N-(3-chloro-1H-indol-7-yl)pyrazole-4-sulfonamide;
N-(3-chloro-1H-indol-7-yl)-1-(2-fluoroethyl)pyrazole-4-sulfonamide;
N-(3-cyano-4-methyl-1H-indol-7-yl)-1-(2-fluoroethyl)pyrazole-4-sulfonamide;
N-(3-chloro-1H-indol-7-yl)-1-phenyl-pyrazole-4-sulfonamide;
N-(3-chloro-1H-indol-7-yl)-1-(2,2,2-trifluoroethyl)pyrazole-4-sulfonamide;
N-(3-chloro-1H-indol-7-yl)-1-isopropyl-pyrazole-4-sulfonamide;
N-(3-chloro-1H-indol-7-yl)-1-(oxetan-3-yl)pyrazole-4-sulfonamide;
N-(3-chloro-1H-indol-7-yl)-1-(4-piperidyl)pyrazole-4-sulfonamide;
*tert*-butyl 3-[[4-[(3-chloro-1H-indol-7-yl)sulfamoyl]pyrazol-1-yl]methyl]azetidine-1-carboxylate;
1-(azetidin-3-ylmethyl)-N-(3-chloro-1H-indol-7-yl)pyrazole-4-sulfonamide;
N-(3 -chloro- 1H-indol-7-yl)-1-[(3 -methylthietan-3 -yl)methyl]pyrazole-4-sulfonamide;
N-(3-chloro-1H-indol-7-yl)-1-[(3-methyl-1,1-dioxo-thietan-3-yl)methyl]pyrazole-4-sulfonamide;
N-(3 -cyano-1H-indol-7-yl)-1-methyl-pyrazole-4-sulfonamide;
N-(3-chloro-1H-indol-7-yl)-1-cyclobutyl-pyrazole-4-sulfonamide;
N-(3-chloro-1H-indol-7-yl)-N,1-bis(cyclopropylmethyl)pyrazole-4-sulfonamide;
N-(3-chloro-1H-indol-7-yl)-1-(2-methoxyethyl)pyrazole-4-sulfonamide;
N-(3-chloro-1H-indol-7-yl)-N,1-bis(2-methoxyethyl)pyrazole-4-sulfonamide;
N-(3-chloro-1H-indol-7-yl)-1-(cyclopropylmethyl)pyrazole-4-sulfonamide;
N-(3-chloro-1H-indol-7-yl)-1-[(1-cyanocyclopropyl)methyl]pyrazole-4-sulfonamide;
N-(3-chloro-1H-indol-7-yl)-1-[(3,3-difluorocyclobutyl)methyl]pyrazole-4-sulfonamide;
N-(3-chloro-4-fluoro-1H-indol-7-yl)-1-methyl-pyrazole-4-sulfonamide;
N-(3-chloro-1H-indol-7-yl)-1-(fluoromethyl)pyrazole-4-sulfonamide;
N-(3-chloro-1H-indol-7-yl)-1-(3-fluoropropyl)pyrazole-4-sulfonamide;
N-(3 -chloro- 1H-indol-7-yl)-1-(1,1-dioxothietan-3 -yl)pyrazole-4-sulfonamide;
N-(3-chloro-1H-indol-7-yl)-1-(2-cyanoethyl)pyrazole-4-sulfonamide;
N-(3-cyano-4-methyl-1H-indol-7-yl)-1-(2,2,2-trifluoroethyl)pyrazole-4-sulfonamide;
N-(3-cyano-4-methyl-1H-indol-7-yl)-1-cyclobutyl-pyrazole-4-sulfonamide;
N-(3-cyano-4-methyl-1H-indol-7-yl)-1H-pyrazole-4-sulfonamide;
N-(3-cyano-4-methyl-1H-indol-7-yl)-1-isopropyl-pyrazole-4-sulfonamide;
N-(3-cyano-4-methyl-1H-indol-7-yl)-1-(oxetan-3-yl)pyrazole-4-sulfonamide;
N-(3-cyano-4-fluoro-1H-indol-7-yl)-1-methyl-pyrazole-4-sulfonamide;
N-(3-cyano-4-methyl-1H-indol-7-yl)-1-(2,2-difluoroethyl)pyrazole-4-sulfonamide;
N-(3-cyano-4-methyl-1H-indol-7-yl)-1-(2-methylsulfonylethyl)pyrazole-4-sulfonamide;
N-(3-cyano-4-methyl-1H-indol-7-yl)-1-ethyl-pyrazole-4-sulfonamide;
N-(3-cyano-4-methyl-1H-indol-7-yl)-1-(cyclopropylmethyl)pyrazole-4-sulfonamide;
N-(3-cyano-4-methyl-1H-indol-7-yl)-1-(fluoromethyl)pyrazole-4-sulfonamide;
N-(3-cyano-4-methyl-1H-indol-7-yl)-1-[(3-methyloxetan-3-yl)methyl]pyrazole-4-sulfonamide;
N-(3-cyano-4-methyl-1H-indol-7-yl)-1-[(3-fluorooxetan-3-yl)methyl]pyrazole-4-sulfonamide;
N-(3-cyano-4-methyl-1H-indol-7-yl)-1-(3-fluoropropyl)pyrazole-4-sulfonamide;
N-(3-chloro-4-methyl-1H-indol-7-yl)-1-methyl-pyrazole-4-sulfonamide;
N-(3-cyano-4-methyl-1H-indol-7-yl)-1-(2-cyano-2-methyl-propyl)pyrazole-4-sulfonamide;
1-(2-cyanoethyl)-N-(3-cyano-4-methyl-1H-indol-7-yl)pyrazole-4-sulfonamide;
N-(3-cyano-4-methyl-1H-indol-7-yl)-1-(cyclobutylmethyl)pyrazole-4-sulfonamide;
N-(3-cyano-4-methyl-1H-indol-7-yl)-1-[(2,2-difluorocyclopropyl)methyl]pyrazole-4-sulfonamide;
N-(3-cyano-4-methyl-1H-indol-7-yl)-1-[1-(fluoromethyl)vinyl]pyrazole-4-sulfonamide;
N-(3,4-dichloro-1H-indol-7-yl)-1-methyl-pyrazole-4-sulfonamide;
N-(4-chloro-3-cyano-1H-indol-7-yl)-1-methyl-pyrazole-4-sulfonamide;
N-(3-chloro-1H-indol-7-yl)-1-(3-hydroxycyclobutyl)pyrazole-4-sulfonamide;
N-(3-cyano-4-methyl-1H-indol-7-yl)-1-(2-methyl-2-methylsulfonyl-propyl)pyrazole-4-sulfonamide;
N-(3-cyano-4-methyl-1H-indol-7-yl)-1-(2-oxaspiro[3.3]heptan-6-yl)pyrazole-4-sulfonamide;
N-(3-cyano-4-methyl-1H-indol-7-yl)-1-(4,4-difluorocyclohexyl)pyrazole-4-sulfonamide;
N-(3-cyano-4-methyl-1H-indol-7-yl)-1-[1-(2,2,2-trifluoroethyl)-4-piperidyl]pyrazole-4-sulfonamide;

N-(3-cyano-4-methyl-1H-indol-7-yl)-1-cyclopentyl-pyrazole-4-sulfonamide;
1-benzyl-N-(3-cyano-4-methyl-1H-indol-7-yl)pyrazole-4-sulfonamide;
N-(3-cyano-4-methyl-1H-indol-7-yl)-1-propyl-pyrazole-4-sulfonamide;
N-(4-chloro-3-cyano-1H-indol-7-yl)-1-(2,2-difluoroethyl)pyrazole-4-sulfonamide;
N-(3-cyano-4-fluoro-1H-indol-7-yl)-1-(2,2-difluoroethyl)pyrazole-4-sulfonamide;
N-(3-cyano-4-methyl-1H-indol-7-yl)-1-methylsulfonyl-pyrazole-4-sulfonamide;
1-*tert*-butyl-N-(3-cyano-4-methyl-1H-indol-7-yl)pyrazole-4-sulfonamide;
1-*tert*-butyl-N-(3-chloro-1H-indol-7-yl)pyrazole-4-sulfonamide;
N-[3-chloro-4-(trifluoromethyl)-1H-indol-7-yl]-1-methyl-pyrazole-4-sulfonamide;
N-(3-chloro-4-methyl-1H-indol-7-yl)-1-(2,2-difluoroethyl)pyrazole-4-sulfonamide;
N-(3-cyano-4-methyl-1H-indol-7-yl)-1-[[1-(trifluoromethyl)cyclopropyl]methyl]pyrazole-4-sulfonamide;
N-(3-cyano-4-methyl-1H-indol-7-yl)-1-(fluoromethylsulfonyl)pyrazole-4-sulfonamide;
1-(benzenesulfonyl)-N-(3-cyano-4-methyl-1H-indol-7-yl)pyrazole-4-sulfonamide;
N-(3-cyano-4-methyl-1H-indol-7-yl)-1-ethyl-5-fluoro-pyrazole-4-sulfonamide;
N-[3-chloro-4-(trifluoromethyl)-1H-indol-7-yl]-1-(2,2-difluoroethyl)pyrazole-4-sulfonamide;
N-[3-cyano-4-(trifluoromethyl)-1H-indol-7-yl]-1-(2,2-difluoroethyl)pyrazole-4-sulfonamide;
N-[3-cyano-4-(trifluoromethyl)-1H-indol-7-yl]-1-methyl-pyrazole-4-sulfonamide;
N-(3-cyano-4-methyl-1H-indol-7-yl)-1-[[3-(hydroxymethyl)oxetan-3-yl]methyl]pyrazole-4-sulfonamide;
N-(3-cyano-4-methyl-1H-indol-7-yl)-1-(3-methyloxetan-3-yl)pyrazole-4-sulfonamide;
N-(3-cyano-4-methyl-1H-indol-7-yl)-1-(2-hydroxy-2-methyl-propyl)pyrazole-4-sulfonamide;
N-(3-cyano-4-methyl-1H-indol-7-yl)-1-(3,3-difluorocyclobutyl)pyrazole-4-sulfonamide;
N-(3-cyano-4-methyl-1H-indol-7-yl)-1-(trideuteriomethyl)pyrazole-4-sulfonamide;
N-(3-cyano-4-methyl-1H-indol-7-yl)-1-(3-fluorocyclobutyl)pyrazole-4-sulfonamide;
N-(3-cyano-4-methyl-1H-indol-7-yl)-5-fluoro-1-methyl-pyrazole-4-sulfonamide;
N-(3-cyano-4-methyl-1H-indol-7-yl)-1,5-dimethyl-pyrazole-4-sulfonamide;
N-(3-cyano-4-methyl-1H-indol-7-yl)-1,3-dimethyl-pyrazole-4-sulfonamide;
N-(3,4-dichloro-1H-indol-7-yl)-1-(3-methyloxetan-3-yl)pyrazole-4-sulfonamide;
N-(3-cyano-4-methyl-1H-indol-7-yl)-1-[3-(hydroxymethyl)oxetan-3-yl]pyrazole-4-sulfonamide;
N-(3-cyano-4-methyl-1H-indol-7-yl)-1-[3-(fluoromethyl)oxetan-3-yl]pyrazole-4-sulfonamide;
N-(4-chloro-3-cyano-1H-indol-7-yl)-1-(3-methyloxetan-3-yl)pyrazole-4-sulfonamide;
N-(4-chloro-3-cyano-1H-indol-7-yl)-1-(difluoromethyl)pyrazole-4-sulfonamide;
N-(4-chloro-3-cyano-1H-indol-7-yl)-1-(2,2,2-trifluoroethyl)pyrazole-4-sulfonamide;
N-(4-chloro-3-cyano-1H-indol-7-yl)-1H-pyrazole-4-sulfonamide;
N-(4-chloro-3-cyano-1H-indol-7-yl)-5-fluoro-1-methyl-pyrazole-4-sulfonamide;
N-(4-chloro-3-cyano-1H-indol-7-yl)-1-(cyclopropylmethyl)pyrazole-4-sulfonamide;
N-(4-chloro-3-cyano-1H-indol-7-yl)-1-(fluoromethyl)pyrazole-4-sulfonamide;
N-(4-chloro-3-cyano-1H-indol-7-yl)-1-(oxetan-3-yl)pyrazole-4-sulfonamide;
N-(3-fluoro-4-methyl-1H-indol-7-yl)-1-methyl-pyrazole-4-sulfonamide;
N-(4-chloro-3-cyano-1H-indol-7-yl)-1-ethyl-pyrazole-4-sulfonamide;
N-(3-cyano-4-methyl-1H-indol-7-yl)-5-fluoro-1-(3-methyloxetan-3-yl)pyrazole-4-sulfonamide;
N-(3-chloro-2-fluoro-1H-indol-7-yl)-1-methyl-pyrazole-4-sulfonamide;
N-(4-chloro-3-cyano-1H-indol-7-yl)-5-fluoro-1-(3-methyloxetan-3-yl)pyrazole-4-sulfonamide;
5-cyano-N-(3-cyano-4-methyl-1H-indol-7-yl)-1-methyl-pyrazole-4-sulfonamide;
N-(4-chloro-3-cyano-1H-indol-7-yl)-5-cyano-1-methyl-pyrazole-4-sulfonamide;
methyl 2-[4-[(3-cyano-4-methyl-1H-indol-7-yl)sulfamoyl]pyrazol-1-yl]acetate;
2-[4-[(3-cyano-4-methyl-1H-indol-7-yl)sulfamoyl]pyrazol-1-yl]acetic acid;
N-(4-chloro-3-cyano-1H-indol-7-yl)-1-(trideuteriomethyl)pyrazole-4-sulfonamide;
N-(4-chloro-3-cyano-1H-indol-7-yl)-1-(2-hydroxy-2-methyl-propyl)pyrazole-4-sulfonamide;
2-[4-[(3-cyano-4-methyl-1H-indol-7-yl)sulfamoyl]pyrazol-1-yl]-N-(3,3-difluorocyclobutyl)acetamide;
N-(3-cyano-4-methyl-1H-indol-7-yl)-1-[2-[(3R,4S)-3,4-difluoropyrrolidin-1-yl]-2-oxoethyl]pyrazole-4-sulfonamide;
2-[4-[(3-cyano-4-methyl-1H-indol-7-yl)sulfamoyl]pyrazol-1-yl]-N-methyl-acetamide;
2-[4-[(3-cyano-4-methyl-1H-indol-7-yl)sulfamoyl]pyrazol-1-yl]-N,N-dimethylacetamide;
2-[4-[(3-cyano-4-methyl-1H-indol-7-yl)sulfamoyl]pyrazol-1-yl]acetamide;
N-(3-cyano-4-methyl-1H-indol-7-yl)-1-(2-oxo-2-pyrrolidin-1-yl-ethyl)pyrazole-4-sulfonamide;
N-(3,4-dichloro-1H-indol-7-yl)-1-(oxetan-3-yl)pyrazole-4-sulfonamide;
N-(3-cyano-4-methyl-1H-indol-7-yl)-5-fluoro-1-(oxetan-3-yl)pyrazole-4-sulfonamide;
1-[(1-cyanocyclopropyl)methyl]-N-(3-cyano-4-methyl-1H-indol-7-yl)pyrazole-4-sulfonamide;

N-(3-cyano-4-methyl-1H-indol-7-yl)-1-(2-oxopyrrolidin-3-yl)pyrazole-4-sulfonamide;
N-(4-chloro-3-cyano-1H-indol-7-yl)-1-(2-oxopyrrolidin-3-yl)pyrazole-4-sulfonamide;
N-(3-cyano-4-methyl-1H-indol-7-yl)-3-fluoro-1-methyl-pyrazole-4-sulfonamide;
N-(3-cyano-4-methyl-1H-indol-7-yl)-5-(difluoromethyl)-1-methyl-pyrazole-4-sulfonamide;
N-(4-chloro-3-cyano-1H-indol-7-yl)-5-(difluoromethyl)-1-methyl-pyrazole-4-sulfonamide;
N-(3-cyano-4-methyl-1H-indol-7-yl)-1-[(3 S,4R)-4-fluoropyrrolidin-3-yl]pyrazole-4-sulfonamide;
N-(4-chloro-3-cyano-1H-indol-7-yl)-1-[(3 S,4R)-4-fluoropyrrolidin-3-yl]pyrazole-4-sulfonamide;
*tert*-butyl (3S,4R)-3-[4-[(3-cyano-4-methyl-1H-indol-7-yl)sulfamoyl]pyrazol-1-yl]-4-fluoro-pyrrolidine-1-carboxylate;
2-[4-[(4-chloro-3-cyano-1H-indol-7-yl)sulfamoyl]pyrazol-1-yl]-2-methyl-propanamide;
5-chloro-N-(3-cyano-4-methyl-1H-indol-7-yl)-1-methyl-pyrazole-4-sulfonamide;
5-chloro-N-(4-chloro-3-cyano-1H-indol-7-yl)-1-methyl-pyrazole-4-sulfonamide;
N-(3-cyano-4-methyl-1H-indol-7-yl)-1-[3-(cyanomethyl)oxetan-3-yl]pyrazole-4-sulfonamide;
N-(4-chloro-3-cyano-1H-indol-7-yl)-1-[3-(cyanomethyl)oxetan-3-yl]pyrazole-4-sulfonamide;
N-(3,4-dichloro-1H-indol-7-yl)-1H-pyrazole-4-sulfonamide;
1-[3-(cyanomethyl)oxetan-3-yl]-N-(3,4-dichloro-1H-indol-7-yl)pyrazole-4-sulfonamide;
5-amino-N-(3-cyano-4-methyl-1H-indol-7-yl)-1-methyl-pyrazole-4-sulfonamide;
N-(4-chloro-3-cyano-1H-indol-7-yl)-1-(2-hydroxyethyl)pyrazole-4-sulfonamide;
N-(3,4-dichloro-1H-indol-7-yl)-1-(2-hydroxyethyl)pyrazole-4-sulfonamide;
N-(4-chloro-3-cyano-1H-indol-7-yl)-1-[1-(chloromethyl)-2-hydroxy-1-(hydroxymethyl)ethyl]pyrazole-4-sulfonamide;
N-(4-chloro-3-cyano-1H-indol-7-yl)-1-[3-(hydroxymethyl)oxetan-3-yl]pyrazole-4-sulfonamide;
1-[1-(chloromethyl)-2-hydroxy-1-(hydroxymethyl)ethyl]-N-(3,4-dichloro-1H-indol-7-yl)pyrazole-4-sulfonamide;
N-(3,4-dichloro-1H-indol-7-yl)-1-[3-(hydroxymethyl)oxetan-3-yl]pyrazole-4-sulfonamide;
methyl 2-[4-[(3,4-dichloro-1H-indol-7-yl)sulfamoyl]pyrazol-1-yl]-2-methyl-propanoate;
methyl 2-[4-[(4-chloro-3-cyano-1H-indol-7-yl)sulfamoyl]pyrazol-1-yl]-2-methylpropanoate;
N-(3,4-dichloro-1H-indol-7-yl)-1-(2-hydroxy-1,1-dimethyl-ethyl)pyrazole-4-sulfonamide;
N-(4-chloro-3-cyano-1H-indol-7-yl)-1-(2-hydroxy-1,1-dimethyl-ethyl)pyrazole-4-sulfonamide;
N-(3,4-dichloro-1H-indol-7-yl)-1-(2-hydroxy-1-methyl-ethyl)pyrazole-4-sulfonamide;
N-(3,4-dichloro-1H-indol-7-yl)-1-[2-hydroxy-1-(hydroxymethyl)-1-methylethyl]pyrazole-4-sulfonamide;
N-(4-chloro-3-cyano-1H-indol-7-yl)-1-(3-hydroxycyclobutyl)pyrazole-4-sulfonamide;
N-(4-chloro-3-cyano-1H-indol-7-yl)-1-(3-hydroxycyclobutyl)pyrazole-4-sulfonamide;
N-(3,4-dichloro-1H-indol-7-yl)-1-[2-hydroxy-1-(hydroxymethyl)ethyl]pyrazole-4-sulfonamide;
N-(3,4-dichloro-1H-indol-7-yl)-1-(2-hydroxy-2-methyl-propyl)pyrazole-4-sulfonamide;
1-(2-amino-2-methyl-propyl)-N-(4-chloro-3-cyano-1H-indol-7-yl)pyrazole-4-sulfonamide;
N-(4-chloro-3-cyano-1H-indol-7-yl)-1-[(1-hydroxycyclobutyl)methyl]pyrazole-4-sulfonamide;
5-amino-N-(4-chloro-3-cyano-1H-indol-7-yl)-1-methyl-pyrazole-4-sulfonamide;
N-(3,4-dichloro-1H-indol-7-yl)-1-(methylsulfonylmethyl)pyrazole-4-sulfonamide;
N-(4-chloro-3-cyano-1H-indol-7-yl)-1-(methylsulfonylmethyl)pyrazole-4-sulfonamide;
N-(4-chloro-3-cyano-1H-indol-7-yl)-1-[(1-cyanocyclopropyl)methyl]pyrazole-4-sulfonamide;
N-(4-chloro-3-cyano-1H-indol-7-yl)-1-[(4-hydroxytetrahydropyran-4-yl)methyl]pyrazole-4-sulfonamide;
N-(4-chloro-3-cyano-1H-indol-7-yl)-5-fluoro-1-(2-hydroxy-2-methyl-propyl)pyrazole-4-sulfonamide;
N-(4-chloro-3-cyano-1H-indol-7-yl)-1-(3-methoxycyclobutyl)pyrazole-4-sulfonamide;
N-(3-chloro-1H-indol-7-yl)-1-(3-methoxycyclobutyl)pyrazole-4-sulfonamide;
N-(4-chloro-3-cyano-1H-indol-7-yl)-1-[1-(hydroxymethyl)cyclobutyl]pyrazole-4-sulfonamide;
N-(3-cyano-4-fluoro-1H-indol-7-yl)-1-(2-hydroxy-2-methyl-propyl)pyrazole-4-sulfonamide;
N-(3-cyano-4-methyl-1H-indol-7-yl)-1-(3-hydroxycyclobutyl)pyrazole-4-sulfonamide;
N-(3-chloro-1H-indol-7-yl)-1-(3-hydroxycyclobutyl)pyrazole-4-sulfonamide;
N-(4-chloro-3-cyano-1H-indol-7-yl)-1-[[1-(hydroxymethyl)cyclopropyl]methyl]pyrazole-4-sulfonamide;
N-(4-chloro-3-cyano-1H-indol-7-yl)-1-[(2S)-2-hydroxypropyl]pyrazole-4-sulfonamide;
N-(4-chloro-3-cyano-1H-indol-7-yl)-1-[(2R)-2-hydroxypropyl]pyrazole-4-sulfonamide;
N-(3-cyano-4-methyl-1H-indol-7-yl)-1-(2-hydroxy-1,1-dimethyl-ethyl)pyrazole-4-sulfonamide;
N-(4-chloro-3-cyano-1H-indol-7-yl)-3-fluoro-1-(2-hydroxy-2-methyl-propyl)pyrazole-4-sulfonamide;
N-(3-cyano-4-methyl-1H-indol-7-yl)-3-fluoro-1-(2-hydroxy-2-methyl-propyl)pyrazole-4-sulfonamide;
N-(3-cyano-4-fluoro-1H-indol-7-yl)-3-fluoro-1-(2-hydroxy-2-methyl-propyl)pyrazole-4-sulfonamide;
N-(3-cyano-4-methyl-1H-indol-7-yl)-1-[(1R)-2-hydroxy-1-methyl-ethyl]pyrazole-4-sulfonamide;
N-(4-chloro-3-cyano-1H-indol-7-yl)-1-[(1R)-2-hydroxy-1-methyl-ethyl]pyrazole-4-sulfonamide;
N-(4-chloro-3-cyano-1H-indol-7-yl)-1-(3-hydroxypropyl)pyrazole-4-sulfonamide;

N-(3-cyano-4-methyl-1H-indol-7-yl)-1-(3-hydroxypropyl)pyrazole-4-sulfonamide;
N-(3-cyano-4-fluoro-1H-indol-7-yl)-1-(3-hydroxypropyl)pyrazole-4-sulfonamide;
N-(3-cyano-4-fluoro-1H-indol-7-yl)-1-[(1R)-2-hydroxy-1-methyl-ethyl]pyrazole-4-sulfonamide;
(2R)-2-[4-[(4-chloro-3-cyano-1H-indol-7-yl)sulfamoyl]pyrazol-1-yl]propenamide;
N-(3-cyano-4-fluoro-1H-indol-7-yl)-1H-pyrazole-4-sulfonamide;
N-(3-cyano-4-fluoro-1H-indol-7-yl)-1-(difluoromethyl)pyrazole-4-sulfonamide;
N-(3-cyano-4-fluoro-1H-indol-7-yl)-1-(2,2,2-trifluoroethyl)pyrazole-4-sulfonamide;
N-(3-cyano-4-fluoro-1H-indol-7-yl)-1-ethyl-pyrazole-4-sulfonamide;
1-(3-bicyclo[1.1.1]pentanyl)-N-(3-cyano-4-methyl-1H-indol-7-yl)pyrazole-4-sulfonamide;
1-(3-bicyclo[1.1.1]pentanyl)-N-(3-cyano-4-fluoro-1H-indol-7-yl)pyrazole-4-sulfonamide;
N-(4-chloro-3-cyano-1H-indol-7-yl)-1-(3-hydroxy-3-methyl-butyl)pyrazole-4-sulfonamide;
N-(3-cyano-4-methyl-1H-indol-7-yl)-1-(3-hydroxy-3-methyl-butyl)pyrazole-4-sulfonamide;
N-(3-cyano-4-fluoro-1H-indol-7-yl)-1-(3-hydroxy-3-methyl-butyl)pyrazole-4-sulfonamide;
1-(3-bicyclo[1.1.1]pentanyl)-N-(4-chloro-3-cyano-1H-indol-7-yl)pyrazole-4-sulfonamide;
N-(3-cyano-4-fluoro-1H-indol-7-yl)-1-(2-hydroxy-1,1-dimethyl-ethyl)pyrazole-4-sulfonamide;
N-(3-cyano-4-fluoro-1H-indol-7-yl)-1-[(1R)-2-fluoro-1-methyl-ethyl]pyrazole-4-sulfonamide;
N-(4-chloro-3-cyano-1H-indol-7-yl)-1-[(1S)-2,2-difluoro-1-(hydroxymethyl)ethyl]pyrazole-4-sulfonamide;
N-(3-cyano-4-methyl-1H-indol-7-yl)-1-[(1S)-2,2-difluoro-1-(hydroxymethyl)ethyl]pyrazole-4-sulfonamide;
N-(3-cyano-4-fluoro-1H-indol-7-yl)-1-[(1S)-2,2-difluoro-1-(hydroxymethyl)ethyl]pyrazole-4-sulfonamide;
N-(3 -cyano-4-methyl- 1H-indol-7-yl)-1-(3-hydroxy-1, 1-dimethyl-propyl)pyrazole-4-sulfonamide;
N-(4-chloro-3-cyano-1H-indol-7-yl)-1-(3-hydroxy-1,1-dimethyl-propyl)pyrazole-4-sulfonamide;
N-(3-cyano-4-methyl-1H-indol-7-yl)-1-(3-hydroxy-1,1,3-trimethyl-butyl)pyrazole-4-sulfonamide;
N-(4-chloro-3-cyano-1H-indol-7-yl)-1-[(1S,2R)-2-hydroxy-1-methyl-propyl]pyrazole-4-sulfonamide;
N-(3-cyano-4-fluoro-1H-indol-7-yl)-1-[(1S,2R)-2-hydroxy-1-methyl-propyl]pyrazole-4-sulfonamide;
N-(3-cyano-4-methyl-1H-indol-7-yl)-1-[(1S,2R)-2-hydroxy-1-methyl-propyl]pyrazole-4-sulfonamide;
N-(3-cyano-1H-indol-7-yl)-1-(2-hydroxy-1,1-dimethyl-ethyl)pyrazole-4-sulfonamide;
N-(3-cyano-4-methyl-1H-indol-7-yl)-1-(3-hydroxy-1-methyl-propyl)pyrazole-4-sulfonamide;
N-(4-chloro-3-cyano-1H-indol-7-yl)-1-(3-hydroxy-1-methyl-propyl)pyrazole-4-sulfonamide;
N-(4-chloro-3-cyano-1H-indol-7-yl)-1-[(1S)-1-(fluoromethyl)-2-hydroxy-ethyl]pyrazole-4-sulfonamide;
N-(3-cyano-4-methyl-1H-indol-7-yl)-5-(2-hydroxyethyl)-1-methyl-pyrazole-4-sulfonamide;
2-[4-[(3-cyano-4-fluoro-1H-indol-7-yl)sulfamoyl]pyrazol-1-yl]-2-methyl-propanamide;
2-[4-[(3-cyano-4-methyl-1H-indol-7-yl)sulfamoyl]pyrazol-1-yl]-2-methyl-propanamide;
N-(3-cyano-4-methyl-1H-indol-7-yl)-1-(3-hydroxy-1,3-dimethyl-butyl)pyrazole-4-sulfonamide;
N-(4-chloro-3-cyano-1H-indol-7-yl)-1-(3-hydroxy-1,3-dimethyl-butyl)pyrazole-4-sulfonamide;
5-chloro-N-(3-cyano-4-methyl-1H-indol-7-yl)-1-(2-hydroxy-1,1-dimethylethyl)pyrazole-4-sulfonamide;
5-chloro-N-(3-cyano-4-fluoro-1H-indol-7-yl)-1-(2-hydroxy-1,1-dimethyl-ethyl)pyrazole-4-sulfonamide;
N-(3-cyano-4-methyl-1H-indol-7-yl)-1-[(1S)-1-(fluoromethyl)-2-hydroxyethyl]pyrazole-4-sulfonamide;
N-(3-cyano-4-fluoro-1H-indol-7-yl)-1-[(1S)-1-(fluoromethyl)-2-hydroxy-ethyl]pyrazole-4-sulfonamide;
1-[(2S)-2-amino-3,3,3-trifluoro-propyl]-N-(4-chloro-3-cyano-1H-indol-7-yl)pyrazole-4-sulfonamide;
1-[(2S)-2-amino-3,3,3-trifluoro-propyl]-N-(3-cyano-4-methyl-1H-indol-7-yl)pyrazole-4-sulfonamide;
5-chloro-N-(4-chloro-3-cyano-1H-indol-7-yl)-1-(2-hydroxy-1,1-dimethyl-ethyl)pyrazole-4-sulfonamide;
5-chloro-N-(3-cyano-1H-indol-7-yl)-1-(2-hydroxy-1,1-dimethyl-ethyl)pyrazole-4-sulfonamide;
3-chloro-N-(3-cyano-4-methyl-1H-indol-7-yl)-1-(2-hydroxy-1,1-dimethylethyl)pyrazole-4-sulfonamide;
3-chloro-N-(3-cyano-1H-indol-7-yl)-1-(2-hydroxy-1,1-dimethyl-ethyl)pyrazole-4-sulfonamide;
*tert*-butyl 3-[[4-[(4-chloro-3-cyano-1H-indol-7-yl)sulfamoyl]pyrazol-1-yl]methyl]-3-fluoro-azetidine-1-carboxylate;
*tert*-butyl 3-[[4-[(3-cyano-4-methyl-1H-indol-7-yl)sulfamoyl]pyrazol-1-yl]methyl]-3-fluoro-azetidine-1-carboxylate;
N-(4-chloro-3-cyano-1H-indol-7-yl)-1-[(3-fluoroazetidin-3-yl)methyl]pyrazole-4-sulfonamide;
N-(3-cyano-4-methyl-1H-indol-7-yl)-1-[(3-fluoroazetidin-3-yl)methyl]pyrazole-4-sulfonamide;
N-(3-cyano-4-methyl-1H-indol-7-yl)-1-(3-hydroxy-2,2-dimethyl-propyl)pyrazole-4-sulfonamide;
N-(4-chloro-3-cyano-1H-indol-7-yl)-1-(3-hydroxy-2,2-dimethyl-propyl)pyrazole-4-sulfonamide;
3-chloro-N-(4-chloro-3-cyano-1H-indol-7-yl)-1-(2-hydroxy-1,1-dimethyl-ethyl)pyrazole-4-sulfonamide;
3-chloro-N-(3-cyano-4-fluoro-1H-indol-7-yl)-1-(2-hydroxy-1,1-dimethyl-ethyl)pyrazole-4-sulfonamide;
N-(4-chloro-3-cyano-1H-indol-7-yl)-1-[1-(hydroxymethyl)-2-methoxy-ethyl]pyrazole-4-sulfonamide;
N-(3-cyano-4-methyl-1H-indol-7-yl)-1-[1-(hydroxymethyl)-2-methoxy-ethyl]pyrazole-4-sulfonamide;
N-(3-cyano-4-methyl-1H-indol-7-yl)-1-[(2R)-3-hydroxy-2-methyl-propyl]pyrazole-4-sulfonamide;
N-(4-chloro-3-cyano-1H-indol-7-yl)-1-[(2R)-3-hydroxy-2-methyl-propyl]pyrazole-4-sulfonamide;

N-(4-chloro-3-cyano-1H-indol-7-yl)-1-[(1S)-2-hydroxy-1-methyl-ethyl]pyrazole-4-sulfonamide;
N-(3-cyano-4-fluoro-1H-indol-7-yl)-1-[(1S)-2-hydroxy-1-methyl-ethyl]pyrazole-4-sulfonamide;
N-(3-cyano-4-methyl-1H-indol-7-yl)-1-[1-(hydroxymethyl)cyclopropyl]pyrazole-4-sulfonamide;
N-(4-chloro-3-cyano-1H-indol-7-yl)-1-[1-(hydroxymethyl)cyclopropyl]pyrazole-4-sulfonamide;
N-(3-cyano-4-methyl-1H-indol-7-yl)-1-(3-hydroxybutyl)pyrazole-4-sulfonamide;
5-amino-N-(3-cyano-4-methyl-1H-indol-7-yl)-1-ethyl-pyrazole-4-sulfonamide;
5-amino-N-(3-cyano-4-fluoro-1H-indol-7-yl)-1-ethyl-pyrazole-4-sulfonamide;
5-amino-N-(4-chloro-3-cyano-1H-indol-7-yl)-1-ethyl-pyrazole-4-sulfonamide;
N-(3-cyano-4-methyl-1H-indol-7-yl)-1-[(1R,2S)-2-hydroxy-1-methyl-propyl]pyrazole-4-sulfonamide;
N-(4-chloro-3-cyano-1H-indol-7-yl)-1-[(1R,2S)-2-hydroxy-1-methyl-propyl]pyrazole-4-sulfonamide;
5-chloro-N-(3-cyano-4-methyl-1H-indol-7-yl)-1-(2-hydroxy-2-methyl-propyl)pyrazole-4-sulfonamide;
5-amino-N-(3-cyano-4-methyl-1H-indol-7-yl)-1-(2,2-difluoroethyl)pyrazole-4-sulfonamide;
5-amino-N-(4-chloro-3-cyano-1H-indol-7-yl)-1-(2,2-difluoroethyl)pyrazole-4-sulfonamide;
5-amino-N-(3-cyano-4-fluoro-1H-indol-7-yl)-1-(2,2-difluoroethyl)pyrazole-4-sulfonamide;
5-chloro-N-(3-cyano-4-fluoro-1H-indol-7-yl)-1-(2-hydroxy-2-methyl-propyl)pyrazole-4-sulfonamide;
N-(3-cyano-4-methyl-1H-indol-7-yl)-1-[(1R)-2-fluoro-1-methyl-ethyl]pyrazole-4-sulfonamide;
5-amino-N-(3-cyano-4-fluoro-1H-indol-7-yl)-1-methyl-pyrazole-4-sulfonamide;
5-amino-N-(3-cyano-4-methyl-1H-indol-7-yl)-1-(2-hydroxy-2-methyl-propyl)pyrazole-4-sulfonamide;
5-amino-N-(3-cyano-4-methyl-1H-indol-7-yl)-1-(difluoromethyl)pyrazole-4-sulfonamide;
5-amino-N-(3-cyano-4-fluoro-1H-indol-7-yl)-1-(difluoromethyl)pyrazole-4-sulfonamide;
N-(4-chloro-3-cyano-1H-indol-7-yl)-1-[(1R,2R)-2-hydroxy-1-methyl-propyl]pyrazole-4-sulfonamide;
N-(3-cyano-4-methyl-1H-indol-7-yl)-1-[(1R,2R)-2-hydroxy-1-methyl-propyl]pyrazole-4-sulfonamide;
N-(3-cyano-4-fluoro-1H-indol-7-yl)-1-[(1R,2R)-2-hydroxy-1-methyl-propyl]pyrazole-4-sulfonamide;
5-amino-N-(4-chloro-3-cyano-1H-indol-7-yl)-1-(2-hydroxy-2-methyl-propyl)pyrazole-4-sulfonamide;
3-amino-N-(3-cyano-4-methyl-1H-indol-7-yl)-1-ethyl-pyrazole-4-sulfonamide;
3-amino-N-(3-cyano-4-fluoro-1H-indol-7-yl)-1-ethyl-pyrazole-4-sulfonamide;
5-amino-N-(3-cyano-1H-indol-7-yl)-1-(difluoromethyl)pyrazole-4-sulfonamide;
N-(3-cyano-4-methyl-1H-indol-7-yl)-1-(1-fluoro-2-hydroxy-1-methyl-ethyl)pyrazole-4-sulfonamide;
N-(3 -cyano-4-fluoro- 1H-indol-7-yl)-1-(1 -fluoro-2-hydroxy-1 -methyl-ethyl)pyrazole-4-sulfonamide
3-amino-N-(3-cyano-4-methyl-1H-indol-7-yl)-1H-pyrazole-4-sulfonamide;
3-amino-N-(4-chloro-3-cyano-1H-indol-7-yl)-1H-pyrazole-4-sulfonamide;
N-(3-cyano-4-methyl-1H-indol-7-yl)-1-(1-fluoro-2-hydroxy-ethyl)pyrazole-4-sulfonamide;
N-(3-cyano-4-fluoro-1H-indol-7-yl)-1-(1-fluoro-2-hydroxy-ethyl)pyrazole-4-sulfonamide;
5-amino-N-(3-cyano-1H-indol-7-yl)-1-methyl-pyrazole-4-sulfonamide;
3-amino-N-(3-cyano-4-fluoro-1H-indol-7-yl)-1H-pyrazole-4-sulfonamide;
N-(3-cyano-4-methyl-1H-indol-7-yl)-1-(1,1-difluoro-2-hydroxy-ethyl)pyrazole-4-sulfonamide;
N-(3-cyano-4-fluoro-1H-indol-7-yl)-1-(fluoromethyl)pyrazole-4-sulfonamide;
5-amino-N-(3-cyano-4-methyl-1H-indol-7-yl)-1-[(1R)-2-hydroxy-1-methylethyl]pyrazole-4-sulfonamide;
5-amino-N-(3-cyano-4-methyl-1H-indazol-7-yl)-1-methyl-pyrazole-4-sulfonamide;
N-(3-cyano-4-methyl-1H-indazol-7-yl)-1-(fluoromethyl)pyrazole-4-sulfonamide;
5-amino-N-(4-chloro-3-cyano-1H-indol-7-yl)-1-[(1R)-2-hydroxy-1-methylethyl]pyrazole-4-sulfonamide;
5-amino-N-(4-chloro-3-cyano-1H-indazol-7-yl)-1-methyl-pyrazole-4-sulfonamide;
5-amino-N-(3-cyano-4-methyl-1H-indol-7-yl)-1-(fluoromethyl)pyrazole-4-sulfonamide;
5-amino-N-(3-cyano-4-fluoro-1H-indol-7-yl)-1-(fluoromethyl)pyrazole-4-sulfonamide;
N-(4-chloro-3-cyano-1H-indazol-7-yl)-1-[(1R)-1-(fluoromethyl)-2-hydroxyethyl]pyrazole-4-sulfonamide;
N-(4-chloro-3-cyano-1H-indol-7-yl)-1-[(1R)-1-(fluoromethyl)-2-hydroxy-ethyl]pyrazole-4-sulfonamide;
5 -amino-N-(3-cyano-1H-indol-7-yl)-1-(fluoromethyl)pyrazole-4-sulfonamide;
N-(3-cyano-4-methyl-1H-indol-7-yl)-1-[(1R)-1-(fluoromethyl)-2-hydroxyethyl]pyrazole-4-sulfonamide;
N-(3-cyano-4-methyl-1H-indazol-7-yl)-1-[(1R)-1-(fluoromethyl)-2-hydroxyethyl]pyrazole-4-sulfonamide;
5-amino-N-(3-cyano-4-methyl-1H-indol-7-yl)-1-(2-hydroxy-1,1-dimethylethyl)pyrazole-4-sulfonamide; and
5-amino-N-(4-chloro-3-cyano-1H-indol-7-yl)-1-(2-hydroxy-1,1-dimethyl-ethyl)pyrazole-4-sulfonamide; or a pharmaceutically acceptable salt thereof.

**11.** A pharmaceutical composition comprising a compound of any one of claims 1-10 or a pharmaceutically acceptable salt thereof, and a pharmaceutically acceptable diluent or carrier.

**12.** A compound of any one of claims 1-10, or a pharmaceutically acceptable salt thereof, for use as a medicament for the treatment of cancer.

**13.** A compound of any one of claims 1-10, or a pharmaceutically acceptable salt thereof, for use in therapy.

**14.** A compound of any one of claims 1-10 or a pharmaceutically acceptable salt thereof for use in a method of treating cancer in a subject in need thereof, the method comprising administering to said subject a therapeutically effective amount of a compound of any one of claims 1-10 or a pharmaceutically acceptable salt thereof.

**15.** A use of the compound of any one of claims 1 to 10, or a pharmaceutically acceptable salt thereof, in the preparation of a medicament for modulating the degradation activity of RBM39 protein, in a patient in need thereof.

**16.** A process for preparing a compound of claim 1, which comprises reacting an intermediate of Formula A:

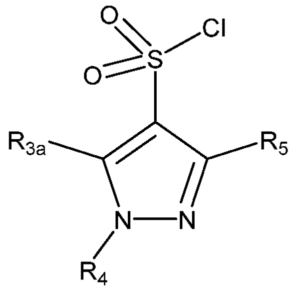

with an intermediate of Formula B:

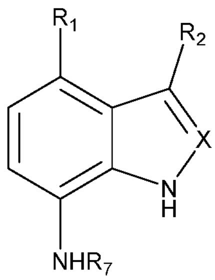

in the presence of a base to give a compound of claim 1, optionally adding or removing any protecting groups if desired, and optionally forming any salt if desired, wherein
$\mathbf{R_{3a}}$ is hydrogen, C1-C4 alkyl, halo, cyano, or -NR$^a$R$^b$, wherein R$^a$ or R$^b$ is independently hydrogen, C3-C6 cycloalkyl, heterocycloalkyl, -C(=O)O(C1-C4 alkyl) or C1-C4 alkyl, or R$^a$/R$^b$ and the nitrogen atom they are attached to form a 5 or 6-membered ring optionally independently substituted by one or more substituents selected from halo, C1-C4 alkyl, cyano, hydroxy and -O(C1-C4 alkyl).

## Patentansprüche

**1.** Eine Verbindung der Formel I:

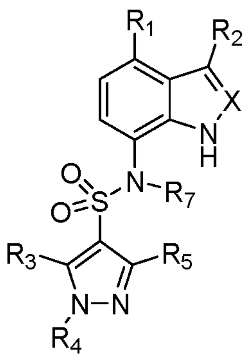

I

oder ein pharmazeutisch verträgliches Salz davon, wobei

**X** N oder **$CR_6$** ist;

**$R_1$** Wasserstoff, eine C1-C4-Alkylgruppe, eine C1-C4-Fluoralkylgruppe oder ein Halogen ist;

**$R_2$** ein Halogen oder eine Cyanogruppe ist;

**$R_3$** Wasserstoff, eine C1-C4-Alkylgruppe, ein Halogen, eine Cyanogruppe, eine C1-C4-Fluoralkylgruppe, -(C1-C4-Alkyl)OH, oder -$NR^aR^b$ ist, wobei $R^a$ oder $R^b$ unabhängig voneinander Wasserstoff, eine C3-C6-Cycloalkylgruppe, eine Heterocycloalkylgruppe oder eine C1-C4-Alkylgruppe sind oder $R^a$/$R^b$ und das Stickstoffatom, an das sie gebunden sind, einen 5- oder 6-gliedrigen Ring bilden, der optional unabhängig voneinander durch einen oder mehrere Substituenten substituiert ist, ausgewählt aus einem Halogen, einer C1-C4-Alkylgruppe, einer Cyanogruppe, einer Hydroxygruppe und -O(C1-C4-Alkyl);

**$R_4$** Wasserstoff, eine C1-C6-Alkylgruppe, eine C1-C10-Heteroalkylgruppe, eine C1-C4-Fluoralkylgruppe, -(C1-C4-Alkyl)$NH_2$, eine C3-C6-Cycloalkylgruppe, -(C1-C4-Alkyl)(C3-C6-Cycloalkyl), -(C1-C4-Alkyl)phenyl, eine Phenylgruppe, eine Heteroarylgruppe, -(C1-C4-Alkyl)heteroaryl, eine C2-C5-Alkenylgruppe, -(C1-C4-Alkyl)heterocycloalkyl, eine Heterocycloalkylgruppe, -CO-C4-AlkylS(=O)$_2$(C1-C4-Alkyl), -S(=O)$_2$phenyl, -S(=O)$_2$heteroaryl, -C0-C4-AlkylC(=O)heterocycloalkyl, -CO-C4-Alkyl C(=O)$NR^aR^b$, -(C0-C4-Alkyl)C(=O)O(C1-C4-Alkyl), -(C0-C4-Alkyl)C(=O)C1-C4-Alkyl, -(C0-C4-Alkyl)C(=O)OH oder -(C1-C8-Alkyl)OH ist, wobei **$R_4$** mit 0, 1, 2 oder 3 Substituenten substituiert ist, ausgewählt aus Deuterium, einem Halogen, einer Hydroxygruppe, -(C0-C4-Alkyl)O(C1-C4-Alkyl), -C1-C4-Alkyl, -(C0-C4-Alkyl)cyano, -S (=O)$_2$(C1-C4-Alkyl), -C0-C4-AlkylC(=O)$NR^aR^b$, -C(=O)O(C1-C4-Alkyl), C1-C4-Fluoralkyloxo, -(C1-C4-Alkyl)OH und -$NH_2$, wobei jedes $R^a$ und $R^b$ unabhängig voneinander mit 0, 1, 2 oder 3 Wasserstoff, C3-C6-Cycloalkyl, Heterocycloalkyl oder C1-C4-Alkyl substituiert ist;

**$R_5$** Wasserstoff, ein Halogen, -$NH_2$ oder eine C1-C4-Alkylgruppe ist;

**$R_6$** Wasserstoff, ein Halogen oder eine C1-C4-Alkylgruppe ist; und

**$R_7$** Wasserstoff, ein Halogen, eine Heteroalkylgruppe, eine C1-C4-Alkylgruppe, -(C0-C4-Alkyl)O( C1-C4-Alkyl), -(C1-C4-Alkyl)(C3-C6-Cycloalkyl), eine C3-C6-Cycloalkylgruppe, -(C1-C4-Alkyl)heterocycloalkyl, oder eine Heterocycloalkylgruppe ist.

**2.** Eine Verbindung nach Anspruch 1 oder ein pharmazeutisch verträgliches Salz davon, wobei $R_1$ eine C1-C4-Alkylgruppe oder ein Halogen ist.

**3.** Eine Verbindung nach Anspruch 2 oder ein pharmazeutisch verträgliches Salz davon, wobei $R_1$ eine Methylgruppe, Chlor oder Fluor ist.

**4.** Eine Verbindung nach Anspruch 1 oder ein pharmazeutisch verträgliches Salz davon, wobei $R_1$ Wasserstoff ist.

**5.** Eine Verbindung nach einem der Ansprüche 1 bis 4 oder ein pharmazeutisch verträgliches Salz davon, wobei $R_2$ Chlor, Fluor, oder eine Cyanogruppe ist.

**6.** Eine Verbindung nach einem der Ansprüche 1 bis 5 oder ein pharmazeutisch verträgliches Salz davon, wobei $R_3$ Wasserstoff, eine Methylgruppe, Fluor, Chlor, eine Difluormethylgruppe, -$NH_2$, oder -$C_2H_4OH$ ist.

**7.** Eine Verbindung nach einem der Ansprüche 1 bis 6 oder ein pharmazeutisch verträgliches Salz davon, wobei $R_4$ Wasserstoff, eine Methylgruppe, eine Ethylgruppe, eine Propylgruppe, eine Isopropylgruppe, eine tert-Butylgruppe, eine Fluormethylgruppe, eine Difluormethylgruppe, eine Difluorethylgruppe, eine Trifluorethylgruppe, eine Fluorpropylgruppe, eine Methoxyethylgruppe, eine Methoxyethoxyethylgruppe, eine Cyclopropylgruppe, eine Cyclobutylgruppe, eine Cyclopentylgruppe, eine Cyclohexylgruppe, eine 3-Bicyclo[1.1.1]pentanylgruppe, eine Cyclopropylmethylgruppe, eine Cyclobutylmethylgruppe, eine Cyclopentylmethylgruppe, eine Cyclohexylmethylgruppe, eine Cyclopropylethylgruppe, eine Cyclobutylethylgruppe, eine Cyclopentylethylgruppe und eine Cyclohexylmethylgruppe, eine Tetrahydrofuranylgruppe, eine Azetidinylgruppe, eine Tetrahydropyranylgruppe, eine Piperidinylgruppe, eine Oxetanylgruppe, eine Thietanylgruppe, eine Pyrrolidinylethylgruppe, eine Pyrrolidinylgruppe, eine Pyrrolidinylmethylgruppe, eine Azetidinylmethylgruppe, eine Tetrahydrothiopyranylmethylgruppe, eine Piperidinylmethylgruppe, eine Tetrahydropyranylmethylgruppe, eine Tetrahydrothiophenylgruppe, eine Oxetanylethylgruppe, eine Oxaspiro[3.3]heptylgruppe, eine (Methylsulfonyl)ethyl, 2-methyl-2-(methylsulfonyl)propylgruppe, eine Pyrazolyl(methylsulfonyl)-Gruppe, eine Methylsulfonylgruppe, eine (Methylsulfonyl)methylgruppe, eine Phenylsulfonylgruppe, eine Hydroxyethylgruppe, eine Aminocarbonylmethylgruppe, eine Carboxymethylgruppe, eine Cyclobutylaminocarbonylmethylgruppe, eine Pyrrolidinylcarbonylmethylgruppe, eine Methylaminocarbonylmethylgruppe, eine (Dimethylamino)carbonylmethyl-gruppe, eine Aminocarbonyl(C1-C4-Alkyl)-Gruppe, eine Methoxycarbonyl(C1-C4-Alkyl)-Gruppe, eine (Aminocarbonyl)C1-C4-Alkylgruppe, eine Phenylgruppe, eine Pyridinylgruppe, eine Propenyl-

gruppe,

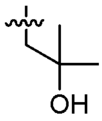, 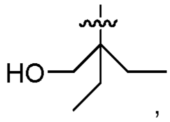, 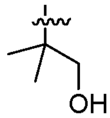, 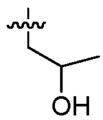, 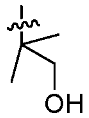,

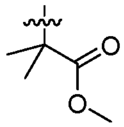, 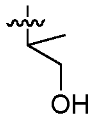, 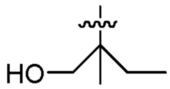, 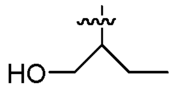,

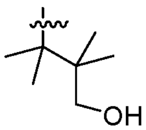, 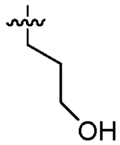, 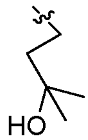, 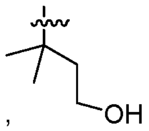, 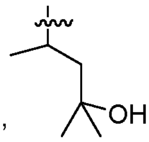, 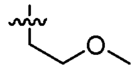,

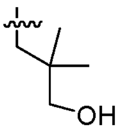, 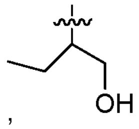, 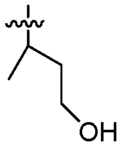, 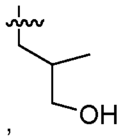, 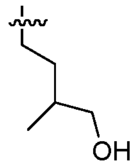,

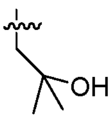, 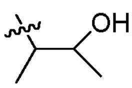, 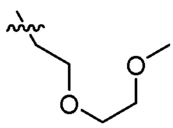 oder 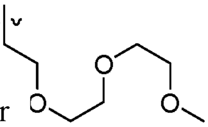

ist, und wobei $\mathbf{R_4}$ mit 0, 1, 2, oder 3 Substituenten substituiert ist, ausgewählt aus Deuterium, einem Halogen, einer Hydroxygruppe, -(C0-C4-Alkyl)O(C1-C4-Alkyl), -C1-C4-Alkyl, -(C0-C4-Alkyl)cyano, - C0-C4-AlkylC(=O)NR$^a$R$^b$, -C(=O)O(C1-C4-Alkyl), einer C1-C4 Fluoralkylgruppe, einer Oxogruppe, -(C1-C4-Alkyl)OH, und -NH$_2$, wobei jedes R$^a$ and R$^b$ unabhängig voneinander mit 0, 1, 2, oder 3 Wasserstoff, C3-C6-Cycloalkyl, Heterocycloalkyl oder C1-C4-Alkyl substituiert ist.

**8.** Eine Verbindung nach einem der Ansprüche 1 bis 7 oder ein pharmazeutisch verträgliches Salz davon, wobei $R_5$ Wasserstoff, eine Methylgruppe, Fluor, Chlor, oder -NH$_2$ ist.

**9.** Eine Verbindung nach einem der Ansprüche 1 bis 8 oder ein pharmazeutisch verträgliches Salz davon, wobei $R_2$ eine Cyanogruppe ist.

**10.** Eine Verbindung nach Anspruch 1, ausgewählt aus der Gruppe bestehend aus:

N-(3 -Chlor-1H-indol-7-yl)-1-methyl-pyrazol-4-sulfonamid;
N-(3-Chlor-1H-indol-7-yl)-1,3,5-trimethyl-pyrazol-4-sulfonamid;
N-(3-Cyano-4-methyl-1H-indol-7-yl)-1-methyl-pyrazol-4-sulfonamid;
N-(3 -Chlor-1H-indol-7-yl)-1-ethyl-pyrazol-4-sulfonamid;
N-(3-Chlor-1H-indol-7-yl)-1,3-dimethyl-pyrazol-4-sulfonamid;
N-(3-Chlor-1H-indol-7-yl)-1-(difluormethyl)pyrazol-4-sulfonamid;
N-(3-Chlor-1H-indol-7-yl)-1-(2,2-difluorethyl)pyrazol-4-sulfonamid;
N-(3-Chlor-1H-indol-7-yl)-1-tetrahydrofuran-3-yl-pyrazol-4-sulfonamid;
N-(3-Chlor-1H-indol-7-yl)-1-(2-methylsulfonylethyl)pyrazol-4-sulfonamid;
N-(3 -Chlor-1H-indol-7-yl)-1H-pyrazol-4-sulfonamid;

N-(3-Cyano-4-methyl-1H-indol-7-yl)-1-(difluormethyl)pyrazol-4-sulfonamid;
*tert*-Butyl-3-[4-[(3-chlor-1H-indol-7-yl)sulfamoyl]pyrazol-1-yl]azetidin-1-carboxylat;
N-(3-Chlor-1H-indol-7-yl)-1-(2-hydroxyethyl)pyrazol-4-sulfonamid;
N-(3-Chlor-1H-indol-7-yl)-1-(cyanomethyl)pyrazol-4-sulfonamid;
N-(3-Chlor-1H-indol-7-yl)-1,3,5-trimethyl-pyrazol-4-sulfonamid;
1-(5-Chlor-3-fluor-2-pyridyl)-N-(3-chlor-1H-indol-7-yl)pyrazol-4-sulfonamid;
2-[4-[(3-Chlor-1H-indol-7-yl)sulfamoyl]pyrazol-1-yl]acetamid;
2-[4-[4-[(3-Chlor-1H-indol-7-yl)sulfamoyl]pyrazol-1-yl]sulfonylpyrazol-1-yl]acetamid;
1-(Azetidin-3-yl)-N-(3-chlor-1H-indol-7-yl)pyrazol-4-sulfonamid;
N-(3-Chlor-1H-indol-7-yl)-1-(1H-pyrazol-4-ylsulfonyl)pyrazol-4-sulfonamid;
N-(3-Chlor-1H-indol-7-yl)-1-tetrahydropyran-4-yl-pyrazol-4-sulfonamid;
N-(3-Chlor-1H-indol-7-yl)-1-(5-fluor-2-pyridyl)pyrazol-4-sulfonamid;
N-(3-Chlor-1H-indol-7-yl)-1-[2-cyano-4-(trifluormethyl)phenyl]pyrazol-4-sulfonamid;
N-(3-Chlor-1H-indol-7-yl)-1-[2-(2-methoxyethoxy)ethyl]pyrazol-4-sulfonamid;
N-(3-Chlor-1H-indol-7-yl)-1-[2-[2-(2-methoxyethoxy)ethoxy]ethyl]pyrazol-4-sulfonamid;
N-(3-Cyano-4-methyl-1H-indol-7-yl)-1-(2-hydroxyethyl)pyrazol-4-sulfonamid;
*tert*-Butyl-4-[4-[(3-chlor-1H-indol-7-yl)sulfamoyl]pyrazol-1-yl]piperidin-1-carboxylat;
1-(1-Acetylazetidin-3-yl)-N-(3-chlor-1H-indol-7-yl)pyrazol-4-sulfonamid;
N-(3-Chlor-1H-indol-7-yl)-1-[(3-methyloxetan-3-yl)methyl]pyrazol-4-sulfonamid;
N-(3-Cyano-4-methyl-1H-indol-7-yl)-1-tetrahydrofuran-3-yl-pyrazol-4-sulfonamid;
N-(3-Chlor-1H-indol-7-yl)-1-[5-(trifluormethyl)-2-pyridyl]pyrazol-4-sulfonamid;
N-(3-Cyano-4-methyl-1H-indol-7-yl)-1-[5-(trifluormethyl)-2-pyridyl]pyrazol-4-sulfonamid;
N-(3-Chlor-1H-indol-7-yl)-1-(2,2-dimethoxyethyl)pyrazol-4-sulfonamid;
*tert*-Butyl-N-[2-[4-[(3-chlor-1H-indol-7-yl)sulfamoyl]pyrazol-1-yl]ethyl]carbamat;
1-(2-Aminoethyl)-N-(3-chlor-1H-indol-7-yl)pyrazol-4-sulfonamid;
N-(3-Chlor-1H-indol-7-yl)-1-(2-fluorethyl)pyrazol-4-sulfonamid;
N-(3-Cyano-4-methyl-1H-indol-7-yl)-1-(2-fluorethyl)pyrazol-4-sulfonamid;
N-(3-Chlor-1H-indol-7-yl)-1-phenyl-pyrazol-4-sulfonamid;
N-(3-Chlor-1H-indol-7-yl)-1-(2,2,2-trifluorethyl)pyrazol-4-sulfonamid;
N-(3-Chlor-1H-indol-7-yl)-1-isopropyl-pyrazol-4-sulfonamid;
N-(3-Chlor-1H-indol-7-yl)-1-(oxetan-3-yl)pyrazol-4-sulfonamid;
N-(3-Chlor-1H-indol-7-yl)-1-(4-piperidyl)pyrazol-4-sulfonamid;
*tert Butyl-3-[[4-[(3-chlor-1H-indol-7-yl)sulfamoyl]pyrazol-1-yl]methyl]azetidin-1-*carboxylat;
1-(Azetidin-3-ylmethyl)-N-(3-chlor-1H-indol-7-yl)pyrazol-4-sulfonamid;
N-(3-Chlor-1H-indol-7-yl)-1-[(3-methylthietan-3-yl)methyl]pyrazol-4-sulfonamid;
N-(3-Chlor-1H-indol-7-yl)-1-[(3-methyl-1,1-dioxothietan-3-yl)methyl]pyrazol-4-sulfonamid;
N-(3-Cyano-1H-indol-7-yl)-1-methyl-pyrazol-4-sulfonamid;
N-(3 -Chlor-1H-indol-7-yl)-1-cyclobutyl-pyrazol-4-sulfonamid;
N-(3-Chlor-1H-indol-7-yl)-N,1-bis(cyclopropylmethyl)pyrazol-4-sulfonamid;
N-(3-Chlor-1H-indol-7-yl)-1-(2-methoxyethyl)pyrazol-4-sulfonamid;
N-(3-Chlor-1H-indol-7-yl)-N,1-bis(2-methoxyethyl)pyrazol-4-sulfonamid;
N-(3-Chlor-1H-indol-7-yl)-1-(cyclopropylmethyl)pyrazol-4-sulfonamid;
N-(3-Chlor-1H-indol-7-yl)-1-[(1-cyanocyclopropyl)methyl]pyrazol-4-sulfonamid;
N-(3-Chlor-1H-indol-7-yl)-1-[(3,3-difluorcyclobutyl)methyl]pyrazol-4-sulfonamid;
N-(3-Chlor-4-fluor-1H-indol-7-yl)-1-methyl-pyrazol-4-sulfonamid;
N-(3 -Chlor-1H-indol-7-yl)-1-(fluormethyl)pyrazol-4-sulfonamid;
N-(3-Chlor-1H-indol-7-yl)-1-(3-fluorpropyl)pyrazol-4-sulfonamid;
N-(3-Chlor-1H-indol-7-yl)-1-(1,1-dioxothietan-3-yl)pyrazol-4-sulfonamid;
N-(3-Chlor-1H-indol-7-yl)-1-(2-cyanoethyl)pyrazol-4-sulfonamid;
N-(3-Cyano-4-methyl-1H-indol-7-yl)-1-(2,2,2-trifluorethyl)pyrazol-4-sulfonamid;
N-(3-Cyano-4-methyl-1H-indol-7-yl)-1-cyclobutyl-pyrazol-4-sulfonamid;
N-(3-Cyano-4-methyl-1H-indol-7-yl)-1H-pyrazol-4-sulfonamid;
N-(3-Cyano-4-methyl-1H-indol-7-yl)-1-isopropyl-pyrazol-4-sulfonamid;
N-(3-Cyano-4-methyl-1H-indol-7-yl)-1-(oxetan-3-yl)pyrazol-4-sulfonamid;
N-(3-Cyano-4-fluor-1H-indol-7-yl)-1-methyl-pyrazol-4-sulfonamid;
N-(3-Cyano-4-methyl-1H-indol-7-yl)-1-(2,2-difluorethyl)pyrazol-4-sulfonamid;
N-(3-Cyano-4-methyl-1H-indol-7-yl)-1-(2-methylsulfonylethyl)pyrazol-4-sulfonamid;
N-(3-Cyano-4-methyl-1H-indol-7-yl)-1-ethyl-pyrazol-4-sulfonamid;

N-(3-Cyano-4-methyl-1H-indol-7-yl)-1-(cyclopropylmethyl)pyrazol-4-sulfonamid;
N-(3-Cyano-4-methyl-1H-indol-7-yl)-1-(fluormethyl)pyrazol-4-sulfonamid;
N-(3-Cyano-4-methyl-1H-indol-7-yl)-1-[(3-methyloxetan-3-yl)methyl]pyrazol-4-sulfonamid;
N-(3-Cyano-4-methyl-1H-indol-7-yl)-1-[(3-fluorooxetan-3-yl)methyl]pyrazol-4-sulfonamid;
N-(3-Cyano-4-methyl-1H-indol-7-yl)-1-(3-fluoropropyl)pyrazol-4-sulfonamid;
N-(3-Chlor-4-methyl-1H-indol-7-yl)-1-methyl-pyrazol-4-sulfonamid;
N-(3-Cyano-4-methyl-1H-indol-7-yl)-1-(2-cyano-2-methyl-propyl)pyrazol-4-sulfonamid;
1-(2-Cyanoethyl)-N-(3 -cyano-4-methyl- 1H-indol-7-yl)pyrazol-4-sulfonamid;
N-(3-Cyano-4-methyl-1H-indol-7-yl)-1-(cyclobutylmethyl)pyrazol-4-sulfonamid;
N-(3-Cyano-4-methyl-1H-indol-7-yl)-1-[(2,2-difluorcyclopropyl)methyl]pyrazol-4-sulfonamid;
N-(3-Cyano-4-methyl-1H-indol-7-yl)-1-[1-(fluormethyl)vinyl]pyrazol-4-sulfonamid;
N-(3,4-Dichlor-1H-indol-7-yl)-1-methyl-pyrazol-4-sulfonamid;
N-(4-Chlor-3-cyano-1H-indol-7-yl)-1-methyl-pyrazol-4-sulfonamid;
N-(3-Chlor-1H-indol-7-yl)-1-(3-hydroxycyclobutyl)pyrazol-4-sulfonamid;
N-(3-Cyano-4-methyl-1H-indol-7-yl)-1-(2-methyl-2-methylsulfonyl-propyl)pyrazol-4-sulf onamid;
N-(3-Cyano-4-methyl-1H-indol-7-yl)-1-(2-oxaspiro[3.3]heptan-6-yl)pyrazol-4-sulfonamid;
N-(3-Cyano-4-methyl-1H-indol-7-yl)-1-(4,4-difluorcyclohexyl)pyrazol-4-sulfonamid;
N-(3-Cyano-4-methyl-1H-indol-7-yl)-1-[1-(2,2,2-trifluorethyl)-4-piperidyl]pyrazol-4-sulf onamid;
N-(3-Cyano-4-methyl-1H-indol-7-yl)-1-cyclopentyl-pyrazol-4-sulfonamid;
1-Benzyl-N-(3 -cyano-4-methyl- 1H-indol-7-yl)pyrazol-4-sulfonamid;
N-(3-Cyano-4-methyl-1H-indol-7-yl)-1-propyl-pyrazol-4-sulfonamid;
N-(4-Chlor-3-cyano-1H-indol-7-yl)-1-(2,2-difluorethyl)pyrazol-4-sulfonamid;
N-(3-Cyano-4-fluor-1H-indol-7-yl)-1-(2,2-difluorethyl)pyrazol-4-sulfonamid;
N-(3-Cyano-4-methyl-1H-indol-7-yl)-1-methylsulfonyl-pyrazol-4-sulfonamid;
1-*tert*-Butyl-N-(3 -cyano-4-methyl- 1H-indol-7-yl)pyrazol-4-sulfonamid;
1-*tert*-Butyl-N-(3 -chlor-1H-indol-7-yl)pyrazol-4-sulfonamid;
N-[3-Chlor-4-(trifluormethyl)-1H-indol-7-yl]-1-methyl-pyrazol-4-sulfonamid;
N-(3-Chlor-4-methyl-1H-indol-7-yl)-1-(2,2-difluorethyl)pyrazol-4-sulfonamid;
N-(3-Cyano-4-methyl-1H-indol-7-yl)-1-[[1-(trifluormethyl)cyclopropyl]methyl]pyrazol-4-sulfonamid;
N-(3-Cyano-4-methyl-1H-indol-7-yl)-1-(fluormethylsulfonyl)pyrazol-4-sulfonamid;
1-(Benzolsulfonyl)-N-(3-cyano-4-methyl-1H-indol-7-yl)pyrazol-4-sulfonamid;
N-(3-Cyano-4-methyl-1H-indol-7-yl)-1-ethyl-5-fluor-pyrazol-4-sulfonamid;
N-[3-Chlor-4-(trifluormethyl)-1H-indol-7-yl]-1-(2,2-difluorethyl)pyrazol-4-sulfonamid;
N-[3-Cyano-4-(trifluormethyl)-1H-indol-7-yl]-1-(2,2-difluorethyl)pyrazol-4-sulfonamid;
N-[3-Cyano-4-(trifluormethyl)-1H-indol-7-yl]-1-methyl-pyrazol-4-sulfonamid;
N-(3-Cyano-4-methyl-1H-indol-7-yl)-1-[[3-(hydroxymethyl)oxetan-3-yl]methyl]pyrazol-4-sulfonamid;
N-(3-Cyano-4-methyl-1H-indol-7-yl)-1-(3-methyloxetan-3-yl)pyrazol-4-sulfonamid;
N-(3-Cyano-4-methyl-1H-indol-7-yl)-1-(2-hydroxy-2-methyl-propyl)pyrazol-4-sulfonamid;
N-(3-Cyano-4-methyl-1H-indol-7-yl)-1-(3,3-difluorcyclobutyl)pyrazol-4-sulfonamid;
N-(3-Cyano-4-methyl-1H-indol-7-yl)-1-(trideuteridmethyl)pyrazol-4-sulfonamid;
N-(3-Cyano-4-methyl-1H-indol-7-yl)-1-(3-fluorcyclobutyl)pyrazol-4-sulfonamid;
N-(3-Cyano-4-methyl-1H-indol-7-yl)-5-fluor-1-methyl-pyrazol-4-sulfonamid;
N-(3-Cyano-4-methyl-1H-indol-7-yl)-1,5-dimethyl-pyrazol-4-sulfonamid;
N-(3-Cyano-4-methyl-1H-indol-7-yl)-1,3-dimethyl-pyrazol-4-sulfonamid;
N-(3,4-Dichlor-1H-indol-7-yl)-1-(3-methyloxetan-3-yl)pyrazol-4-sulfonamid;
N-(3-Cyano-4-methyl-1H-indol-7-yl)-1-[3-(hydroxymethyl)oxetan-3-yl]pyrazol-4-sulfonamid;
N-(3-Cyano-4-methyl-1H-indol-7-yl)-1-[3-(fluormethyl)oxetan-3-yl]pyrazol-4-sulfonamid;
N-(4-Chlor-3-cyano-1H-indol-7-yl)-1-(3-methyloxetan-3-yl)pyrazol-4-sulfonamid;
N-(4-Chlor-3-cyano-1H-indol-7-yl)-1-(difluormethyl)pyrazol-4-sulfonamid;
N-(4-Chlor-3-cyano-1H-indol-7-yl)-1-(2,2,2-trifluorethyl)pyrazol-4-sulfonamid;
N-(4-Chlor-3-cyano-1H-indol-7-yl)-1H-pyrazol-4-sulfonamid;
N-(4-Chlor-3-cyano-1H-indol-7-yl)-5-fluor-1-methyl-pyrazol-4-sulfonamid;
N-(4-Chlor-3-cyano-1H-indol-7-yl)-1-(cyclopropylmethyl)pyrazol-4-sulfonamid;
N-(4-Chlor-3-cyano-1H-indol-7-yl)-1-(fluormethyl)pyrazol-4-sulfonamid;
N-(4-Chlor-3-cyano-1H-indol-7-yl)-1-(oxetan-3-yl)pyrazol-4-sulfonamid;
N-(3-Fluor-4-methyl-1H-indol-7-yl)-1-methyl-pyrazol-4-sulfonamid;
N-(4-Chlor-3-cyano-1H-indol-7-yl)-1-ethyl-pyrazol-4-sulfonamid;
N-(3-Cyano-4-methyl-1H-indol-7-yl)-5-fluor-1-(3-methyloxetan-3-yl)pyrazol-4-sulfonamid;

N-(3-Chlor-2-fluor-1H-indol-7-yl)-1-methyl-pyrazol-4-sulfonamid;
N-(4-Chlor-3-cyano-1H-indol-7-yl)-5-fluor-1-(3-methyloxetan-3-yl)pyrazol-4-sulfonamid;
5-Cyano-N-(3-cyano-4-methyl-1H-indol-7-yl)-1-methyl-pyrazol-4-sulfonamid;
N-(4-Chlor-3-cyano-1H-indol-7-yl)-5-cyano-1-methyl-pyrazol-4-sulfonamid;
Methyl-2-[4-[(3-cyano-4-methyl-1H-indol-7-yl)sulfamoyl]pyrazol-1-yl]acetat;
2-[4-[(3-cyano-4-methyl-1H-indol-7-yl)sulfamoyl]pyrazol-1-yl]essigsäure;
N-(4-Chlor-3-cyano-1H-indol-7-yl)-1-(trideuteriomethyl)pyrazol-4-sulfonamid;
N-(4-Chlor-3-cyano-1H-indol-7-yl)-1-(2-hydroxy-2-methyl-propyl)pyrazol-4-sulfonamid;
2-[4-[(3-Cyano-4-methyl-1H-indol-7-yl)sulfamoyl]pyrazol-1-yl]-N-(3,3-difluorcy-clobutyl)acetamid;
N-(3-Cyano-4-methyl-1H-indol-7-yl)-1-[2-[(3R,4S)-3,4-difluorpyrrolidin-1-yl]-2-oxo-ethyl]pyrazol-4-sulfonamid;
2-[4-[(3-Cyano-4-methyl-1H-indol-7-yl)sulfamoyl]pyrazol-1-yl]-N-methylacetamid;
2-[4-[(3-Cyano-4-methyl-1H-indol-7-yl)sulfamoyl]pyrazol-1-yl]-N,N-dimethylacetamid;
2-[4-[(3-Cyano-4-methyl-1H-indol-7-yl)sulfamoyl]pyrazol-1-yl]acetamid;
N-(3-Cyano-4-methyl-1H-indol-7-yl)-1-(2-oxo-2-pyrrolidin-1-yl-ethyl)pyrazol-4-sulfonamid;
N-(3,4-Dichlor-1H-indol-7-yl)-1-(oxetan-3-yl)pyrazol-4-sulfonamid;
N-(3-Cyano-4-methyl-1H-indol-7-yl)-5-fluor-1-(oxetan-3-yl)pyrazol-4-sulfonamid;
1-[(1-Cyanocyclopropyl)methyl]-N-(3-cyano-4-methyl-1H-indol-7-yl)pyrazol-4-sulfonamid;
N-(3-Cyano-4-methyl-1H-indol-7-yl)-1-(2-oxopyrrolidin-3-yl)pyrazol-4-sulfonamid;
N-(4-Chlor-3-cyano-1H-indol-7-yl)-1-(2-oxopyrrolidin-3-yl)pyrazol-4-sulfonamid;
N-(3-Cyano-4-methyl-1H-indol-7-yl)-3-fluor-1-methyl-pyrazol-4-sulfonamid;
N-(3-Cyano-4-methyl-1H-indol-7-yl)-5-(difluormethyl)-1-methyl-pyrazol-4-sulfonamid;
N-(4-Chlor-3-cyano-1H-indol-7-yl)-5-(difluormethyl)-1-methyl-pyrazol-4-sulfonamid;
N-(3-Cyano-4-methyl-1H-indol-7-yl)-1-[(3 S,4R)-4-fluorpyrrolidin-3-yl]pyrazol-4-sulfonamid;
N-(4-Chlor-3-cyano-1H-indol-7-yl)-1-[(3S,4R)-4-fluorpyrrolidin-3-yl]pyrazol-4-sulfonamid;
*tert*-Butyl (3S,4R)-3-[4-[(3-Cyano-4-methyl-1H-indol-7-yl)sulfamoyl]pyrazol-1-yl]-4-fluorpyrrolidin-1 -carboxylat;
2-[4-[(4-Chlor-3-cyano-1H-indol-7-yl)sulfamoyl]pyrazol-1-yl]-2-methylpropanamid;
5-Chlor-N-(3-cyano-4-methyl-1H-indol-7-yl)-1-methyl-pyrazol-4-sulfonamid;
5-Chlor-N-(4-chlor-3-cyano-1H-indol-7-yl)-1-methyl-pyrazol-4-sulfonamid;
N-(3-Cyano-4-methyl-1H-indol-7-yl)-1-[3-(cyanomethyl)oxetan-3-yl]pyrazol-4-sulfonamid;
N-(4-Chlor-3-cyano-1H-indol-7-yl)-1-[3-(cyanomethyl)oxetan-3-yl]pyrazol-4-sulfonamid;
N-(3,4-Dichlor-1H-indol-7-yl)-1H-pyrazol-4-sulfonamid;
1-[3-(Cyanmethyl)oxetan-3-yl]-N-(3,4-dichlor-1H-indol-7-yl)pyrazol-4-sulfonamid;
5-Amino-N-(3-cyano-4-methyl-1H-indol-7-yl)-1-methyl-pyrazol-4-sulfonamid;
N-(4-Chlor-3-cyano-1H-indol-7-yl)-1-(2-hydroxyethyl)pyrazol-4-sulfonamid;
N-(3,4-Dichlor-1H-indol-7-yl)-1-(2-hydroxyethyl)pyrazol-4-sulfonamid;
N-(4-Chlor-3-cyano-1H-indol-7-yl)-1-[1-(chlormethyl)-2-hydroxy-1-(hydroxymethyl)ethyl]pyrazol-4-sulfonamid;
N-(4-Chlor-3-cyano-1H-indol-7-yl)-1-[3-(hydroxymethyl)oxetan-3-yl]pyrazol-4-sulfonamid;
1-[1-(Chlormethyl)-2-hydroxy-1-(hydroxymethyl)ethyl]-N-(3,4-dichlor-1H-indol-7-yl)pyrazol-4-sulfonamid;
N-(3,4-Dichlor-1H-indol-7-yl)-1-[3-(hydroxymethyl)oxetan-3-yl]pyrazol-4-sulfonamid;
Methyl-2-[4-[(3,4-dichlor-1H-indol-7-yl)sulfamoyl]pyrazol-1-yl]-2-methyl-propanoat;
Methyl-2-[4-[(4-chlor-3-cyano-1H-indol-7-yl)sulfamoyl]pyrazol-1-yl]-2-methyl-propanoat;
N-(3,4-Dichlor-1H-indol-7-yl)-1-(2-hydroxy-1, 1-dimethylethyl)pyrazol-4-sulfonamid;
N-(4-Chlor-3-cyano-1H-indol-7-yl)-1-(2-hydroxy-1,1-dimethylethyl)pyrazol-4-sulfonamid;
N-(3,4-Dichlor-1H-indol-7-yl)-1-(2-hydroxy-1-methylethyl)pyrazol-4-sulfonamid;
N-(3,4-Dichlor-1H-indol-7-yl)-1-[2-hydroxy-1-(hydroxymethyl)-1-methylethyl]pyrazol-4-sulfonamid;
N-(4-Chlor-3-cyano-1H-indol-7-yl)-1-(3-hydroxycyclobutyl)pyrazol-4-sulfonamid;
N-(4-Chlor-3-cyano-1H-indol-7-yl)-1-(3-hydroxycyclobutyl)pyrazol-4-sulfonamid;
N-(3,4-Dichlor-1H-indol-7-yl)-1-[2-hydroxy-1-(hydroxymethyl)ethyl]pyrazol-4-sulfonamid;
N-(3,4-Dichlor-1H-indol-7-yl)-1-(2-hydroxy-2-methyl-propyl)pyrazol-4-sulfonamid;
1-(2-Amino-2-methyl-propyl)-N-(4-chlor-3-cyano-1H-indol-7-yl)pyrazol-4-sulfonamid;
N-(4-Chlor-3-cyano-1H-indol-7-yl)-1-[(1-hydroxycyclobutyl)methyl]pyrazol-4-sulfonamid;
5-Amino-N-(4-chlor-3-cyano-1H-indol-7-yl)-1-methyl-pyrazol-4-sulfonamid;
N-(3,4-Dichlor-1H-indol-7-yl)-1-(methylsulfonylmethyl)pyrazol-4-sulfonamid;
N-(4-Chlor-3-cyano-1H-indol-7-yl)-1-(methylsulfonylmethyl)pyrazol-4-sulfonamid;
N-(4-Chlor-3-cyano-1H-indol-7-yl)-1-[(1-cyanocyclopropyl)methyl]pyrazol-4-sulfonamid;
N-(4-Chlor-3-cyano-1H-indol-7-yl)-1-[(4-hydroxytetrahydropyran-4-yl)methyl]pyrazol-4-sulfonamid;
N-(4-Chlor-3-cyano-1H-indol-7-yl)-5-fluor-1-(2-hydroxy-2-methyl-propyl)pyrazol-4-sulfonamid;
N-(4-Chlor-3-cyano-1H-indol-7-yl)-1-(3-methoxycyclobutyl)pyrazol-4-sulfonamid;

N-(3-Chlor-1H-indol-7-yl)-1-(3-methoxycyclobutyl)pyrazol-4-sulfonamid;
N-(4-Chlor-3-cyano-1H-indol-7-yl)-1-[1-(hydroxymethyl)cyclobutyl]pyrazol-4-sulfonamid;
N-(3-Cyano-4-fluor-1H-indol-7-yl)-1-(2-hydroxy-2-methyl-propyl)pyrazol-4-sulfonamid;
N-(3-Cyano-4-methyl-1H-indol-7-yl)-1-(3-hydroxycyclobutyl)pyrazol-4-sulfonamid;
N-(3-Chlor-1H-indol-7-yl)-1-(3-hydroxycyclobutyl)pyrazol-4-sulfonamid;
N-(4-Chlor-3-cyano-1H-indol-7-yl)-1-[[1-(hydroxymethyl)cyclopropyl]methyl]pyrazol-4-sulfonamid;
N-(4-Chlor-3-cyano-1H-indol-7-yl)-1-[(2S)-2-hydroxypropyl]pyrazol-4-sulfonamid;
N-(4-Chlor-3-cyano-1H-indol-7-yl)-1-[(2R)-2-hydroxypropyl]pyrazol-4-sulfonamid;
N-(3-Cyano-4-methyl-1H-indol-7-yl)-1-(2-hydroxy-1,1-dimethylethyl)pyrazol-4-sulfonamid;
N-(4-Chlor-3-cyano-1H-indol-7-yl)-3-fluor-1-(2-hydroxy-2-methylpropyl)pyrazol-4-sulfonamid;
N-(3-Cyano-4-methyl-1H-indol-7-yl)-3-fluor-1-(2-hydroxy-2-methyl-propyl)pyrazol-4-sulfonamid;
N-(3-Cyano-4-fluor-1H-indol-7-yl)-3-fluor-1-(2-hydroxy-2-methyl-propyl)pyrazol-4-sulfonamid;
N-(3-Cyano-4-methyl-1H-indol-7-yl)-1-[(1R)-2-hydroxy-1-methyl-ethyl]pyrazol-4-sulfonamid;
N-(4-Chlor-3-cyano-1H-indol-7-yl)-1-[(1R)-2-hydroxy-1-methyl-ethyl]pyrazol-4-sulfonamid;
N-(4-Chlor-3-cyano-1H-indol-7-yl)-1-(3-hydroxypropyl)pyrazol-4-sulfonamid;
N-(3-Cyano-4-methyl-1H-indol-7-yl)-1-(3-hydroxypropyl)pyrazol-4-sulfonamid;
N-(3-Cyano-4-fluor-1H-indol-7-yl)-1-(3-hydroxypropyl)pyrazol-4-sulfonamid;
N-(3-Cyano-4-fluor-1H-indol-7-yl)-1-[(1R)-2-hydroxy-1-methyl-ethyl]pyrazol-4-sulfonamid;
(2R)-2-[4-[(4-Chlor-3-cyano-1H-indol-7-yl)sulfamoyl]pyrazol-1-yl]propenamid;
N-(3-Cyano-4-fluor-1H-indol-7-yl)-1H-pyrazol-4-sulfonamid;
N-(3-Cyano-4-fluor-1H-indol-7-yl)-1-(difluormethyl)pyrazol-4-sulfonamid;
N-(3-Cyano-4-fluor-1H-indol-7-yl)-1-(2,2,2-trifluorethyl)pyrazol-4-sulfonamid;
N-(3-Cyano-4-fluor-1H-indol-7-yl)-1-ethyl-pyrazol-4-sulfonamid;
1-(3-Bicyclo[1. 1. 1]pentanyl)-N-(3-cyano-4-methyl-1H-indol-7-yl)pyrazol-4-sulfonamid;
1-(3-Bicyclo[1.1.1]pentanyl)-N-(3-cyano-4-fluor-1H-indol-7-yl)pyrazol-4-sulfonamid;
N-(4-Chlor-3-cyano-1H-indol-7-yl)-1-(3-hydroxy-3-methyl-butyl)pyrazol-4-sulfonamid;
N-(3-Cyano-4-methyl-1H-indol-7-yl)-1-(3-hydroxy-3-methyl-butyl)pyrazol-4-sulfonamid;
N-(3-Cyano-4-fluor-1H-indol-7-yl)-1-(3-hydroxy-3-methyl-butyl)pyrazol-4-sulfonamid;
1-(3-Bicyclo[1. 1. 1]pentanyl)-N-(4-chlor-3-cyano-1H-indol-7-yl)pyrazol-4-sulfonamid;
N-(3-Cyano-4-fluor-1H-indol-7-yl)-1-(2-hydroxy-1,1-dimethylethyl)pyrazol-4-sulfonamid;
N-(3-Cyano-4-fluor-1H-indol-7-yl)-1-[(1R)-2-fluor-1-methylethyl]pyrazol-4-sulfonamid;
N-(4-Chlor-3-cyano-1H-indol-7-yl)-1-[(1S)-2,2-difluor-1-(hydroxymethyl)ethyl]pyrazol-4-sulfonamid;
N-(3-Cyano-4-methyl-1H-indol-7-yl)-1-[(1S)-2,2-difluor-1-(hydroxymethyl)ethyl]pyrazol-4-sulfonamid;
N-(3-Cyano-4-fluor-1H-indol-7-yl)-1-[(1S)-2,2-difluor-1-(hydroxymethyl)ethyl]pyrazol-4-sulfonamid;
N-(3-Cyano-4-methyl-1H-indol-7-yl)-1-(3-hydroxy-1,1-dimethyl-propyl)pyrazol-4-sulfonamid;
N-(4-Chlor-3-cyano-1H-indol-7-yl)-1-(3-hydroxy-1,1-dimethyl-propyl)pyrazol-4-sulfonamid;
N-(3-Cyano-4-methyl-1H-indol-7-yl)-1-(3-hydroxy-1,1,3-trimethylbutyl)pyrazol-4-sulfonamid;
N-(4-Chlor-3-cyano-1H-indol-7-yl)-1-[(1S,2R)-2-hydroxy-1-methyl-propyl]pyrazol-4-sulfonamid;
N-(3-Cyano-4-fluor-1H-indol-7-yl)-1-[(1S,2R)-2-hydroxy-1-methyl-propyl]pyrazol-4-sulfonamid;
N-(3-Cyano-4-methyl-1H-indol-7-yl)-1-[(1S,2R)-2-hydroxy-1-methyl-propyl]pyrazol-4-sulfonamid;
N-(3-Cyano-1H-indol-7-yl)-1-(2-hydroxy-1, 1-dimethyl-ethyl)pyrazol-4-sulfonamid;
N-(3-Cyano-4-methyl-1H-indol-7-yl)-1-(3-hydroxy-1-methyl-propyl)pyrazol-4-sulfonamid;
N-(4-Chlor-3-cyano-1H-indol-7-yl)-1-(3-hydroxy-1-methyl-propyl)pyrazol-4-sulfonamid;
N-(4-Chlor-3-cyano-1H-indol-7-yl)-1-[(1S)-1-(fluormethyl)-2-hydroxyethyl]pyrazol-4-sulfonamid;
N-(3-Cyano-4-methyl-1H-indol-7-yl)-5-(2-hydroxyethyl)-1-methyl-pyrazol-4-sulfonamid;
2-[4-[(3-Cyano-4-fluor-1H-indol-7-yl)sulfamoyl]pyrazol-1-yl]-2-methyl-propanamid;
2-[4-[(3-Cyano-4-methyl-1H-indol-7-yl)sulfamoyl]pyrazol-1-yl]-2-methyl-propanamid;
N-(3-Cyano-4-methyl-1H-indol-7-yl)-1-(3-hydroxy-1,3-dimethylbutyl)pyrazol-4-sulfonamid;
N-(4-Chlor-3-cyano-1H-indol-7-yl)-1-(3-hydroxy-1,3-dimethylbutyl)pyrazol-4-sulfonamid;
5-Chlor-N-(3-cyano-4-methyl-1H-indol-7-yl)-1-(2-hydroxy-1,1-dimethylethyl)pyrazol-4-sulfonamid;
5-Chlor-N-(3-cyano-4-fluor-1H-indol-7-yl)-1-(2-hydroxy-1, 1 -dimethylethyl)pyrazol-4-sulfonamid;
N-(3-Cyano-4-methyl-1H-indol-7-yl)-1-[(1S)-1-(fluormethyl)-2-hydroxy-ethyl]pyrazol-4-sulfonamid;
N-(3-Cyano-4-fluor-1H-indol-7-yl)-1-[(1S)-1-(fluormethyl)-2-hydroxy-ethyl]pyrazol-4-sulfonamid;
1-[(2S)-2-Amino-3,3,3-trifluorpropyl]-N-(4-chlor-3-cyano-1H-indol-7-yl)pyrazol-4-sulfonamid;
1-[(2S)-2-Amino-3,3,3-trifluorpropyl]-N-(3-cyano-4-methyl-1H-indol-7-yl)pyrazol-4-sulfonamid;
5-Chlor-N-(4-chlor-3-cyano-1H-indol-7-yl)-1-(2-hydroxy-1,1-dimethylethyl)pyrazol-4-sulfonamid;
5-Chlor-N-(3-cyano-1H-indol-7-yl)-1-(2-hydroxy-1, 1-dimethylethyl)pyrazol-4-sulfonamid;
3-Chlor-N-(3-cyano-4-methyl-1H-indol-7-yl)-1-(2-hydroxy-1,1-dimethylethyl)pyrazol-4-sulfonamid;

3-Chlor-N-(3-cyano-1H-indol-7-yl)-1-(2-hydroxy-1,1-dimethylethyl)pyrazol-4-sulfonamid;
*tert*-Butyl-3-[[4-[(4-chlor-3-cyano-1H-indol-7-yl)sulfamoyl]pyrazol-1-yl]methyl]-3-fluorazetidin-1 -carboxylat;
*tert*-Butyl-3-[[4-[(3-cyano-4-methyl-1H-indol-7-yl)sulfamoyl]pyrazol-1-yl]methyl]-3-fluorazetidin-1-carboxylat;
N-(4-Chlor-3-cyano-1H-indol-7-yl)-1-[(3-fluorazetidin-3-yl)methyl]pyrazol-4-sulfonamid;
N-(3-Cyano-4-methyl-1H-indol-7-yl)-1-[(3-fluorazetidin-3-yl)methyl]pyrazol-4-sulfonamid;
N-(3-Cyano-4-methyl-1H-indol-7-yl)-1-(3-hydroxy-2,2-dimethylpropyl)pyrazol-4-sulfonamid;
N-(4-Chlor-3-cyano-1H-indol-7-yl)-1-(3-hydroxy-2,2-dimethylpropyl)pyrazol-4-sulfonamid;
3-Chlor-N-(4-chlor-3-cyano-1H-indol-7-yl)-1-(2-hydroxy-1,1-dimethylethyl)pyrazol-4-sulfonamid;
3 -Chlor-N-(3 -cyano-4-fluor-1H-indol-7-yl)-1-(2-hydroxy-1, 1 -dimethylethyl)pyrazol-4-sulfonamid;
N-(4-Chlor-3-cyano-1H-indol-7-yl)-1-[1-(hydroxymethyl)-2-methoxy-ethyl]pyrazol-4-sulf onamid;
N-(3-Cyano-4-methyl-1H-indol-7-yl)-1-[1-(hydroxymethyl)-2-methoxy-ethyl]pyrazol-4-sulfonamid;
N-(3-Cyano-4-methyl-1H-indol-7-yl)-1-[(2R)-3-hydroxy-2-methyl-propyl]pyrazol-4-sulfonamid;
N-(4-Chlor-3-cyano-1H-indol-7-yl)-1-[(2R)-3-hydroxy-2-methyl-propyl]pyrazol-4-sulfonamid;
N-(4-Chlor-3-cyano-1H-indol-7-yl)-1-[(1S)-2-hydroxy-1-methylethyl]pyrazol-4-sulfonamid;
N-(3 -Cyano-4-fluor-1H-indol-7-yl)-1-[(1S)-2-hydroxy-1-methylethyl]pyrazol-4-sulfonamid;
N-(3-Cyano-4-methyl-1H-indol-7-yl)-1-[1-(hydroxymethyl)cyclopropyl]pyrazol-4-sulfonamid;
N-(4-Chlor-3-cyano-1H-indol-7-yl)-1-[1-(hydroxymethyl)cyclopropyl]pyrazol-4-sulfonamid;
N-(3-Cyano-4-methyl-1H-indol-7-yl)-1-(3-hydroxybutyl)pyrazol-4-sulfonamid;
5-Amino-N-(3-cyano-4-methyl-1H-indol-7-yl)-1-ethyl-pyrazol-4-sulfonamid;
5-Amino-N-(3-cyano-4-fluor-1H-indol-7-yl)-1-ethyl-pyrazol-4-sulfonamid;
5-Amino-N-(4-chlor-3-cyano-1H-indol-7-yl)-1-ethyl-pyrazol-4-sulfonamid;
N-(3-Cyano-4-methyl-1H-indol-7-yl)-1-[(1R,2S)-2-hydroxy-1-methyl-propyl]pyrazol-4-sulfonamid;
N-(4-Chlor-3-cyano-1H-indol-7-yl)-1-[(1R,2S)-2-hydroxy-1-methyl-propyl]pyrazol-4-sulfonamid;
5-Chlor-N-(3-cyano-4-methyl-1H-indol-7-yl)-1-(2-hydroxy-2-methyl-propyl)pyrazol-4-sulfonamid;
5-Amino-N-(3-cyano-4-methyl-1H-indol-7-yl)-1-(2,2-difluorethyl)pyrazol-4-sulfonamid;
5-Amino-N-(4-chlor-3-cyano-1H-indol-7-yl)-1-(2,2-difluorethyl)pyrazol-4-sulfonamid;
5-Amino-N-(3-cyano-4-fluor-1H-indol-7-yl)-1-(2,2-difluorethyl)pyrazol-4-sulfonamid;
5-Chlor-N-(3-cyano-4-fluor-1H-indol-7-yl)-1-(2-hydroxy-2-methyl-propyl)pyrazol-4-sulfonamid;
N-(3-Cyano-4-methyl-1H-indol-7-yl)-1-[(1R)-2-fluor-1-methylethyl]pyrazol-4-sulfonamid;
5-Amino-N-(3-cyano-4-fluor-1H-indol-7-yl)-1-methyl-pyrazol-4-sulfonamid;
5-Amino-N-(3-cyano-4-methyl-1H-indol-7-yl)-1-(2-hydroxy-2-methyl-propyl)pyrazol-4-sulfonamid;
5-Amino-N-(3-cyano-4-methyl-1H-indol-7-yl)-1-(difluormethyl)pyrazol-4-sulfonamid;
5-Amino-N-(3-cyano-4-fluor-1H-indol-7-yl)-1-(difluormethyl)pyrazol-4-sulfonamid;
N-(4-Chlor-3-cyano-1H-indol-7-yl)-1-[(1R,2R)-2-hydroxy-1-methyl-propyl]pyrazol-4-sulfonamid;
N-(3-Cyano-4-methyl-1H-indol-7-yl)-1-[(1R,2R)-2-hydroxy-1-methyl-propyl]pyrazol-4-sulfonamid;
N-(3 -Cyano-4-fluor-1H-indol-7-yl)-1-[(1R,2R)-2-hydroxy-1-methyl-propyl]pyrazol-4-sulfonamid;
5-Amino-N-(4-chlor-3-cyano-1H-indol-7-yl)-1-(2-hydroxy-2-methyl-propyl)pyrazol-4-sulfonamid;
3-Amino-N-(3-cyano-4-methyl-1H-indol-7-yl)-1-ethyl-pyrazol-4-sulfonamid;
3-Amino-N-(3-cyano-4-fluor-1H-indol-7-yl)-1-ethyl-pyrazol-4-sulfonamid;
5-Amino-N-(3-cyano-1H-indol-7-yl)-1-(difluormethyl)pyrazol-4-sulfonamid;
N-(3-Cyano-4-methyl-1H-indol-7-yl)-1-(1-fluor-2-hydroxy-1-methylethyl)pyrazol-4-sulfonamid;
N-(3-Cyano-4-fluor-1H-indol-7-yl)-1-(1-fluor-2-hydroxy-1-methylethyl)pyrazol-4-sulfonamid;
3-Amino-N-(3-cyano-4-methyl-1H-indol-7-yl)-1H-pyrazol-4-sulfonamid;
3-Amino-N-(4-chlor-3-cyano-1H-indol-7-yl)-1H-pyrazol-4-sulfonamid;
N-(3-Cyano-4-methyl-1H-indol-7-yl)-1-(1-fluor-2-hydroxy-ethyl)pyrazol-4-sulfonamid;
N-(3-Cyano-4-fluor-1H-indol-7-yl)-1-(1-fluor-2-hydroxy-ethyl)pyrazol-4-sulfonamid;
5-Amino-N-(3-cyano-1H-indol-7-yl)-1-methyl-pyrazol-4-sulfonamid;
3-Amino-N-(3-cyano-4-fluor-1H-indol-7-yl)-1H-pyrazol-4-sulfonamid;
N-(3-Cyano-4-methyl-1H-indol-7-yl)-1-(1,1-difluor-2-hydroxyethyl)pyrazol-4-sulfonamid;
N-(3-Cyano-4-fluor-1H-indol-7-yl)-1-(fluormethyl)pyrazol-4-sulfonamid;
5-Amino-N-(3-cyano-4-methyl-1H-indol-7-yl)-1-[(1R)-2-hydroxy-1-methylethyl]pyrazol-4-sulfonamid;
5-Amino-N-(3-cyano-4-methyl-1H-indazol-7-yl)-1-methyl-pyrazol-4-sulfonamid;
N-(3-Cyano-4-methyl-1H-indazol-7-yl)-1-(fluormethyl)pyrazol-4-sulfonamid;
5-Amino-N-(4-chlor-3-cyano-1H-indol-7-yl)-1-[(1R)-2-hydroxy-1-methyl-ethyl]pyrazol-4-sulfonamid;
5-Amino-N-(4-chlor-3-cyano-1H-indazol-7-yl)-1-methyl-pyrazol-4-sulfonamid;
5-Amino-N-(3-cyano-4-methyl-1H-indol-7-yl)-1-(fluormethyl)pyrazol-4-sulfonamid;
5-Amino-N-(3-cyano-4-fluor-1H-indol-7-yl)-1-(fluormethyl)pyrazol-4-sulfonamid;
N-(4-Chlor-3-cyano-1H-indazol-7-yl)-1-[(1R)-1-(fluormethyl)-2-hydroxy-ethyl]pyrazol-4-sulfonamid;

N-(4-Chlor-3-cyano-1H-indol-7-yl)-1-[(1R)-1-(fluormethyl)-2-hydroxyethyl]pyrazol-4-sulfonamid;
5-Amino-N-(3-cyano-1H-indol-7-yl)-1-(fluormethyl)pyrazol-4-sulfonamid;
N-(3-Cyano-4-methyl-1H-indol-7-yl)-1-[(1R)-1-(fluormethyl)-2-hydroxy-ethyl]pyrazol-4-sulfonamid;
N-(3-Cyano-4-methyl-1H-indazol-7-yl)-1-[(1R)-1-(fluormethyl)-2-hydroxy-ethyl]pyrazol-4-sulfonamid;
5-Amino-N-(3-cyano-4-methyl-1H-indol-7-yl)-1-(2-hydroxy-1,1-dimethylethyl)pyrazol-4-sulfonamid und
5-Amino-N-(4-chlor-3-cyano-1H-indol-7-yl)-1-(2-hydroxy-1,1-dimethylethyl)pyrazol-4-sulfonamid; oder ein pharmazeutisch verträgliches Salz davon.

**11.** Eine pharmazeutische Zusammensetzung, die eine Verbindung nach einem der Ansprüche 1-10 oder ein pharmazeutisch verträgliches Salz davon und ein pharmazeutisch verträgliches Verdünnungsmittel oder Träger umfasst.

**12.** Eine Verbindung nach einem der Ansprüche 1-10 oder ein pharmazeutisch verträgliches Salz davon, zur Verwendung als ein Medikament zur Behandlung von Krebs.

**13.** Eine Verbindung nach einem der Ansprüche 1-10 oder ein pharmazeutisch verträgliches Salz davon, zur Verwendung in einer Therapie.

**14.** Eine Verbindung nach einem der Ansprüche 1-10 oder ein pharmazeutisch verträgliches Salz davon zur Verwendung in einem Verfahren zur Behandlung von Krebs in einem Subjekt, der diese benötigt, wobei das Verfahren das Verabreichen einer therapeutisch effektiven Menge einer Verbindung nach einem der Ansprüche 1 bis 10 oder eines pharmazeutisch verträglichen Salzes davon an das Subjekt umfasst.

**15.** Eine Verwendung der Verbindung nach einem der Ansprüche 1 bis 10 oder eines pharmazeutisch verträglichen Salzes davon zur Herstellung eines Medikaments zum Modulieren der Degradationsaktivität des RBM39-Proteins in einem Patienten, der dies benötigt.

**16.** Ein Verfahren zur Herstellung einer Verbindung nach Anspruch 1, umfassend das Reagieren eines Zwischenprodukts der Formel A:

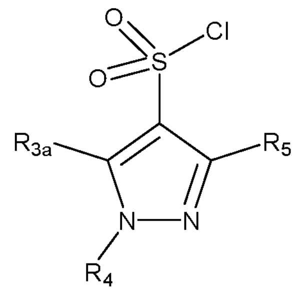

mit einem Zwischenprodukt der Formel B:

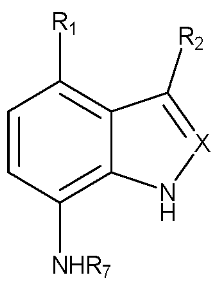

in Gegenwart einer Base, um eine Verbindung nach Anspruch 1 zu erhalten, wobei optional, sofern gewünscht, beliebige Schutzgruppen hinzugefügt oder entfernt werden und wobei, optional, sofern gewünscht, ein beliebiges Salz gebildet wird, wobei
$\mathbf{R_{3a}}$ Wasserstoff, eine C1-C4-Alkylgruppe, ein Halogen, eine Cyanogruppe, oder $-NR^aR^b$ ist, wobei $R^a$ oder $R^b$ unabhängig voneinander Wasserstoff, eine C3-C6-Cycloalkylgruppe, eine Heterocycloalkylgruppe, -C(=O)O(C1-C4-Alkyl) oder eine C1-C4-Alkylgruppe ist, oder $R^a/R^b$ und das Stickstoffatom, an das sie gebunden

sind, einen 5- oder 6-gliedrigen Ring bilden, der optional unabhängig voneinander durch einen oder mehrere Substituenten substituiert ist, ausgewählt aus einem Halogen, einer C1-C4-Alkylgruppe, einer Cyanogruppe, einer Hydroxygruppe und -O(C1-C4-Alkyl).

## Revendications

**1.** Composé de formule I :

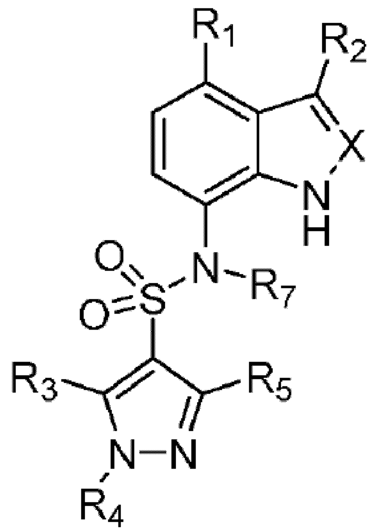

I

ou un sel pharmaceutiquement acceptable de celui-ci, dans lequel
X représente un atome d'azote N ou un groupe $CR_6$ ;
$R_1$ représente un atome d'hydrogène, un groupe alkyle en $C_1$-$C_4$, un groupe fluoroalkyle en $C_1$-$C_4$ ou un atome d'halogène ;
$R_2$ représente un atome d'halogène ou un groupe cyano ;
$R_3$ représente un atome d'hydrogène, un groupe alkyle en $C_1$-$C_4$, un atome d'halogène, un groupe cyano, un groupe fluoroalkyle en $C_1$-$C_4$, un groupe -(alkyle en $C_1$-$C_4$)OH, ou un groupe -$NR^aR^b$, dans lequel $R^a$ ou $R^b$ représente indépendamment un atome d'hydrogène, un groupe cycloalkyle en $C_3$-$C_6$, un groupe hétérocycloalkyle ou un groupe alkyle en $C_1$-$C_4$, ou $R^a$/$R^b$ et l'atome d'azote auquel ils sont liés forment un cycle à 5 ou 6 chaînons éventuellement indépendamment substitués par un ou plusieurs substituants choisis parmi un atome d'halogène, un groupe alkyle en $C_1$-$C_4$, un groupe cyano, un groupe hydroxyle et un groupe -O(alkyle en $C_1$-$C_4$) ;
$R_4$ représente un atome d'hydrogène, un groupe alkyle en $C_1$-$C_6$, un groupe hétéroalkyle en $C_1$-$C_{10}$, un groupe fluoroalkyle en $C_1$-$C_4$, un groupe -(alkyle en $C_1$-$C_4$)$NH_2$, un groupe cycloalkyle en $C_3$-$C_6$, un groupe -(alkyle en $C_1$-$C_4$)(cycloalkyle en $C_3$-$C_6$), un groupe -(alkyle en $C_1$-$C_4$)phényle, un groupe phényle, un groupe hétéroaryle, un groupe -(alkyle en $C_1$-$C_4$)hétéroaryle, un groupe alcényle en $C_2$-$C_5$, un groupe -(alkyle en $C_1$-$C_4$)hétérocycloalkyle, un groupe hétérocycloalkyle, un groupe (alkyle en $C_0$-$C_4$)$S(=O)_2$(alkyle en $C_1$-$C_4$), un groupe -$S(=O)_2$phényle, un groupe -$S(=O)_2$hétéroaryle, un groupe (alkyle en $C_0$-$C_4$)(=O)hétérocycloalkyle, un groupe -((alkyle en $C_0$-$C_4$)C(=O)$NR^aR^b$, un groupe -(alkyle en $C_0$-$C_4$)C(=O)O(alkyle en $C_1$-$C_4$), un groupe -(alkyle en $C_0$-$C_4$)C(=O)(alkyle en $C_1$-$C_4$), un groupe -(alkyle en $C_0$-$C_4$)C(=O)OH ou un groupe -(alkyle en $C_1$-$C_8$)OH, dans lequel $R_4$ est substitué par 0, 1, 2 ou 3 substituants choisis parmi le deutérium, un atome d'halogène, un groupe hydroxyle, un groupe -(alkyle en $C_0$-$C_4$)O(alkyl een $C_1$-$C_4$), un groupe alkyle en $C_1$-$C_4$, un groupe (alkyle en $C_0$-$C_4$)cyano, un groupe -$S(=O)_2$(alkyle en $C_1$-$C_4$), un groupe (alkyle en $C_0$-$C_4$)C(=O)$NR^aR^b$, un groupe -C(=O)O(alkyle en $C_1$-$C_4$), un groupe (fluoroalkyle en $C_1$-$C_4$)oxo, un groupe -(alkyle en $C_1$-$C_4$)OH, et un groupe -$NH_2$, dans lesquels chacun des groupes $R^a$ et $R^b$ sont tous deux indépendamment substitués par 0, 1, 2 ou 3 atomes d'hydrogène, groupes cycloalkyles en $C_3$-$C_6$, groupes hétérocycloalkyles ou groupes alkyles en $C_1$-$C_4$;
$R_5$ représente un atome d'hydrogène, un atome d'halogène, un groupe -$NH_2$ ou un groupe alkyle en $C_1$-$C_4$;
$R_6$ représente un atome d'hydrogène, un atome d'halogène ou un groupe alkyle en $C_1$-$C_4$; et
$R_7$ représente un atome d'hydrogène, un atome d'halogène, un groupe hétéroalkyle, un groupe alkyle en $C_1$-$C_4$, un groupe -(alkyle en $C_0$-$C_4$)O(alkyle en $C_1$-$C_4$), un groupe -(alkyle en $C_1$-$C_4$)(cycloalkyle en $C_3$-$C_6$), un groupe cycloalkyle en $C_3$-$C_6$, un groupe -(alkyle en $C_1$-$C_4$)hétérocycloalkyle, ou un groupe hétérocycloalkyle.

**2.** Composé selon la revendication 1 ou l'un de ses sels pharmaceutiquement acceptables, dans lequel $R_1$ représente un groupe alkyle en $C_1$-$C_4$ ou un atome d'halogène.

**3.** Composé selon la revendication 2 ou l'un de ses sels pharmaceutiquement acceptables, dans lequel $R_1$ représente un groupe méthyle, un atome de chlore ou un atome de fluor.

**4.** Composé selon la revendication 1 ou l'un de ses sels pharmaceutiquement acceptables, dans lequel $R_1$ représente un atome d'hydrogène.

**5.** Composé selon l'une quelconque des revendications 1 à 4, ou l'un de ses sels pharmaceutiquement acceptables, dans lequel $R_2$ représente un atome de chlore, un atome de fluor ou un groupe cyano.

**6.** Composé selon l'une quelconque des revendications 1 à 5, ou l'un de ses sels pharmaceutiquement acceptables, dans lequel $R_3$ représente un atome d'hydrogène, un groupe méthyle, un atome de fluor, un atome de chlore, un groupe difluorométhyle, un groupe $-NH_2$, ou un groupe $-C_2H_4OH$.

**7.** Composé selon l'une quelconque des revendications 1 à 6 ou l'un de ses sels pharmaceutiquement acceptables, dans lequel $R_4$ représente un atome d'hydrogène, un groupe méthyle, un groupe éthyle, un groupe propyle, un groupe isopropyle, un groupe tert-butyle, un groupe fluorométhyle, un groupe difluorométhyle, un groupe difluoroéthyle, un groupe trifluoroéthyle, un groupe fluoropropyle, un groupe méthoxyéthyle, un groupe méthoxyéthoxyéthyle, un groupe cyclopropyle, un groupe cyclobutyle, un groupe cyclopentyle, un groupe cyclohexyle, un groupe 3-bicyclo[1.1.1]pentanylve, un groupe cyclopropylméthyle, un groupe cyclobutylméthyle, un groupe cyclopentylméthyle, un groupe cyclohexylméthyle, un groupe cyclopropyléthyle, un groupe cyclobutyléthyle, un groupe cyclopentyléthyle et un groupe cyclohexylméthyle, un groupe tétrahydrofuranyle, un groupe azétidinyle, un groupe tétrahydropyranyle, un groupe pipéridinyle, un groupe oxétanyle, un groupe thiétanyle, un groupe pyrrolidinyléthyle, un groupe pyrrolidinyle, un groupe pyrrolidinylméthyle, un groupe azétidinylméthyle, un groupe tétrahydrothiopyranylméthyle, un groupe pipéridinylméthyle, un groupe tétrahydropyranylméthyle, un groupe tétrahydrothiophényle, un groupe oxétanyléthyle, un groupe oxaspiro[3.3]heptyle, un groupe (méthylsulfonyl)éthyle, un groupe 2-méthyl-2-(méthylsulfonyl)propyle, un groupe pyrazolyl(méthylsulfonyl), un groupe méthylsulfonyle, un groupe (méthylsulfonyl)méthyle, un groupe phénylsulfonyle, un groupe hydroxyéthyle, un groupe aminocarbonylméthyle, un groupe carboxyméthyle, un groupe cyclobutylaminocarbonylméthyle, un groupe pyrrolidinylcarbonylméthyle, un groupe méthylaminocarbonylméthyle, un groupe (diméthylamino)carbonylméthyle, un groupe aminocarbonyl(alkyle en $C_1$-$C_4$), méthoxycarbonyl(alkyle en $C_1$-$C_4$), (aminocarbonyl)(alkyle en $C_1$-$C_4$), un groupe phényle, un groupe pyridinyle, un groupe propényle,

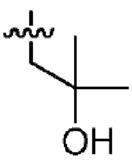 , 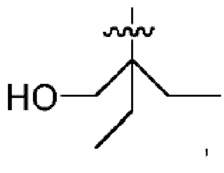 , 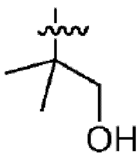 , 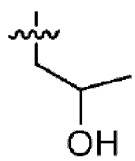 , 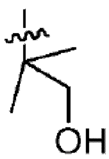 ,

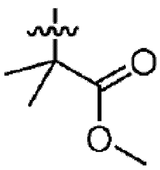 , 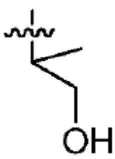 , 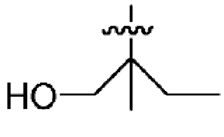 , 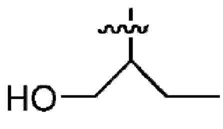 ,

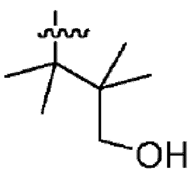 , 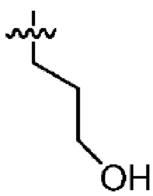 , 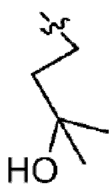 , 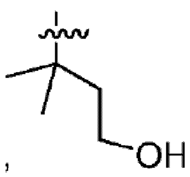 , 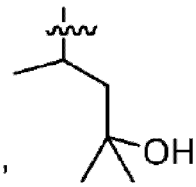 , 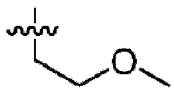 ,

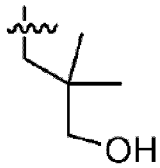,
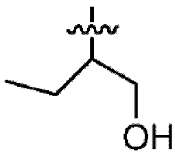,
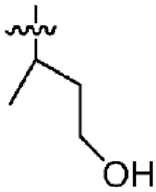,
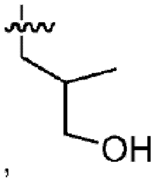,
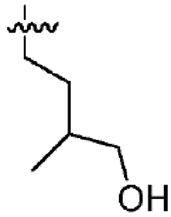,
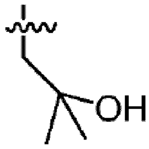,
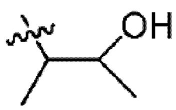,
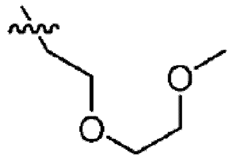
or
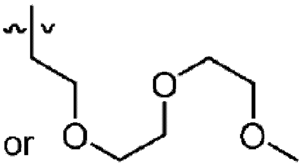,

et dans lequel $R_4$ est substitué par 0, 1, 2 ou 3 substituants choisis parmi le deutérium, un atome d'halogène, un groupe hydroxyle, un groupe (alkyle en $C_0$-$C_4$)O(alkyle en $C_1$-$C_4$), un groupe alkyle en $C_1$-$C_4$, un groupe -(alkyle en $C_0$-$C_4$)cyano, un groupe (alkyle en $C_0$-$C_4$)C(=O)$NR^aR^b$, un groupe C(=O)O(alkyle en $C_1$-$C_4$), un groupe (fluoroalkyle en $C_1$-$C_4$)oxo, un groupe -(alkyle en $C_1$-$C_4$)OH, et un groupe -$NH_2$, dans lequel chacun des groupes $R^a$ et $R^b$ est indépendamment substitué par 0, 1, 2, ou 3 atomes d'hydrogène, groupes cycloalkyles en $C_3$-$C_6$, groupes hétérocycloalkyles ou groupes alkyles en $C_1$-$C_4$.

**8.** Composé selon l'une quelconque des revendications 1 à 7, ou l'un de ses sels pharmaceutiquement acceptables, dans lequel $R_5$ représente un atome d'hydrogène, un groupe méthyle, un atome de fluor, un atome de chlore ou un groupe -$NH_2$.

**9.** Composé selon l'une quelconque des revendications 1 à 8, ou l'un de ses sels pharmaceutiquement acceptables, dans lequel $R_2$ représente un groupe cyano.

**10.** Composé selon la revendication 1 qui est choisi dans le groupe comprenant

le N-(3-chloro-1H-indol-7-yl)-1-méthyl-pyrazole-4-sulfonamide;
le N-(3-chloro-1H-indol-7-yl)-1,3,5-triméthyl-pyrazole-4-sulfonamide;
le N-(3-cyano-4-méthyl-1H-indol-7-yl)-1-méthyl-pyrazole-4-sulfonamide;
le N-(3-chloro-1H-indol-7-yl)-1-éthyl-pyrazole-4-sulfonamide;
le N-(3-chloro-1H-indol-7-yl)-1,3-diméthyl-pyrazole-4-sulfonamide;
le N-(3-chloro-1H-indol-7-yl)-1-(difluorométhyl)pyrazole-4-sulfonamide;
le N-(3-chloro-1H-indol-7-yl)-1-(2,2-difluoroéthyl)pyrazole-4-sulfonamide;
le N-(3-chloro-1H-indol-7-yl)-1-tétrahydrofuran-3-yl-pyrazole-4-sulfonamide;
le N-(3-chloro-1H-indol-7-yl)-1-(2-méthylsulfonyléthyl)pyrazole-4-sulfonamide;
le N-(3-chloro-1H-indol-7-yl)-1H-pyrazole-4-sulfonamide;
le N-(3-cyano-4-méthyl-1H-indol-7-yl)-1-(difluorométhyl)pyrazole-4-sulfonamide;
le 3-[4-[(3-chloro-1H-indol-7-yl)sulfamoyl]pyrazol-1-yl]azétidine-1-carboxylate de tert-butyle;
le N-(3-chloro-1H-indol-7-yl)-1-(2-hydroxyéthyl)pyrazole-4-sulfonamide;
le N-(3-chloro-1H-indol-7-yl)-1-(cyanométhyl)pyrazole-4-sulfonamide;
le 1-(5-chloro-3-fluoro-2-pyridyl)-N-(3-chloro-1H-indol-7-yl)pyrazole-4-sulfonamide;
le 2-[4-[(3-chloro-1H-indol-7-yl)sulfamoyl]pyrazol-1-yl]acétamide;
le 2-[4-[4-[(3-chloro-1H-indol-7-yl)sulfamoyl]pyrazol-1-yl]sulfonylpyrazol-1-yl]acétamide;
le 1-(azétidin-3-yl)-N-(3-chloro-1H-indol-7-yl)pyrazole-4-sulfonamide;
le N-(3-chloro-1H-indol-7-yl)-1-(1H-pyrazol-4-ylsulfonyl)pyrazole-4-sulfonamide;
le N-(3-chloro-1H-indol-7-yl)-1-tétrahydropyran-4-yl-pyrazole-4-sulfonamide;
le N-(3-chloro-1H-indol-7-yl)-1-(5-fluoro-2-pyridyl)pyrazole-4-sulfonamide;
le N-(3-chloro-1H-indol-7-yl)-1-[2-cyano-4-(trifluorométhyl)phényl]pyrazole-4-sulfonamide;
le N-(3-chloro-1H-indol-7-yl)-1-[2-(2-méthoxyéthoxy)éthyl]pyrazole-4-sulfonamide;
le N-(3-chloro-1H-indol-7-yl)-1-[2-[2-(2-méthoxyéthoxy)éthoxy]éthyl]pyrazole-4-sulfonamide;
le N-(3-cyano-4-méthyl-1H-indol-7-yl)-1-(2-hydroxyéthyl)pyrazole-4-sulfonamide;

le 4-[4-[(3-chloro-1H-indol-7-yl)sulfamoyl]pyrazol-1-yl]pipéridine-1- carboxylate de tert-butyle;
le 1-(1-acétylazétidin-3-yl)-N-(3-chloro-1H-indol-7-yl)pyrazole-4-sulfonamide;
le N-(3-chloro-1H-indol-7-yl)-1-[(3-méthyloxetan-3-yl)méthyl]pyrazole-4-sulfonamide;
le N-(3-cyano-4-méthyl-1H-indol-7-yl)-1-tétrahydrofuran-3-yl-pyrazole-4-sulfonamide;
le N-(3-chloro-1H-indol-7-yl)-1-[5-(trifluorométhyl)-2-pyridyl]pyrazole-4-sulfonamide;
le N-(3-cyano-4-méthyl-1H-indol-7-yl)-1-[5-(trifluorométhyl)-2-pyridyl]pyrazole-4-sulfonamide;
le N-(3-chloro-1H-indol-7-yl)-1-(2,2-diméthoxyéthyl)pyrazole-4-sulfonamide;
le N-[2-[4-[(3-chloro-1H-indol-7-yl)sulfamoyl]pyrazol-1-yl]éthyl]carbamate de tert-butyle;
le 1-(2-aminoéthyl)-N-(3-chloro-1H-indol-7-yl)pyrazole-4-sulfonamide;
le N-(3-chloro-1H-indol-7-yl)-1-(2-fluoroéthyl)pyrazole-4-sulfonamide;
le N-(3-cyano-4-méthyl-1H-indol-7-yl)-1-(2-fluoroéthyl)pyrazole-4-sulfonamide;
le N-(3-chloro-1H-indol-7-yl)-1-phényl-pyrazole-4-sulfonamide;
le N-(3-chloro-1H-indol-7-yl)-1-(2,2,2-trifluoroéthyl)pyrazole-4-sulfonamide;
le N-(3-chloro-1H-indol-7-yl)-1-isopropyl-pyrazole-4-sulfonamide;
le N-(3-chloro-1H-indol-7-yl)-1-(oxétan-3-yl)pyrazole-4-sulfonamide;
le N-(3-chloro-1H-indol-7-yl)-1-(4-pipéridyl)pyrazole-4-sulfonamide;
le 3-[[4-[(3-chloro-1H-indol-7-yl)sulfamoyl]pyrazol-1-yl]méthyl]azétidine-1-carboxylate de tert-butyle;
le 1-(azétidin-3-ylméthyl)-N-(3-chloro-1H-indol-7-yl)pyrazole-4-sulfonamide;
le N-(3-chloro-1H-indol-7-yl)-1-[(3-méthylthietan-3-yl)méthyl]pyrazole-4-sulfonamide;
le N-(3-chloro-1H-indol-7-yl)-1-[(3-méthyl-1,1-dioxo-thietan-3-yl)méthyl]pyrazole-4-sulfonamide;
le N-(3-cyano-1H-indol-7-yl)-1-méthyl-pyrazole-4-sulfonamide;
le N-(3-chloro-1H-indol-7-yl)-1-cyclobutyl-pyrazole-4-sulfonamide;
le N-(3-chloro-1H-indol-7-yl)-N,1-bis(cyclopropylméthyl)pyrazole-4-sulfonamide;
le N-(3-chloro-1H-indol-7-yl)-1-(2-méthoxyéthyl)pyrazole-4-sulfonamide;
le N-(3-chloro-1H-indol-7-yl)-N,1-bis(2-méthoxyéthyl)pyrazole-4-sulfonamide;
le N-(3-chloro-1H-indol-7-yl)-1-(cyclopropylméthyl)pyrazole-4-sulfonamide;
le N-(3-chloro-1H-indol-7-yl)-1-[(1-cyanocyclopropyl)méthyl]pyrazole-4-sulfonamide;
le N-(3-chloro-1H-indol-7-yl)-1-[(3,3-difluorocyclobutyl)méthyl]pyrazole-4-sulfonamide;
le N-(3-chloro-4-fluoro-1H-indol-7-yl)-1-méthyl-pyrazole-4-sulfonamide;
le N-(3-chloro-1H-indol-7-yl)-1-(fluorométhyl)pyrazole-4-sulfonamide;
le N-(3-chloro-1H-indol-7-yl)-1-(3-fluoropropyl)pyrazole-4-sulfonamide;
le N-(3-chloro-1H-indol-7-yl)-1-(1,1-dioxothietan-3-yl)pyrazole-4-sulfonamide;
le N-(3-chloro-1H-indol-7-yl)-1-(2-cyanoéthyl)pyrazole-4-sulfonamide;
le N-(3-cyano-4-méthyl-1H-indol-7-yl)-1-(2,2,2-trifluoroéthyl)pyrazole-4-sulfonamide;
le N-(3-cyano-4-méthyl-1H-indol-7-yl)-1-cyclobutyl-pyrazole-4-sulfonamide;
le N-(3-cyano-4-méthyl-1H-indol-7-yl)-1H-pyrazole-4-sulfonamide;
le N-(3-cyano-4-méthyl-1H-indol-7-yl)-1-isopropyl-pyrazole-4-sulfonamide;
le N-(3-cyano-4-méthyl-1H-indol-7-yl)-1-(oxetan-3-yl)pyrazole-4-sulfonamide;
le N-(3-cyano-4-fluoro-1H-indol-7-yl)-1-méthyl-pyrazole-4-sulfonamide;
le N-(3-cyano-4-méthyl-1H-indol-7-yl)-1-(2,2-difluoroéthyl)pyrazole-4-sulfonamide;
le N-(3-cyano-4-méthyl-1H-indol-7-yl)-1-(2-méthylsulfonyléthyl)pyrazole-4-sulfonamide;
le N-(3-cyano-4-méthyl-1H-indol-7-yl)-1-éthyl-pyrazole-4-sulfonamide;
LE N-(3-cyano-4-méthyl-1H-indol-7-yl)-1-(cyclopropylméthyl)pyrazole-4-sulfonamide;
le N-(3-cyano-4-méthyl-1H-indol-7-yl)-1-(fluorométhyl)pyrazole-4-sulfonamide;
le N-(3-cyano-4-méthyl-1H-indol-7-yl)-1-[(3-méthyloxetan-3-yl)méthyl] pyrazole-4-sulfonamide;
le N-(3-cyano-4-méthyl-1H-indol-7-yl)-1-[(3-fluorooxetan-3-yl)méthyl]pyrazole-4-sulfonamide;
le N-[3-chloro-4-(trifluorométhyl)-1H-indol-7-yl]-1-(2,2-difluoroéthyl)pyrazole-4-sulfonamide;
le N-[3-cyano-4-(trifluorométhyl)-1H-indol-7-yl]-1-(2,2-difluoroéthyl)pyrazole-4-sulfonamide;
le N-[3-cyano-4-(trifluorométhyl)-1H-indol-7-yl]-1-méthyl-pyrazole-4-sulfonamide;
le N-(3-cyano-4-méthyl-1H-indol-7-yl)-1-[[3-(hydroxyméthyl)oxetan-3-yl]méthyl]pyrazole-4-sulfonamide;
le N-(3-cyano-4-méthyl-1H-indol-7-yl)-1-(3-méthyloxetan-3-yl)pyrazole-4-sulfonamide;
le N-(3-cyano-4-méthyl-1H-indol-7-yl)-1-(2-hydroxy-2-méthyl-propyl)pyrazole-4-sulfonamide;
le N-(3-cyano-4-méthyl-1H-indol-7-yl)-1-(3,3-difluorocyclobutyl)pyrazole-4-sulfonamide;
le N-(3-cyano-4-méthyl-1H-indol-7-yl)-1-(trideuteriométhyl)pyrazole-4-sulfonamide;
le N-(3-cyano-4-méthyl-1H-indol-7-yl)-1-(3-fluorocyclobutyl)pyrazole-4-sulfonamide;
le N-(3-cyano-4-méthyl-1H-indol-7-yl)-5-fluoro-1-méthyl-pyrazole-4-sulfonamide;
le N-(3-cyano-4-méthyl-1H-indol-7-yl)-1,5-diméthyl-pyrazole-4-sulfonamide;
le N-(3-cyano-4-méthyl-1H-indol-7-yl)-1,3-diméthyl-pyrazole-4-sulfonamide;

le N-(3,4-dichloro-1H-indol-7-yl)-1-(3-méthyloxetan-3-yl)pyrazole-4-sulfonamide;
le N-(3-cyano-4-méthyl-1H-indol-7-yl)-1-[3-(hydroxyméthyl)oxetan-3-yl]pyrazole-4-sulfonamide;
le N-(3-cyano-4-méthyl-1H-indol-7-yl)-1-[3-(fluorométhyl)oxetan-3-yl]pyrazole-4-sulfonamide;
le N-(4-chloro-3-cyano-1H-indol-7-yl)-1-(3-méthyloxetan-3-yl)pyrazole-4-sulfonamide;
le N-(4-chloro-3-cyano-1H-indol-7-yl)-1-(difluorométhyl)pyrazole-4-sulfonamide;
le N-(4-chloro-3-cyano-1H-indol-7-yl)-1-(2,2,2-trifluoroéthyl)pyrazole-4-sulfonamide;
le N-(4-chloro-3-cyano-1H-indol-7-yl)-1H-pyrazole-4-sulfonamide;
le N-(4-chloro-3-cyano-1H-indol-7-yl)-5-fluoro-1-méthyl-pyrazole-4-sulfonamide;
le N-(4-chloro-3-cyano-1H-indol-7-yl)-1-(cyclopropylméthyl)pyrazole-4-sulfonamide;
le N-(4-chloro-3-cyano-1H-indol-7-yl)-1-(fluorométhyl)pyrazole-4-sulfonamide;
le N-(4-chloro-3-cyano-1H-indol-7-yl)-1-(oxetan-3-yl)pyrazole-4-sulfonamide;
le N-(3-fluoro-4-méthyl-1H-indol-7-yl)-1-méthyl-pyrazole-4-sulfonamide;
le N-(4-chloro-3-cyano-1H-indol-7-yl)-1-éthyl-pyrazole-4-sulfonamide;
le N-(3-cyano-4-méthyl-1H-indol-7-yl)-5-fluoro-1-(3-méthyloxetan-3-yl)pyrazole-4-sulfonamide;
le N-(3-chloro-2-fluoro-1H-indol-7-yl)-1-méthyl-pyrazole-4-sulfonamide;
le N-(3-cyano-4-méthyl-1H-indol-7-yl)-1-(3-fluoropropyl)pyrazole-4-sulfonamide;
le N-(3-chloro-4-méthyl-1H-indol-7-yl)-1-méthyl-pyrazole-4-sulfonamide;
le N-(3-cyano-4-méthyl-1H-indol-7-yl)-1-(2-cyano-2-méthyl-propyl)pyrazole-4-sulfonamide;
le 1-(2-cyanoéthyl)-N-(3-cyano-4-méthyl-1H-indol-7-yl)pyrazole-4-sulfonamide;
le N-(3-cyano-4-méthyl-1H-indol-7-yl)-1-(cyclobutylméthyl)pyrazole-4-sulfonamide;
le N-(3-cyano-4-méthyl-1H-indol-7-yl)-1-[(2,2-difluorocyclopropyl)méthyl]pyrazole-4-sulfonamide;
le N-(3-cyano-4-méthyl-1H-indol-7-yl)-1-[1-(fluorométhyl)vinyl]pyrazole-4-sulfonamide;
le N-(3,4-dichloro-1H-indol-7-yl)-1-méthyl-pyrazole-4-sulfonamide;
le N-(4-chloro-3-cyano-1H-indol-7-yl)-1-méthyl-pyrazole-4-sulfonamide;
le N-(3-chloro-1H-indol-7-yl)-1-(3-hydroxycyclobutyl)pyrazole-4-sulfonamide;
le N-(3-cyano-4-méthyl-1H-indol-7-yl)-1-(2-méthyl-2-méthylsulfonyl-propyl)pyrazole-4-sulfonamide;
le N-(3-cyano-4-méthyl-1H-indol-7-yl)-1-(2-oxaspiro[3.3]heptan-6-yl)pyrazole-4-sulfonamide;
le N-(3-cyano-4-méthyl-1H-indol-7-yl)-1-(4,4-difluorocyclohexyl)pyrazole-4-sulfonamide;
le N-(3-cyano-4-méthyl-1H-indol-7-yl)-1-[1-(2,2,2-trifluoroéthyl)-4-pipéridyl]pyrazole-4-sulfonamide;
le N-(3-cyano-4-méthyl-1H-indol-7-yl)-1-cyclopentyl-pyrazole-4-sulfonamide;
le 1-benzyl-N-(3-cyano-4-méthyl-1H-indol-7-yl)pyrazole-4-sulfonamide;
le N-(3-cyano-4-méthyl-1H-indol-7-yl)-1-propyl-pyrazole-4-sulfonamide;
le N-(4-chloro-3-cyano-1H-indol-7-yl)-1-(2,2-difluoroéthyl)pyrazole-4-sulfonamide;
le N-(3-cyano-4-fluoro-1H-indol-7-yl)-1-(2,2-difluoroéthyl)pyrazole-4-sulfonamide;
le N-(3-cyano-4-méthyl-1H-indol-7-yl)-1-méthylsulfonyl-pyrazole-4-sulfonamide;
le 1-tert-butyl-N-(3-cyano-4-méthyl-1H-indol-7-yl)pyrazole-4-sulfonamide;
le 1-tert-butyl-N-(3-chloro-1H-indol-7-yl)pyrazole-4-sulfonamide;
le N-[3-chloro-4-(trifluorométhyl)-1H-indol-7-yl]-1-méthyl-pyrazole-4-sulfonamide;
le N-(3-chloro-4-méthyl-1H-indol-7-yl)-1-(2,2-difluoroéthyl)pyrazole-4-sulfonamide;
le N-(3-cyano-4-méthyl-1H-indol-7-yl)-1-[[1-(trifluorométhyl)cyclopropyl]méthyl]pyrazole-4-sulfonamide;
le N-(3-cyano-4-méthyl-1H-indol-7-yl)-1-(fluorométhylsulfonyl)pyrazole-4-sulfonamide;
le 1-(benzenesulfonyl)-N-(3-cyano-4-méthyl-1H-indol-7-yl)pyrazole-4-sulfonamide;
le N-(3-cyano-4-méthyl-1H-indol-7-yl)-1-éthyl-5-fluoro-pyrazole-4-sulfonamide;
le N-(4-chloro-3-cyano-1H-indol-7-yl)-5-fluoro-1-(3-méthyloxetan-3-yl)pyrazole-4-sulfonamide;
le 5-cyano-N-(3-cyano-4-méthyl-1H-indol-7-yl)-1-méthyl-pyrazole-4-sulfonamide;
le N-(4-chloro-3-cyano-1H-indol-7-yl)-5-cyano-1-méthyl-pyrazole-4-sulfonamide;
le 2-[4-[(3-cyano-4-méthyl-1H-indol-7-yl)sulfamoyl]pyrazol-1-yl]acétate de méthyle;
l'acide 2-[4-[(3-cyano-4-méthyl-1H-indol-7-yl)sulfamoyl]pyrazol-1-yl]acétique;
le N-(4-chloro-3-cyano-1H-indol-7-yl)-1-(trideuteriométhyl)pyrazole-4-sulfonamide;
le N-(4-chloro-3-cyano-1H-indol-7-yl)-1-(2-hydroxy-2-méthyl-propyl)pyrazole-4-sulfonamide;
le 2-[4-[(3-cyano-4-méthyl-1H-indol-7-yl)sulfamoyl]pyrazol-1-yl]-N-(3,3-difluorocyclobutyl)acétamide;
le N-(3-cyano-4-méthyl-1H-indol-7-yl)-1-[2-[(3R,4S)-3,4-difluoropyrrolidin-1-yl]-2-oxo-éthyl]pyrazole-4-sulfonamide;
le 2-[4-[(3-cyano-4-méthyl-1H-indol-7-yl)sulfamoyl]pyrazol-1-yl]-N-méthyl-acétamide;
le 2-[4-[(3-cyano-4-méthyl-1H-indol-7-yl)sulfamoyl]pyrazol-1-yl]-N,N-diméthyl- acétamide;
le 2-[4-[(3-cyano-4-méthyl-1H-indol-7-yl)sulfamoyl]pyrazol-1-yl]acétamide;
le N-(3-cyano-4-méthyl-1H-indol-7-yl)-1-(2-oxo-2-pyrrolidin-1-yl-éthyl)pyrazole-4-sulfonamide; le N-(3,4-dichloro-1H-indol-7-yl)-1-(oxetan-3-yl)pyrazole-4-sulfonamide;

le N-(3-cyano-4-méthyl-1H-indol-7-yl)-5-fluoro-1-(oxetan-3-yl)pyrazole-4-sulfonamide;
le 1-[(1-cyanocyclopropyl)méthyl]-N-(3-cyano-4-méthyl-1H-indol-7-yl)pyrazole-4-sulfonamide;
le N-(3-cyano-4-méthyl-1H-indol-7-yl)-1-(2-oxopyrrolidin-3-yl)pyrazole-4-sulfonamide;
le N-(4-chloro-3-cyano-1H-indol-7-yl)-1-(2-oxopyrrolidin-3-yl)pyrazole-4-sulfonamide;
le N-(3-cyano-4-méthyl-1H-indol-7-yl)-3-fluoro-1-méthyl-pyrazole-4-sulfonamide;
le N-(3-cyano-4-méthyl-1H-indol-7-yl)-5-(difluorométhyl)-1-méthyl-pyrazole-4-sulfonamide;
le N-(4-chloro-3-cyano-1H-indol-7-yl)-5-(difluorométhyl)-1-méthyl-pyrazole-4-sulfonamide;
le N-(3-cyano-4-méthyl-1H-indol-7-yl)-1-[(3S,4R)-4-fluoropyrrolidin-3-yl]pyrazole-4-sulfonamide;
le N-(4-chloro-3-cyano-1H-indol-7-yl)-1-[(3S,4R)-4-fluoropyrrolidin-3-yl]pyrazole-4-sulfonamide;
le (3S,4R)-3-[4-[(3-cyano-4-méthyl-1H-indol-7-yl)sulfamoyl]pyrazol-1-yl]-4-fluoro-pyrrolidine-1-carboxylate de tert-butyle;
le 2-[4-[(4-chloro-3-cyano-1H-indol-7-yl)sulfamoyl]pyrazol-1-yl]-2-méthyl-propanamide;
le 5-chloro-N-(3-cyano-4-méthyl-1H-indol-7-yl)-1-méthyl-pyrazole-4-sulfonamide;
le 5-chloro-N-(4-chloro-3-cyano-1H-indol-7-yl)-1-méthyl-pyrazole-4-sulfonamide;
le N-(3-cyano-4-méthyl-1H-indol-7-yl)-1-[3-(cyanométhyl)oxetan-3-yl]pyrazole-4-sulfonamide;
le N-(4-chloro-3-cyano-1H-indol-7-yl)-1-[3-(cyanométhyl)oxetan-3-yl]pyrazole-4-sulfonamide;
le N-(3,4-dichloro-1H-indol-7-yl)-1H-pyrazole-4-sulfonamide;
le 1-[3-(cyanométhyl)oxetan-3-yl]-N-(3,4-dichloro-1H-indol-7-yl)pyrazole-4-sulfonamide;
le 5-amino-N-(3-cyano-4-méthyl-1H-indol-7-yl)-1-méthyl-pyrazole-4-sulfonamide;
le N-(4-chloro-3-cyano-1H-indol-7-yl)-1-(2-hydroxyéthyl)pyrazole-4-sulfonamide;
le N-(3,4-dichloro-1H-indol-7-yl)-1-(2-hydroxyéthyl)pyrazole-4-sulfonamide;
le N-(4-chloro-3-cyano-1H-indol-7-yl)-1-[1-(chlorométhyl)-2-hydroxy-1-(hydroxyméthyl)éthyl]pyrazole-4-sulfonamide;
le N-(4-chloro-3-cyano-1H-indol-7-yl)-1-[3-(hydroxyméthyl)oxetan-3-yl]pyrazole-4-sulfonamide;
le 1-[1-(chlorométhyl)-2-hydroxy-1-(hydroxyméthyl)éthyl]-N-(3,4-dichloro-1H-indol-7-yl)pyrazole-4-sulfonamide;
le N-(3,4-dichloro-1H-indol-7-yl)-1-[3-(hydroxyméthyl)oxetan-3-yl]pyrazole-4-sulfonamide;
le 2-[4-[(3,4-dichloro-1H-indol-7-yl)sulfamoyl]pyrazol-1-yl]-2-méthyl-propanoate de méthyle;
le 2-[4-[(4-chloro-3-cyano-1H-indol-7-yl)sulfamoyl]pyrazol-1-yl]-2-méthyl- propanoate de méthyle;
le N-(3,4-dichloro-1H-indol-7-yl)-1-(2-hydroxy-1,1-diméthyl-éthyl)pyrazole-4-sulfonamide;
le N-(4-chloro-3-cyano-1H-indol-7-yl)-1-(2-hydroxy-1,1-diméthyl-éthyl)pyrazole-4-sulfonamide;
le N-(3,4-dichloro-1H-indol-7-yl)-1-(2-hydroxy-1-méthyl-éthyl)pyrazole-4-sulfonamide;
le N-(3,4-dichloro-1H-indol-7-yl)-1-[2-hydroxy-1-(hydroxyméthyl)-1-méthyl-éthyl]pyrazole-4-sulfonamide;
le N-(4-chloro-3-cyano-1H-indol-7-yl)-1-(3-hydroxycyclobutyl)pyrazole-4-sulfonamide;
le N-(4-chloro-3-cyano-1H-indol-7-yl)-1-(3-hydroxycyclobutyl)pyrazole-4-sulfonamide;
le N-(3,4-dichloro-1H-indol-7-yl)-1-[2-hydroxy-1-(hydroxyméthyl)éthyl]pyrazole-4-sulfonamide;
le N-(3,4-dichloro-1H-indol-7-yl)-1-(2-hydroxy-2-méthyl-propyl)pyrazole-4-sulfonamide;
le 1-(2-amino-2-méthyl-propyl)-N-(4-chloro-3-cyano-1H-indol-7-yl)pyrazole-4-sulfonamide;
le N-(4-chloro-3-cyano-1H-indol-7-yl)-1-[(1-hydroxycyclobutyl)méthyl]pyrazole-4-sulfonamide;
le 5-amino-N-(4-chloro-3-cyano-1H-indol-7-yl)-1-méthyl-pyrazole-4-sulfonamide;
le N-(3,4-dichloro-1H-indol-7-yl)-1-(méthylsulfonylméthyl)pyrazole-4-sulfonamide;
le N-(4-chloro-3-cyano-1H-indol-7-yl)-1-(méthylsulfonylméthyl)pyrazole-4-sulfonamide;
le N-(4-chloro-3-cyano-1H-indol-7-yl)-1-[(1-cyanocyclopropyl)méthyl]pyrazole-4-sulfonamide;
le N-(4-chloro-3-cyano-1H-indol-7-yl)-1-[(4-hydroxytétrahydropyran-4- yl)méthyl]pyrazole-4-sulfonamide;
le N-(4-chloro-3-cyano-1H-indol-7-yl)-5-fluoro-1-(2-hydroxy-2-méthyl-propyl)pyrazole-4-sulfonamide;
le N-(4-chloro-3-cyano-1H-indol-7-yl)-1-(3-méthoxycyclobutyl)pyrazole-4-sulfonamide;
le N-(3-chloro-1H-indol-7-yl)-1-(3-méthoxycyclobutyl)pyrazole-4-sulfonamide;
le N-(4-chloro-3-cyano-1H-indol-7-yl)-1-[1-(hydroxyméthyl)cyclobutyl]pyrazole-4-sulfonamide;
le N-(3-cyano-4-fluoro-1H-indol-7-yl)-1-(2-hydroxy-2-méthyl-propyl)pyrazole-4-sulfonamide;
le N-(3-cyano-4-méthyl-1H-indol-7-yl)-1-(3-hydroxycyclobutyl)pyrazole-4-sulfonamide;
le N-(3-chloro-1H-indol-7-yl)-1-(3-hydroxycyclobutyl)pyrazole-4-sulfonamide;
le N-(4-chloro-3-cyano-1H-indol-7-yl)-1-[[1(hydroxyméthyl)cyclopropyl]méthyl]pyrazole-4-sulfonamide;
le N-(4-chloro-3-cyano-1H-indol-7-yl)-1-[(2S)-2-hydroxypropyl]pyrazole-4-sulfonamide;
le N-(4-chloro-3-cyano-1H-indol-7-yl)-1-[(2R)-2-hydroxypropyl]pyrazole-4-sulfonamide;
le N-(3-cyano-4-méthyl-1H-indol-7-yl)-1-(2-hydroxy-1,1-diméthyl-éthyl)pyrazole-4-sulfonamide;
le N-(4-chloro-3-cyano-1H-indol-7-yl)-3-fluoro-1-(2-hydroxy-2-méthyl-propyl)pyrazole-4-sulfonamide;
le N-(3-cyano-4-méthyl-1H-indol-7-yl)-3-fluoro-1-(2-hydroxy-2-méthyl-propyl)pyrazole-4-sulfonamide;
le N-(3-cyano-4-fluoro-1H-indol-7-yl)-3-fluoro-1-(2-hydroxy-2-méthyl-propyl)pyrazole-4-sulfonamide;

le N-(3-cyano-4-méthyl-1H-indol-7-yl)-1-[(1R)-2-hydroxy-1-méthyl-éthyl]pyrazole-4-sulfonamide;
le N-(4-chloro-3-cyano-1H-indol-7-yl)-1-[(1R)-2-hydroxy-1-méthyl-éthyl]pyrazole-4-sulfonamide;
le N-(4-chloro-3-cyano-1H-indol-7-yl)-1-(3-hydroxypropyl)pyrazole-4-sulfonamide;
le N-(3-cyano-4-méthyl-1H-indol-7-yl)-1-(3-hydroxypropyl)pyrazole-4-sulfonamide;
le N-(3-cyano-4-fluoro-1H-indol-7-yl)-1-(3-hydroxypropyl)pyrazole-4-sulfonamide;
le N-(3-cyano-4-fluoro-1H-indol-7-yl)-1-[(1R)-2-hydroxy-1-méthyl-éthyl]pyrazole-4-sulfonamide;
le (2R)-2-[4-[(4-chloro-3-cyano-1H-indol-7-yl)sulfamoyl]pyrazol-1-yl]propénamide;
le N-(3-cyano-4-fluoro-1H-indol-7-yl)-1H-pyrazole-4-sulfonamide;
le N-(3-cyano-4-fluoro-1H-indol-7-yl)-1-(difluorométhyl)pyrazole-4-sulfonamide;
le N-(3-cyano-4-fluoro-1H-indol-7-yl)-1-(2,2,2-trifluoroéthyl)pyrazole-4-sulfonamide;
le N-(3-cyano-4-fluoro-1H-indol-7-yl)-1-éthyl-pyrazole-4-sulfonamide;
le 1-(3-bicyclo[1.1.1]pentanyl)-N-(3-cyano-4-méthyl-1H-indol-7-yl)pyrazole-4-sulfonamide;
le 1-(3-bicyclo[1.1.1]pentanyl)-N-(3-cyano-4-fluoro-1H-indol-7-yl)pyrazole-4-sulfonamide;
le N-(4-chloro-3-cyano-1H-indol-7-yl)-1-(3-hydroxy-3-méthyl-butyl)pyrazole-4-sulfonamide;
le N-(3-cyano-4-méthyl-1H-indol-7-yl)-1-(3-hydroxy-3-méthyl-butyl)pyrazole-4-sulfonamide;
le N-(3-cyano-4-fluoro-1H-indol-7-yl)-1-(3-hydroxy-3-méthyl-butyl)pyrazole-4-sulfonamide;
le 1-(3-bicyclo[1.1.1]pentanyl)-N-(4-chloro-3-cyano-1H-indol-7-yl)pyrazole-4-sulfonamide;
le N-(3-cyano-4-fluoro-1H-indol-7-yl)-1-(2-hydroxy-1,1-diméthyl-éthyl)pyrazole-4-sulfonamide;
le N-(3-cyano-4-fluoro-1H-indol-7-yl)-1-[(1R)-2-fluoro-1-méthyl-éthyl]pyrazole-4-sulfonamide;
le N-(4-chloro-3-cyano-1H-indol-7-yl)-1-[(1S)-2,2-difluoro-1- (hydroxyméthyl)éthyl]pyrazole-4-sulfonamide;
le N-(3-cyano-4-méthyl-1H-indol-7-yl)-1-[(1S)-2,2-difluoro-1- (hydroxyméthyl)éthyl]pyrazole-4-sulfonamide;
le N-(3-cyano-4-fluoro-1H-indol-7-yl)-1-[(1S)-2,2-difluoro-1- (hydroxyméthyl)éthyl]pyrazole-4-sulfonamide;
le N-(3-cyano-4-méthyl-1H-indol-7-yl)-1-(3-hydroxy-1,1-diméthyl-propyl)pyrazole-4-sulfonamide;
le N-(4-chloro-3-cyano-1H-indol-7-yl)-1-(3-hydroxy-1,1-diméthyl-propyl)pyrazole-4-sulfonamide;
le N-(3-cyano-4-méthyl-1H-indol-7-yl)-1-(3-hydroxy-1,1,3-triméthyl-butyl)pyrazole-4-sulfonamide;
le N-(4-chloro-3-cyano-1H-indol-7-yl)-1-[(1S,2R)-2-hydroxy-1-méthyl-propyl]pyrazole-4-sulfonamide;
le N-(3-cyano-4-fluoro-1H-indol-7-yl)-1-[(1S,2R)-2-hydroxy-1-méthyl-propyl]pyrazole-4-sulfonamide;
le N-(3-cyano-4-méthyl-1H-indol-7-yl)-1-[(1S,2R)-2-hydroxy-1-méthyl-propyl]pyrazole-4-sulfonamide;
le N-(3-cyano-1H-indol-7-yl)-1-(2-hydroxy-1,1-diméthyl-éthyl)pyrazole-4-sulfonamide;
le N-(3-cyano-4-méthyl-1H-indol-7-yl)-1-(3-hydroxy-1-méthyl-propyl)pyrazole-4-sulfonamide;
le N-(4-chloro-3-cyano-1H-indol-7-yl)-1-(3-hydroxy-1-méthyl-propyl)pyrazole-4-sulfonamide;
le N-(4-chloro-3-cyano-1H-indol-7-yl)-1-[(1S)-1-(fluorométhyl)-2-hydroxy-éthyl]pyrazole-4-sulfonamide;
le N-(3-cyano-4-méthyl-1H-indol-7-yl)-5-(2-hydroxyéthyl)-1-méthyl-pyrazole-4-sulfonamide;
le 2-[4-[(3-cyano-4-fluoro-1H-indol-7-yl)sulfamoyl]pyrazol-1-yl]-2-méthyl-propanamide;
le 2-[4-[(3-cyano-4-méthyl-1H-indol-7-yl)sulfamoyl]pyrazol-1-yl]-2-méthyl-propanamide;
le N-(3-cyano-4-méthyl-1H-indol-7-yl)-1-(3-hydroxy-1,3-diméthyl-butyl)pyrazole-4-sulfonamide;
le N-(4-chloro-3-cyano-1H-indol-7-yl)-1-(3-hydroxy-1,3-diméthyl-butyl)pyrazole-4-sulfonamide;
le 5-chloro-N-(3-cyano-4-méthyl-1H-indol-7-yl)-1-(2-hydroxy-1,1-diméthyl-éthyl)pyrazole-4-sulfonamide;
le 5-chloro-N-(3-cyano-4-fluoro-1H-indol-7-yl)-1-(2-hydroxy-1,1-diméthyl-éthyl)pyrazole-4-sulfonamide;
le N-(3-cyano-4-méthyl-1H-indol-7-yl)-1-[(1S)-1-(fluorométhyl)-2-hydroxy-éthyl]pyrazole-4-sulfonamide;
le N-(3-cyano-4-fluoro-1H-indol-7-yl)-1-[(1S)-1-(fluorométhyl)-2-hydroxy-éthyl]pyrazole-4-sulfonamide;
le 1-[(2S)-2-amino-3,3,3-trifluoro-propyl]-N-(4-chloro-3-cyano-1H-indol-7-yl)pyrazole-4-sulfonamide;
le 1-[(2S)-2-amino-3,3,3-trifluoro-propyl]-N-(3-cyano-4-méthyl-1H-indol-7-yl)pyrazole-4-sulfonamide;
le 5-chloro-N-(4-chloro-3-cyano-1H-indol-7-yl)-1-(2-hydroxy-1,1-diméthyl-éthyl)pyrazole-4-sulfonamide;
le 5-chloro-N-(3-cyano-1H-indol-7-yl)-1-(2-hydroxy-1,1-diméthyl-éthyl)pyrazole-4-sulfonamide;
le 3-chloro-N-(3-cyano-4-méthyl-1H-indol-7-yl)-1-(2-hydroxy-1,1-diméthyl-éthyl)pyrazole-4-sulfonamide;
le 3-chloro-N-(3-cyano-1H-indol-7-yl)-1-(2-hydroxy-1,1-diméthyl-éthyl)pyrazole-4-sulfonamide;
le 3-[[4-[(4-chloro-3-cyano-1H-indol-7-yl)sulfamoyl]pyrazol-1-yl]méthyl]-3-fluoro-azétidine-1-carboxylate de tert-butyle;
le 3-[[4-[(3-cyano-4-méthyl-1H-indol-7-yl)sulfamoyl]pyrazol-1-yl]méthyl]-3-fluoro-azétidine-1-carboxylate de tert-butyle;
le N-(4-chloro-3-cyano-1H-indol-7-yl)-1-[(3-fluoroazétidin-3-yl)méthyl]pyrazole-4-sulfonamide;
le N-(3-cyano-4-méthyl-1H-indol-7-yl)-1-[(3-fluoroazétidin-3-yl)méthyl]pyrazole-4-sulfonamide;
le N-(3-cyano-4-méthyl-1H-indol-7-yl)-1-(3-hydroxy-2,2-diméthyl-propyl)pyrazole-4-sulfonamide;
le N-(4-chloro-3-cyano-1H-indol-7-yl)-1-(3-hydroxy-2,2-diméthyl-propyl)pyrazole-4-sulfonamide;
le 3-chloro-N-(4-chloro-3-cyano-1H-indol-7-yl)-1-(2-hydroxy-1,1-diméthyl-éthyl)pyrazole-4-sulfonamide;
le 3-chloro-N-(3-cyano-4-fluoro-1H-indol-7-yl)-1-(2-hydroxy-1,1-diméthyl-éthyl)pyrazole-4-sulfonamide;
le N-(4-chloro-3-cyano-1H-indol-7-yl)-1-[1-(hydroxyméthyl)-2-méthoxy-éthyl]pyrazole-4-sulfonamide;

le N-(3-cyano-4-méthyl-1H-indol-7-yl)-1-[1-(hydroxyméthyl)-2-méthoxy-éthyl]pyrazole-4-sulfonamide;
le N-(3-cyano-4-méthyl-1H-indol-7-yl)-1-[(2R)-3-hydroxy-2-méthyl-propyl]pyrazole-4-sulfonamide;
le N-(4-chloro-3-cyano-1H-indol-7-yl)-1-[(2R)-3-hydroxy-2-méthyl-propyl]pyrazole-4-sulfonamide;
le N-(4-chloro-3-cyano-1H-indol-7-yl)-1-[(1S)-2-hydroxy-1-méthyl-éthyl]pyrazole-4-sulfonamide;
le N-(3-cyano-4-fluoro-1H-indol-7-yl)-1-[(1S)-2-hydroxy-1-méthyl-éthyl]pyrazole-4-sulfonamide;
le N-(3-cyano-4-méthyl-1H-indol-7-yl)-1-[1-(hydroxyméthyl)cyclopropyl]pyrazole-4-sulfonamide;
le N-(4-chloro-3-cyano-1H-indol-7-yl)-1-[1-(hydroxyméthyl)cyclopropyl]pyrazole-4-sulfonamide;
le N-(3-cyano-4-méthyl-1H-indol-7-yl)-1-(3-hydroxybutyl)pyrazole-4-sulfonamide;
le 5-amino-N-(3-cyano-4-méthyl-1H-indol-7-yl)-1-éthyl-pyrazole-4-sulfonamide;
le 5-amino-N-(3-cyano-4-fluoro-1H-indol-7-yl)-1-éthyl-pyrazole-4-sulfonamide;
le 5-amino-N-(4-chloro-3-cyano-1H-indol-7-yl)-1-éthyl-pyrazole-4-sulfonamide;
le N-(3-cyano-4-méthyl-1H-indol-7-yl)-1-[(1R,2S)-2-hydroxy-1-méthyl-propyl]pyrazole-4-sulfonamide;
le N-(4-chloro-3-cyano-1H-indol-7-yl)-1-[(1R,2S)-2-hydroxy-1-méthyl-propyl]pyrazole-4-sulfonamide;
le 5-chloro-N-(3-cyano-4-méthyl-1H-indol-7-yl)-1-(2-hydroxy-2-méthyl-propyl)pyrazole-4-sulfonamide;
le 5-amino-N-(3-cyano-4-méthyl-1H-indol-7-yl)-1-(2,2-difluoroéthyl)pyrazole-4-sulfonamide;
le 5-amino-N-(4-chloro-3-cyano-1H-indol-7-yl)-1-(2,2-difluoroéthyl)pyrazole-4-sulfonamide;
le 5-amino-N-(3-cyano-4-fluoro-1H-indol-7-yl)-1-(2,2-difluoroéthyl)pyrazole-4-sulfonamide;
le 5-chloro-N-(3-cyano-4-fluoro-1H-indol-7-yl)-1-(2-hydroxy-2-méthyl-propyl)pyrazole-4-sulfonamide;
le N-(3-cyano-4-méthyl-1H-indol-7-yl)-1-[(1R)-2-fluoro-1-méthyl-éthyl]pyrazole-4-sulfonamide;
le 5-amino-N-(3-cyano-4-fluoro-1H-indol-7-yl)-1-méthyl-pyrazole-4-sulfonamide;
le 5-amino-N-(3-cyano-4-méthyl-1H-indol-7-yl)-1-(2-hydroxy-2-méthyl-propyl)pyrazole-4-sulfonamide;
le 5-amino-N-(3-cyano-4-méthyl-1H-indol-7-yl)-1-(difluorométhyl)pyrazole-4-sulfonamide;
le 5-amino-N-(3-cyano-4-fluoro-1H-indol-7-yl)-1-(difluorométhyl)pyrazole-4-sulfonamide;
le N-(4-chloro-3-cyano-1H-indol-7-yl)-1-[(1R,2R)-2-hydroxy-1-méthyl-propyl]pyrazole-4-sulfonamide;
le N-(3-cyano-4-méthyl-1H-indol-7-yl)-1-[(1R,2R)-2-hydroxy-1-méthyl-propyl]pyrazole-4-sulfonamide;
le N-(3-cyano-4-fluoro-1H-indol-7-yl)-1-[(1R,2R)-2-hydroxy-1-méthyl-propyl]pyrazole-4-sulfonamide;
le 5-amino-N-(4-chloro-3-cyano-1H-indol-7-yl)-1-(2-hydroxy-2-méthyl-propyl)pyrazole-4-sulfonamide;
le 3-amino-N-(3-cyano-4-méthyl-1H-indol-7-yl)-1-éthyl-pyrazole-4-sulfonamide;
le 3-amino-N-(3-cyano-4-fluoro-1H-indol-7-yl)-1-éthyl-pyrazole-4-sulfonamide;
le 5-amino-N-(3-cyano-1H-indol-7-yl)-1-(difluorométhyl)pyrazole-4-sulfonamide;
le N-(3-cyano-4-méthyl-1H-indol-7-yl)-1-(1-fluoro-2-hydroxy-1-méthyl-éthyl)pyrazole-4-sulfonamide;
le N-(3-cyano-4-fluoro-1H-indol-7-yl)-1-(1-fluoro-2-hydroxy-1-méthyl-éthyl)pyrazole-4-sulfonamide;
le 3-amino-N-(3-cyano-4-méthyl-1H-indol-7-yl)-1H-pyrazole-4-sulfonamide;
le 3-amino-N-(4-chloro-3-cyano-1H-indol-7-yl)-1H-pyrazole-4-sulfonamide;
le N-(3-cyano-4-méthyl-1H-indol-7-yl)-1-(1-fluoro-2-hydroxy-éthyl)pyrazole-4-sulfonamide;
le N-(3-cyano-4-fluoro-1H-indol-7-yl)-1-(1-fluoro-2-hydroxy-éthyl)pyrazole-4-sulfonamide;
le 5-amino-N-(3-cyano-1H-indol-7-yl)-1-méthyl-pyrazole-4-sulfonamide;
le 3-amino-N-(3-cyano-4-fluoro-1H-indol-7-yl)-1H-pyrazole-4-sulfonamide;
le N-(3-cyano-4-méthyl-1H-indol-7-yl)-1-(1,1-difluoro-2-hydroxy-éthyl)pyrazole-4-sulfonamide;
le N-(3-cyano-4-fluoro-1H-indol-7-yl)-1-(fluorométhyl)pyrazole-4-sulfonamide;
le 5-amino-N-(3-cyano-4-méthyl-1H-indol-7-yl)-1-[(1R)-2-hydroxy-1-méthyl-éthyl]pyrazole-4-sulfonamide;
le 5-amino-N-(3-cyano-4-méthyl-1H-indazol-7-yl)-1-méthyl-pyrazole-4-sulfonamide;
le N-(3-cyano-4-méthyl-1H-indazol-7-yl)-1-(fluorométhyl)pyrazole-4-sulfonamide;
le 5-amino-N-(4-chloro-3-cyano-1H-indol-7-yl)-1-[(1R)-2-hydroxy-1-méthyl-éthyl]pyrazole-4-sulfonamide;
le 5-amino-N-(4-chloro-3-cyano-1H-indazol-7-yl)-1-méthyl-pyrazole-4-sulfonamide;
le 5-amino-N-(3-cyano-4-méthyl-1H-indol-7-yl)-1-(fluorométhyl)pyrazole-4-sulfonamide;
le 5-amino-N-(3-cyano-4-fluoro-1H-indol-7-yl)-1-(fluorométhyl)pyrazole-4-sulfonamide;
le N-(4-chloro-3-cyano-1H-indazol-7-yl)-1-[(1R)-1-(fluorométhyl)-2-hydroxy-éthyl]pyrazole-4-sulfonamide;
le N-(4-chloro-3-cyano-1H-indol-7-yl)-1-[(1R)-1-(fluorométhyl)-2-hydroxy-éthyl]pyrazole-4-sulfonamide;
le 5-amino-N-(3-cyano-1H-indol-7-yl)-1-(fluorométhyl)pyrazole-4-sulfonamide;
le N-(3-cyano-4-méthyl-1H-indol-7-yl)-1-[(1R)-1-(fluorométhyl)-2-hydroxy-éthyl]pyrazole-4-sulfonamide;
le N-(3-cyano-4-méthyl-1H-indazol-7-yl)-1-[(1R)-1-(fluorométhyl)-2-hydroxy-éthyl]pyrazole-4-sulfonamide;
le 5-amino-N-(3-cyano-4-méthyl-1H-indol-7-yl)-1-(2-hydroxy-1,1-diméthyl-éthyl)pyrazole-4-sulfonamide; et
le 5-amino-N-(4-chloro-3-cyano-1H-indol-7-yl)-1-(2-hydroxy-1,1-diméthyl-éthyl)pyrazole-4-sulfonamide ;
ou un sel pharmaceutiquement acceptable de celui-ci.

**11.** Composition pharmaceutique comprenant un composé selon l'une quelconque des revendications 1 à 10 ou un sel pharmaceutiquement acceptable de celui-ci, et un diluant ou un support pharmaceutiquement acceptable.

**12.** Composé selon l'une quelconque des revendications 1 à 10, ou un sel pharmaceutiquement acceptable de celui-ci, destiné à être utilisé en tant que médicament pour le traitement du cancer.

**13.** Composé selon l'une quelconque des revendications 1 à 10, ou un sel pharmaceutiquement acceptable de celui-ci, destiné à être utilisé en thérapie.

**14.** Composé selon l'une quelconque des revendications 1 à 10 ou un sel pharmaceutiquement acceptable de celui-ci destiné à être utilisé dans un procédé de traitement du cancer chez un sujet qui en a besoin, le procédé comprenant l'administration audit sujet d'une quantité thérapeutiquement efficace d'un composé selon l'une quelconque des revendications 1 à 10 ou d'un sel pharmaceutiquement acceptable de celui-ci.

**15.** Utilisation du composé selon l'une quelconque des revendications 1 à 10, ou d'un sel pharmaceutiquement acceptable de celui-ci, dans la préparation d'un médicament afin de moduler l'activité de dégradation de la protéine RBM39, chez un patient qui en a besoin.

**16.** Procédé de préparation d'un composé selon la revendication 1, qui comprend le fait de faire réagir un intermédiaire de formule A :

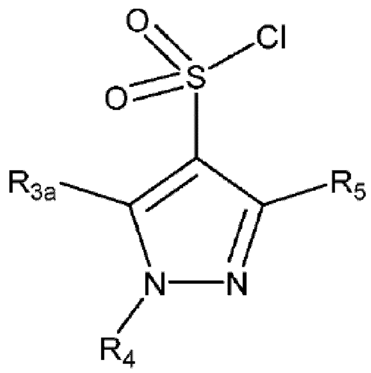

avec un intermédiaire de formule B :

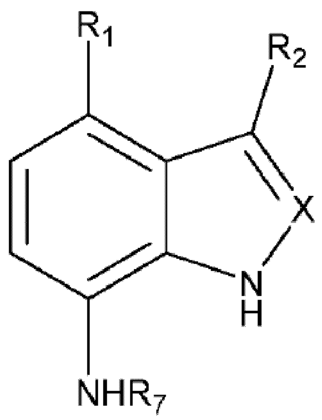

en présence d'une base afin d'obtenir un composé selon la revendication 1, en ajoutant ou en éliminant éventuellement un groupe protecteur si souhaité, et en formant éventuellement un sel si souhaité, dans lequel $R_{3a}$ représente un atome d'hydrogène, un groupe alkyle en $C_1$-$C_4$, un atome d'halogène, un groupe cyano ou un groupe -$NR^aR^b$, dans lequel $R^a$ ou $R^b$ représente indépendamment un atome hydrogène, un groupe cycloalkyle en $C_3$-$C_6$, un groupe hétérocycloalkyle, un groupe -C(=O)O(alkyle en $C_1$-$C_4$) ou un groupe alkyle en $C_1$-$C_4$, ou $R^a$/$R^b$ et l'atome d'azote auquel ils sont liés forment un cycle à 5 ou 6 chaînons éventuellement substitué indépendamment par un ou plusieurs substituants choisis parmi un groupe halogène, un groupe alkyle en $C_1$-$C_4$, un groupe cyano, un groupe hydroxyle et un groupe -O(alkyle en $C_1$-$C_4$).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8957070 B2
- US 6638964 B2
- WO 2019147783 A1
- WO 9633172 A
- WO 9627583 A
- EP 0818442 A
- EP 1004578 A
- WO 9807697 A
- WO 9803516 A
- WO 9834918 A
- WO 9834915 A
- WO 9833768 A
- WO 9830566 A
- EP 606046 A
- EP 931788 A
- WO 9005719 A
- WO 9952910 A
- WO 9952889 A
- WO 9929667 A
- WO 1999007675 A
- EP 1786785 A
- EP 1181017 A
- US 20090012085 A
- US 5863949 A
- US 5861510 A
- EP 0780386 A
- US 20030162712 A, Ceretti
- US 6413932 B
- US 6727225 B, Wiley
- US 20020042368 A, Fanslow
- US 5981245 A
- US 5728813 A
- US 5969110 A
- US 6596852 B
- US 6232447 B
- US 6057124 A
- WO 06044453 A
- WO 09036082 A
- WO 09055730 A
- WO 06122806 A
- WO 05011700 A
- US 6656963 B
- WO 9409010 A
- AP 23573
- WO 9802441 A
- WO 0114387 A
- WO 05005434 A
- US 5258389 A
- WO 94090101 A
- WO 9205179 A
- US 5118677 A
- US 5118678 A
- US 5100883 A
- US 5151413 A
- US 5120842 A
- WO 93111130 A
- WO 9402136 A
- WO 9402485 A
- WO 9514023 A
- WO 9516691 A
- WO 9641807 A
- US 5256790 A
- WO 05016252 A


### Non-patent literature cited in the description


- **E. WANG et al.** Targeting an RNA-Binding Protein network in Acute Myeliod Leukemia. *Cancer Cell,* 2019, vol. 35, 369-382
- **D. HSIEHCHEN et al.** Biomarkers for RBM39 degradation in acute myeloid leukemia. *Springer Nature,* February 2020
- **MODJTAHEDI, H. et al.** *Br. J. Cancer,* 1993, vol. 67, 247-253
- **TERAMOTO, T. et al.** *Cancer,* 1996, vol. 77, 639-645
- **GOLDSTEIN et al.** *Clin. Cancer Res.,* 1995, vol. 1, 1311-1318
- **HUANG, S. M. et al.** *Cancer Res.,* 1999, vol. 59 (8), 1935-40
- **YANG, X. et al.** *Cancer Res.,* 1999, vol. 59, 1236-1243
- **BARNETT et al.** *Biochem. J.,* 2005, vol. 385 (2), 399-408
- **JIN et al.** *Br. J. Cancer,* 2004, vol. 91, 1808-12
- **SARKAR ; LI.** *J Nutr.,* 2004, vol. 134 (12), 3493S-3498S
- **DASMAHAPATRA et al.** *Clin. Cancer Res.,* 2004, vol. 10 (15), 5242-52
- **GILLS ; DENNIS.** *Expert. Opin. Investig. Drugs,* 2004, vol. 13, 787-97

- **YANG et al.** *Cancer Res.,* 2004, vol. 64, 4394-9
- **HAN et al.** *Science,* 28 April 2017, vol. 356 (6336
- **UEHARA et al.** *Nat Chem Biol.,* June 2017, vol. 13 (6), 675-680